# EUROPEAN PATENT APPLICATION

(11) **EP 4 821 510 A1**
(43) Date of publication of application: **14.10.2026**
(21) Application number: 24899970.8
(22) Date of filing: 06.12.2024
(51) Int. Cl.: A61K 47/68, A61P 31/16

(54) **ANTIVIRAL CONJUGATE**

(30) Priority: 08.12.2023 CN 202311676711; 05.08.2024 CN 202411063815
(71) Applicant: Suzhou Renolynx Therapeutics Co., Ltd., Suzhou, Jiangsu 215000 (CN)
(72) Inventor: LIAO, Cheng, Lianyungang, Jiangsu 222047 (CN); HUANG, Jian, Shanghai 201203 (CN); REN, Wenming, Shanghai 201210 (CN); GAO, Han, Shanghai 201210 (CN); ZHU, Lingjian, Shanghai 201203 (CN); QIN, Yuhao, Shanghai 201210 (CN); LIN, Yuan, Shanghai 201210 (CN); QIAN, Mengxin, Shanghai 201210 (CN); CHI, Yushi, Lianyungang, Jiangsu 222047 (CN); HU, Kuan, Lianyungang, Jiangsu 222047 (CN)
(74) Representative: Regimbeau
(86) International application number: PCT/CN2024/137478
(87) International publication number: WO 2025/119337

(57) **Abstract**

The present invention relates to an antiviral conjugate. Specifically, the present invention relates to a conjugate represented by formula (I), wherein E comprises a protein or a polypeptide; L is a linker for covalently linking E to D; and D is each independently selected from the structure represented by formula (A). The antiviral conjugate can effectively treat related diseases such as virus infection.

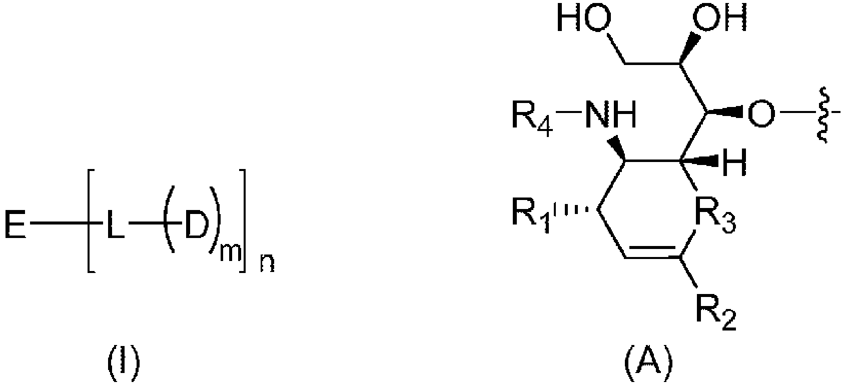

## Description

### FIELD OF THE INVENTION

The present disclosure belongs to the pharmaceutical field, and specifically relates to an antiviral conjugate.

### BACKGROUND OF THE INVENTION

Influenza viruses, including human influenza viruses and animal influenza viruses, can cause infection and morbidity in a variety of animals such as humans, avians, swines, equines, and bats. Influenza viruses are classified into four types: A, B, C, and D. Among them, influenza A virus spreads rapidly and is prone to human-to-human transmission, and even cross-species transmission. Due to the antigenic variability of influenza viruses, they have repeatedly caused worldwide pandemics. Influenza pandemics bring heavy disasters to human health and the global economy.

At present, anti-influenza drugs used in clinical practice are mainly classified into neuraminidase (NA) inhibitors (such as Oseltamivir) and M2 ion channel protein inhibitors (such as Amantadine and Rimantadine). Symptomatic use of such drugs in the early stage of influenza can shorten the course of the disease and alleviate symptoms, but such drugs also have drawbacks. Currently, the problem of drug resistance has become an important problem that cannot be ignored in the application of anti-influenza virus drugs.

Studies have shown that, since 2003, drug-resistant influenza A virus (H₃N₂) has emerged globally; in 2007, seasonal influenza A virus (H₁N₁) resistant to Oseltamivir emerged worldwide. The incidence of oseltamivir-resistant influenza A virus (H₃N₂) was almost 100%, while the incidence of oseltamivir-resistant influenza A virus (H₁N₁) reached 15.5%. Therefore, there is an urgent need to develop novel anti-influenza drugs.

Several drugs for inhibiting the growth of influenza viruses have been disclosed, including novel conjugates. For example, WO2020051498 discloses a series of antiviral conjugate drugs.

### SUMMARY OF THE INVENTION

An objective of the present disclosure is to provide a novel antiviral conjugate.

In one aspect, the present disclosure provides a conjugate represented by Formula (I), 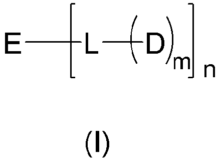 wherein,
E comprises a protein or a polypeptide;
L is a linker covalently connecting E to D;
m is selected from the group consisting of 3, 4, 5, 6, 7, 8, 9, and 10;
n is 1 to 20 (including 1, 2, 3, 4, 5, 6, 7, 8, 9, 10, 11, 12, 13, 14, 15, 16, 17, 18, 19, 20 or any value between any two values);
each D is independently selected from structure represented by Formula (A), 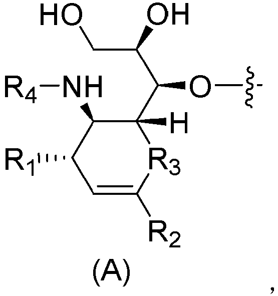
R₁ is selected from the group consisting of -OH, -NH₂, and -NHC(=NH)NHR₅;
R₂ is selected from the group consisting of -CO₂H, -P(=O)(OH)₂, and -SO₃H;
R₃ is -O- or -S-;
R₄ is selected from the group consisting of -COCH₃, -COCF₃, and -SO₂CH₃;
R₅ is selected from the group consisting of hydrogen, hydroxy, sulfhydryl, nitro, cyano, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, C₁-C₁₀ alkyl, C₁-C₁₀ alkoxy, C₂-C₁₀ alkenyl, and C₂-C₁₀ alkynyl, wherein the alkyl, alkoxy, alkenyl, and alkynyl are optionally substituted with one or more substituents selected from the group consisting of C₁-C₆ alkyl, halogen, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
Rᵢ and Rⱼ are each independently selected from the group consisting of hydrogen atom, hydroxy, C₁-C₆ alkyl, and C₁-C₆ alkoxy;
Rₖ is independently selected from the group consisting of hydrogen atom, C₁-C₆ alkyl, C₁-C₆ haloalkyl, C₁-C₆ alkoxy, hydroxy, and -NRᵢRⱼ, wherein the alkyl, alkoxy, and haloalkyl are optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3-to 10-membered cycloalkyl, 3- to 10-membered heterocyclyl, 6- to 10-membered aryl, and 5- to 10-membered heteroaryl.

In some embodiments, R₅ is selected from the group consisting of hydrogen, hydroxy, amino, and -C(O)Rₖ, wherein Rₖ is selected from the group consisting of hydrogen atom, C₁-C₆ alkyl, C₁-C₆ alkoxy, and hydroxy, wherein the alkyl and alkoxy are optionally substituted with one or more substituents selected from the group consisting of halogen, hydroxy, oxo, thio, carboxyl, C₁-C₆ alkoxy, C₁-C₆ alkylthio, 6- to 10-membered aryl, and 5- to 10-membered heteroaryl.

In some embodiments, D is selected from structure represented by Formula (A-1),

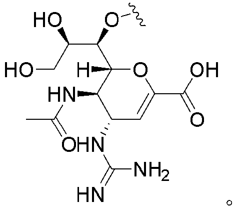

In some embodiments, m is 3 or 4.

In some embodiments, the linker comprises a backbone unit connected to D. The backbone unit may comprise a core moiety L₂ and branch moieties L₁ and L₃. The branch moiety L₁ is used for connecting E to the core moiety. The branch moiety L₃ is used for connecting D to the core moiety. There may be a plurality of branches L₃ connected to the core moiety.

In some embodiments, the linker comprises a repeating unit structure of (-CH₂(CH₂)ₓ₂-Y₁-)ₓ₁, wherein x1 is an integer from 0 to 100, x2 is an integer from 0 to 10, and Y₁ is selected from the group consisting of O and CH₂, such as O. In some embodiments, x2 is 1. In some embodiments, the linker comprises a polyethylene glycol (PEG) structure.

In some embodiments, the core moiety structure L₂ is selected from 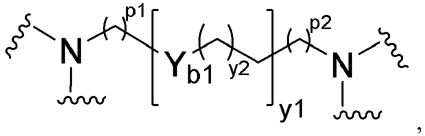 wherein, y1 is an integer from 0 to 100, such as an integer from 0 to 30; y2 is an integer from 0 to 10, such as an integer from 0 to 6, such as 1 or 2; Y_{b1} is selected from O or CH₂, such as O; p1 and p2 are each independently selected from the group consisting of integers from 0 to 10, such as an integer from 0 to 6, such as 1 or 2; or 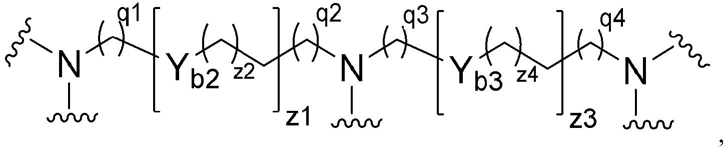 wherein, z1 is an integer from 0 to 100, such as an integer from 0 to 30; z2 is an integer from 0 to 10, such as an integer from 0 to 6, such as 1 or 2; Y_{b2} is selected from the group consisting of O and CH₂, such as O; z3 is an integer from 0 to 100, such as an integer from 0 to 30; z4 is an integer from 0 to 10, such as an integer from 0 to 6, such as 1 or 2; Y_{b3} is selected from the group consisting of O and CH₂; q1, q2, q3, and q4 are each independently selected from the group consisting of integers from 0 to 10, such as an integer from 0 to 6; or

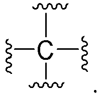

E or D may be connected to any one nitrogen atom.

In some embodiments, the linker comprises a branch structure L₁, which is a chemical structural fragment having one end covalently connected to E through a carbon atom and the other end optionally connected to the core moiety of the linker through a repeating unit structure.

In some embodiments, a molecule containing an azide group can be used to form an end group, wherein the azide group can undergo cycloaddition with an alkyne to form a 1,2,3-triazole linkage. In some embodiments, a molecule containing an alkynyl can be used to form an end group, wherein the alkynyl can undergo cycloaddition with an azide to form a 1,2,3-triazole linkage. In some embodiments, a molecule containing a maleimide group can be used to form an end group, wherein the maleimide group can react with cysteine to form a C-S linkage. In some embodiments, a molecule containing one or more sulfonic acid groups can be used to form an end group, wherein the sulfonic acid group can form a sulfonamide linkage with a connecting nitrogen in the neuraminidase inhibitor. In some embodiments, a molecule containing one or more isocyanate groups can be used to form an end group, wherein the isocyanate group can form a urea linkage with a connecting nitrogen in the neuraminidase inhibitor. In some embodiments, a molecule containing one or more haloalkyl can be used to form an end group, wherein the haloalkyl can form a covalent linkage, such as a C-N and C-O linkage, with the neuraminidase inhibitor.

Covalently conjugating two or more components in the conjugate using end group can be achieved using well-known organic chemistry synthesis techniques and methods. Complementary functional groups on the two components can react with each other to form a covalent bond. Examples of complementary reactive functional groups include, but are not limited to, for example, maleimide and cysteine, amine and activated carboxylic acid, thiol and maleimide, activated sulfonic acid and amine, isocyanate and amine, azide and alkyne, and alkene and tetrazine. Site-specific conjugation to a polypeptide (for example, an Fc monomer, an Fc domain, an Fc-binding peptide, albumin, or an albumin-binding domain, etc.) can be achieved using techniques known in the art.

In some embodiments, the polypeptide E and the small molecule D of the present disclosure can be conjugated by means of the following manners: (a) a thiourea linkage (*i.e.,* -NH(C=S)NH-) to lysine of E; (b) a carbamate linkage (*i.e.,* -NH(C=O)-O) to lysine of E; (c) an amine linkage achieved through reductive amination between lysine and E (*i.e.,* -NHCH₂); (d) an amide linkage (*i.e.,* -NH-(C=O)CH₂) to lysine of E; (e) cysteine-maleimide binding between a maleimide in an end group and cysteine of E; (f) an amine linkage achieved through reductive amination between an end group and a carbohydrate of E (for example, a glycosyl of an Fc monomer, an Fc domain, an Fc-binding peptide, albumin, or an albumin-binding domain) (*i.e.,* -NHCH₂); (g) re-bridging cysteine binding, wherein the end group is conjugated to two cysteines of E; (h) an oxime linkage between an end group and a carbohydrate of E (for example, a glycosyl of an Fc monomer, an Fc domain, an Fc-binding peptide, albumin, or an albumin-binding domain); (i) an oxime linkage between an end group and an amino acid residue of E; (j) an azide linkage between an end group and E; (k) direct acylation of an end group to E; or (l) a thioether linkage between an end group and E.

In some embodiments, conjugation to E is via an active ester or the like (such as a nitrophenyl ester or an N-hydroxysuccinimide ester or a derivative thereof (such as a functionalized PEG linker (such as an azido-PEG₂-PEG₄₀-NHS ester))). In these cases, an E-(PEG₂-PEG₄₀)-azide can be conjugated via click chemistry to an intermediate having a terminal alkyne linker. During click conjugation, the catalyzed azide (such as an Fc-(PEG₂-PEG₄₀)-azide) reacts with the terminal alkynyl of the intermediate to form a 5-membered heteroatom ring.

In some embodiments, L₁ may comprise the following reactive group Gₓ directly or indirectly connected to E, 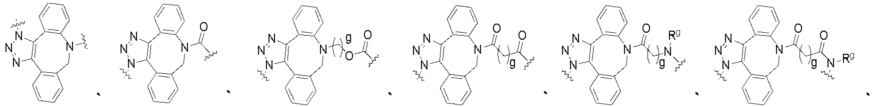 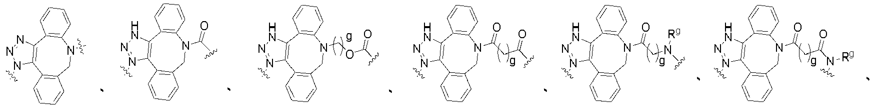 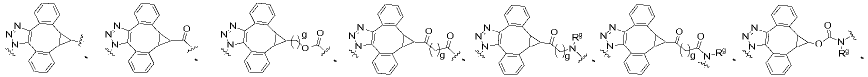 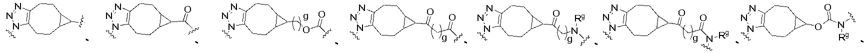 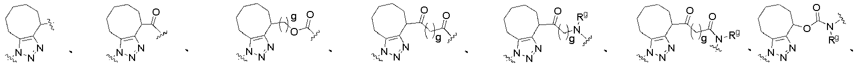 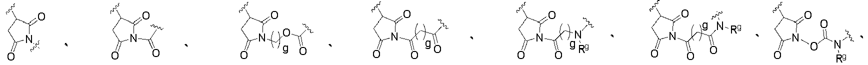 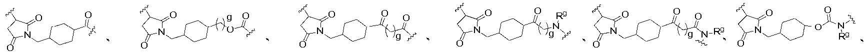 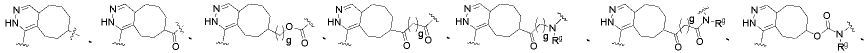 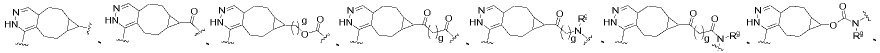 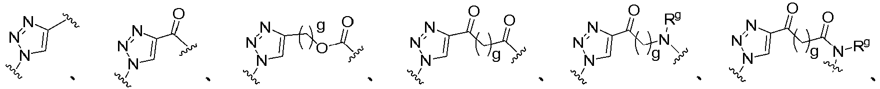 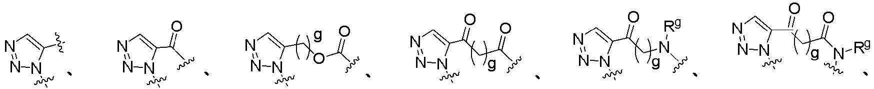 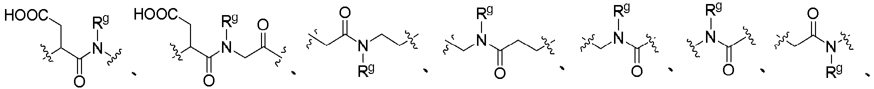 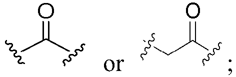 g is each independently selected from the group consisting of 0, 1, 2, 3, and 4; R^{g} is each independently selected from the group consisting of hydrogen and methyl.

In some embodiments, L₁ and E form an E-L₁- structure, and L₁ is selected from the group consisting of -Gₐ₁-(CH₂)_{wa1}-[Yₐ₁-CH₂(CH₂)ₓₐ₂]ₓₐ₁-(CH₂)_{wa2}-Gₐ₂-(CH₂)_{Wa3}-[Yₐ₂-CH₂(CH₂)ₓₐ₄]ₓₐ₃-(C H₂)_{wa4}-Gₐ₃-, wherein xa1 and xa3 are each independently selected from the group consisting of integers from 0 to 100, such as an integer from 0 to 30; xa2 and xa4 are each independently selected from the group consisting of integers from 0 to 10, such as an integer from 0 to 6, for example 1 or 2; wa1, wa2, wa3 and wa4 are each independently selected from the group consisting of integers from 0 to 10, such as an integer from 0 to 6; Yₐ₁ and Yₐ₂ are each independently selected from the group consisting of O and CH₂, such as O; Gₐ₁ is selected from the group Gₓ; and Gₐ₂ and Gₐ₃ are each independently selected from the group Gₓ or being absent.

In some embodiments, L₁ is selected from -Gₐ₁-(CH₂)_{wa1}-[O-CH₂CH₂]ₓₐ₁-(CH₂)_{wa2}-Gₐ₂-(CH₂)_{wa3}-[O-CH₂CH₂]ₓₐ₃-(CH₂)_{wa4}-Gₐ₃-.

In some embodiments, the linker comprises an amino acid unit, the amino acid unit, for example, comprising a peptide residue composed of 2 to 7 amino acids selected from the group consisting of phenylalanine, glycine, valine, lysine, citrulline, serine, glutamic acid, aspartic acid, homolysine, n-methyl-valine, and 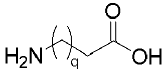 (wherein q is an integer from 1 to 6). Exemplary amino acid units include, but are not limited to, valine-citrulline (Val-Cit), alanine-phenylalanine (Ala-Phe), phenylalanine-lysine (Phe-Lys), phenylalanine-homolysine (Phe-Homolys), n-methyl-valine-citrulline (Me-Val-Cit), alanine-alanine (Ala-Ala), glycine-glutamic acid (Gly-Glu), glutamic acid-alanine-alanine (Glu-Ala-Ala), glycine-lysine (Gly-Lys), glycine-valine-citrulline (Glv-Val-Cit), glycine-glycine-glycine (Gly-Gly-Gly), and

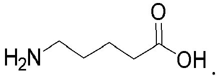

In some embodiments, the branch moiety L₃ comprises an end group G_{c1} connected to D, forming a -G_{c1}-D structure. The end group G_{c1} may be selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, for example, -NR₆(C=O)-, -NR₆(C=S)-, -O(C=O)-, -(C=O)-, and -NR₆(C=O)CH₂-,

In some embodiments, L₃ is -(CH₂)_{wc1}-[Y_{c1}-CH₂(CH₂)_{xc2}]_{xc1}-(CH₂)_{wc2}-G_{c1}-,
wherein, each xc1 is independently an integer from 0 to 100, such as an integer from 0 to 30, for example 0, 1, 2, 3, 4, 5, 6, 7, 8, 9, or 10;
each xc2 is independently an integer from 0 to 10, such as an integer from 0 to 6, for example 1 or 2;
each Y_{c1} is independently selected from the group consisting of O and CH₂, such as O;
wc1 and wc2 are each independently selected from the group consisting of integers from 0 to 10, such as an integer from 0 to 6, for example 0, 1, 2, or 3;
each G_{c1} is independently selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, for example, -NR₆(C=O)-, -NR₆(C=S)-, -O(C=O)-, -(C=O)-, and -NR₆(C=O)CH₂-;
m is 3 or 4.

In some embodiments, L₃ is -(CH₂)_{wc1}-[O-CH₂CH₂]_{xc1}-(CH₂)_{wc2}-G_{c1}-.

In some embodiments, each xc1 is independently an integer from 2 to 30, such as 2, 3, 4, 5, 6, 7, 8, 9, or 10.

In some embodiments, the number of atoms in the L₃ backbone is greater than 7, such as greater than 8, for example greater than 9, such as 8, 9, 10, 11, 12, 13, 14, 15, 16, 17, 18, 19, 20, 21, 22, 23, 24, 25, 26, 27, 28, 29, 30, or any numerical range therebetween, such as 8 to 30 or 8 to 20.

The number of atoms in the L₃ backbone can be understood as the number of atoms in the straight-chain moiety between the group -(CH₂)_{wc1} and the group -G_{c1}-. For example, if L₃ is 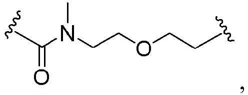 the backbone is 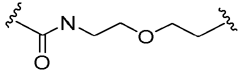 and the number of backbone atoms is 7; if L₃ is 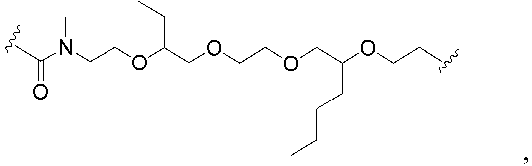 the backbone is 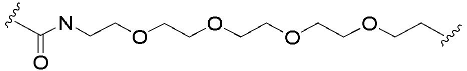 and the number of backbone atoms is 16.

In some embodiments, -L(-D)ₘ is -L₁-L₂(-L₃-D)ₘ, wherein,
L₁ is a moiety connected to E, and L₁ is selected from -Gₐ₁-(CH₂)_{wa1}-[Yₐ₁-CH₂(CH₂)ₓₐ₂]ₓₐ₁-(CH₂)_{wa2}-Gₐ₂-(CH₂)_{wa3}-[Yₐ₂-CH₂(CH₂)ₐ₄]ₓₐ₃-(CH₂) _{wa4}-Gₐ₃-, wherein,
xa1 and xa3 are each independently selected from the group consisting of integers from 0 to 100, such as an integer from 0 to 30;
xa2 and xa4 are each independently selected from the group consisting of integers from 0 to 10, such as an integer from 0 to 6, for example 1 or 2;
wa1, wa2, wa3 and wa4 are each independently selected from the group consisting of integers from 0 to 10, such as an integer from 0 to 6, for example 0, 1, or 2;
Yₐ₁ and Yₐ₂ are each independently selected from the group consisting of O and CH₂, such as O;
Gₐ₁ is selected from the group Gₓ; Gₐ₂ and Gₐ₃ are each independently selected from the group Gₓ or being absent; Gₓ is as described above;
L₂ is selected from the group consisting of 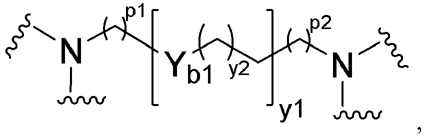
wherein, y1 is an integer from 0 to 100, such as an integer from 0 to 30, for example 0, 1, 2, 3, 4, 5, 6, 7, 8, 9, or 10;
y2 is an integer from 0 to 10, such as an integer from 0 to 6, for example 1 or 2;
Y_{b1} is selected from the group consisting of O and CH₂, such as O;
p1 and p2 are each independently selected from the group consisting of integers from 0 to 10, such as an integer from 0 to 6, for example 1 or 2; or 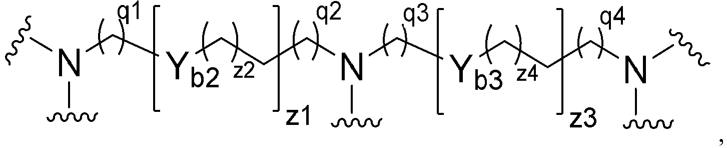
wherein, z1 is an integer from 0 to 100, such as an integer from 0 to 30, for example 0, 1, 2, 3, 4, 5, 6, 7, 8, 9, or 10;
z2 is an integer from 0 to 10, such as an integer from 0 to 6, for example 1 or 2;
Y_{b2} is selected from the group consisting of O and CH₂, such as O;
z3 is an integer from 0 to 100, such as an integer from 0 to 30, for example 0, 1, 2, 3, 4, 5, 6, 7, 8, 9, or 10;
z4 is an integer from 0 to 10, such as an integer from 0 to 6, for example 1 or 2;
Y_{b3} is selected from the group consisting of O and CH₂, preferably O;
q1, q2, q3, and q4 are each independently selected from the group consisting of integers from 0 to 10, such as an integer from 0 to 6; or 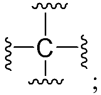
L₃ is -(CH₂)_{wc1}-[Y_{c1}-CH₂(CH₂)ₓₑ₂]x_{c1}-(CH₂)_{wc2}-G_{c1}-,
wherein each xc1 is independently an integer from 0 to 100, such as an integer from 0 to 30, for example 0, 1, 2, 3, 4, 5, 6, 7, 8, 9, or 10;
each xc2 is independently an integer from 0 to 10, such as an integer from 0 to 6, for example 1 or 2;
each Y_{c1} is independently selected from the group consisting of O and CH₂, such as O;
wc1 and wc2 are each independently selected from the group consisting of integers from 0 to 10, such as an integer from 0 to 6, for example 0, 1, 2, or 3;
each G_{c1} is independently selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, for example, -NR₆(C=O)-, -NR₆(C=S)-, -O(C=O)-, -(C=O)-, and -NR₆(C=O)CH₂-;
m is 3 or 4.

In some embodiments, each xc1 is independently an integer from 2 to 30, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, or any numerical range therebetween, such as 2 to 20, and the like.

In some embodiments, the number of atoms in the L₃ backbone is greater than 7, such as greater than 8, for example greater than 9, such as 8, 9, 10, 11, 12, 13, 14, 15, 16, 17, 18, 19, 20, 21, 22, 23, 24, 25, 26, 27, 28, 29, 30, or any numerical range therebetween, such as 8 to 30 or 8 to 20. In some embodiments, n is any value between 1 and 15, such as any value between 2 and 12. n can be an integer or a decimal.

In some embodiments, E comprises a half-life extension domain; for example, E is selected from the group consisting of an Fc domain, albumin, and an albumin-binding domain.

In some embodiments, the Fc domain is a monomer or a dimer. In some embodiments, the Fc domain is an Fc domain monomer, which comprises at least a portion of a C_{H}2 antibody constant domain and a C_{H}3 antibody constant domain. In some embodiments, the Fc domain monomer may have an immunoglobulin antibody isotype IgG, IgE, IgM, IgA, or IgD. The Fc domain monomer may also have any immunoglobulin antibody isotype (for example, IgG1, IgG2a, IgG2b, IgG3, or IgG4). The Fc domain monomer may have any immunoglobulin antibody allotype (for example, IGHG1*01 (i.e., G1m(za)), IGHG1*07 (i.e., G1m(zax)), IGHG1*04 (i.e., G1m(zav)), IGHG1*03 (G1m(f)), IGHG1*08 (i.e., G1m(fa)), IGHG2*01, IGHG2*06, IGHG2*02, IGHG3*01, IGHG3*05, IGHG3*10, IGHG3*04, IGHG3*09, IGHG3*11, IGHG3*12, IGHG3*06, IGHG3*07, IGHG3*08, IGHG3*13, IGHG3*03, IGHG3*14, IGHG3*15, IGHG3*16, IGHG3*17, IGHG3*18, IGHG3*19, IGHG2*04, IGHG4*01, IGHG4*03, or IGHG4*02). The Fc domain monomer may also belong to any species, such as human, murine, or mouse. A dimerized system of the Fc domain monomers can bind to an Fc domain of an Fc receptor, which is a receptor located on the surface of leukocytes. In some embodiments, the Fc domain comprises a hinge domain; in some embodiments, the Fc domain does not comprise a hinge domain.

In some embodiments, the Fc domain of the present disclosure comprises two Fc domain monomers dimerized through an interaction between C_{H}3 antibody constant domains, and one or more disulfide bonds formed between the hinge domains of the two dimerized Fc domain monomers. In some embodiments, the two Fc domain monomers may be the same or different. The Fc domain comprises a minimal structure capable of binding to an Fc receptor, such as an Fc-γ receptor (i.e., Fcγ receptor (FcγR)), an Fc-α receptor (i.e., Fcα receptor (FcαR)), an Fc-ε receptor (i.e., Fcε receptor (FcεR)), and/or a neonatal Fc receptor (FcRn). In some embodiments, the Fc domain of the present disclosure binds to an Fcy receptor (for example, FcRn, FcγRI (CD64), FcγRIIa (CD32), FcγRIIb (CD32), FcγRIIIa (CD16a), FcγRIIIb (CD16b)) and/or FcγRIV and/or a neonatal Fc receptor (FcRn).

In some embodiments, the Fc domain comprises a native Fc region, or an Fc region variant having one or more alterations relative to the native Fc region. The alterations may include amino acid substitutions, additions, and/or deletions, the attachment of additional moieties, and/or alterations of native glycans. In some embodiments, the Fc domain also encompasses single-chain Fc regions, wherein the constituent Fc domains are linked together through a linker moiety.

In some embodiments, the Fc domain of the present disclosure is an aglycosylated Fc domain (for example, an Fc domain that maintains the capability to bind to an Fc receptor (for example, FcRn)). For example, the Fc domain is an aglycosylated IgG1 variant that maintains the capability to bind to an Fc receptor (for example, an IgG1 having an amino acid substitution at N297 and/or T299 of a glycosylation motif). Exemplary aglycosylated Fc domains and methods for preparing aglycosylated Fc domains are known in the art.

The Fc domain of the present disclosure may be selected from an antibody.

Antibodies include full-length antibodies, antigen-binding fragments of full-length antibodies, and molecules comprising antibody CDRs, VH domains (VH), or VL domains (VL). Examples of antibodies include monoclonal antibodies, recombinantly produced antibodies, monospecific antibodies, multispecific antibodies (including bispecific antibodies), human antibodies, humanized antibodies, chimeric antibodies, immunoglobulins, synthetic antibodies, tetrameric antibodies comprising two heavy chain and two light chain molecules, antibody light chain monomers, antibody heavy chain monomers, antibody light chain dimers, antibody heavy chain dimers, antibody light chain-antibody heavy chain pairs, intrabodies, heteroconjugated antibodies, antibody-drug conjugates, single-domain antibodies (sdAb), monovalent antibodies, single-chain antibodies or single-chain Fv (scFv), camelid antibodies, affibody molecules, VHH fragments, Fab fragments, F(ab')₂ fragments, disulfide-linked Fvs (sdFv), and antigen-binding fragments of any of the above antibodies.

In some embodiments, the antibody is a human, mouse, camelid (for example, llama, alpaca, or camel), goat, sheep, rabbit, chicken, guinea pig, hamster, horse, or rat antibody or an antigen-binding fragment thereof. In some embodiments, the antibody is IgG, IgA, IgD, IgE, IgM. In some embodiments, the antigen-binding fragment comprises a Fab, a modified Fab, a Fab', a modified Fab', an F(ab')₂, an Fv, a Fab-Fv, a Fab-dsFv, a single-domain antibody (for example, VH, VL, or VHH), an scFv, a bivalent, trivalent, or tetravalent antibody, a Bis-scFv, a diabody, a tribody, a triabody, a tetrabody, and an epitope-binding fragment of any of the above.

In some embodiments, the antibody confers binding specificity for one or more targets (for example, antigens).

In some embodiments, the one or more targets (for example, antigens) bound by the antibody is a viral (for example, influenza) protein, such as neuraminidase or hemagglutinin. In some embodiments, the antibody or antibody fragment recognizes a viral surface antigen. In some embodiments, the antibody targets hemagglutinin. Hemagglutinin-targeting antibodies include monoclonal antibodies, such as CR6261, CR8020, MEDI8852, MHAA4549A, and VIS410. In some embodiments, the antibody is a broadly neutralizing antibody or an antigen-binding fragment targeting influenza hemagglutinin. In some embodiments, the antibody targets a viral matrix protein (for example, matrix 2 protein). TCN032 is a matrix 2 protein-targeting monoclonal antibody. In some embodiments, the antibody targets human serum albumin.

In some embodiments, the antibody comprises one or more single-domain antibodies (sdAb). In some embodiments, the Fc domain-containing composition is an antibody or an antibody fragment as follows, which comprises an sdAb having influenza A reactivity, such as an sdAb conjugated to hemagglutinin of influenza A. In some embodiments, the antibody comprises an sdAb having influenza B reactivity, such as an sdAb conjugated to hemagglutinin of influenza B.

In some embodiments, the antibody is a multi-domain antibody (MDAb) or a multi-domain antibody fragment comprising 2 or more (for example, 2, 3, 4, 5, 6, 7, 8, 9, or 10 or more) sdAbs. In some embodiments, the MDAb or the fragment thereof comprises one or more sdAbs conjugated to hemagglutinin of influenza A, and one or more sdAbs conjugated to hemagglutinin of influenza B.

In some embodiments, the Fc domain has an enhanced effector function, for example, the effector function is selected from the group consisting of C1q binding and complement-dependent cytotoxicity, Fc receptor (for example, FcyR) binding, antibody-dependent cellular cytotoxicity (ADCC), phagocytosis, downregulation of cell surface receptors (for example, B cell receptors), and B cell activation.

In some embodiments, the Fc domain may be the following sequence:
(I) a sequence as set forth in SEQ ID NO: 1 or a sequence having at least 70%, 80%, 90%, 95%, 98%, or 99% identity to SEQ ID NO: 1; or
(II) a sequence as set forth in SEQ ID NO: 2 or a sequence having at least 70%, 80%, 90%, 95%, 98%, or 99% identity to SEQ ID NO: 2;
(III) a sequence as set forth in SEQ ID NO: 3 or a sequence having at least 70%, 80%, 90%, 95%, 98%, or 99% identity to SEQ ID NO: 3; or
(IV) a sequence as set forth in SEQ ID NO: 4 or a sequence having at least 70%, 80%, 90%, 95%, 98%, or 99% identity to SEQ ID NO: 4; or
(V) a sequence as set forth in SEQ ID NO: 5 or a sequence having at least 70%, 80%, 90%, 95%, 98%, or 99% identity to SEQ ID NO: 2;
(VI) a sequence as set forth in SEQ ID NO: 6 or a sequence having at least 70%, 80%, 90%, 95%, 98%, or 99% identity to SEQ ID NO: 3; or
(VII) a sequence as set forth in SEQ ID NO: 7 or a sequence having at least 70%, 80%, 90%, 95%, 98%, or 99% identity to SEQ ID NO: 4; or
(VIII) a sequence as set forth in SEQ ID NO: 8 or a sequence having at least 70%, 80%, 90%, 95%, 98%, or 99% identity to SEQ ID NO: 8.

In some embodiments, the Fc domain is an antibody. Exemplarily, the antibody comprises a heavy chain and a light chain. Exemplarily, the heavy chain comprises a sequence as set forth in SEQ ID NO: 9 or a sequence having at least 70%, 80%, 90%, 95%, 98%, or 99% identity to SEQ ID NO: 9; the light chain comprises a sequence as set forth in SEQ ID NO: 10 or a sequence having at least 70%, 80%, 90%, 95%, 98%, or 99% identity to SEQ ID NO: 10.

All mutations are named and numbered according to the EU numbering system, and bold underlines indicate specific mutation sites.
>Human IgG1 Fc fragment (Asn201-Gly446) with C220S, M252Y, S254T, and T256E mutations 
NVNHKPSNTKVDKKVEPKSSDKTHTCPPCPAPELLGGPSVFLFPPKPKDTLYITREPEVTCVVVDVSHEDP
EVKFNWYVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPG
SEQ ID NO: 1
>Human IgG1 Fc fragment (Asn201-Gly446) with C220S, M252Y, S254T, T256E, H268C, and L443C mutations 
NVNHKPSNTKVDKKVEPKSSDKTHTCPPCPAPELLGGPSVFLFPPKPKDTLYITREPEVTCVVVDVSCEDP
EVKFNWYVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSDGSFFLYSKL
TVDKSRWQQGNVFSCSVMHEALHNHYTQKSLSCSPG
SEQ ID NO: 2
>Human IgG1 Fc fragment (Asn201-Gly446) with C220S, M252Y, S254T, T256E, K290C, and L443C mutations 
NVNHKPSNTKVDKKVEPKSSDKTHTCPPCPAPELLGGPSVFLFPPKPKDTLYITREPEVTCVVVDVSHEDP
EVKFNWYVDGVEVHNAKTCPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSDGSFFLYSKL
TVDKSRWQQGNVFSCSVMHEALHNHYTQKSLSCSPG
SEQ ID NO: 3
>Human IgG1 Fc fragment (Asn201-Gly446) with C220S, M252Y, S254T, T256E, L398C, and L443C mutations 
NVNHKPSNTKVDKKVEPKSSDKTHTCPPCPAPELLGGPSVFLFPPKPKDTLYITREPEVTCVVVDVSHEDP
EVKFNWYVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVCDSDGSFFLYSKL
TVDKSRWQQGNVFSCSVMHEALHNHYTQKSLSCSPG
SEQ ID NO: 4
>Human IgG1 Fc fragment (Asn201-Gly446) with C220S, M252Y, S254T, T256E, T223Y, and P329Y mutations 
NVNHKPSNTKVDKKVEPKSSDKYHTCPPCPAPELLGGPSVFLFPPKPKDTLYITREPEVTCVVVDVSHED
PEVKFNWYVDGVEVHNAKTKPREEQYNSTYRVVSVLTVLHQDWLNGKEYKCKVSNKALYAPIEKTISK
AKGQPREPQVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG
SEQ ID NO: 5
>Human IgG1 Fc fragment (Asn201-Gly446) with C220S, M252Y, S254T, T256E, and P329Y mutations, and a GGGGY sequence added at the C-terminus 
NVNHKPSNTKVDKKVEPKSSDKTHTCPPCPAPELLGGPSVFLFPPKPKDTLYITREPEVTCVVVDVSHEDP
EVKFNWYVDGVEVHNAKTKPREEQYNSTYRVVSVLTVLHQDWLNGKEYKCKVSNKALYAPIEKTISKA
KGQPREPQVYTLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGGGGGY
SEQ ID NO: 6
>Human IgG1 Fc fragment (Asn201-Gly446) with C220S, M252Y, S254T, T256E, and T223Y mutations, and a GGGGY sequence added at the C-terminus 
NVNHKPSNTKVDKKVEPKSSDKYHTCPPCPAPELLGGPSVFLFPPKPKDTLYITREPEVTCVVVDVSHED
PEVKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGY
SEQ ID NO: 7
>Human IgG1 Fc fragment (Asn201-Gly446) with C220A, M252Y, S254T, and T256E mutations 
NVNHKPSNTKVDKKVEPKSADKTHTCPPCPAPELLGGPSVFLFPPKPKDTLYITREPEVTCVVVDVSHED
PEVKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG
SEQ ID NO：8
>hIgG antibody heavy chain 
QVQLVQSGAEVKKPGASVKVSCKASGYTFTNSWIGWFRQAPGQGLEWIGDIYPGGGYTNYNEIFKGKAT
MTADTSTNTAYMELSSLRSEDTAVYYCSRGIPGYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSKLTVDKSRWQQ
GNVFSCSVMHEALHNHYTQKSLSLSPGK
SEQ ID NO：9
>hIgG antibody light chain

DIQMTQSPSSLSASVGDRVTMSCKSSQSLLNSGDQKNYLTWYQQKPGKAPKLLIYWASTGESGVPSRFSG
SGSGTDFTFTISSLQPEDIATYYCQNDYSYPWTFGQGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSPV
TKSFNRGEC
SEQ ID NO: 10

In some embodiments, the albumin is human albumin, such as human serum albumin.

In some embodiments, the albumin-binding domain is a polypeptide capable of binding to albumin, or a domain that binds to albumin, for example, an antibody or an antigen-binding fragment thereof (for example, VHH).

In some embodiments, the albumin or the albumin-binding domain is modified. For example, it contains one or more (for example, 2, 3, 4, 5, 6, 7, or 8) solvent-exposed cysteine (Cys) or lysine (Lys) residues after modification. In some embodiments, the modification mode is an amino acid mutation (for example, substitution). In some embodiments, the albumin or the albumin-binding domain is conjugated to a compound of the present disclosure (for example, a neuraminidase inhibitor monomer or dimer) through a modified or naturally occurring Cys and/or Lys.

In some embodiments, one of the aforementioned conjugates of the present disclosure may contain one or more (for example, 2, 3, or 4) proteins or polypeptides, and the protein or polypeptide may be any of the aforementioned Fc domains, albumins, or albumin-binding domains of the present disclosure. In some embodiments, one of the aforementioned conjugates of the present disclosure may contain one or more (for example, 2, 3, or 4) identical proteins or polypeptides; in other embodiments, one of the aforementioned conjugates of the present disclosure may contain a plurality of (for example, 2, 3, or 4) different proteins or polypeptides.

In some embodiments, the antiviral conjugate of the present disclosure is selected from the group consisting of: 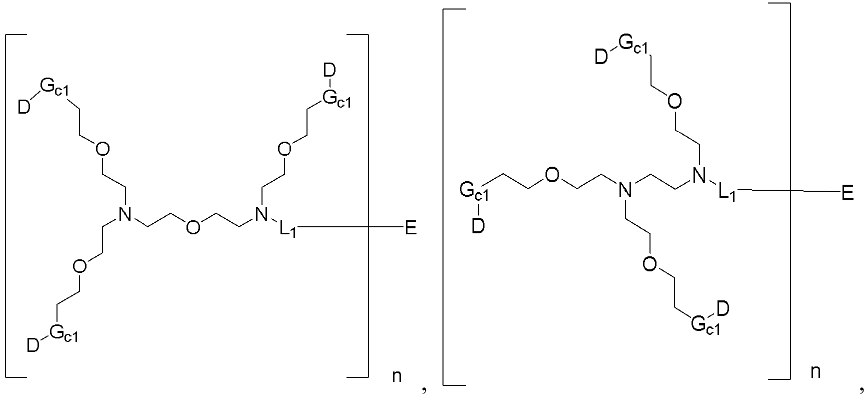 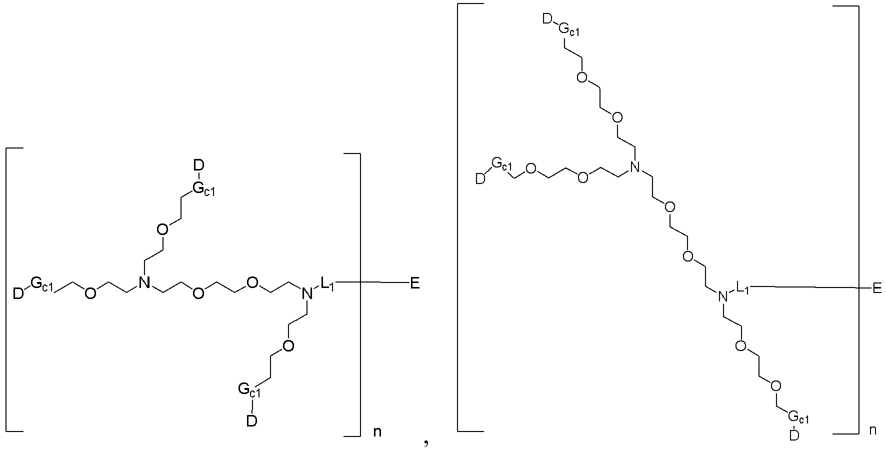 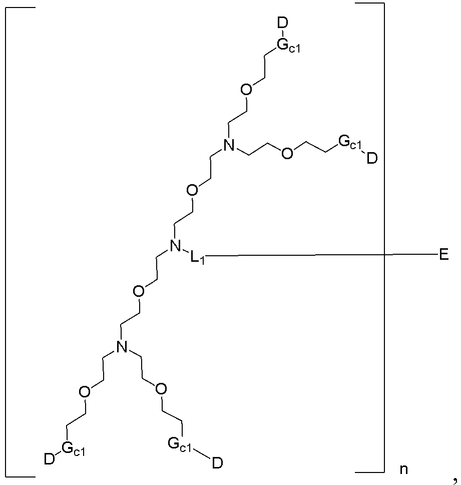 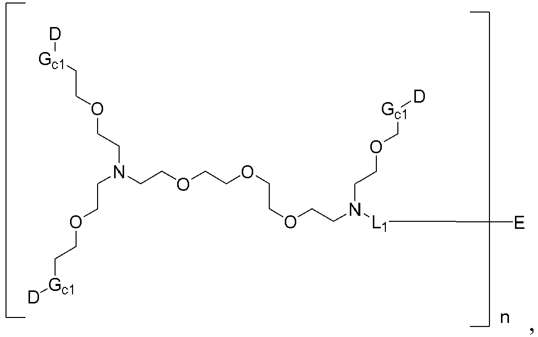 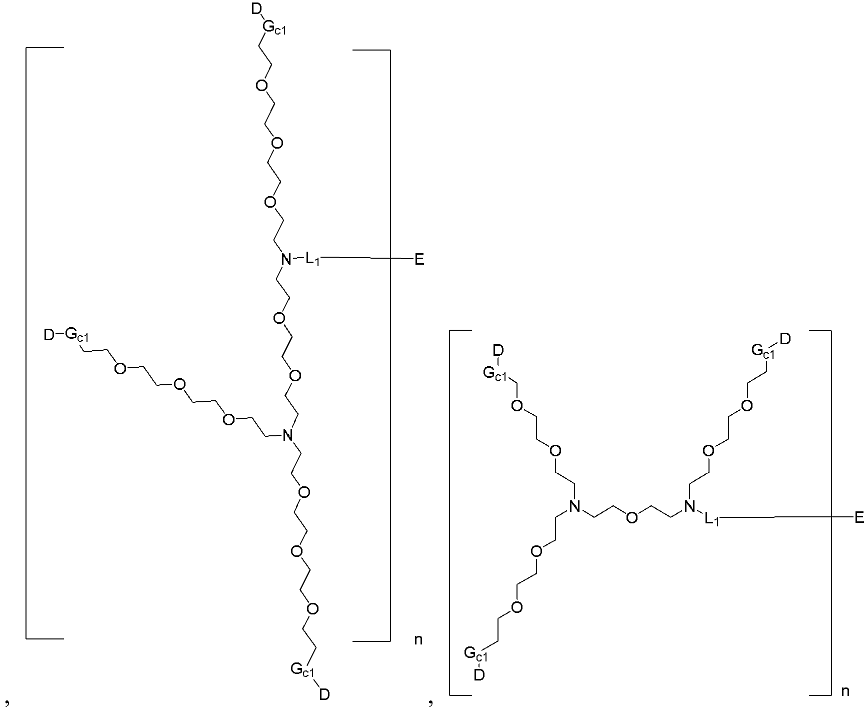 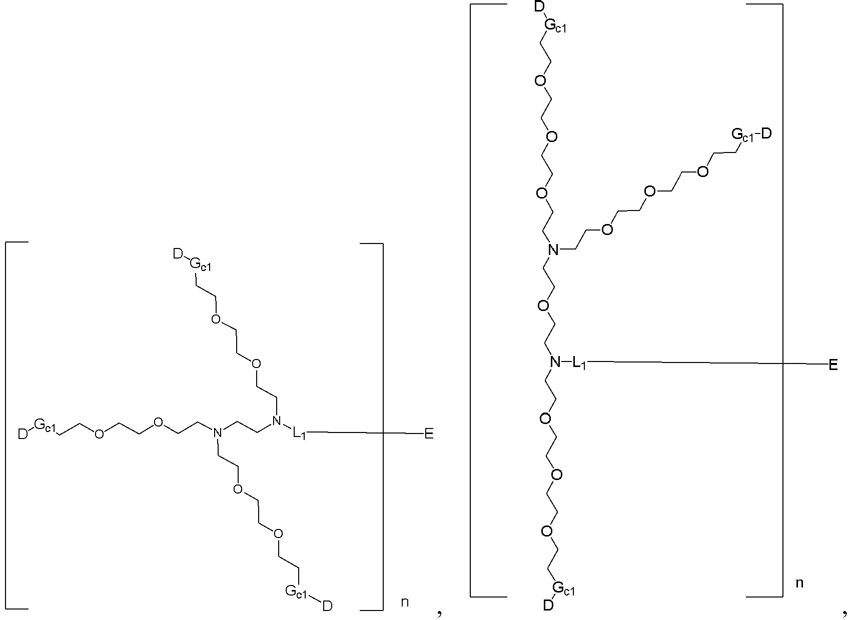 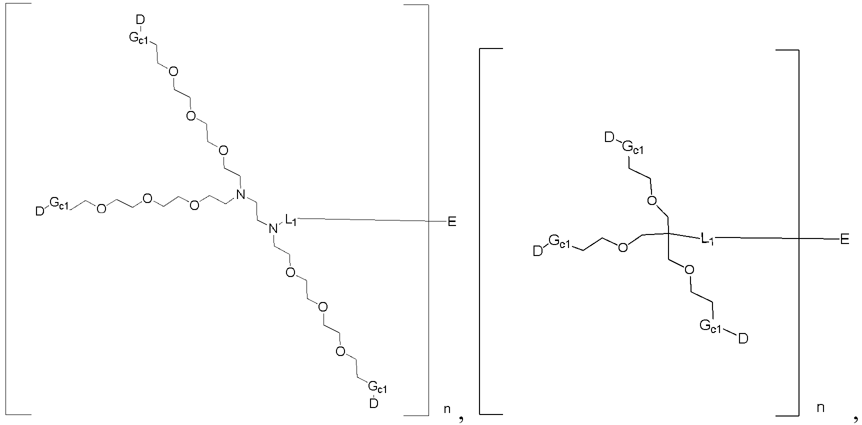 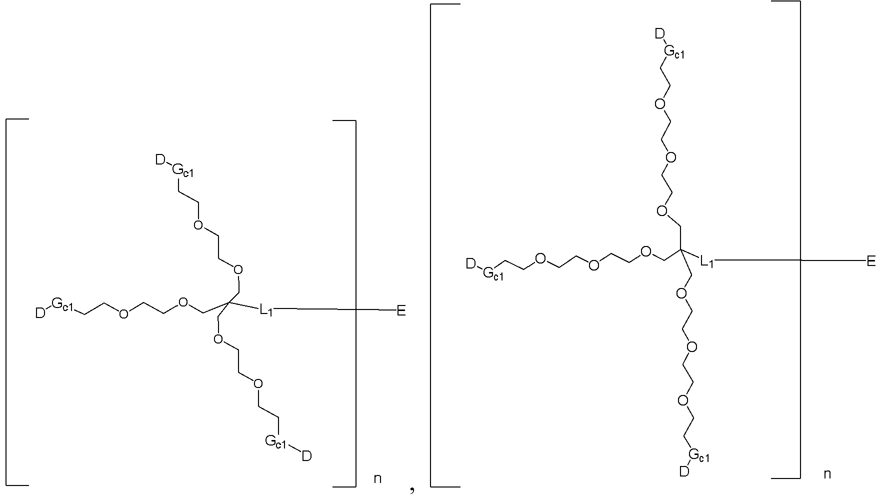 and 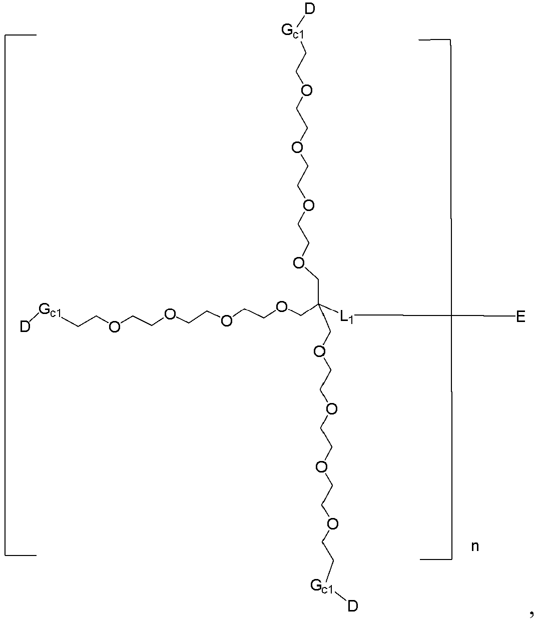
L₁ and E form an E-L₁- structure, and L₁ is selected from -Gₐ₁-(CH₂)_{wa1}-[Yₐ₁-CH₂(CH₂)ₓₐ₂]ₓₐ₁-(CH₂)_{wa2}-Gₐ₂-(CH₂)_{wa3}-[Yₐ₂-CH₂(CH₂)ₓₐ₄]ₓₐ₃-(CH₂) _{wa4}-Gₐ₃-, wherein xa1 and xa3 are each independently selected from the group consisting of integers from 0 to 100, such as an integer from 0 to 30; xa2 and xa4 are each independently selected from the group consisting of integers from 0 to 10, such as an integer from 0 to 6, for example 1 or 2; wa1, wa2, wa3 and wa4 are each independently selected from the group consisting of integers from 0 to 10, such as an integer from 0 to 6; Yₐ₁ and Yₐ₂ are each independently selected from the group consisting of O and CH₂; Gₐ₁ is selected from group Gₓ; Gₐ₂ and Gₐ₃ are each independently selected from group Gₓ or being absent;
Gₓ is selected from the group consisting of: 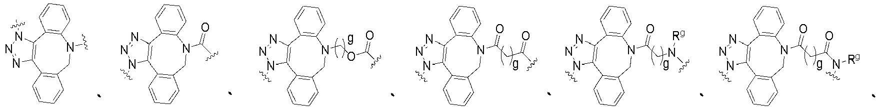 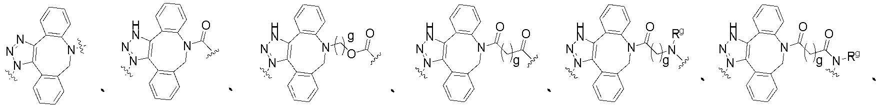 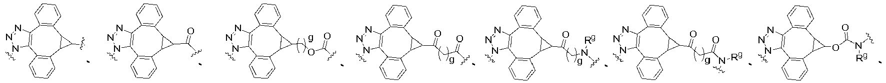 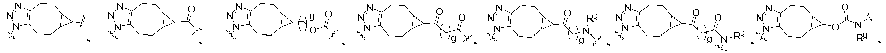 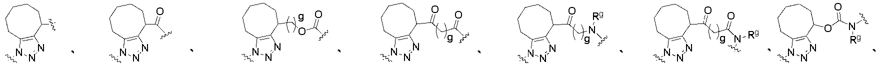 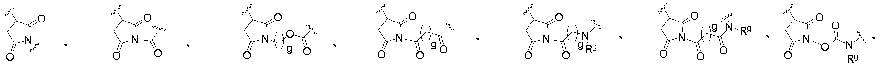 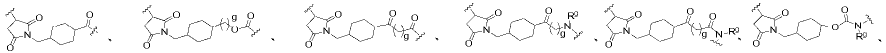 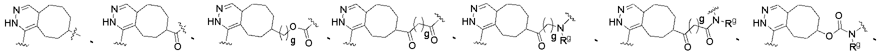 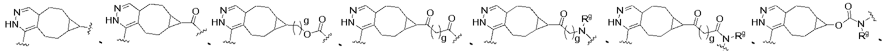 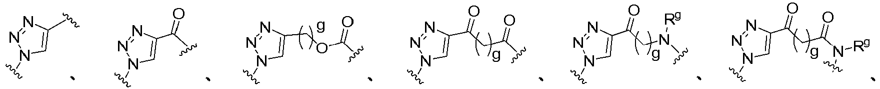 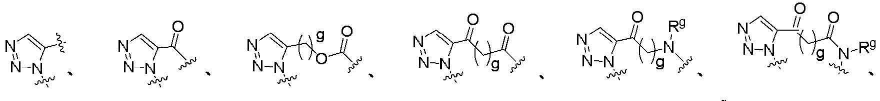 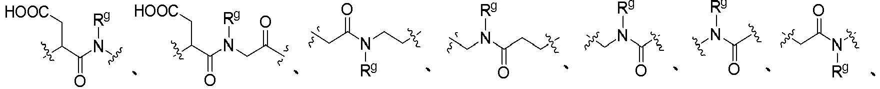 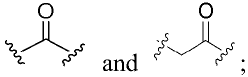 g is each independently selected from the group consisting of 0, 1, 2, 3, and 4; R^{g} is each independently selected from the group consisting of hydrogen and methyl;
G_{c1} is selected from the group consisting of -NR₆(C=O)-, -NR₆(C=S)-, -O(C=O)-, -(C=O)-, and -NR₆(C=O)CH₂-, wherein R₆ is selected from the group consisting of hydrogen and C₁-C₆ alkyl;
n is any value between 1 and 15.

In some embodiments, the antiviral conjugate of the present disclosure is selected from the group consisting of: 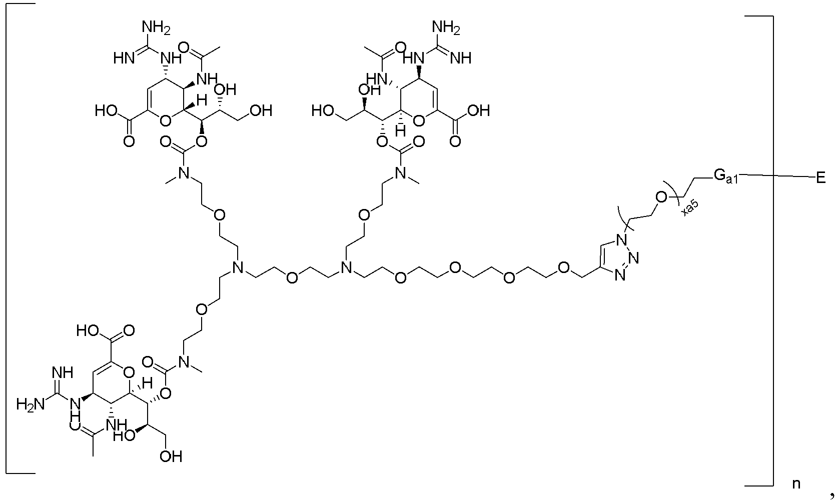 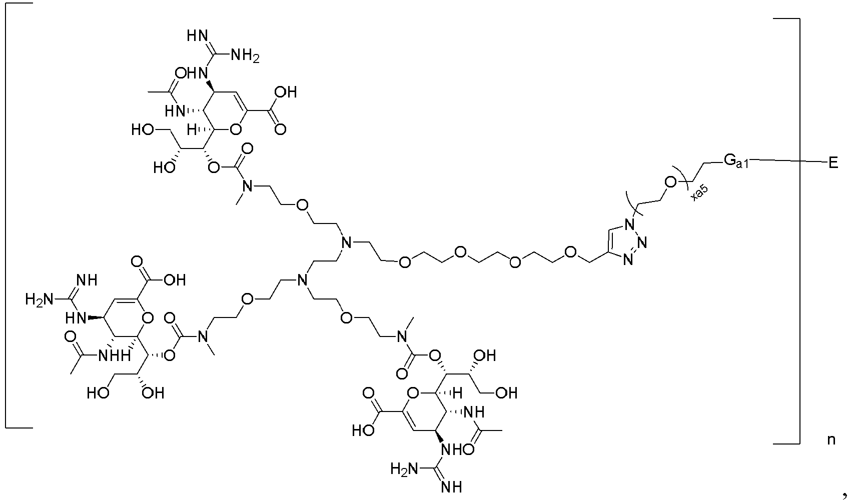 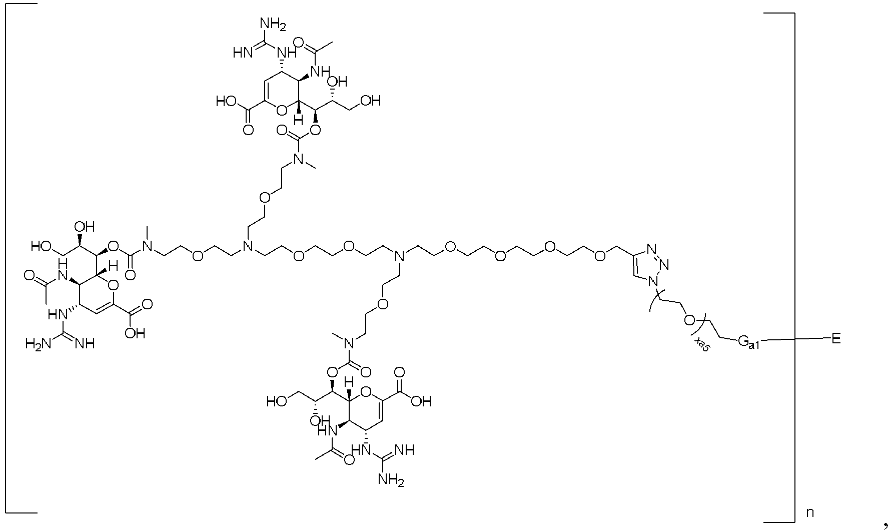 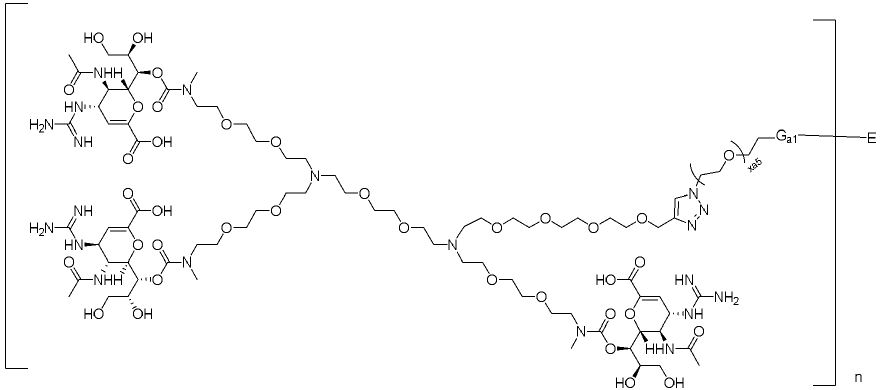 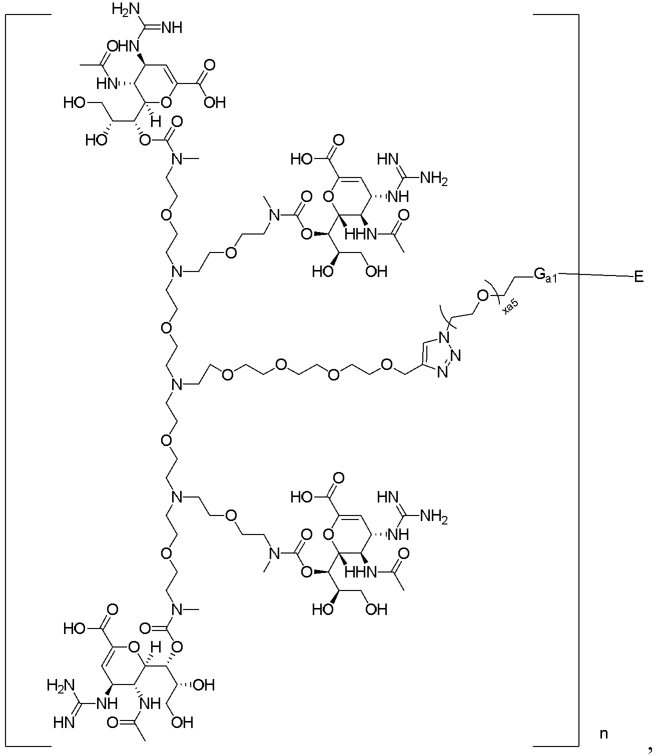 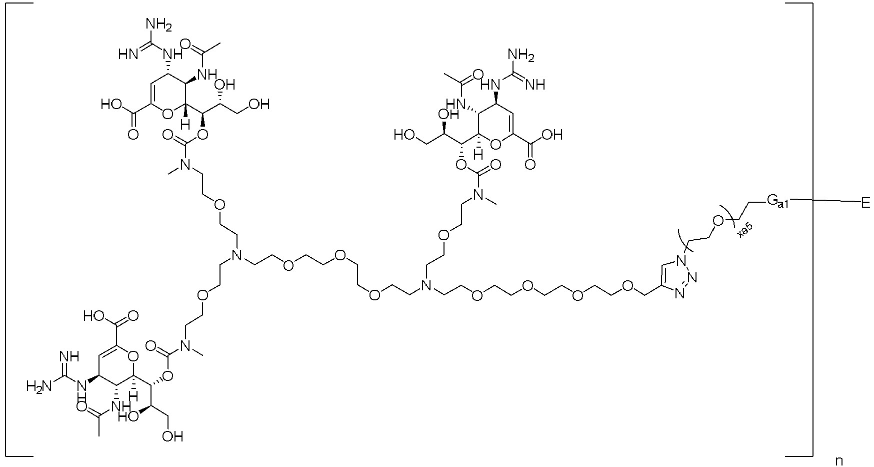 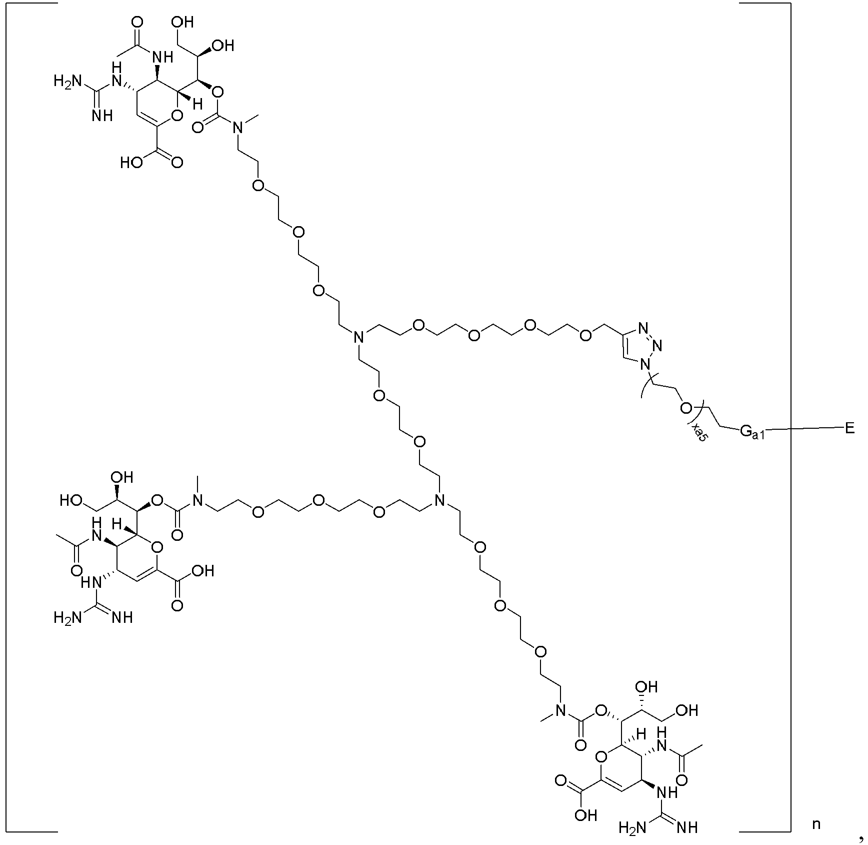 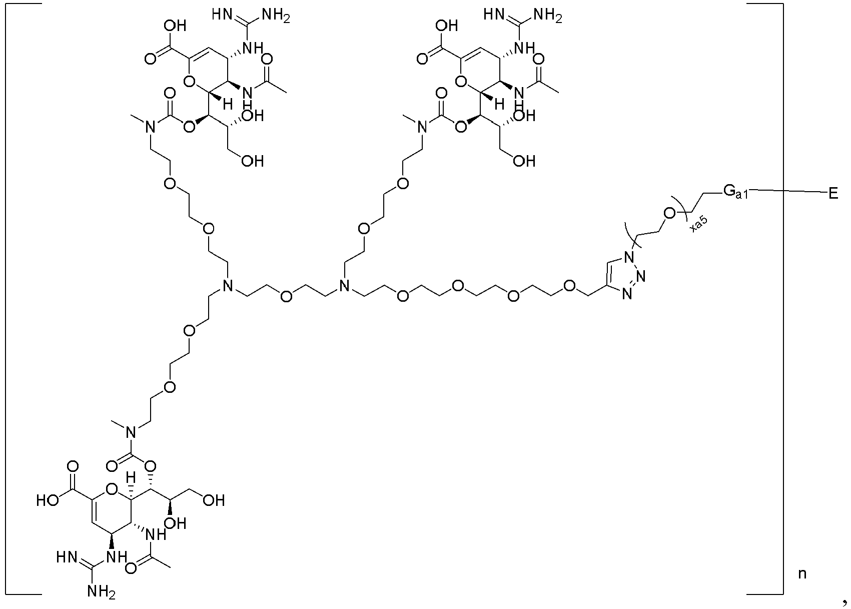 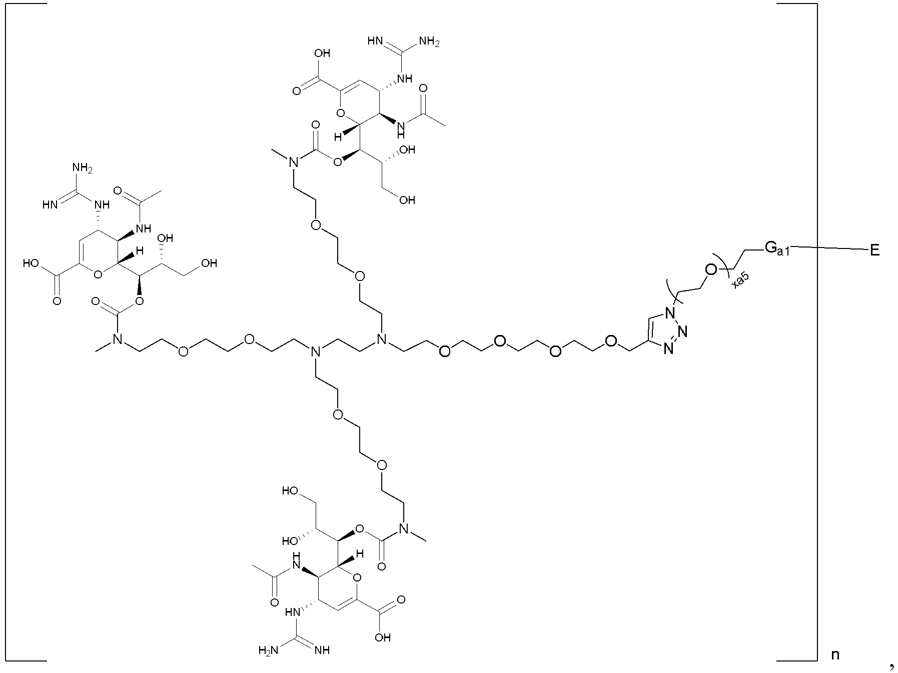 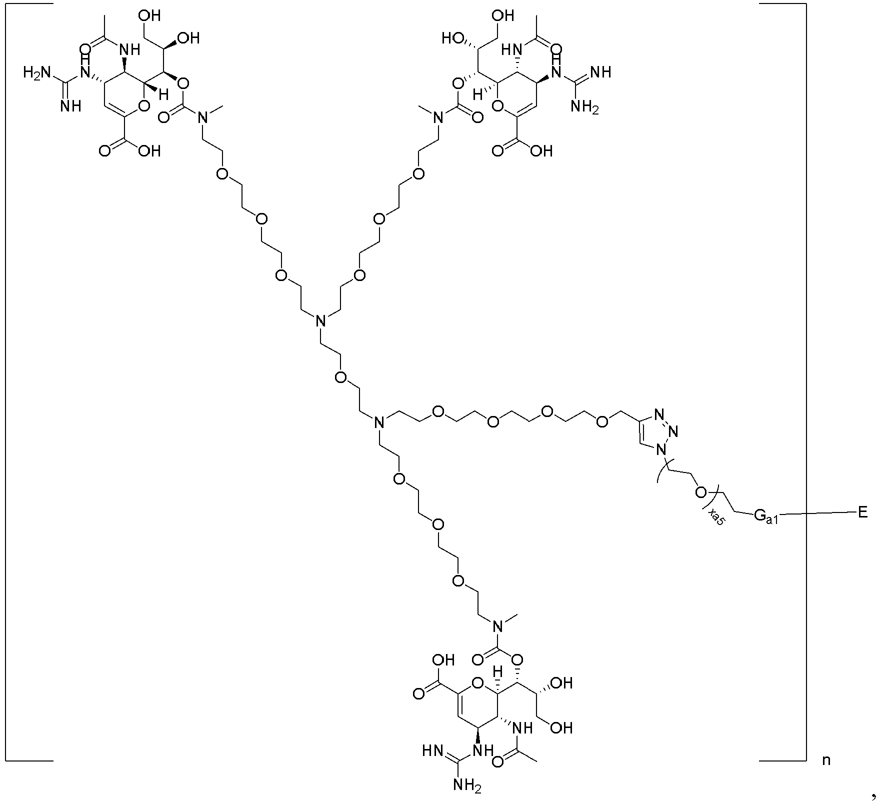 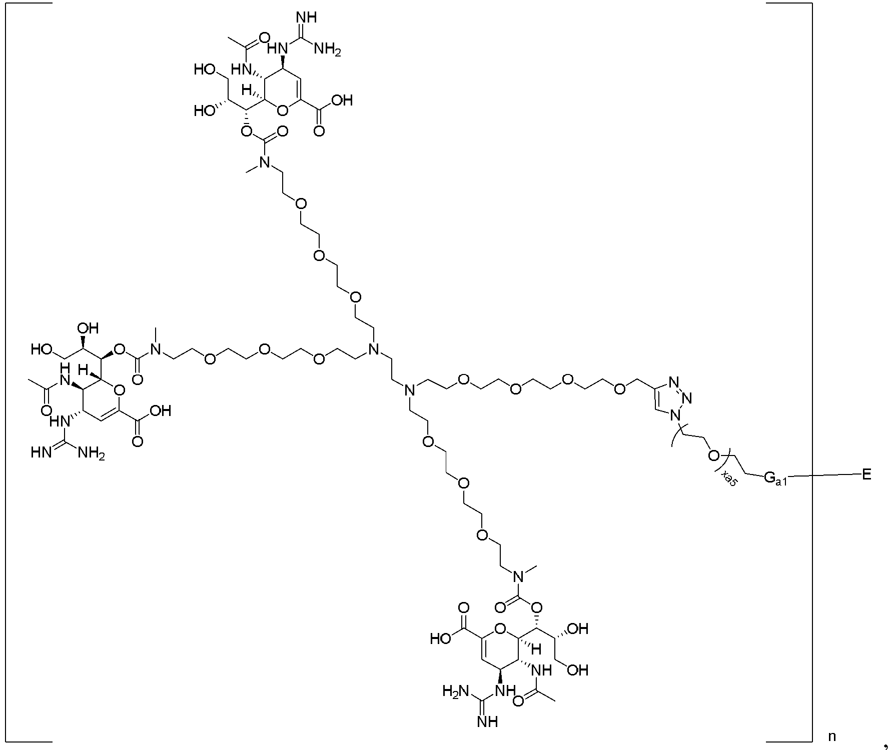 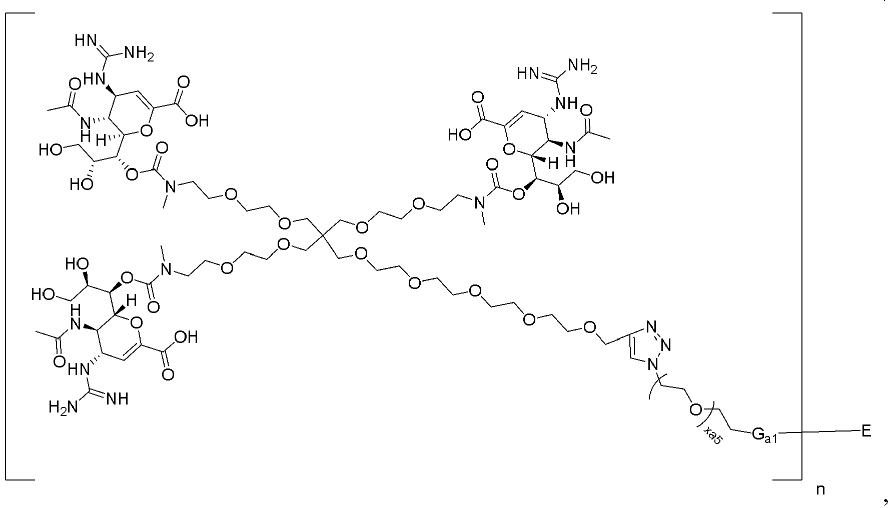 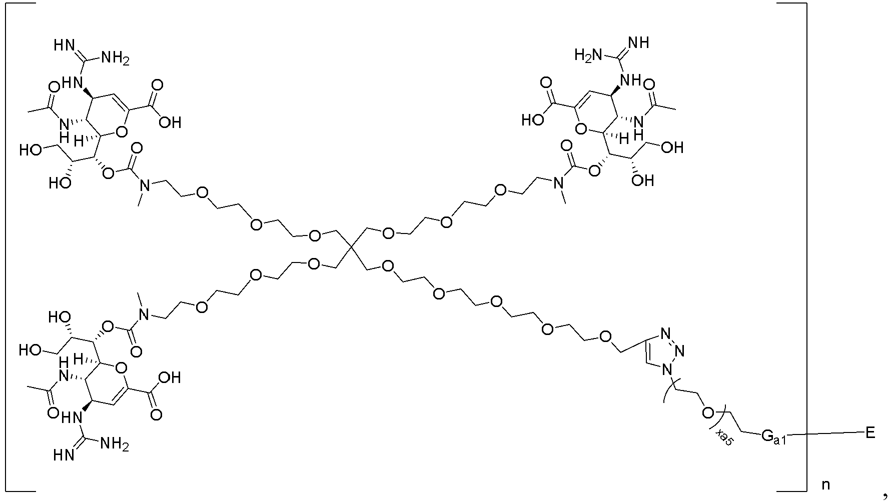 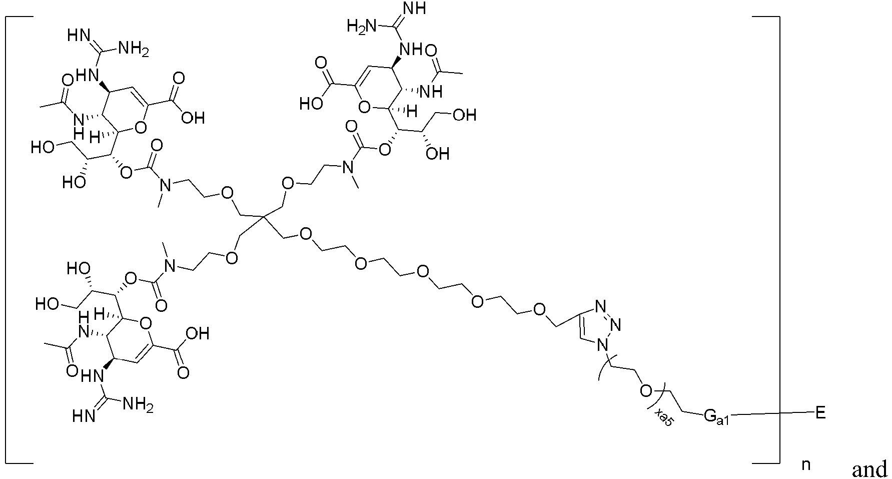 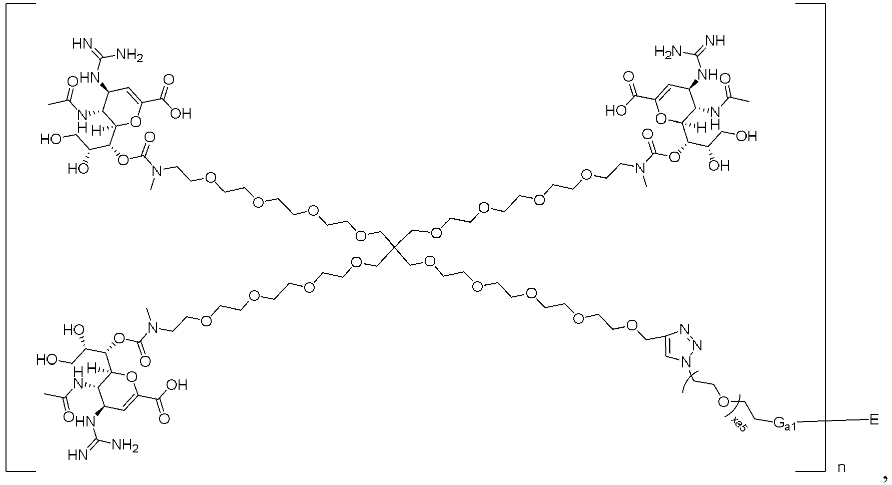 wherein,
each xa5 is independently selected from the group consisting of integers from 2 to 8,
Gₐ₁ is each independently selected from the group consisting of 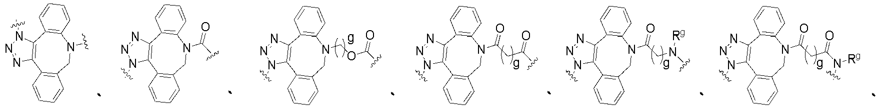 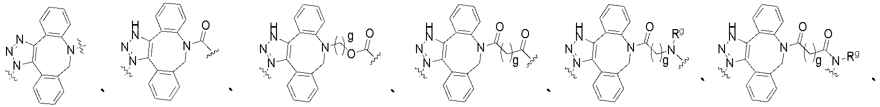 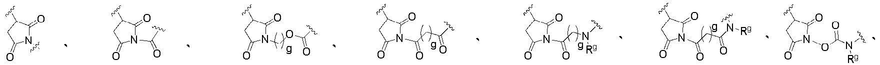 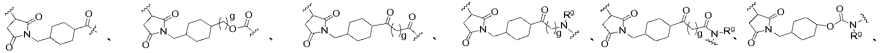 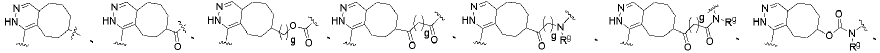 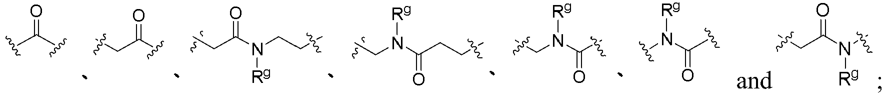
g is each independently selected from the group consisting of 0, 1, 2, 3, and 4; R^{g} is each independently selected from the group consisting of hydrogen and methyl;
n is any value between 1 and 15.

The present disclosure also provides a compound represented as follows,

L₁'-L₂(-L₃-D)ₘ,

wherein,
L₁' is selected from the group consisting of Gₓ'-(CH₂)_{wa1}-[Yₐ₁-CH₂(CH₂)ₓₐ₂]ₓₐ₁-(CH₂))_{wa2}-Gₐ₂-(CH₂)_{wa3}-[Yₐ₂-CH₂(CH₂)ₓₐ₄]ₓₐ₃-(CH₂)_{w a4}-Gₐ₃-, wherein,
xa1 and xa3 are each independently selected from the group consisting of integers from 0 to 100, such as an integer from 0 to 30;
xa2 and xa4 are each independently selected from the group consisting of integers from 0 to 10, such as an integer from 0 to 6, for example 1 or 2;
wa1, wa2, wa3 and wa4 are each independently selected from the group consisting of integers from 0 to 10, such as an integer from 0 to 6, for example 0, 1, or 2;
Yₐ₁ and Yₐ₂ are each independently selected from the group consisting of O and CH₂, such as O;
Gx' is each independently selected from the group consisting of 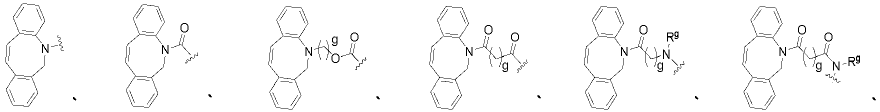 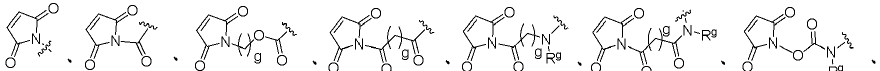 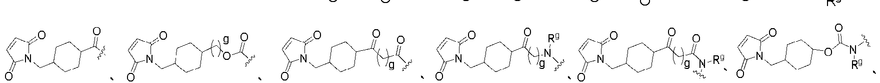 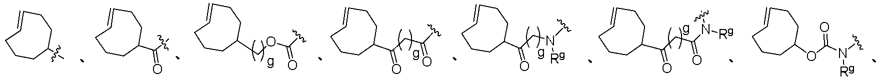 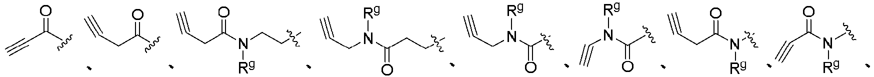 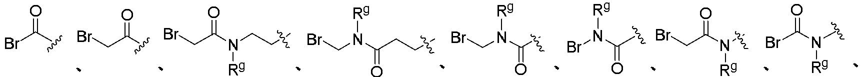 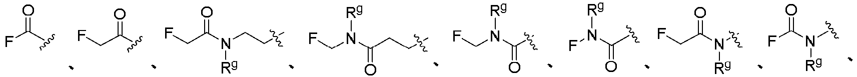 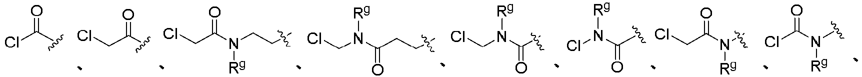 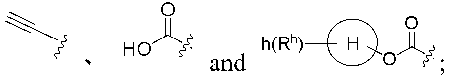
Gₐ₂ and Gₐ₃ are each independently selected from the group consisting of: 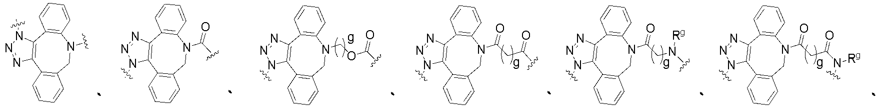 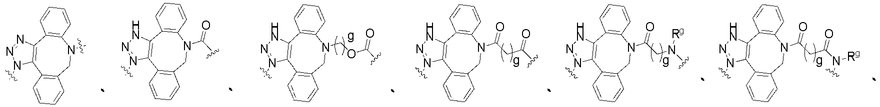 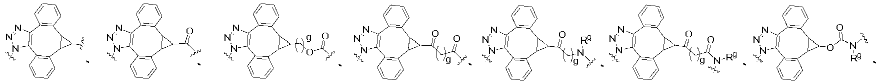 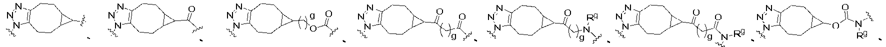 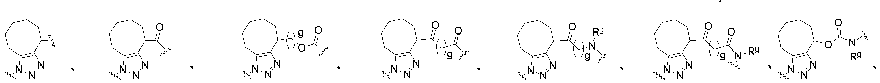 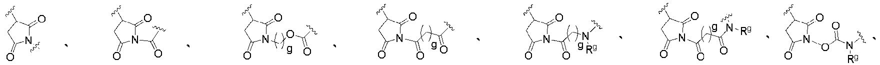 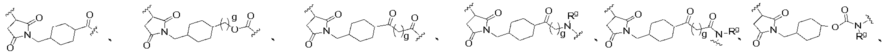 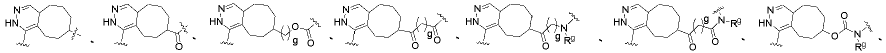 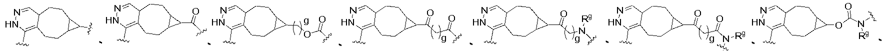 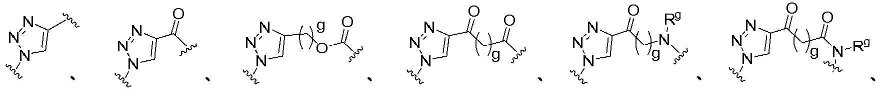 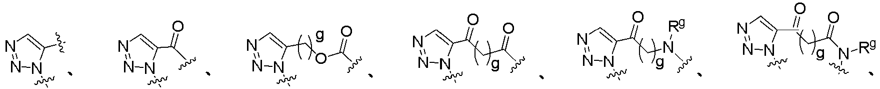 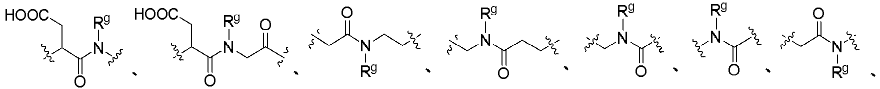 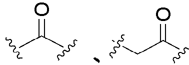 or being absent;
g is each independently selected from the group consisting of 0, 1, 2, 3, and 4; R^{g} is each independently selected from the group consisting of hydrogen and methyl;
Ring H is a 5- to 10-membered heteroaryl, 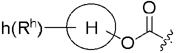 may be selected from the group consisting of 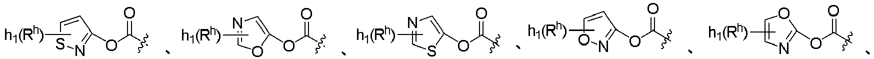 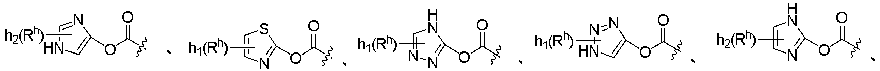 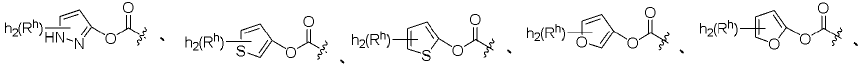 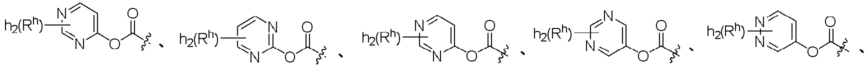 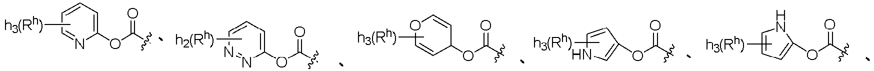 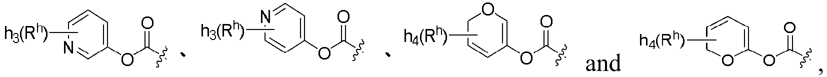 such as 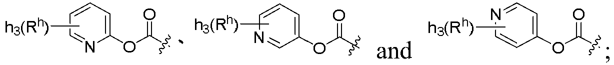
each Rₕ is independently selected from halogen;
h is selected from the group consisting of 0, 1, 2, 3, 4, and 5;
each h1 is independently selected from the group consisting of 0, 1, and 2;
each h2 is independently selected from the group consisting of 0, 1, 2, and 3;
each h3 is independently selected from the group consisting of 0, 1, 2, 3, and 4;
each h4 is independently selected from the group consisting of 0, 1, 2, 3, 4, and 5;
L₂ is selected from 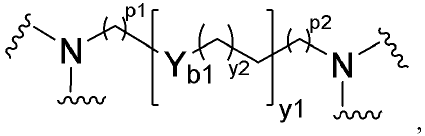
wherein, y1 is an integer from 0 to 100, such as an integer from 0 to 30, for example 0, 1, 2, 3, 4, 5, 6, 7, 8, 9, or 10;
y2 is an integer from 0 to 10, such as an integer from 0 to 6, for example 1 or 2;
Y_{b1} is selected from the group consisting of O and CH₂, such as O;
p1 and p2 are each independently selected from the group consisting of integers from 0 to 10, such as an integer from 0 to 6, for example 1 and 2; or 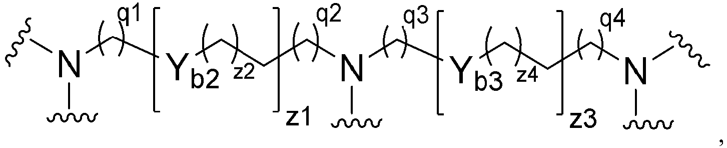
wherein, z1 is an integer from 0 to 100, such as an integer from 0 to 30, for example 0, 1, 2, 3, 4, 5, 6, 7, 8, 9, or 10;
z2 is an integer from 0 to 10, such as an integer from 0 to 6, for example 1 or 2;
Y_{b2} is selected from the group consisting of O and CH₂, such as O;
z3 is an integer from 0 to 100, such as an integer from 0 to 30, for example 0, 1, 2, 3, 4, 5, 6, 7, 8, 9, or 10;
z4 is an integer from 0 to 10, such as an integer from 0 to 6, for example 1 or 2;
Y_{b3} is selected from the group consisting of O and CH₂, such as O;
q1, q2, q3 and q4 are each independently selected from the group consisting of integers from 0 to 10, such as an integer from 0 to 6; or 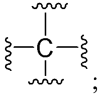
L₃ is -(CH₂)_{wc1}-[Y_{c1}-CH₂(CH₂)_{xc2}]_{xc1}-(CH₂)_{wc2}-G_{c1}-, wherein,
each xc1 is independently an integer from 0 to 100, such as an integer from 0 to 30, for example 0, 1, 2, 3, 4, 5, 6, 7, 8, 9, or 10;
each xc2 is independently an integer from 0 to 10, such as an integer from 0 to 6, for example 1 or 2;
each Y_{c1} is independently selected from the group consisting of O and CH₂, such as O;
wc1 and wc2 are each independently selected from the group consisting of integers from 0 to 10, such as an integer from 0 to 6, for example 0, 1, 2, and 3;
each G_{c1} is independently selected from the group consisting of NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, for example, -NR₆(C=O)-, -NR₆(C=S)-, -O(C=O)-, -(C=O)-, and -NR₆(C=O)CH₂;
D is 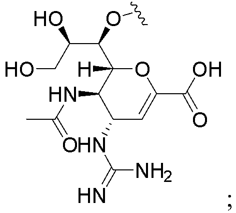
m is 3 or 4.

In some embodiments, each xc1 is independently an integer from 2 to 30, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, or any numerical range therebetween, such as 2 to 20, and the like.

In some embodiments, the number of atoms in the L₃ backbone is greater than 7, such as greater than 8, for example greater than 9, such as 8, 9, 10, 11, 12, 13, 14, 15, 16, 17, 18, 19, 20, 21, 22, 23, 24, 25, 26, 27, 28, 29, 30, or any numerical range therebetween, such as 8 to 30 or 8 to 20.

In some embodiments, the compound is selected from the group consisting of: 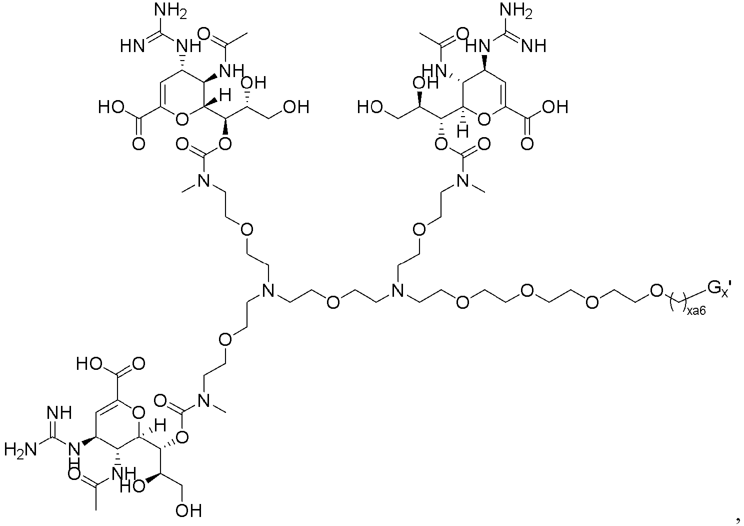 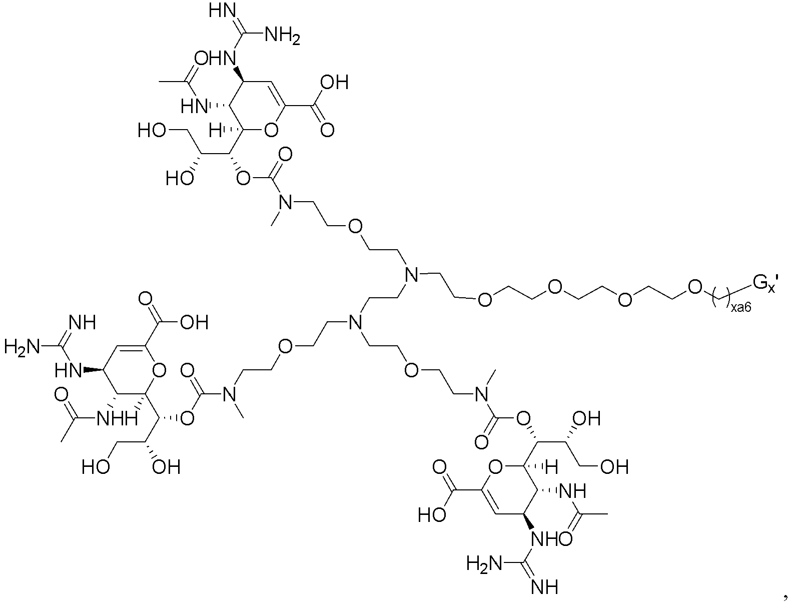 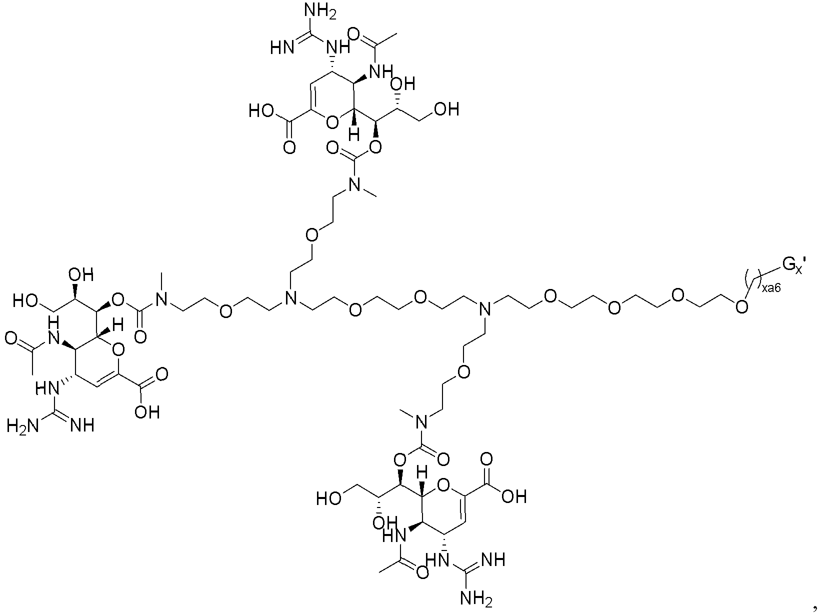 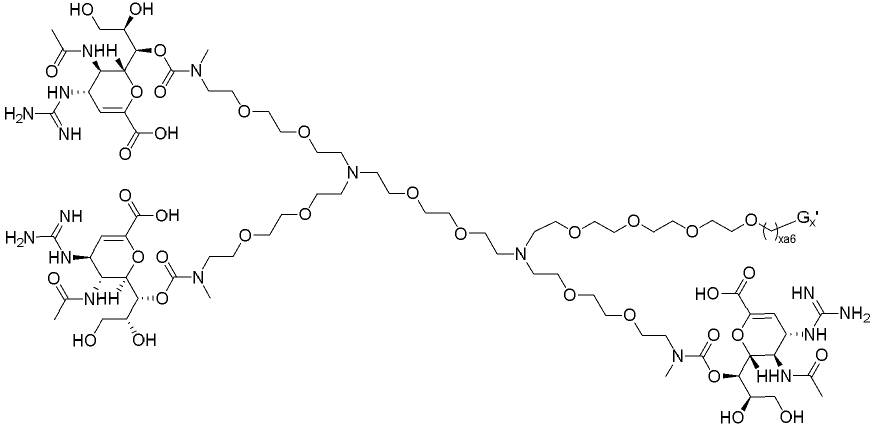 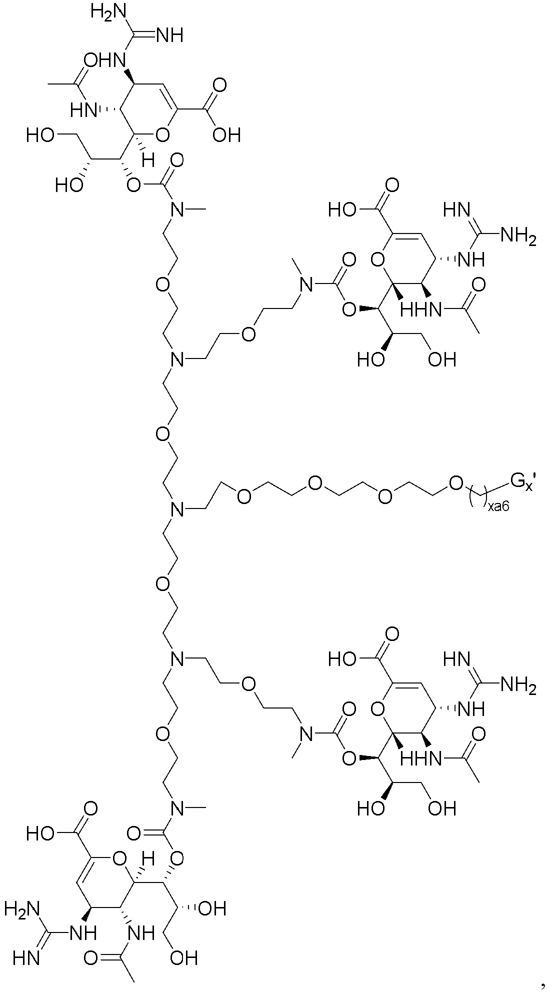 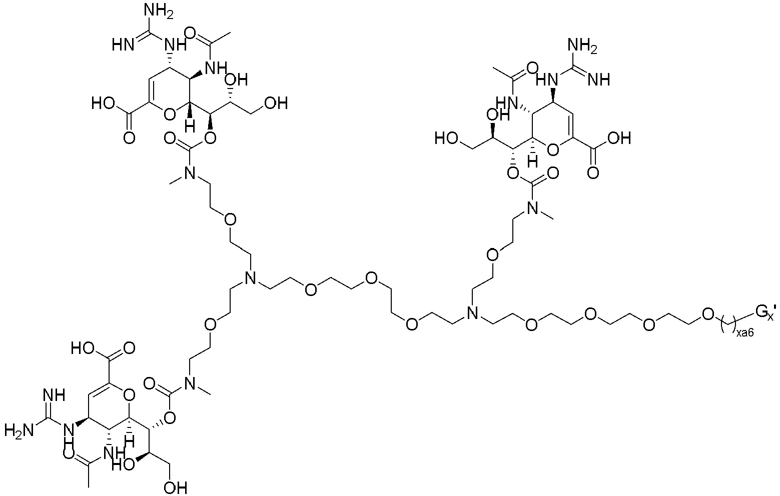 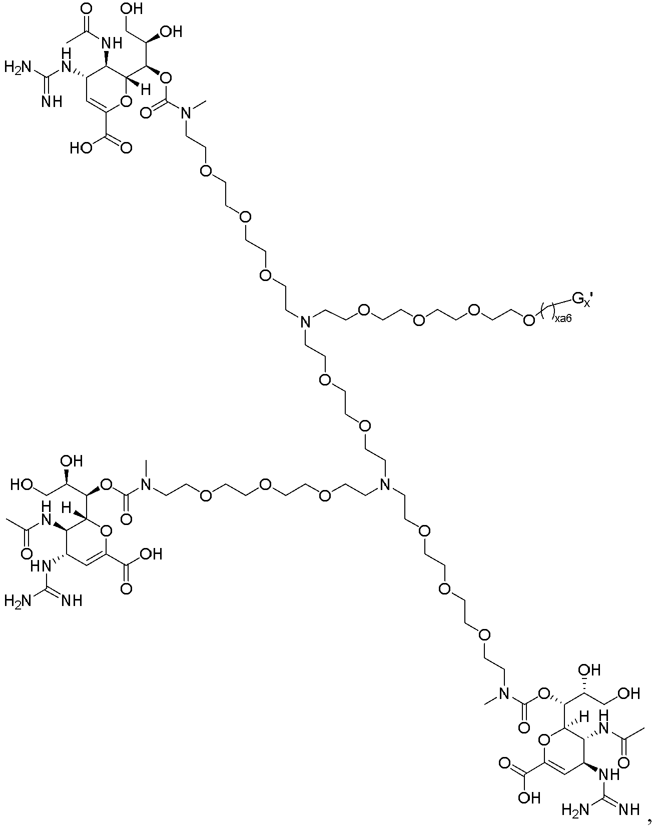 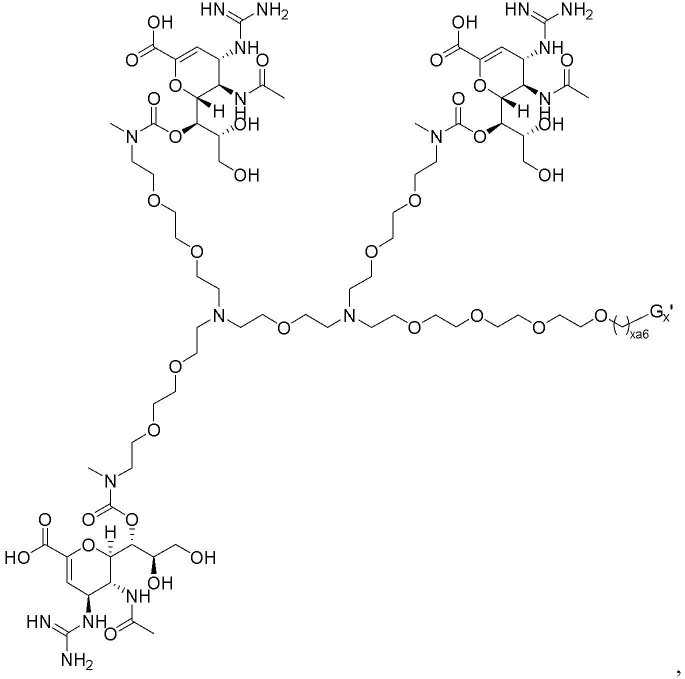 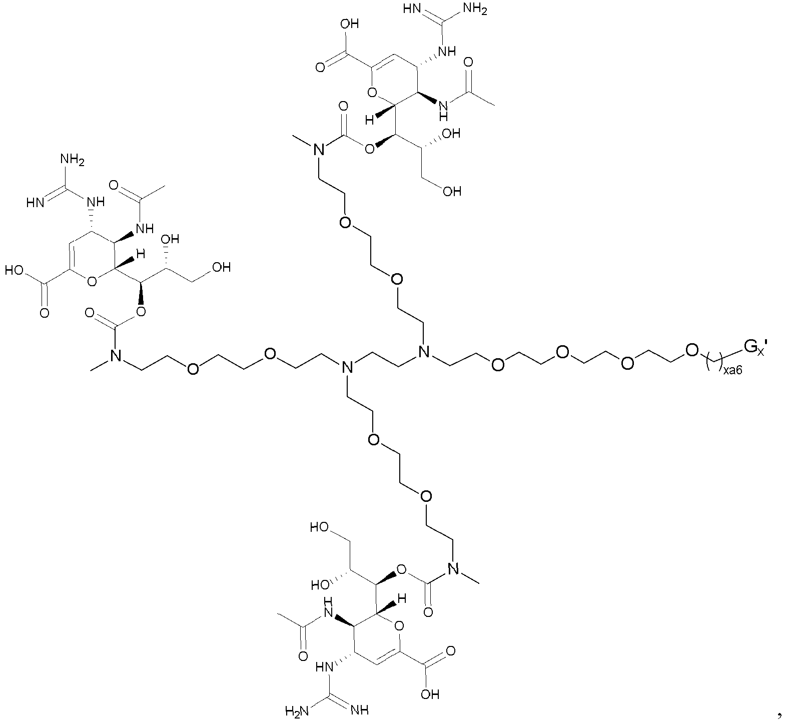 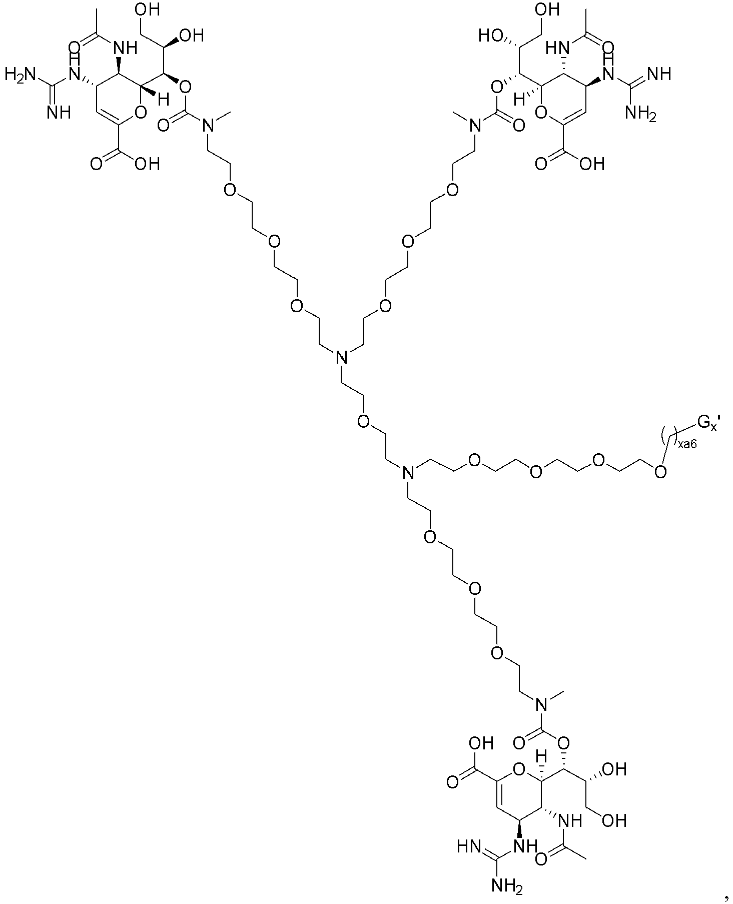 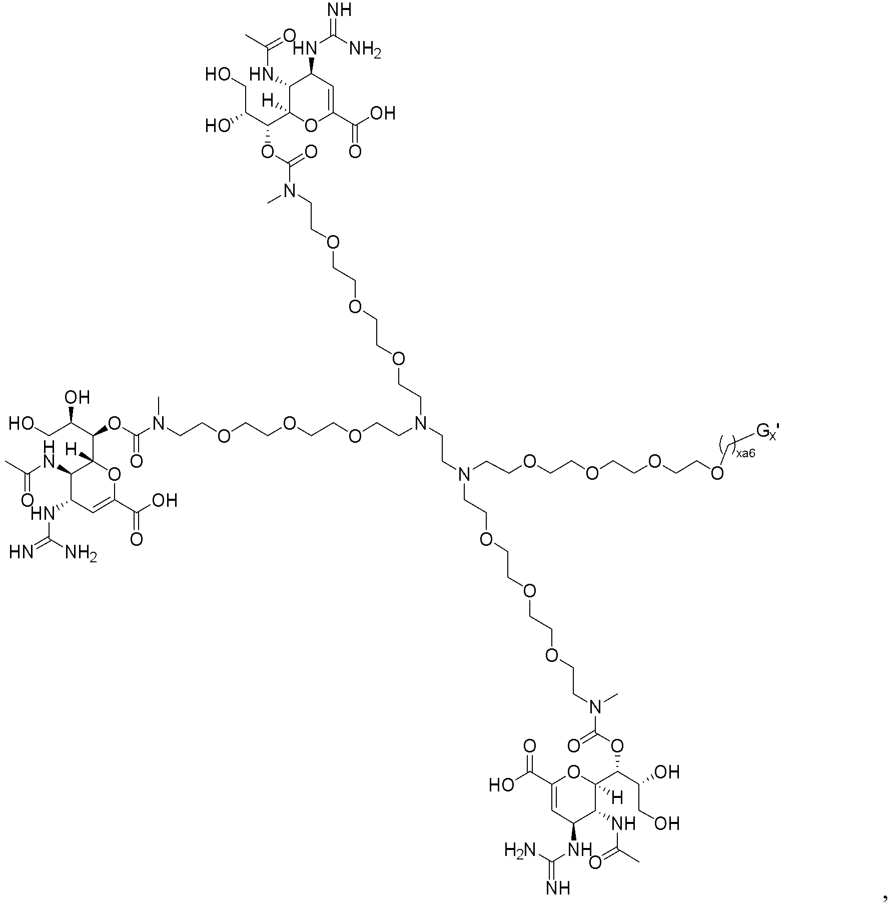 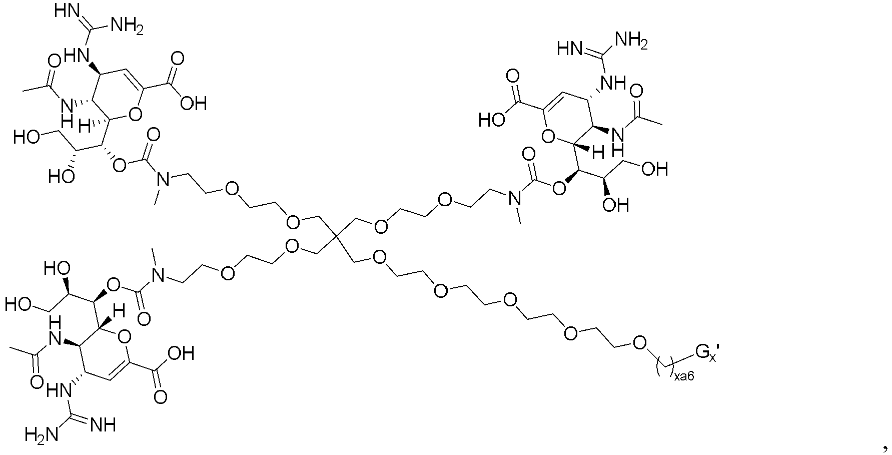 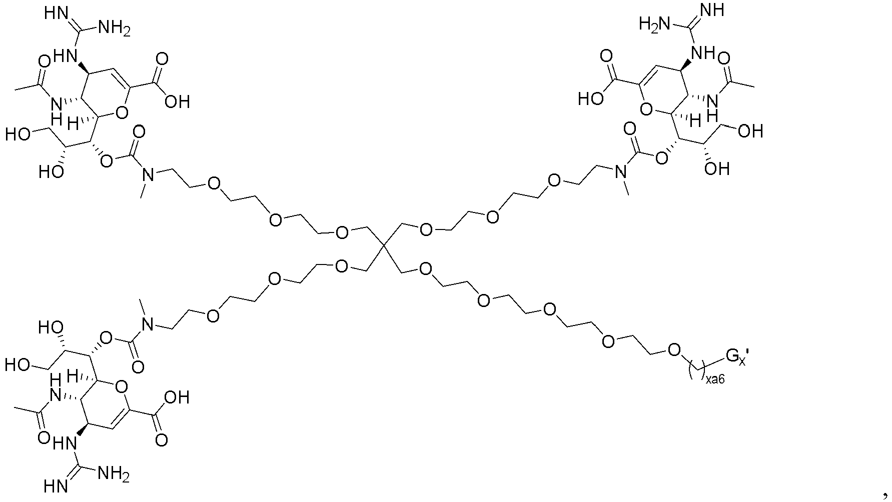 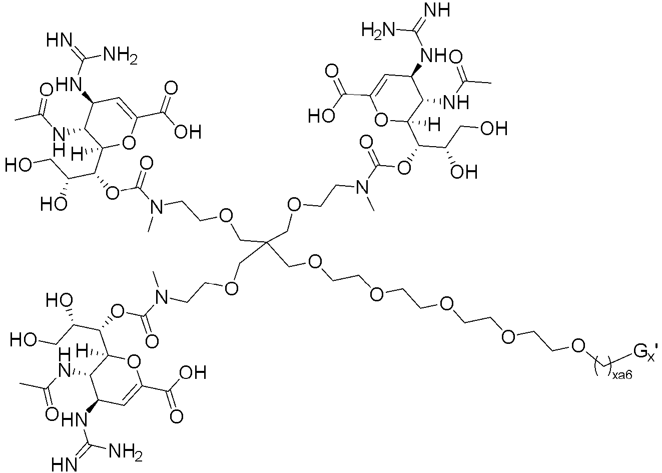 and 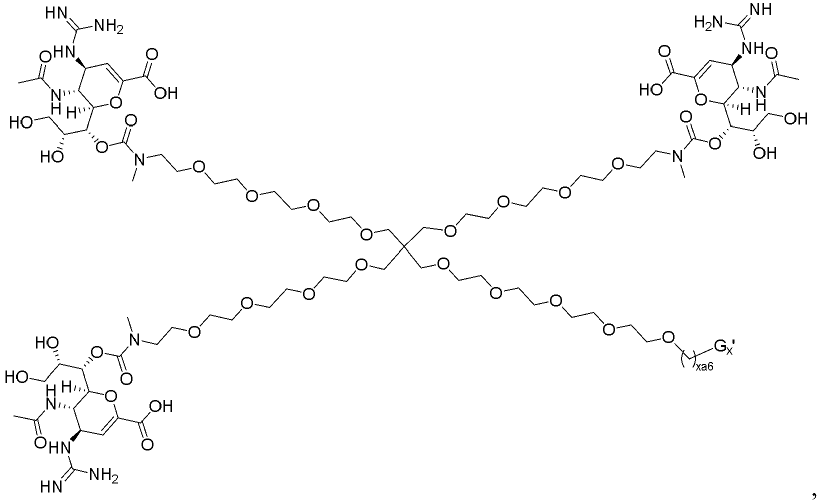 wherein,
each xa6 is independently selected from the group consisting of integers from 0 to 8,
Gₓ' is each independently selected from the group consisting of 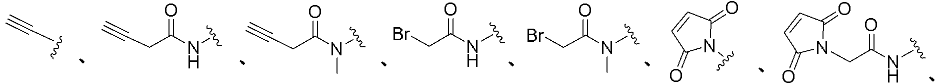 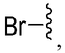 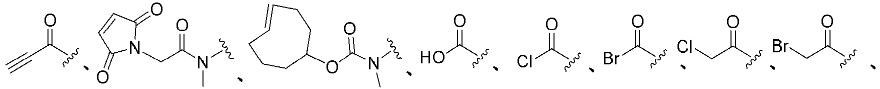 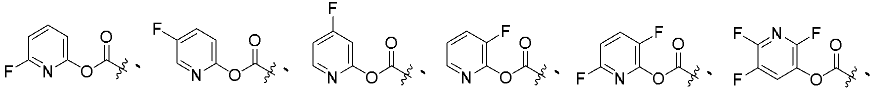 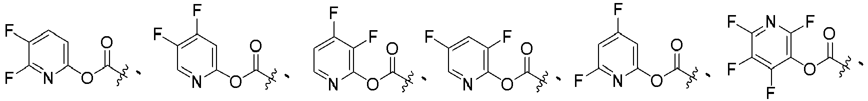 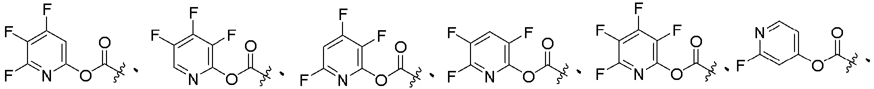 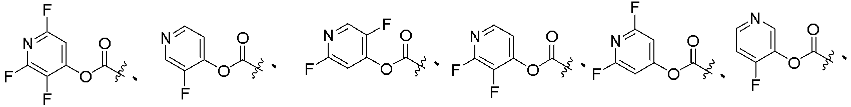 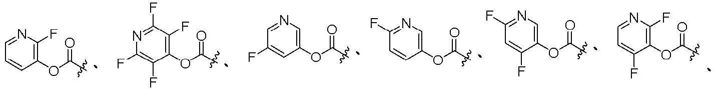 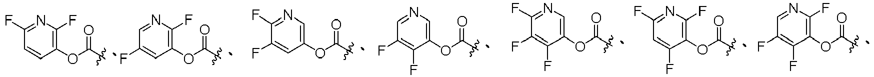 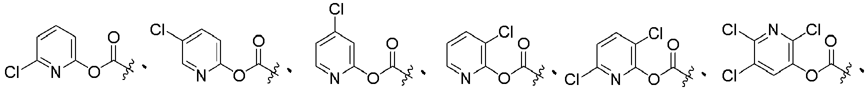 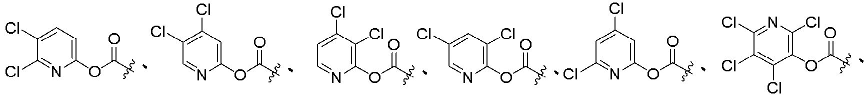 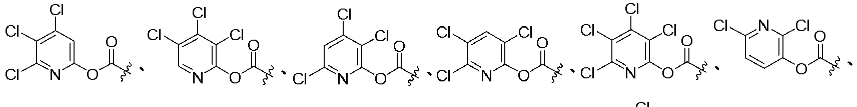 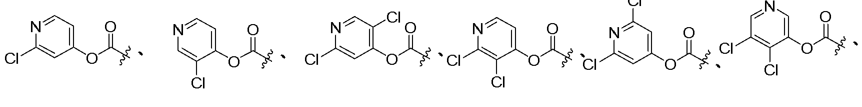 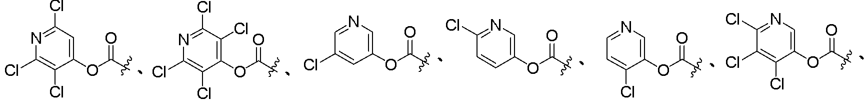 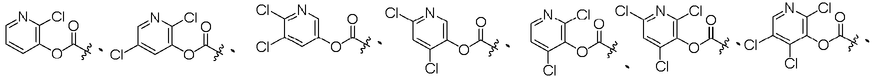 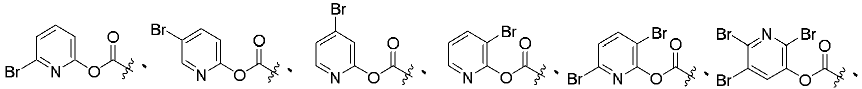 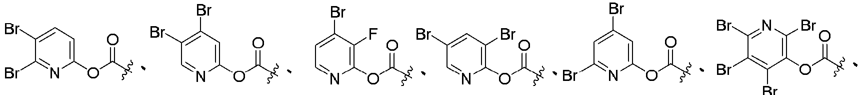 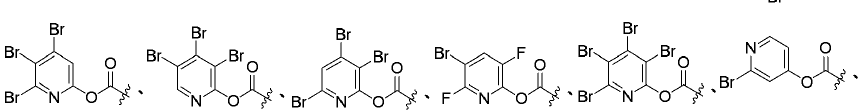 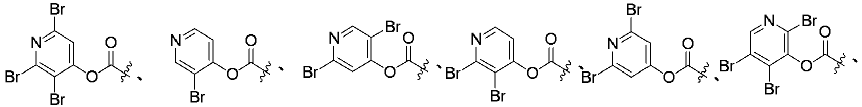 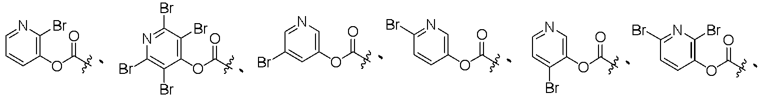 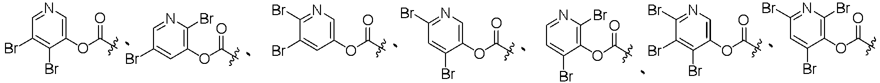 and

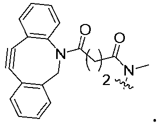

In some embodiments, the compound is selected from the group consisting of: 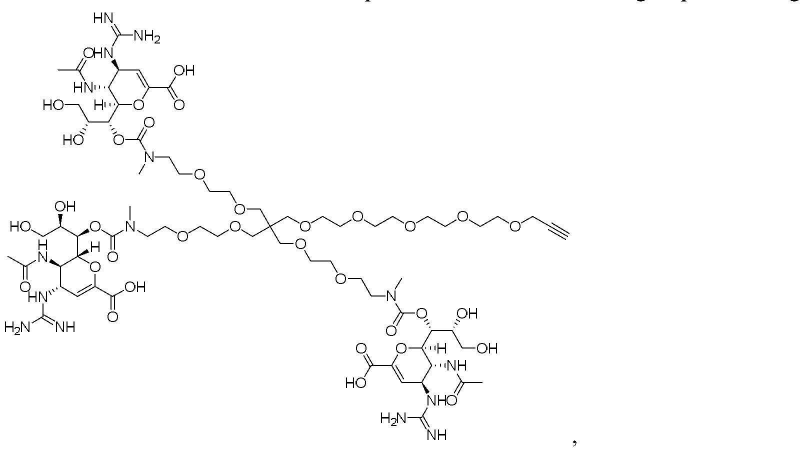 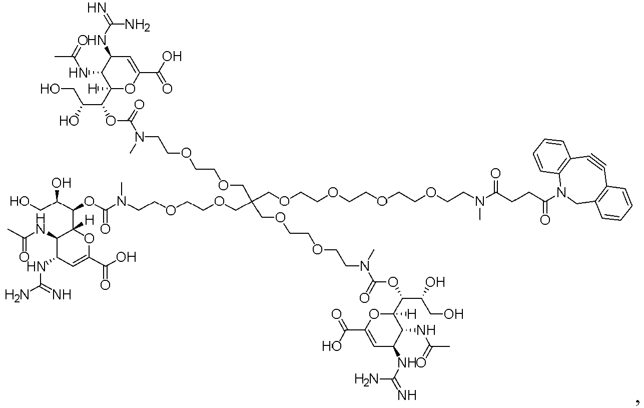 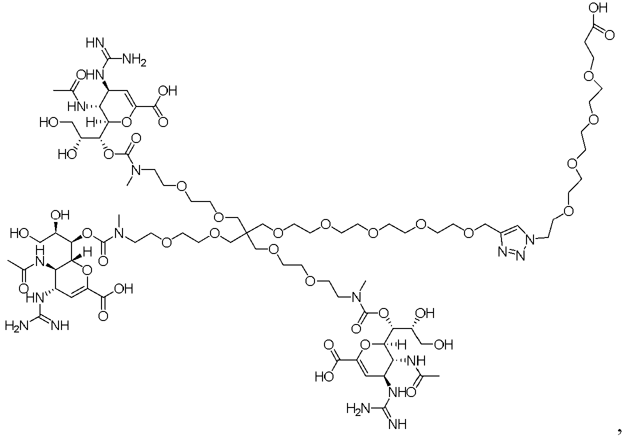 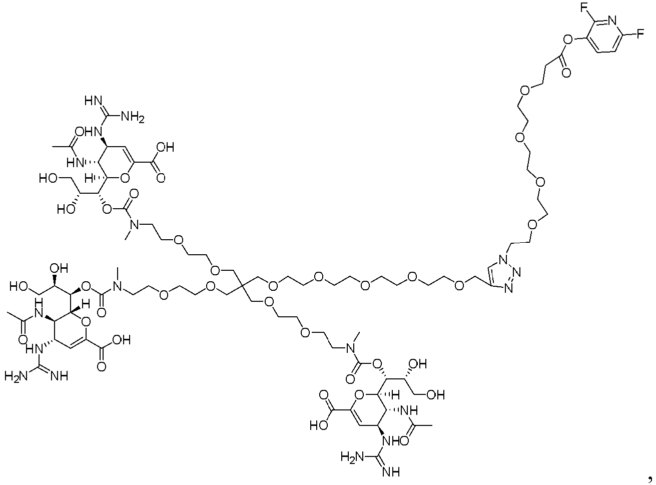 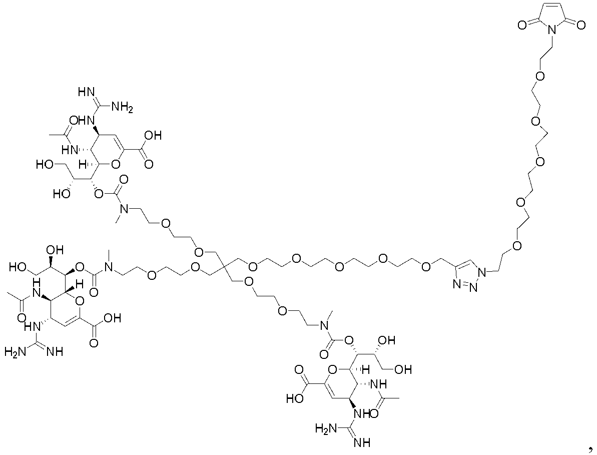 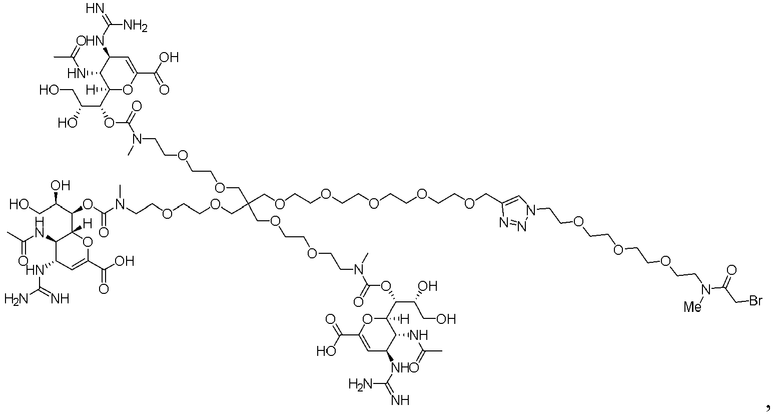 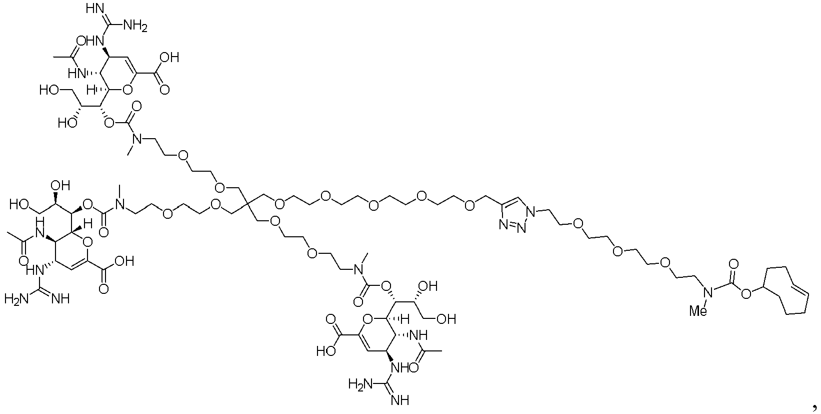 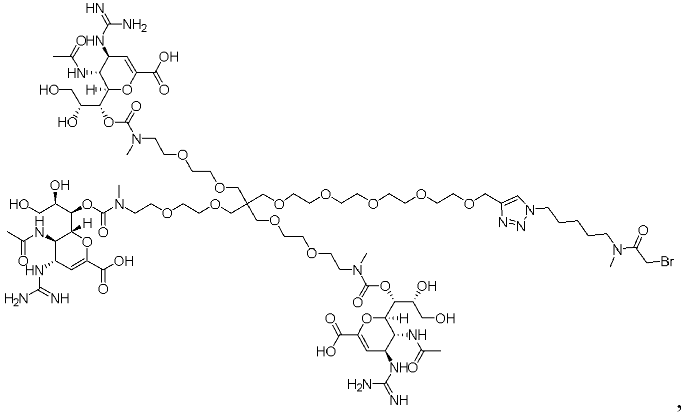 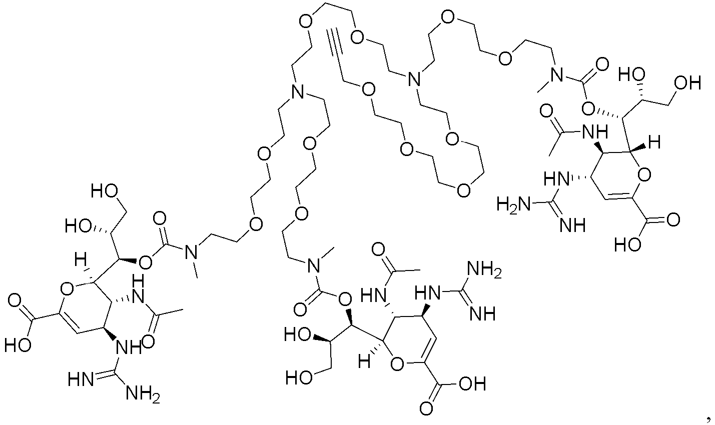 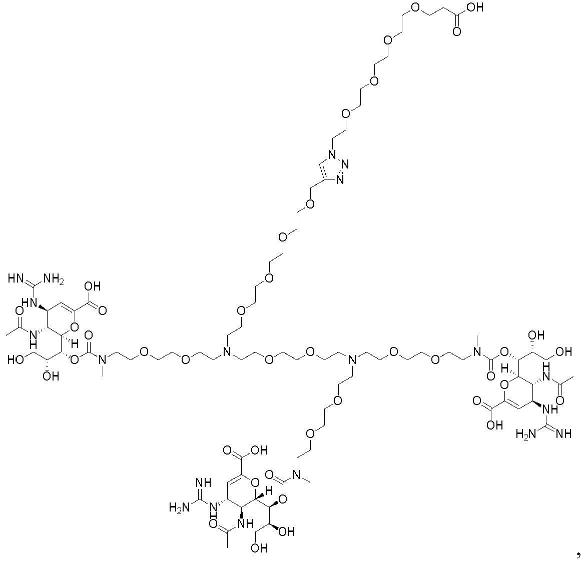 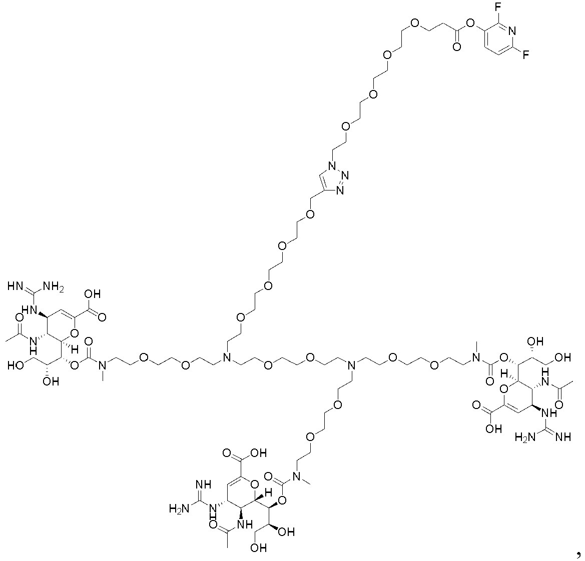 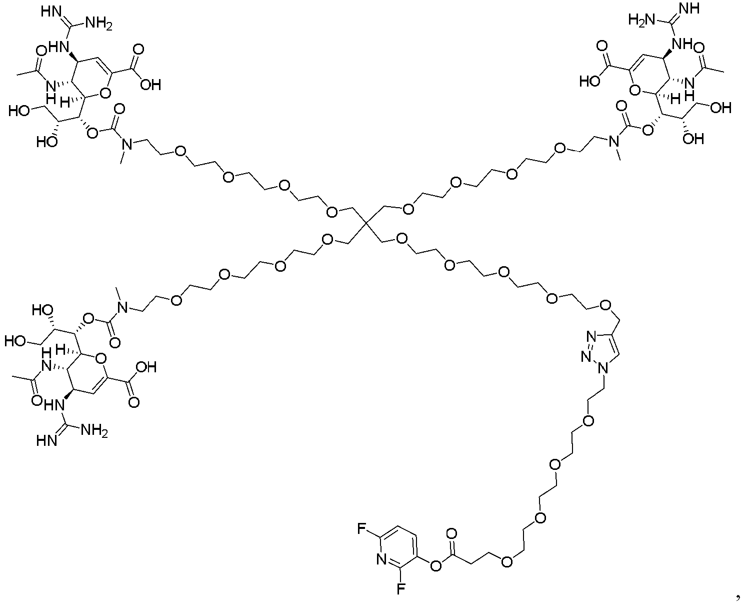 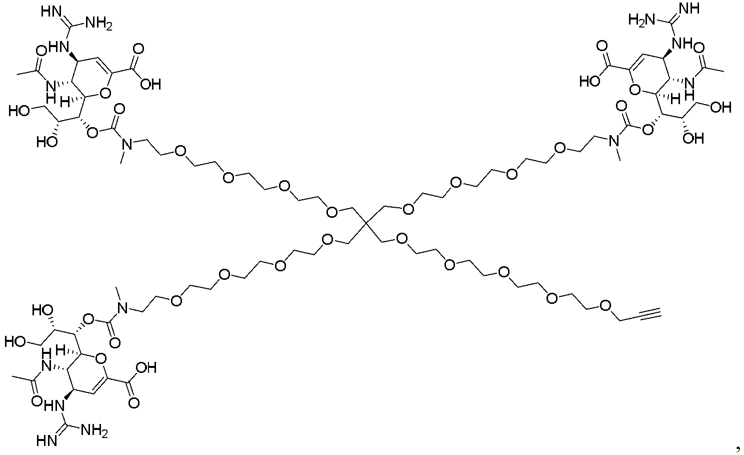 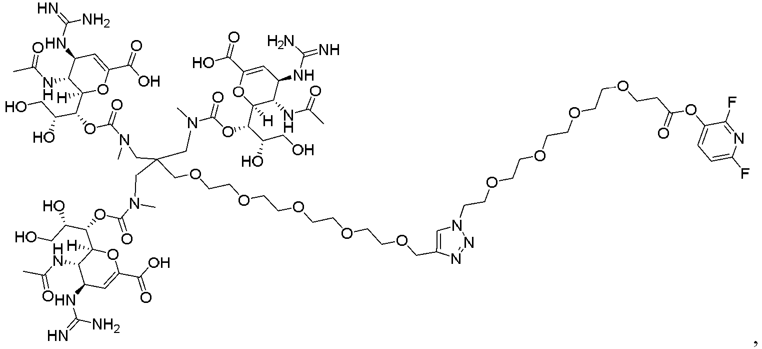 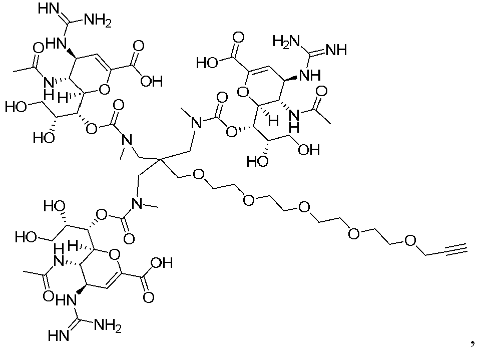 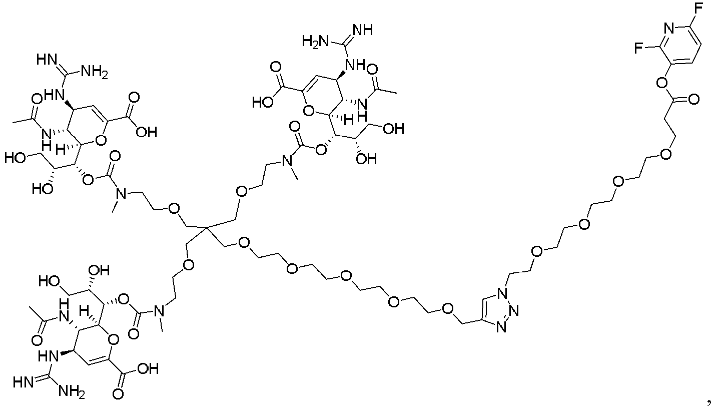 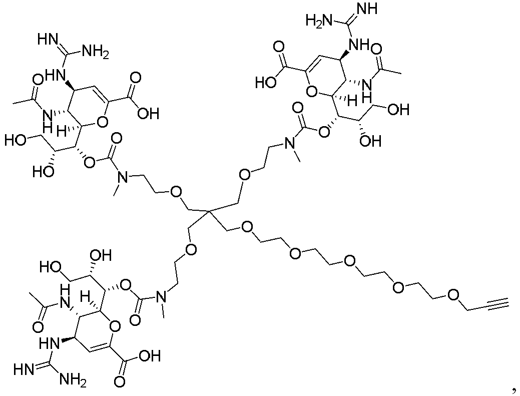 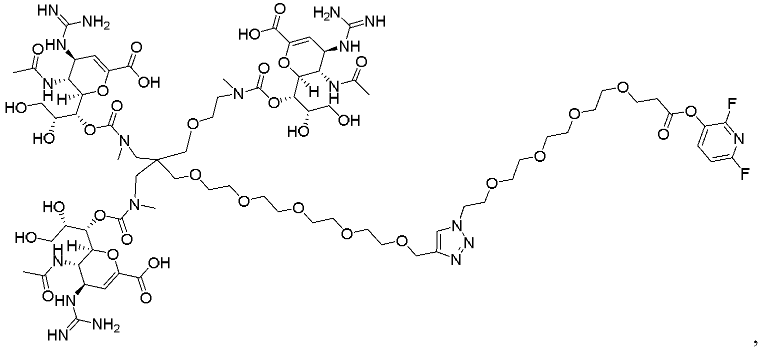 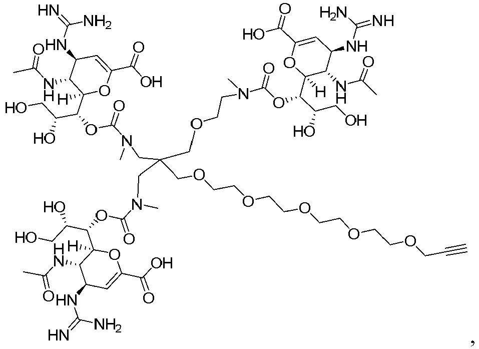 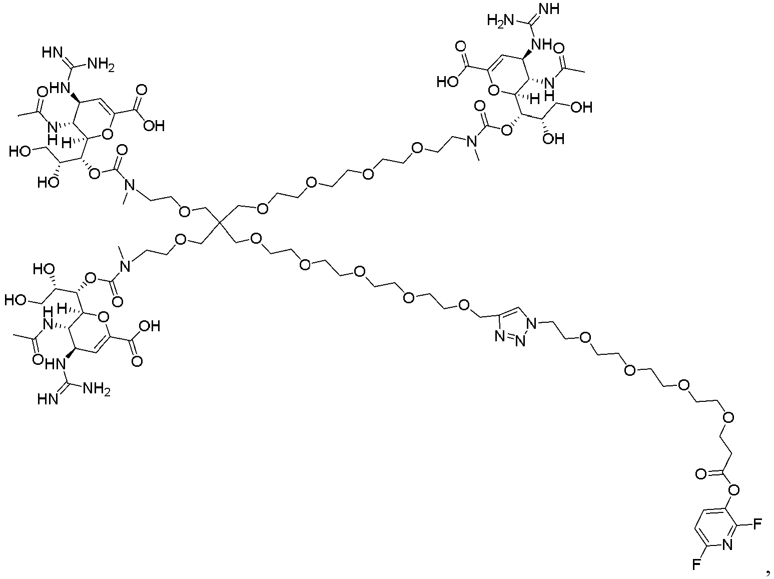 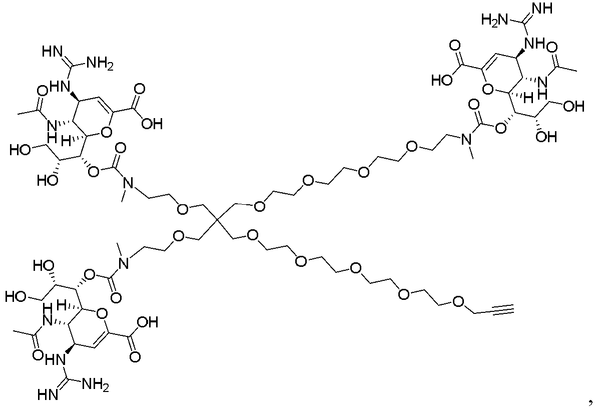 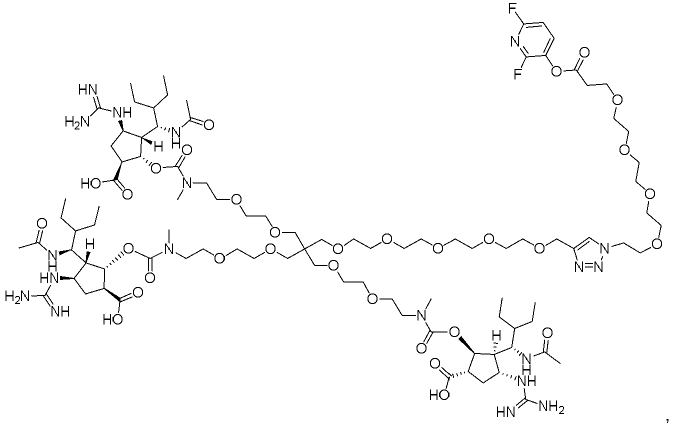 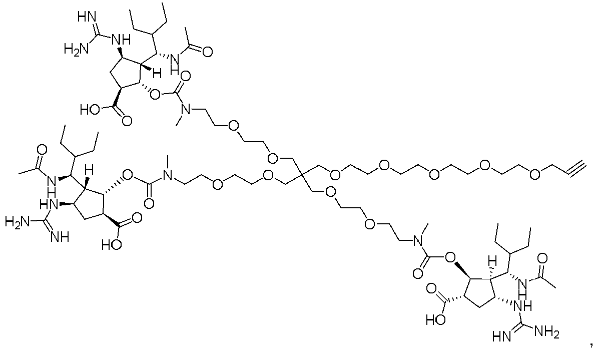 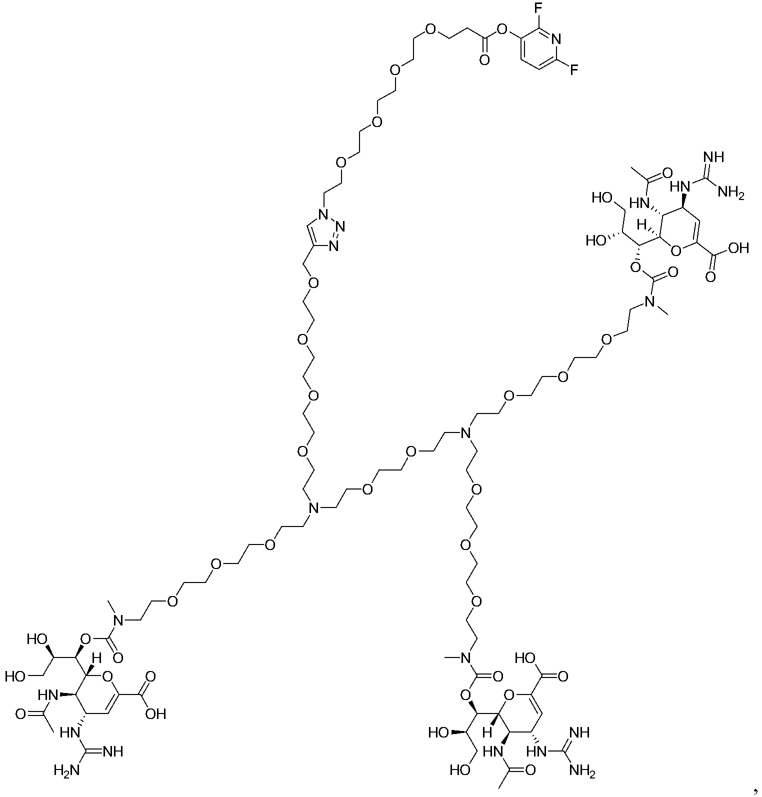 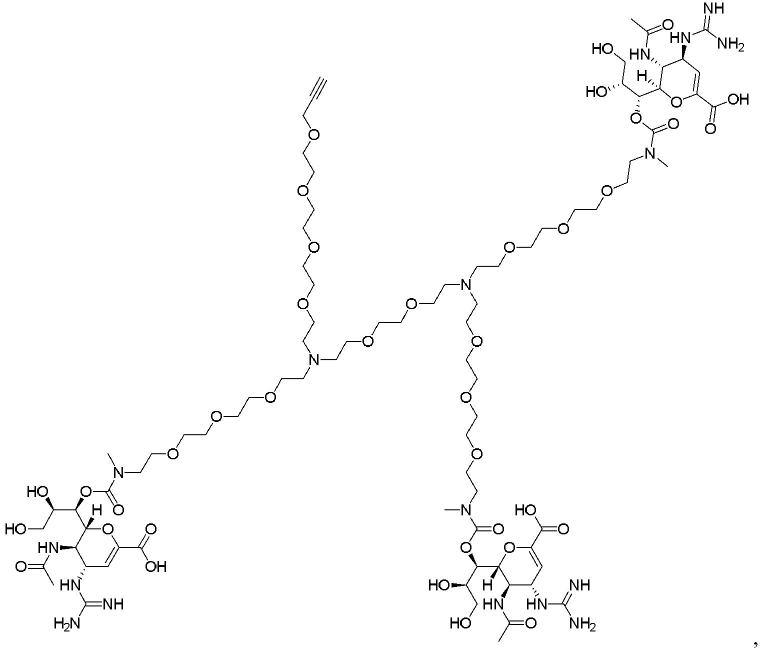 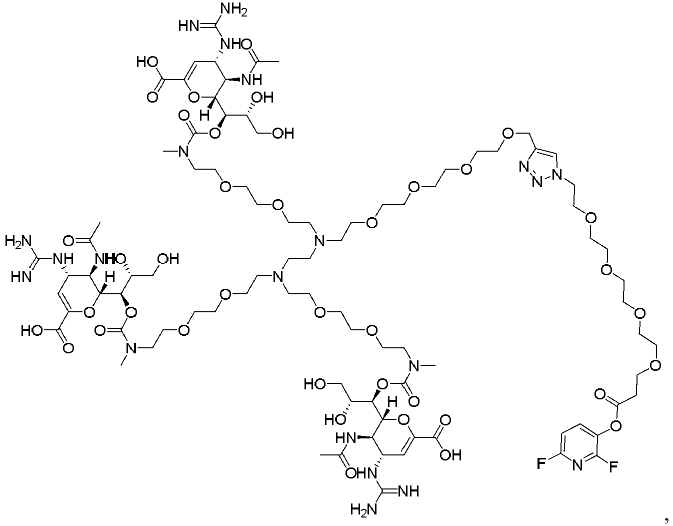 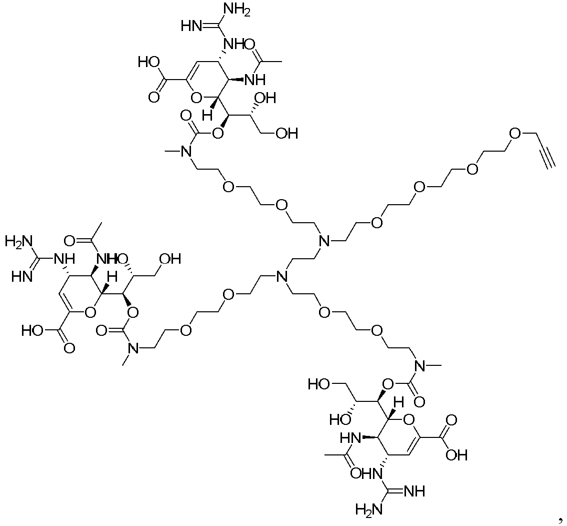 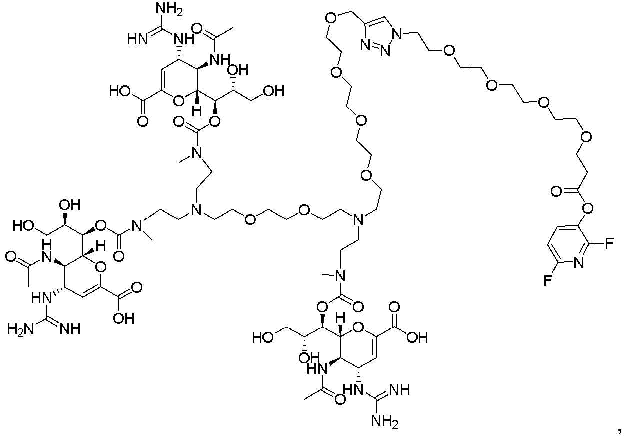 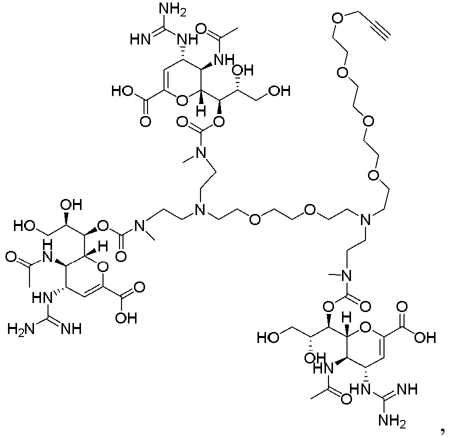 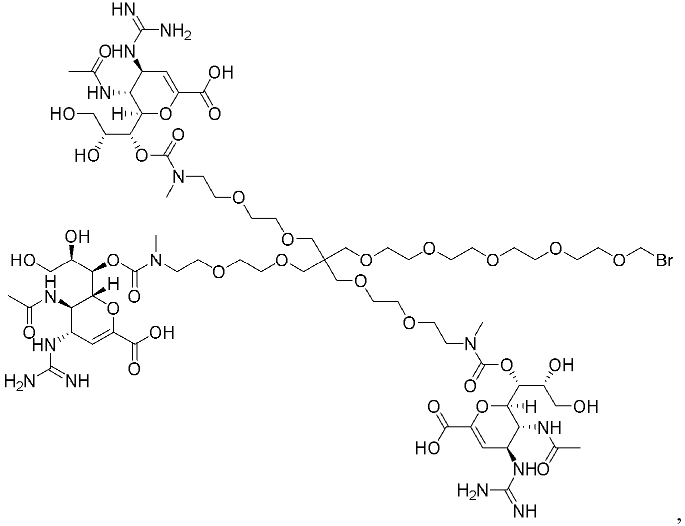 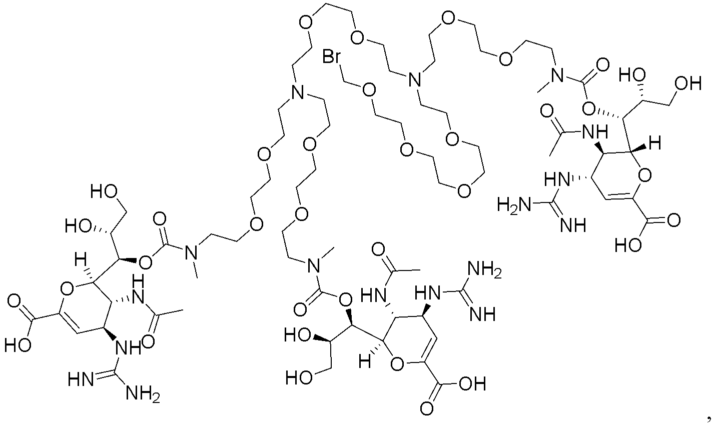 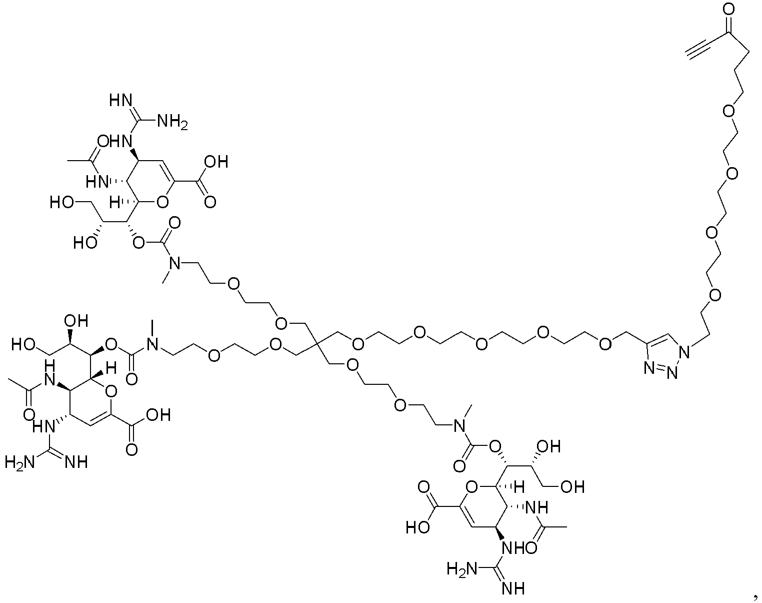 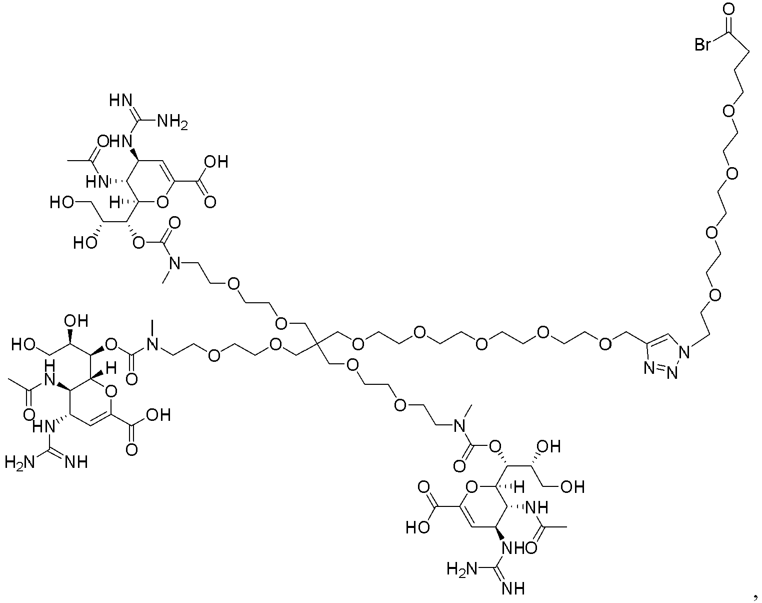 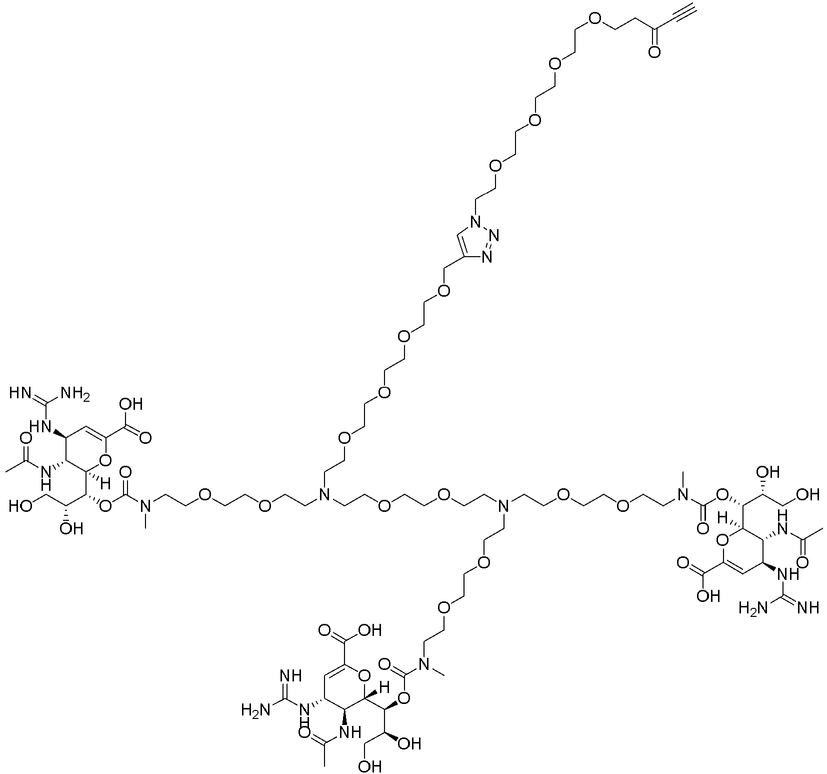 and

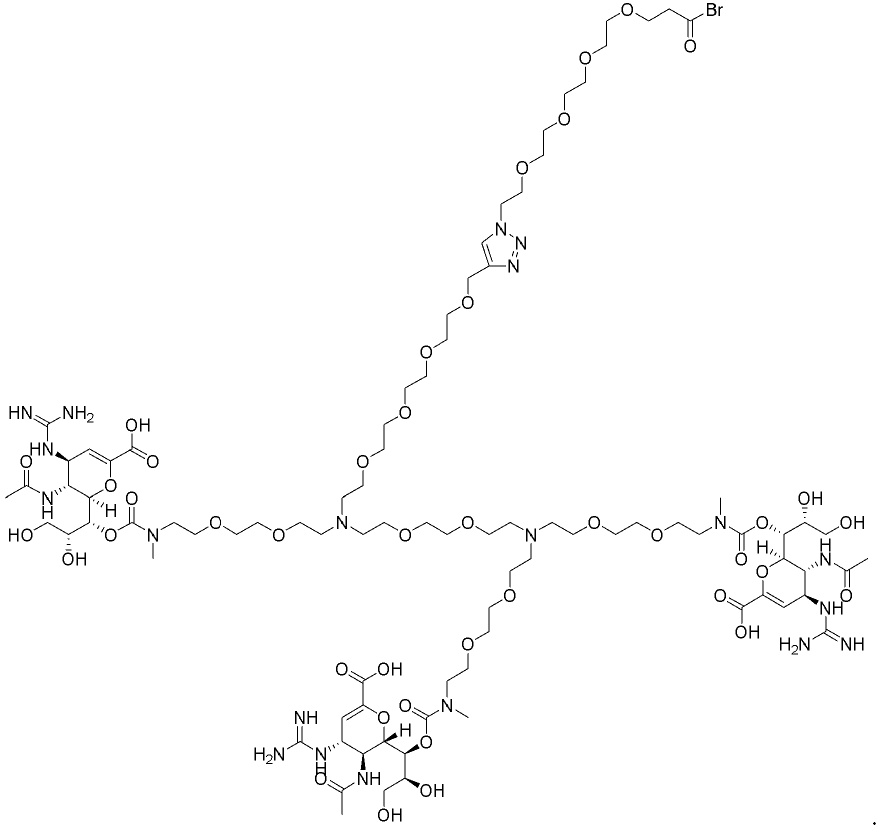

The present disclosure also provides a method for preparing the antiviral conjugate of the present disclosure, comprising a step of conjugating the polypeptide E and the small molecule D, 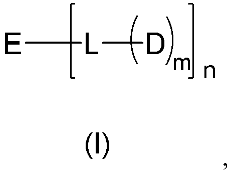
the polypeptide E and the small molecule D may be conjugated by means of the following manners, including but not limited to (a) a thiourea linkage (i.e., -NH(C=S)NH-) to lysine of E; (b) a carbamate linkage (i.e., -NH(C=O)-O) to lysine of E; (c) an amine linkage achieved through reductive amination between lysine and E (i.e., -NHCH₂); (d) an amide linkage (i.e., -NH-(C=O)CH₂) to lysine of E; (e) cysteine-maleimide binding between a maleimide in an end group and cysteine of E; (f) an amine linkage achieved through reductive amination between an end group and a carbohydrate of E (for example, a glycosyl of an Fc domain, albumin, or an albumin-binding domain) (i.e., -NHCH₂); (g) a bridging cysteine binding, wherein the end group is conjugated to two cysteines of E; (h) an oxime linkage between an end group and a carbohydrate of E (for example, a glycosyl of an Fc domain, albumin, or an albumin-binding domain); (i) an oxime linkage between an end group and an amino acid residue of E; (j) an azide linkage between an end group and E; (k) direct acylation of an end group to E; or (l) a thioether linkage between an end group and E;
alternatively, site-specific labeling is performed through a tyrosine specific site mutation or a tyrosine tag, and the tyrosine tag is genetically fused to the C-terminus of E. Tyrosinase oxidizes the phenol structure of tyrosine into a quinone, thereby allowing cycloaddition with various bicyclo[6.1.0]nonyne (BCN) derivatives, trans-cyclooctene (TCO) derivatives, and the like, thereby connecting E and D.

The present disclosure also provides a pharmaceutical composition, comprising at least one of the aforementioned antiviral conjugates, and a pharmaceutically acceptable carrier, diluent, or excipient.

In some embodiments, the unit dose of the pharmaceutical composition is 0.001 mg to 1000 mg.

In some embodiments, based on the total weight of the composition, the pharmaceutical composition contains 0.01% to 99.99% of the aforementioned antiviral conjugate. In some embodiments, the pharmaceutical composition contains 0.1% to 99.9% of the aforementioned antiviral conjugate. In some embodiments, the pharmaceutical composition contains 0.5% to 99.5% of the aforementioned antiviral conjugate. In some embodiments, the pharmaceutical composition contains 1% to 99% of the aforementioned antiviral conjugate. In some embodiments, the pharmaceutical composition contains 2% to 98% of the aforementioned antiviral conjugate.

In some embodiments, based on the total weight of the composition, the pharmaceutical composition contains 0.01% to 99.99% of the pharmaceutically acceptable carrier, diluent, or excipient. In some embodiments, the pharmaceutical composition contains 0.1% to 99.9% of the pharmaceutically acceptable carrier, diluent, or excipient. In some embodiments, the pharmaceutical composition contains 0.5% to 99.5% of the pharmaceutically acceptable carrier, diluent, or excipient. In some embodiments, the pharmaceutical composition contains 1% to 99% of the pharmaceutically acceptable carrier, diluent, or excipient. In some embodiments, the pharmaceutical composition contains 2% to 98% of the pharmaceutically acceptable carrier, diluent, or excipient.

The present disclosure also provides use of the antiviral conjugate or the pharmaceutical composition of the present disclosure in the preparation of a medicament for treating a viral infection. In some embodiments, the viral infection is caused by an influenza virus or a parainfluenza virus. In some embodiments, the viral infection is caused by influenza A, B, or C virus or a parainfluenza virus.

The present disclosure further provides a method for treating a viral infection, comprising administering the antiviral conjugate or the pharmaceutical composition of the present disclosure to a mammal for therapeutic purpose, wherein the mammal may be a human or a non-human mammal.

The present disclosure further provides a kit comprising the antiviral conjugate or the pharmaceutical composition of the present disclosure.

### Definitions of Terms:

Unless stated to the contrary, the terms used in the specification and claims have the following meanings.

When a trade name is used in the present disclosure, the applicant intends to include formulations of products under trade name, generic drugs of products under the trade name, and the active pharmaceutical ingredients.

Unless stated to the contrary, the terms used in the specification and claims have the following meanings.

The term "linker", "linking unit", "linker unit", "joint", or "linking fragment" refers to a chemical structural fragment or a bond having one end connected to a protein or a polypeptide and the other end connected to a drug. It may also be connected to another linker before being connected to the drug.

The term "amino acid" refers to an organic compound containing an amino group and a carboxyl group in its molecular structure, and both the amino group and the carboxyl group are directly connected to a -CH- structure. Its general formula is H₂NCHRCOOH, wherein R is H, a substituted or unsubstituted alkyl, etc. Based on the position of the carbon atom in the carboxylic acid to which the amino is connected, it can be classified into α, β, γ, δ, ε...-amino acids. In the biological field, the amino acids constituting natural proteins have their specific structural characteristics, that is, their amino groups are directly connected to the α-carbon atoms, i.e., α-amino acids, including Glycine, Alanine, Valine, Leucine, Isoleucine, Phenylalanine, Tryptophan, Tyrosine, Aspartic acid, Histidine, Asparagine, Glutamic acid, Lysine, Glutamine, Methionine, Arginine, Serine, Threonine, Cysteine, Proline, and the like. Unnatural amino acids include, for example, Citrulline. As is well known to those skilled in the art, unnatural amino acids do not constitute natural proteins, and therefore do not participate in the synthesis of the antibodies in the present disclosure. The three-letter codes and single-letter codes for amino acids used in the present disclosure are as described in J. Biol. Chem., 243, p3558 (1968).

| Abbreviation | Three-letter code | Name | Structure |
|---|---|---|---|
| G | Gly | Glycine | 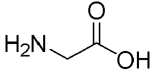 |
| A | Ala | Alanine | 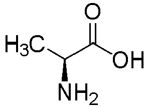 |
| V | Val | Valine | 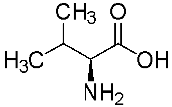 |
| L | Leu | Leucine | 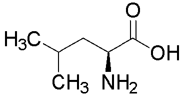 |
| I | Ile | Isoleucine | 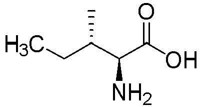 |
| F | Phe | Phenylalanine | 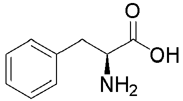 |
| W | Trp | Tryptophan | 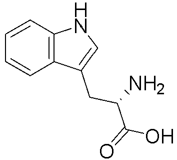 |
| Y | Tyr | Tyrosine | 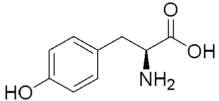 |
| D | Asp | Aspartic acid | 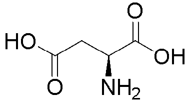 |
| H | His | Histidine | 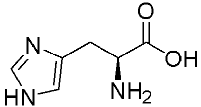 |
| N | Asn | Asparagine | 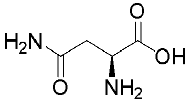 |
| E | Glu | Glutamic acid | 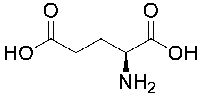 |
| K | Lys | Lysine | 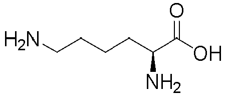 |
| Q | Gln | Glutamine | 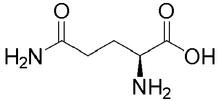 |
| C | Cit | Citrulline | 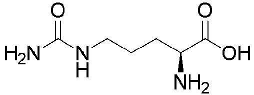 |

The term "drug loading" refers to the average number of drugs loaded on each conjugate molecule in a conjugate population. It can also be expressed as the ratio of the drug amount to the protein or polypeptide. Drug loading can range from 1 to 20, for example, 1 to 15, for example, 1 to 10 neuraminidase inhibitors (D) connected to each protein or polypeptide. In the embodiments of the present disclosure, the drug loading is represented by n, which exemplarily can be a value of 1, 2, 3, 4, 5, 6, 7, 8, 9, 10, 11, 12, 13, 14, 15, 16, 17, 18, 19, 20 or the average of any two values. For example, it can be an average of 1 to 10, for example, 1 to 8, or 2 to 8, or 2 to 7, or 3 to 8, or 3 to 7, or 3 to 6, or 4 to 7, or 4 to 6, or 4 to 5. The average number of drugs per conjugate after the conjugation reaction can be identified using conventional methods such as UV/visible spectroscopy, mass spectrometry, ELISA assay, capillary electrophoresis-sodium dodecyl sulfate (CE-SDS) assay, and HPLC.

The monoclonal antibody molecular size variant assay (CE-SDS) of the present disclosure employs a capillary electrophoresis-sodium dodecyl sulfate (CE-SDS) ultraviolet detection method. Under reducing and non-reducing conditions, based on the molecular weight size, the purity of a recombinant monoclonal antibody product is quantitatively determined according to the capillary electrophoresis method (Chinese Pharmacopoeia, 2015 Edition, 0542).

In one embodiment of the present disclosure, the neuraminidase inhibitor is conjugated to the N-terminal amino and/or the ε-amino of a lysine residue of a ligand through a linking unit. Generally, the number of drug molecules capable of conjugating to an antibody in the conjugation reaction will be less than the theoretical maximum.

The loading of the conjugate can be controlled by the following non-limiting methods, including:
(1) controlling the molar ratio of a linking reagent to a monoclonal antibody;
(2) controlling the reaction time and temperature; and
(3) selecting different reaction reagents.

The term "antibody" encompasses various antibody structures, including, but not limited to, a monoclonal antibody, a polyclonal antibody; a monospecific antibody, a multispecific antibody (for example, a bispecific antibody), a full-length antibody, and an antibody fragment (or an antigen-binding fragment, or an antigen-binding portion), so long as they exhibit a desired antigen-binding activity. The antibody may refer to an immunoglobulin, which is a tetrapeptide chain structure formed by connecting two identical heavy chains and two identical light chains through interchain disulfide bonds. The amino acid composition and sequence of the immunoglobulin heavy chain constant regions are different, so their antigenicity is also different. Accordingly, immunoglobulins can be classified into five classes, or immunoglobulin isotypes, namely IgM, IgD, IgG, IgA, and IgE, and their corresponding heavy chains areµ chain,δ chain,γ chain,α chain, andε chain, respectively. Ig of the same class can be further classified into different subclasses based on differences in the amino acid composition of their hinge regions and the number and location of heavy chain disulfide bonds. For example, IgG can be classified into IgG1, IgG2, IgG3, and IgG4. Light chains are classified intoκ chain orλ chain based on different constant regions. Each of the five classes of Ig can haveκ chain orλ chain.

The sequence of about 110 amino acids near the N-terminus of the antibody heavy chain and light chain varies greatly and is defined as a variable region (Fv region); the remaining amino acid sequence near the C-terminus is relatively stable and is defined as a constant region. The variable region comprises 3 hypervariable regions (HVRs) and 4 relatively conserved framework regions (FRs). The 3 hypervariable regions determine the specificity of the antibody and are also known as complementarity determining regions (CDRs). Each light chain variable region (LCVR) and heavy chain variable region (HCVR) is composed of 3 CDRs and 4 FRs, arranged sequentially from the amino terminus to the carboxyl terminus as follows: FR1, CDR1, FR2, CDR2, FR3, CDR3, and FR4. The 3 CDRs of the light chain refer to LCDR1, LCDR2, and LCDR3; the 3 CDRs of the heavy chain refer to HCDR1, HCDR2, and HCDR3.

The antibody of the present disclosure includes a murine antibody, a chimeric antibody, a humanized antibody, and a fully human antibody, for example, a humanized antibody and a fully human antibody.

The term "murine antibody" in the present disclosure refers to an antibody prepared from a mouse according to the knowledge and skills in the art. During the preparation, a test subject is injected with a specific antigen, and then a hybridoma expressing an antibody having desired sequence or functional characteristics is isolated.

The term "chimeric antibody" refers to an antibody formed by fusing a variable region of a murine antibody with a constant region of a human antibody, which can alleviate an immune response induced by the murine antibody. To establish a chimeric antibody, a hybridoma secreting a murine specific monoclonal antibody is first established. Then a variable region gene is cloned from the murine hybridoma cells, and a human antibody constant region gene is cloned as required. The murine variable region gene and the human constant region gene are ligated into a chimeric gene and inserted into an expression vector. Finally, the chimeric antibody molecule is expressed in a eukaryotic system or a prokaryotic system.

The term "humanized antibody", also known as a CDR-grafted antibody, refers to an antibody generated by grafting murine CDR sequences into a human antibody variable region framework, i.e., framework sequences of different types of human germline antibodies. This can overcome the heterologous response induced by the chimeric antibody due to carrying a large amount of murine protein components. Such framework sequences can be obtained from public DNA databases containing germline antibody gene sequences or from published references. For example, germline DNA sequences for human heavy and light chain variable region genes can be found in the "VBase" human germline sequence database (available on the Internet at www.mrccpe.com.ac.uk/vbase), as well as in Kabat, E.A. et al., 1991 Sequences of Proteins of Immunological Interest, 5th Edition. In order to avoid a decrease in activity along with reducing immunogenicity, minimal back mutations or reverse mutations can be performed on the human antibody variable region framework sequence to maintain activity. The humanized antibody of the present disclosure also includes a humanized antibody further subjected to affinity maturation of CDRs by phage display. Further literatures describing methods that can be used to participate in humanization of murine antibodies include, for example, Queen et al., Proc. Natl. Acad. Sci. USA, 88, 2869, 1991, and the methods of Winter and colleagues [Jones et al., Nature, 321, 522 (1986), Riechmann, et al., Nature, 332, 323-327 (1988), Verhoeyen, et al., Science, 239, 1534 (1988)].

The term "fully human source antibody", "fully human antibody", or "completely humanized antibody", also known as a "fully human monoclonal antibody", has its antibody variable region and constant region all derived from human, eliminating immunogenicity and toxic side effects. The development of monoclonal antibodies has gone through four stages: murine monoclonal antibodies, chimeric monoclonal antibodies, humanized monoclonal antibodies, and fully human monoclonal antibodies. The antibody of the present disclosure is a fully human monoclonal antibody. Related technologies for preparing fully human antibodies mainly include: human hybridoma technology, EBV-transformed B lymphocyte technology, phage display technology, transgenic mouse antibody preparation technology, and single B cell antibody preparation technology.

The term "antigen-binding fragment" refers to one or more fragments of an antibody that retain the capability to specifically bind to an antigen. It has been shown that fragments of a full-length antibody can be utilized to perform the antigen-binding function of the antibody. Examples of binding fragments encompassed within the "antigen-binding fragment" include (i) Fab fragment, a monovalent fragment consisting of VL, VH, CL, and CH1 domains; (ii) F(ab')₂ fragment, a bivalent fragment comprising two Fab fragments linked by a disulfide bridge at the hinge region; (iii) Fd fragment consisting of VH and CH1 domains; (iv) Fv fragment consisting of VH and VL domains of a single arm of an antibody; (v) single-domain or dAb fragment (Ward et al., (1989) Nature 341: 544-546), which consists of a VH domain; and (vi) isolated complementarity determining region (CDR); or (vii) a combination of two or more isolated CDRs that may optionally be joined by a synthetic linker. Furthermore, although the two domains VL and VH of the Fv fragment are encoded by separate genes, they can be joined, using recombinant methods, by a synthetic linker that enables them to be made as a single protein chain in which the VL and VH regions pair to form monovalent molecules (known as single-chain Fv (scFv); see, e.g., Bird et al. (1988) Science 242: 423-426; and Huston et al. (1988) Proc. Natl. Acad. Sci. USA 85: 5879-5883). Such single-chain antibodies are also intended to be encompassed within the term "antigen-binding fragment" of an antibody. Such antibody fragments are obtained using conventional techniques known to those skilled in the art, and the fragments are screened for functionality in the same manner as for intact antibodies. Antigen-binding portions can be produced by recombinant DNA techniques or by enzymatic or chemical cleavage of intact immunoglobulins. The antibody can be different isotypes, for example, an IgG (for example, IgG1, IgG2, IgG3, or IgG4 subtype), IgA1, IgA2, IgD, IgE, or IgM antibody.

Fab is an antibody fragment having a molecular weight of about 50,000 and having antigen-binding activity, obtained by treating an IgG antibody molecule with the protease papain (cleaving the amino acid residue at position 224 of the H chain), wherein about half of the N-terminal side of the H chain and the entire L chain are bound together through a disulfide bond.

F(ab')2 is an antibody fragment having a molecular weight of about 100,000 and having antigen-binding activity, obtained by digesting the portion below two disulfide bonds in the hinge region of IgG with the pepsin, and comprising two Fab regions connected at the hinge position.

Fab' is an antibody fragment having a molecular weight of about 50,000 and having antigen-binding activity, obtained by cleaving the disulfide bond in the hinge region of the above F(ab')2.

In addition, the Fab' can be produced by inserting a DNA encoding the Fab' fragment of the antibody into a prokaryotic expression vector or a eukaryotic expression vector and then introducing the vector into a prokaryote or a eukaryote to express the Fab'.

The term "single-chain antibody", "single-chain Fv", or "scFv" means a molecule comprising an antibody heavy chain variable domain (or region; VH) and an antibody light chain variable domain (or region; VL) connected by a linker. Such scFv molecules can have the general structure: NH₂-VL-linker-VH-COOH or NH₂-VH-linker-VL-COOH. A suitable linker in the prior art consists of repeated GGGGS amino acid sequences or variants thereof, for example, using variants with 1 to 4 repeats (Holliger et al. (1993), Proc. Natl. Acad. Sci. USA 90: 6444-6448). Other linkers that can be used in the present disclosure are described by Alfthan et al. (1995), Protein Eng. 8: 725-731, Choi et al. (2001), Eur. J. Immunol. 31: 94-106, Hu et al. (1996), Cancer Res. 56: 3055-3061, Kipriyanov et al. (1999), J. Mol. Biol. 293: 41-56, and Roovers et al. (2001), Cancer Immunol.

The term "CDR" refers to one of the 6 hypervariable regions within the variable domains of an antibody that primarily contribute to antigen binding. One of the most commonly used definitions for the 6 CDRs is provided by Kabat E.A. et al., (1991) Sequences of proteins of immunological interest. NIH Publication 91-3242. As used herein, the Kabat definition of CDRs applies only to CDR1, CDR2, and CDR3 of the light chain variable domain (CDR L1, CDR L2, CDR L3, or L1, L2, L3), and CDR2 and CDR3 of the heavy chain variable domain (CDR H2, CDR H3, or H2, H3). Typically, there are three CDRs (HCDR1, HCDR2, HCDR3) in each heavy chain variable region and three CDRs (LCDR1, LCDR2, LCDR3) in each light chain variable region. The amino acid sequence boundaries of CDRs can be determined using any of various well-known schemes, including the "Kabat" numbering rules (see Kabat et al. (1991), "Sequences of Proteins of Immunological Interest", 5th Edition, Public Health Service, National Institutes of Health, Bethesda, MD), the "Chothia" numbering rules (see Al-Lazikani et al., (1997) JMB 273: 927-948), and the ImMunoGeneTics (IMGT) numbering rules (see Lefranc M.P., Immunologist, 7, 132-136 (1999); Lefranc, M.P. et al., Dev. Comp. Immunol., 27, 55-77 (2003)), and the like. For example, for a classical format, following the Kabat rules, the CDR amino acid residues in the heavy chain variable domain (VH) are numbered 31-35 (HCDR1), 50-65 (HCDR2), and 95-102 (HCDR3); the CDR amino acid residues in the light chain variable domain (VL) are numbered 24-34 (LCDR1), 50-56 (LCDR2), and 89-97 (LCDR3). Following the Chothia rules, the CDR amino acid residues in VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3). By combining the CDR definitions of both Kabat and Chothia, the CDRs consist of amino acid residues 26-35 (HCDR1), 50-65 (HCDR2), and 95-102 (HCDR3) in human VH, and amino acid residues 24-34 (LCDR1), 50-56 (LCDR2), and 89-97 (LCDR3) in human VL. Following the IMGT rules, the CDR amino acid residues in VH are numbered approximately 26-35 (CDR1), 51-57 (CDR2), and 93-102 (CDR3), and the CDR amino acid residues in VL are numbered approximately 27-32 (CDR1), 50-52 (CDR2), and 89-97 (CDR3). Following the IMGT rules, the CDR regions of an antibody can be determined using the program IMGT/DomainGap Align.

The term "Fc domain monomer" or "Fc monomer" refers to a polypeptide chain comprising at least one hinge domain and second and third antibody constant domains (C_{H}2 and C_{H}3), or a functional fragment thereof (for example, capable of (i) dimerizing with another Fc domain monomer to form an Fc domain, and (ii) binding to an Fc receptor, or a fragment thereof). In some embodiments, an Fc domain monomer sequentially comprises a hinge domain, a C_{H}2, and a C_{H}3 from the N-terminus to the C-terminus. The Fc domain monomer can be of any immunoglobulin antibody isotype, including IgG, IgE, IgM, IgA, or IgD (for example, IgG). In addition, the Fc domain monomer can be of an IgG subtype (for example, IgG1, IgG2a, IgG2b, IgG3, or IgG4) (for example, IgG1). The Fc domain monomer in the conjugate as described herein may contain one or more alterations relative to a wild-type Fc domain monomer sequence (for example, 1 to 10, 1 to 8, 1 to 6, 1 to 4 amino acid substitutions, additions, or deletions) that alter the interaction between the Fc domain and an Fc receptor. Examples of suitable alterations are known in the art. In some embodiments, a human Fc domain monomer (for example, an IgG heavy chain, such as IgG1) comprises a region extending from any one of Asn201, Asn208, Glu216, Asp221, Lys222, or Cys226 to the heavy chain carboxyl terminus at Lys447. The C-terminal Lys447 of the Fc region may or may not be present without affecting the structure or stability of the Fc region. Unless otherwise specified herein, the numbering of amino acid residues in the IgG or Fc domain monomer is according to the EU numbering system for antibodies, also referred to as the Kabat EU index, as described, for example, in Kabat et al., Sequences of Proteins of Immunological Interest, 5th Ed. Public Health Service, National Institutes of Health, Bethesda, MD, 1991.

The term "antibody framework" refers to a portion of a variable domain VL or VH that serves as a scaffold for the antigen-binding loops (CDRs) of that variable domain. Essentially, it is a variable domain without the CDRs.

The term "epitope" or "antigenic determinant" refers to a site on an antigen to which an immunoglobulin or antibody specifically binds. An epitope typically includes at least 3, 4, 5, 6, 7, 8, 9, 10, 11, 12, 13, 14, or 15 contiguous or non-contiguous amino acids in a unique spatial conformation (see, e.g., Epitope Mapping Protocols in Methods in Molecular Biology, Vol. 66, G.E. Morris, Ed. (1996)).

The term "albumin" refers to a polypeptide comprising amino acids corresponding to naturally occurring albumin (for example, human serum albumin) or a variant thereof (such as an engineered variant of naturally occurring albumin). Variants of albumin include polymorphisms, fragments such as domains and sub-domains, and fusion proteins (for example, albumin fused at the C-terminus or N-terminus, such as with a polypeptide linker). The albumin may comprise only naturally occurring amino acid residues, or may comprise one or more non-naturally occurring amino acid residues. In some embodiments, a non-naturally occurring amino acid residue (for example, a side chain of a non-naturally occurring amino acid residue) can be used as an attachment point for the compound of the present disclosure (for example, a neuraminidase inhibitor monomer or dimer, including via a linker).

The term "albumin-binding domain" encompasses a polypeptide having albumin-binding activity, as well as an antibody or a fragment thereof that binds to albumin. The "albumin-binding domain" has an affinity for albumin (for example, human serum albumin) and the function of binding to said albumin. The albumin-binding domain can be of different origins, for example, artificially synthesized or derived from a human, a mouse, or a rat. The albumin-binding domain can be linear or cyclic, including any albumin-binding domain known to those skilled in the art. Exemplary albumin-binding domains are found in US2005/0287153, which is incorporated herein by reference in its entirety.

The terms "specifically bind", "selectively bind", "selectively binds", and "specifically binds" refer to the binding of an antibody to an epitope on a predetermined antigen. Typically, the antibody binds with an affinity (KD) of about less than 10⁻⁷ M, for example, about less than 10⁻⁸ M, 10⁻⁹ M, or 10⁻¹⁰ M or less.

The term "nucleic acid molecule" refers to a DNA molecule and an RNA molecule. The nucleic acid molecule can be single-stranded or double-stranded, for example, it is double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid", which refers to a circular double-stranded DNA loop into which additional DNA segments can be ligated. In another embodiment, the vector is a viral vector, wherein additional DNA segments can be ligated into the viral genome. The vectors disclosed herein are capable of autonomous replication in a host cell into which they have been introduced (for example, bacterial vectors having a bacterial origin of replication and episomal mammalian vectors) or can integrate into the genome of a host cell upon introduction into the host cell, thereby replicating along with the host genome (for example, non-episomal mammalian vectors).

Methods for producing and purifying antibodies and antigen-binding fragments are well known in the prior art, such as Chapters 5-8 and 15 of Antibodies: A Laboratory Manual, Cold Spring Harbor. Antigen-binding fragments can also be prepared using conventional methods. The antibody or antigen-binding fragment of the present disclosure can be prepared by genetic engineering methods by adding one or more human FR regions to a non-human CDR region. Human FR germline sequences can be obtained by aligning with the IMGT human antibody variable region germline gene database and MOE software, from the website of ImMunoGeneTics (IMGT) at http://imgt.cines.fr, or from the Journal of Immunology, 2001 ISBN 012441351.

The term "host cell" refers to a cell into which an expression vector has been introduced. Host cells can include bacterial, microbial, plant, or animal cells. Bacteria susceptible to transformation include members of the enterobacteriaceae, such as strains of Escherichia coli or Salmonella; Bacillaceae, such as Bacillus subtilis; Pneumococcus; Streptococcus, and Haemophilus influenzae. Suitable microorganisms include Saccharomyces cerevisiae and Pichia pastoris. Suitable animal host cell lines include CHO (Chinese Hamster Ovary cell line) and NS0 cells.

The engineered antibody or antigen-binding fragment of the present disclosure can be prepared and purified using conventional methods. For example, cDNA sequences encoding the heavy and light chains can be cloned and recombined into a GS expression vector. The recombinant immunoglobulin expression vector can stably transfect CHO cells. As a more recommended prior art, a mammalian expression system results in glycosylation of the antibody, particularly at the highly conserved N-terminal site in the Fc region. Positive clones are expanded in serum-free medium in a bioreactor to produce antibodies. The culture broth secreting the antibody can be purified using conventional techniques. For example, purification is performed using an A or G Sepharose FF column with an adjusted buffer. Non-specifically bound components are washed away. The bound antibody is then eluted using a pH gradient method, and antibody fragments are detected by SDS-PAGE and collected. The antibody can be filtered and concentrated using conventional methods. Soluble mixtures and multimers can also be removed using conventional methods, such as molecular sieves and ion exchange. The resulting product needs to be immediately frozen, for example, at -70°C, or lyophilized.

Amino acid sequence "identity" refers to the percentage of amino acid residues in a first sequence that are identical to the amino acid residues in a second sequence, after aligning the amino acid sequences and introducing gaps if necessary to achieve the maximum percent sequence identity, and not considering any conservative substitutions as part of the sequence identity. For the purpose of determining the percent amino acid sequence identity, alignment can be achieved in various ways that are within the skill in the art, for example, using publicly available computer software such as BLAST, BLAST-2, ALIGN, ALIGN-2, or Megalign (DNASTAR) software. Those skilled in the art can determine appropriate parameters for measuring alignment, including any algorithms needed to achieve maximal alignment over the full length of the sequences being compared.

The term "peptide" refers to a compound fragment intermediate between amino acids and proteins, formed by linking two or more amino acid molecules to each other through peptide bonds. It is a structural and functional fragment of a protein; for example, hormones, enzymes, etc. are essentially peptides.

The term "saccharide" refers to a biological macromolecule composed of three elements: C, H, and O, which can be classified into monosaccharides, disaccharides, polysaccharides, etc.

The term "fluorescent probe" refers to a class of fluorescent molecules that have characteristic fluorescence in the ultraviolet-visible-near-infrared region, and whose fluorescence properties (excitation and emission wavelengths, intensity, lifetime, polarization, etc.) can sensitively change with the properties of the environment they are in, such as polarity, refractive index, viscosity, etc. Their non-covalent interaction with macromolecular structures such as nucleic acids (DNA or RNA), proteins, or other macromolecules alters one or several fluorescence properties, which can be used to study the properties and behaviors of macromolecular substances.

"Alkyl" refers to a saturated aliphatic hydrocarbon group, including straight-chain and branched-chain groups of 1 to 20 carbon atoms. Alkyl groups containing 1 to 6 carbon atoms are also included. Non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, and various branched isomers thereof, etc. The alkyl can be substituted or unsubstituted. When substituted, the substituent can be substituted at any available attachment point, preferably one or more of the following groups independently selected from the group consisting of halogen, hydroxy, oxo, cyano, amino, C₁₋₆ alkyl, C₁₋₆ alkoxy, 3- to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino.

The term "cycloalkyl" refers to a saturated or partially unsaturated monocyclic or polycyclic cyclic hydrocarbon substituent, wherein the cycloalkyl ring contains 3 to 20 carbon atoms, preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc.; polycyclic cycloalkyls include spirocyclic, fused, and bridged cycloalkyls. The cycloalkyl can be substituted or unsubstituted. When substituted, the substituent can be substituted at any available attachment point, preferably one or more of the following groups, independently selected from the group consisting of halogen, hydroxy, oxo, cyano, amino, C₁₋₆ alkyl, C₁₋₆ alkoxy, 3- to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino.

The term "heterocycloalkyl" refers to a saturated or partially unsaturated monocyclic or polycyclic cyclic hydrocarbon substituent comprising 3 to 20 ring atoms, wherein one or more ring atoms are heteroatoms selected from the group consisting of nitrogen, oxygen, and S(O)ₘ (wherein m is an integer from 0 to 2), but excluding ring portions of -O-O-, -O-S-, or -S-S-, with the remaining ring atoms being carbon. Preferably, it comprises 3 to 12 ring atoms, of which 1 to 4 are heteroatoms; more preferably, it comprises 3 to 7 ring atoms. Non-limiting examples of "heterocycloalkyl" include: 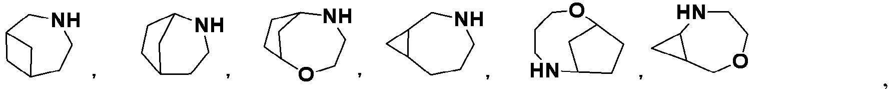 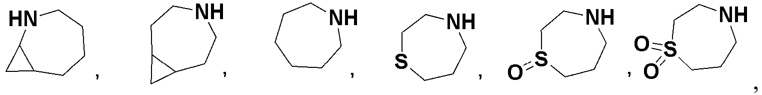 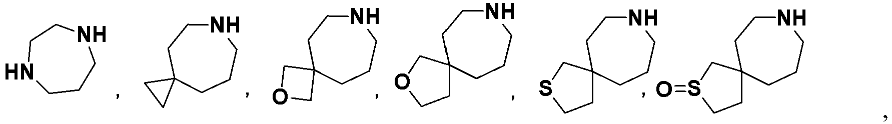 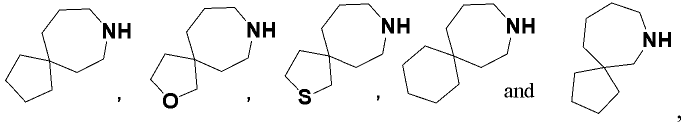 etc.

The heterocycloalkyl ring can be fused to an aryl or heteroaryl ring, wherein the ring connected to the parent structure is the heterocycloalkyl, non-limiting examples of which include: 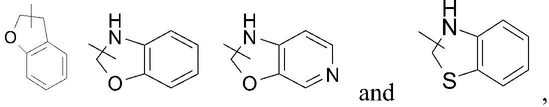 etc.

The heterocycloalkyl can be optionally substituted or unsubstituted. When substituted, the substituent is preferably one or more of the following groups, independently selected from the group consisting of halogen, hydroxy, oxo, cyano, amino, C₁₋₆ alkyl, C₁₋₆ alkoxy, 3- to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino (adjustments are required here according to the claims!).

The term "alkoxy" refers to -O-(alkyl), wherein the definition of the alkyl is as described above. Non-limiting examples of alkoxys include: methoxy, ethoxy, propoxy, and butoxy. The alkoxy can be optionally substituted or unsubstituted. When substituted, the substituent is preferably one or more of the following groups, independently selected from the group consisting of halogen, hydroxy, oxo, cyano, amino, C₁₋₆ alkyl, C₁₋₆ alkoxy, 3- to 7-membered cycloalkyl, and 3- to 7-membered heterocycloalkyl, wherein the alkyl, alkoxy, cycloalkyl, or heterocycloalkyl is optionally substituted with halogen, hydroxy, nitro, cyano, or amino (adjustments are required here according to the claims!).

Similarly, "cycloalkoxy" and "heterocycloalkoxy" are defined the same as the above "alkoxy".

The term "alkylthio" refers to -S-(alkyl), wherein the definition of the alkyl is as described above. Non-limiting examples of alkoxy include: methylthio, ethylthio, propylthio, and butylthio. The alkylthio can be optionally substituted or unsubstituted. When substituted, the substituent is preferably one or more of the following groups, independently selected from the group consisting of C₁₋₆ alkoxy, 3- to 6-membered cycloalkyl, 3- to 6-membered heterocycloalkyl, 3- to 6-membered cycloalkoxy, 3- to 6-membered heterocycloalkoxy, C₁₋₆ alkylthio, 3- to 6-membered cycloalkylthio, and 3-to 6-membered heterocycloalkylthio, wherein the alkoxy, cycloalkyl, heterocycloalkyl, cycloalkoxy, heterocyclooxy, alkylthio, cycloalkylthio, or heterocycloalkylthio is optionally substituted with halogen, hydroxy, cyano, or amino (adjustments are required here according to the claims!).

Similarly, "cycloalkylthio" and "heterocycloalkylthio" are defined the same as the above "alkylthio".

A "monovalent group" refers to a group formed by the "formal" elimination of one monovalent atom or group from a compound. A "ylene group" refers to a group formed by the "formal" elimination of two monovalent atoms or one divalent atom from a compound.

The term "alkylene" refers to the portion of an alkane molecule remaining after the removal of 2 hydrogen atoms, including straight-chain and branched-chain subgroups with 1 to 20 carbon atoms. For an alkylene containing 1 to 6 carbon atoms, non-limiting examples include methylene (-CH₂-) and ethylene (such as -CH₂CH₂- or -CH(CH₃)-). Unless otherwise specified, the alkylene can be substituted or unsubstituted. When substituted, the substituent can be substituted at any available attachment point, preferably one or more of the following groups, independently selected from the group consisting of halogen, hydroxy, cyano, amino, C₁₋₆ alkyl, and C₁₋₆ alkoxy (adjustments are required here according to claims!).

Similarly, the definitions of "alkeneoxy", "alkenyl", "alkenyloxy", "cycloalkylene", and "heterocycloalkylene" are the same as that of "alkylene".

The term "aryl" refers to a 6- to 14-membered all-carbon monocyclic or fused polycyclic (i.e., rings sharing adjacent pairs of carbon atoms) group having a conjugated π-electron system, preferably 6- to 12-membered, such as phenyl and naphthyl. The aryl ring can be fused to a heteroaryl, heterocycloalkyl, or cycloalkyl ring, wherein the ring connected to the parent structure is the aryl ring, non-limiting examples of which include:

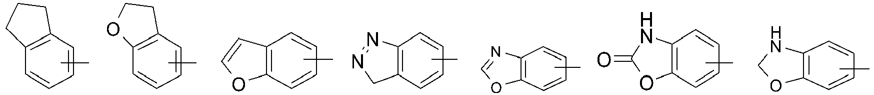

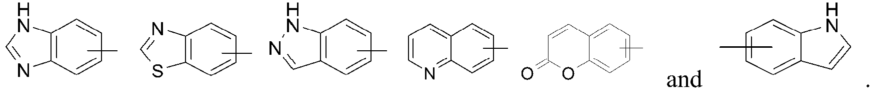

The aryl can be substituted or unsubstituted. When substituted, the substituent is preferably one or more of the following groups, independently selected from the group consisting of halogen, hydroxy, oxo, nitro, cyano, C₁₋₆ alkyl, C₁₋₆ alkoxy, C₂₋₆ alkenoxy, C₂₋₆ alkynoxy, 3- to 6-membered cycloalkoxy, 3- to 6-membered heterocycloalkoxy, C₃₋₈ cycloalkenoxy, and 5- to 6-membered aryl or heteroaryl, wherein the C₁₋₆ alkyl, C₁₋₆ alkoxy, C₂₋₆ alkenoxy, C₂₋₆ alkynoxy, 3- to 6-membered cycloalkoxy, 3- to 6-membered heterocycloalkoxy, 3- to 8-membered cycloalkenoxy, and 5- to 6-membered aryl or heteroaryl is optionally substituted with one or more substituents selected from the group consisting of halogen, hydroxy, cyano, amino, C₁₋₆ alkyl, and C₁₋₆ alkoxy (adjustments are _{required} here according to the claims!).

The term "heteroaryl" refers to a heteroaromatic system comprising 1 to 4 heteroatoms and 5 to 14 ring atoms, wherein the heteroatoms are selected from the group consisting of oxygen, sulfur, and nitrogen. The heteroaryl is preferably 6- to 12-membered, more preferably 5-membered or 6-membered. Non-limiting examples thereof include: imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzoimidazolyl, 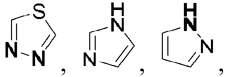 etc.

The heteroaryl ring can be fused to an aryl, heterocycloalkyl, or cycloalkyl ring, wherein the ring connected to the parent structure is the heteroaryl ring, non-limiting examples of which include:

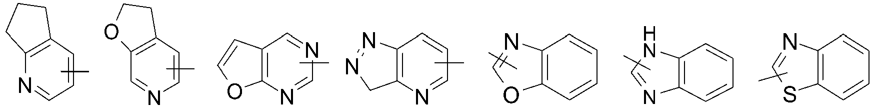

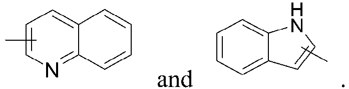

The heteroaryl can be optionally substituted or unsubstituted. When substituted, the substituent is preferably one or more of the following groups, independently selected from the group consisting of halogen, hydroxy, cyano, amino, C₁₋₆ alkyl, and C₁₋₆ alkoxy (adjustments are required here according to the claims!).

The term "spiro ring" refers to a compound in which two rings share one atom. Non-limiting examples of spiro cycloalkyl include:

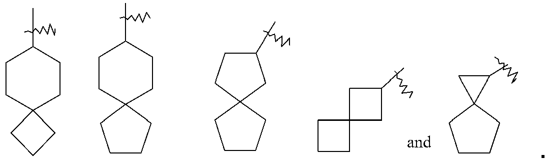

The term "fused ring" refers to a compound in which two or more rings are fused together by sharing two adjacent atoms. Non-limiting examples of fused cycloalkyl include:

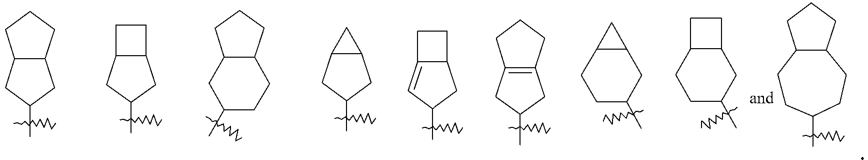

The term "bridged ring" refers to a structure formed by two or more cyclic structures sharing two non-adjacent ring atoms with each other. Depending on the number of constituent rings, it can be classified into bicyclic, tricyclic, tetracyclic, or polycyclic bridged cycloalkyls, preferably bicyclic, tricyclic, or tetracyclic, and more preferably bicyclic or tricyclic. Non-limiting examples of bridged cycloalkyl include:

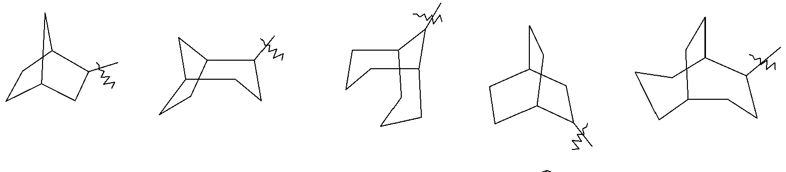

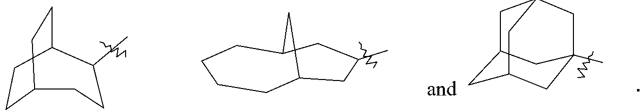

The term "heterocycle" refers to a ring composed of atoms other than carbon atoms, including heterocycloalkyl and heteroaromatic ring.

The term "hydroxy" refers to an -OH group.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to an =O substituent.

"Substituted" means that one or more hydrogen atoms in a group, preferably up to 5, more preferably 1 to 3 hydrogen atoms, are independently replaced by a corresponding number of substituents. When a substituent is a keto or oxo (i.e., =O), two (2) hydrogens on the atom are replaced.

"Optional" or "optionally" means that the event or environment described below may but does not have to occur, and the description includes the cases in which the event or environment occurs or does not occur. For example, "a heterocycloalkyl optionally substituted with an alkyl" means that the alkyl may or may not be present, and the description includes cases where the heterocycloalkyl is substituted with an alkyl and cases where the heterocycloalkyl is not substituted with an alkyl.

The term "pharmaceutical composition" refers to a mixture containing one or more of the compounds described herein or physiologically/pharmaceutically acceptable salts or prodrugs thereof with other chemical components, as well as other components such as physiological/pharmaceutical carriers and excipients. The purpose of a pharmaceutical composition is to facilitate administration to an organism, favoring the absorption of the active ingredient and thereby exerting biological activity.

The term "pharmaceutical carrier" used for the medicament of the present disclosure refers to a system capable of altering the manner in which the drug enters the human body and its distribution in the body, controlling the release rate of the drug, and delivering the drug to targeted organs. Drug carrier release and targeting system can reduce drug degradation and loss, decrease side effects, and improve bioavailability. For example, a polymer surfactant that can be used as a carrier can self-assemble due to its unique amphiphilic structure to form aggregates in various forms; preferred examples are micelles, microemulsions, gels, liquid crystals, vesicles, etc. These aggregates have the capability to encapsulate drug molecules, while simultaneously having good membrane permeability, and can serve as excellent pharmaceutical carriers.

The term "excipient" refers to an additive other than the active pharmaceutical ingredient in a pharmaceutical preparation, which may also be referred to as an auxiliary material. For example, binders, fillers, disintegrants, and lubricants in tablets; matrix portions in semi-solid preparations such as ointments and creams; and preservatives, antioxidants, flavoring agents, fragrances, solubilizers, emulsifiers, solvents, osmotic pressure regulators, colorants, etc. in liquid preparations can all be referred to as excipients.

The term "diluent", also known as a filler, is primarily used to increase the weight and volume of a tablet. The addition of a diluent not only ensures a certain volume size but also reduces the dose deviation of the main ingredient, improves the compressibility and formability of the drug, etc. When the tablet contains an oily component, an absorbent is added to absorb the oily substance to maintain a "dry" state, facilitating formulation into tablets.

The compound in the present disclosure may contain one or more asymmetric centers, thus producing enantiomers, diastereomers, and other stereoisomeric forms can be defined by absolute stereochemistry, as (*R*)- or (*S*)-, or as (*D*)*-* or (*L*)- for amino acids. The present disclosure includes all possible isomers as well as their racemic and optically pure forms. Optically active (+) and (-), (R)- and (S)-, or (D)- and (L)-isomers can be prepared using chiral synthons or chiral reagents, or can be prepared using conventional methods such as chromatography and fractional crystallization. Conventional methods for the preparation/isolation of individual enantiomers include chiral synthesis from a suitable optically pure precursor or resolution of the racemate (or the racemate of a salt or derivative) using, for example, chiral high-performance liquid chromatography (HPLC). When the compounds described herein contain an olefinic double bond or other centers of geometric asymmetry, unless otherwise specified, it means that the compounds include E and Z geometric isomers. Moreover, all tautomeric forms are also meant to be included.

In the chemical structures of the compounds of the present disclosure, a bond " 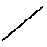 " indicates an unspecified configuration; that is, if a chiral isomer is present in the chemical structure, the bond " 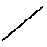 " can be " 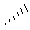 " or " 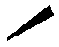 ", or it can encompass both " 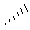 " and " 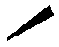 " configurations. In the chemical structures of the compounds of the present disclosure, a bond " 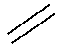 " indicates an unspecified configuration; that is, it can be a Z configuration or an E configuration, or it can encompass both configurations.

"Stereoisomers" refer to compounds consisting of the same atoms bonded by the same bonds but having different three-dimensional structures, which are not interchangeable. Various stereoisomers and mixtures thereof are contemplated in the present disclosure, including "enantiomers", which refer to two stereoisomers whose molecules are non-superimposable mirror images of one another.

"Tautomers" refer to the transfer of a proton from one atom of a molecule to another atom of the same molecule. Tautomers of any of said compounds are included in the present disclosure.

The present disclosure also includes isotopically-labeled compounds of the present disclosure which are identical to those recited herein, but in which one or more atoms are replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Unless otherwise specified, when a position is specifically designated as deuterium (D), the position should be understood to have deuterium abundance that is at least 1000 times greater than the natural abundance of deuterium (which is 0.015%) (i.e., at least 10% deuterium incorporation). The compounds in the examples can have deuterium abundance at least 1000 times, at least 2000 times, at least 3000 times, at least 4000 times, at least 5000 times, at least 6000 times greater than the natural abundance of deuterium, or an even higher abundance of deuterium. The present disclosure also includes various deuterated forms of the compound of Formula (I). Each available hydrogen atom attached to a carbon atom can be independently replaced by a deuterium atom. Those skilled in the art can synthesize deuterated forms of the compound of Formula (I) with reference to relevant literature. Commercially available deuterated starting materials can be used in the preparation of deuterated forms of the compound of Formula (I), or they can be synthesized using conventional techniques employing deuterated reagents, including but not limited to deuterated borane, trideuterated borane tetrahydrofuran solutions, deuterated lithium aluminum hydride, deuterated iodoethane, deuterated iodomethane, and the like.

### DESCRIPTION OF DRAWINGS

Figure 1 shows the determined cellular level of cytotoxicity and anti-influenza virus activity of the conjugate molecules of the present disclosure.
Figure 2 shows the PK curves of different test articles administered to cynomolgus monkeys via a single intravenous injection.

### DETAILED DESCRIPTION

The preparation of the compounds and pharmaceutically acceptable salts of the present disclosure is further described below in conjunction with examples, but these examples do not limit the scope of the present disclosure.

Experimental methods without specific conditions noted in the examples of the present disclosure were generally carried out according to conventional conditions or conditions suggested by the raw material or commercial manufacturers. Reagents whose specific sources were not noted were conventional reagents purchased from the market.

NMR shifts (δ) were given in units of 10⁻⁶ (ppm). NMR was determined using a Bruker AVANCE-400 nuclear magnetic resonance spectrometer, the determination solvents were deuterated dimethyl sulfoxide (DMSO-d⁶), deuterated chloroform (CDCl₃), and deuterated methanol (CD₃OD), and the internal standard was tetramethylsilane (TMS).

MS was determined using a Shimadzu 2010 Mass Spectrometer or an Agilent 6110A MSD mass spectrometer.

HPLC was determined using Shimadzu LC-20A systems, Shimadzu LC-2010HT series, or an Agilent 1200 LC high-pressure liquid chromatograph (Ultimate XB-C18 3.0*150 mm column or Xtimate C18 2.1*30 mm column).

Chiral HPLC analysis was carried out using Chiralpak IC-3 100×4.6 mm I.D., 3 µm; Chiralpak AD-3 150×4.6 mm I.D., 3 µm; Chiralpak AD-3 50×4.6 mm I.D., 3 µm; Chiralpak AS-3 150×4.6 mm I.D., 3 µm; Chiralpak AS-3 100×4.6 mm I.D., 3 µm; ChiralCel OD-3 150×4.6 mm I.D., 3 µm; Chiralcel OD-3 100×4.6 mm I.D., 3 µm; ChiralCel OJ-H 150×4.6 mm I.D., 5 µm; and Chiralcel OJ-3 150×4.6 mm I.D., 3 µm columns.

Yantai Huanghai HSGF254 or Qingdao GF254 silica gel plates were used for thin-layer chromatography (TLC) silica gel plates; the specification of the silica gel plates used for TLC was 0.15 mm to 0.2 mm, and the specification used for product separation and purification by TLC was 0.4 mm to 0.5 mm.

Column chromatography was generally performed with Yantai Huanghai silica gel 100 to 200 mesh, 200 to 300 mesh, or 300 to 400 mesh silica gel as a carrier.

A DAICEL CHIRALPAK IC (250 mm*30 mm, 10 µm) or Phenomenex-Amylose-1 (250 mm*30 mm, 5 µm) was used as a chiral preparative column.

A Combiflash Rf150 (TELEDYNE ISCO) was used as a CombiFlash rapid preparative instrument.

A NovoStar microplate reader (BMG, Germany) was used to determine the average kinase inhibition rate and IC₅₀ values.

The known starting materials of the present disclosure could be synthesized by using or according to methods known in the art, or could be purchased from companies such as ABCR GmbH & Co. KG, Acros Organics, Aldrich Chemical Company, Accela ChemBio Inc, and Darui Chemicals.

Unless otherwise specified in the examples, reactions could all be carried out under an argon atmosphere or a nitrogen atmosphere.

An argon atmosphere or a nitrogen atmosphere means that the reaction flask was connected to an argon or nitrogen balloon with a volume of about 1 L.

A hydrogen atmosphere means that the reaction flask was connected to a hydrogen balloon with a volume of about 1 L.

A Parr 3916EKX hydrogenator and a Qinglan QL-500 hydrogen generator or an HC2-SS hydrogenator were used for pressurized hydrogenation reactions.

Hydrogenation reactions were generally carried out under vacuum, filled with hydrogen gas, and repeated 3 times.

A CEM Discover-S 908860 microwave reactor was used for microwave reactions.

Unless otherwise specified in the examples, "solution" refers to an aqueous solution.

Unless otherwise specified in the examples, the reaction temperature was room temperature, which was 20°C to 30°C.

The reaction progress in the examples was monitored by thin-layer chromatography (TLC). The developing solvent used in the reaction, the eluent systems for column chromatography used to purify compounds, and the developing solvent systems for TLC included: A: dichloromethane/methanol system, B: *n*-hexane/ethyl acetate system, C: petroleum ether/ethyl acetate system, and D: petroleum ether/ethyl acetate/methanol. The volume ratio of the solvent was adjusted according to the different polarities of the compounds, and could also be adjusted by adding a small amount of basic or acidic reagents such as triethylamine and acetic acid.

The abbreviations used in the following experiments have the following meanings:
EtOAc: ethyl acetate; DCM: dichloromethane; DIPEA: N,N-diisopropylethylamine; PPTS: pyridinium p-toluenesulfonate; Boc: tert-butoxycarbonyl; MeOH: methanol.
PEG4-azido NHS ester: 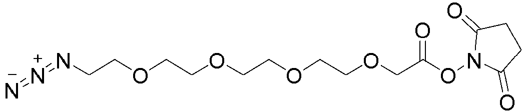
Azido-PEG3-Maleimide: 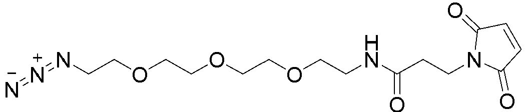
N3-PEG3-Oxa:

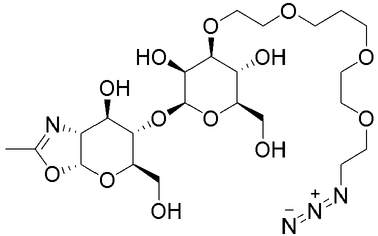

### Example 1 Synthesis of Intermediate Int-A

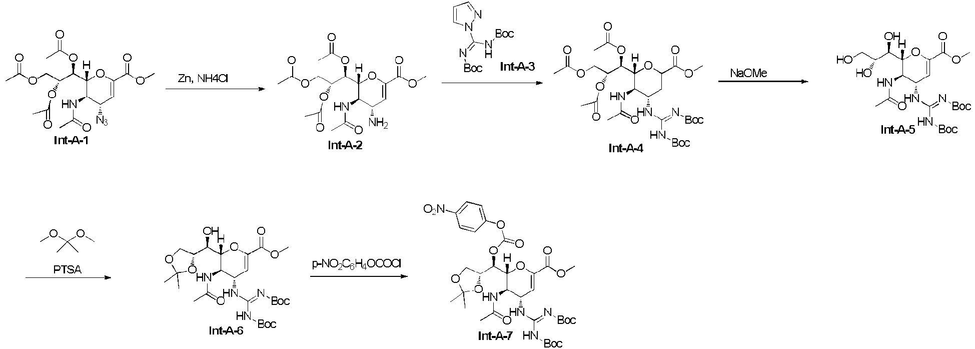

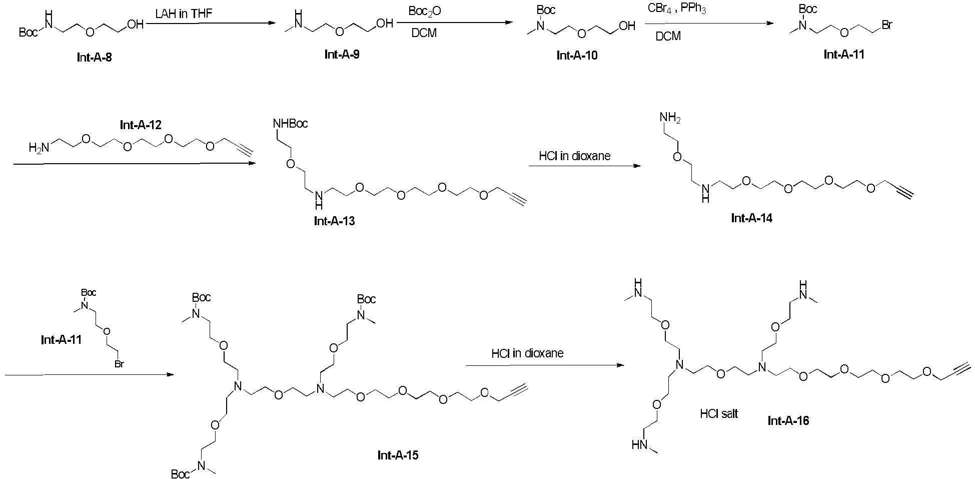

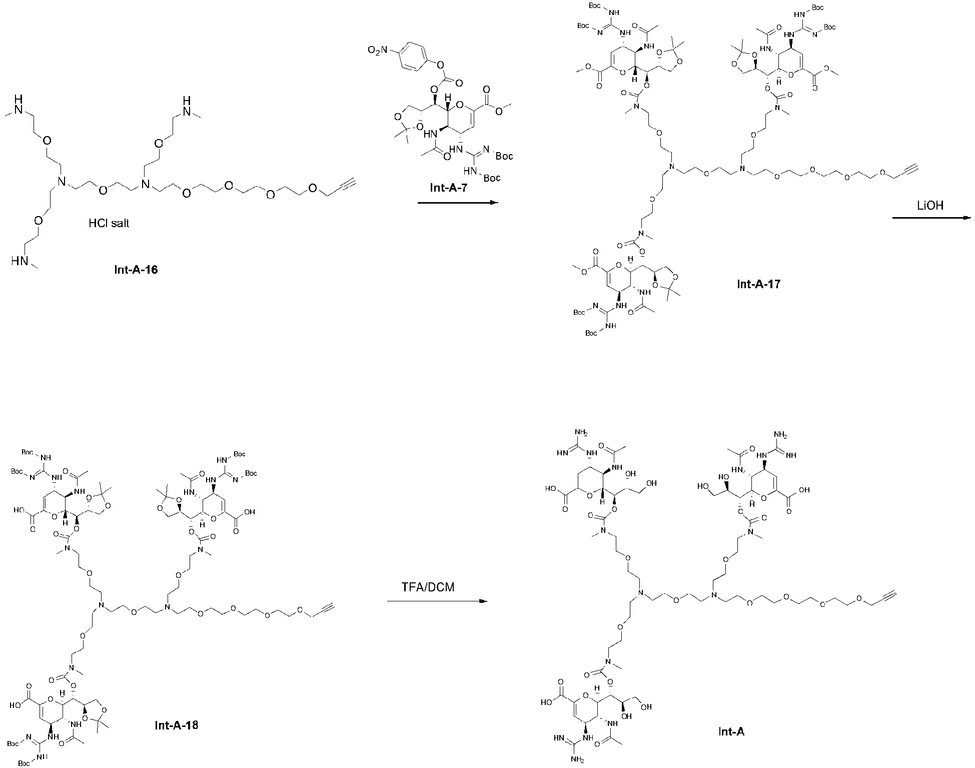

### Step 1:

### Compound

(1S,2R)-1-((2R,3R,4S)-3-acetamido-4-azido-6-(methoxycarbonyl)-3,4-dihydro-2H-pyra n-2-yl)propane-1,2,3-triyl triacetate Int-A-1 (8.5 g, 18.6 mmol) was dissolved in a mixed solution of ethanol (90 mL) and water (15 mL), and ammonium chloride (3.0 g, 56 mmol) and zinc powder (3.9 g, 60 mmol) were added sequentially. The reaction was stirred at 30°C for 30 minutes. After the reaction was completed, the mixture was cooled to 0°C and filtered. After the filtrate was concentrated, ethyl acetate (100 mL) was added, followed by filtration and concentration to give compound Int-A-2 (8.0 g, yield 100%).

¹H NMR (400 MHz, CDCl₃): *δ* 6.14 (s, 1H), 5.49 (s, 1H), 5.29 (s, 1H), 4.61 (d, 1H), 4.50-4.15 (m, 2H), 3.78-3.71 (m, 4H), 2.10-2.04 (m, 12H).

### Step 2:

Int-A-2 (8.0 g, 18.6 mmol) was dissolved in dry tetrahydrofuran (80 mL), and N,N'-di-Boc-1H-pyrazole-1-carboxamidine (6.3 g, 20.5 mmol) and triethylamine (4 mL, 27.9 mmol) were added sequentially. The reaction was stirred at room temperature overnight. After the reaction was completed, the reaction was concentrated in the cold, and ethyl acetate (200 mL) was added, followed by sequential washing with water (100 mL) and saturated brine (100 mL). The organic phase was dried over anhydrous sodium sulfate, filtered, and concentrated to obtain a crude product. The crude product was purified by silica gel column chromatography (petroleum ether : ethyl acetate = 1:1) to obtain compound Int-A-4 (11 g, yield 87.9%).

¹H NMR (400 MHz, CDCl₃): *δ* 11.40 (s, 1H), 8.51 (d, 1H), 6.75 (br, 1H), 5.86 (s, 1H), 5.46 (d, 1H), 5.31 - 5.24 (m, 1H), 5.12 (t, 1H), 4.64 (dd, 1H), 4.30 - 4.12 (m, 3H), 3.77 (s, 3H), 2.10 (s, 3H), 2.05 (s, 3H), 2.03 (s, 3H), 1.84 (s, 3H), 1.46 (d, 18H).

### Step 3:

Compound Int-A-4 (12.3 g, 18.3 mmol) was dissolved in anhydrous methanol (240 mL), and sodium methoxide solution (0.5 M in MeOH, 6.5 mL, 3.25 mmol) was added. The reaction was stirred at room temperature for 30 minutes. After the reaction was completed, IRN-77 resin was added for neutralization to neutral pH. The neutralized reaction solution was filtered and concentrated to obtain compound Int-A-5 (9.0 g, crude product), which was directly used in the next step.

¹H NMR (400 MHz, CDCl₃): *δ* 11.37 (s, 1H), 8.59 (d, 1H), 8.23 (d, 1H), 5.80 (s, 1H), 5.36 - 5.18 (m, 2H), 4.21 (d, 1H), 4.11 - 3.82 (m, 5H), 3.79 (s, 3H), 3.61 (d, 1H), 2.02 (s, 3H), 1.50 (d, 18H).

### Step 4:

Compound Int-A-5 (9.0 g, crude product) was dissolved in acetone (180 mL), and 2,2-dimethoxypropane (24 mL, 196 mmol) and p-toluenesulfonic acid monohydrate (740 mg, 3.92 mmol) were added sequentially. The reaction was stirred at room temperature overnight. After the reaction was completed, the reaction solution was concentrated. The crude product was purified by silica gel column chromatography (petroleum ether:ethyl acetate = 1:1) to obtain compound Int-A-6 (8.5 g, two-step yield 79.2%).

¹H NMR (400 MHz, CDCl₃): *δ* 11.36 (s, 1H), 8.65 (d, 1H), 8.02 (d, 1H), 5.80 (d, 1H), 5.27 (s, 1H), 5.16 (t, 1H), 4.43 - 4.36 (m, 1H), 4.21 - 4.07 (m, 2H), 4.05 - 3.93 (m, 2H), 3.79 (s, 3H), 3.50 (d, 1H), 2.01 (s, 3H), 1.50 (d, 18H), 1.43 (s, 3H), 1.37 (s, 3H).

### Step 5:

Compound Int-A-6 (8.2 g, 14.0 mmol) was dissolved in dry dichloromethane (160 mL). 4-Dimethylaminopyridine (13.7 g, 112 mmol) was added under an ice bath, and then 4-nitrophenyl chloroformate (16.8 g, 84 mmol) was added in batches. The reaction was stirred at room temperature overnight. After the reaction was completed, the reaction solution was concentrated. The crude product was diluted with ethyl acetate to precipitate a solid, followed by filtration. The filtrate was concentrated and then purified by silica gel column chromatography (petroleum ether:ethyl acetate = 1:1) to obtain compound Int-A-7 (5.2 g, yield 49.4%).

¹H NMR (400 MHz, CDCl₃): *δ* 11.35 (s, 1H), 8.56 (d, 1H), 8.35 (d, 2H), 7.54 (d, 2H), 6.60 (d, 1H), 5.90 (d, 1H), 5.26 (d, 1H), 5.23 - 5.16 (m, 1H), 4.49 - 4.37 (m, 2H), 4.27 - 4.20 (m, 2H), 4.18 - 4.12 (m, 1H), 3.81 (s, 3H), 1.92 (s, 3H), 1.49 (s, 18H), 1.42 (s, 3H), 1.39 (s, 3H).

### Step 6:

A solution of lithium aluminum hydride in tetrahydrofuran (2.5 M, 29.2 mL, 73.0 mmol) was added dropwise to a solution of compound Int-A-8 (5.0 g, 24.4 mmol) in tetrahydrofuran at 0°C. After completion of the addition, the mixture was heated to 80°C and stirred overnight. After the reaction was completed, the cooled reaction solution was slowly poured into a system of tetrahydrofuran (30 mL) containing sodium sulfate decahydrate (10 g), and stirred at room temperature for one hour. The solid was removed by filtration, and the mother liquor was evaporated to dryness to obtain compound Int-A-9 (2.90 g, crude product). It was directly used in the next step without further purification.

MS m/z (ESI):120.2 [M+1]⁺.

### Step 7:

A solution of di-tert-butyl dicarbonate (6.67 g, 30.5 mmol) in dichloromethane (20 mL) was added to a solution of compound Int-A-9 (2.90 g, crude product) in dichloromethane (50 mL) at 0°C, and the mixture was stirred at room temperature overnight. The reaction solution was concentrated, and purified by silica gel column chromatography (dichloromethane/ethyl acetate = 10/1 to 2/1) to obtain compound Int-A-10 (5.27 g, two-step yield: 98.5%).

¹H NMR (400 MHz, CDCl₃): *δ* 3.77 - 3.68 (m, 2H), 3.60-3.55 (m, 4H), 3.42 (s, 2H), 2.91 (s, 3H), 1.46 (s, 9H).

### Step 8:

Triphenylphosphine (1.79 g, 28.5 mmol) was added to a solution of compound Int-A-10 (4.17 g, 19.0 mmol) in dichloromethane (60 mL) at 0°C. After continuing stirring for 15 minutes, carbon tetrabromide (9.45 g, 28.5 mmol) was added in batches. After completion of the addition, the reaction solution was stirred at room temperature for 1 hour. The reaction solution was directly evaporated to dryness, and purified by silica gel column chromatography (dichloromethane/ethyl acetate = 10/1 to 2/1) to obtain compound Int-A-11 (4.14 g, yield 77.2%).

¹H NMR (400 MHz, CDCl₃): *δ* 3.77 (t, 2H), 3.61 (s, 2H), 3.50-3.41 (m, 4H), 2.93 (s,3H), 1.46 (s, 9H).

### Step 9:

Compound Int-A-12 (1.00 g, 4.32 mmol) and potassium carbonate (0.896 g, 6.48 mmol) were added to a solution of compound Int-A-11 (1.16 g, 4.11 mmol) in N,N-dimethylformamide (20 mL), and then the mixture was heated and stirred at 50°C overnight. The reaction solution was concentrated to remove N,N-dimethylformamide, and water (10 mL) was added. The solution was extracted with dichloromethane (10 mL × 3). The organic phases were combined, washed with saturated brine (20 mL), dried over anhydrous sodium sulfate, evaporated to dryness, and purified by silica gel column chromatography (dichloromethane/methanol = 20/1 to 10/1) to obtain compound Int-A-13 (0.860 g, yield 49.9%).

¹H NMR (400 MHz, CDCl₃): *δ* 5.24 (s, 1H), 4.21 (s, 2H), 3.75-3.64 (m, 13H), 3.61 (t, 2H), 3.52 (t, 2H), 3.35-3.26 (m, 2H), 2.95-2.82 (m, 4H), 2.45 (s, 1H), 1.44 (s, 9H).

### Step 10:

A solution of HCl in dioxane (4 M, 10 mL) was added to a solution of compound Int-A-13 (0.76 g, 1.82 mmol) in dichloromethane (5 mL) at 0°C. The reaction solution was then stirred at room temperature for 2 hours and concentrated to obtain compound Int-A-14 (0.72 g, crude product). It was directly used in the next step without further purification.

¹H NMR (400 MHz, DMSO-*d₆*): *δ* 8.80 (br, 2H), 8.02 (br, 3H), 4.15 (s, 2H), 3.78 - 3.73 (m, 2H), 3.72 - 3.67 (m, 2H), 3.64 - 3.52 (m, 14H), 3.45 (t, 1H), 3.25-3.15 (m, 4H), 3.02 (q, 2H).

### Step 11:

Compound Int-A-11 (1.98 g, 8.76 mmol) and potassium carbonate (1.21 g, 8.76 mmol) were added to a solution of compound Int-A-14 (0.56 g, 1.42 mmol) in N,N-dimethylformamide (10 mL), and then the reaction solution was heated and stirred at 60°C overnight. The reaction solution was concentrated under reduced pressure to remove N,N-dimethylformamide, and water (15 mL) was added. The solution was extracted with dichloromethane (15 mL × 3). The organic phases were combined, washed with saturated brine (20 mL), dried over anhydrous sodium sulfate, evaporated to dryness, and purified by silica gel column chromatography (dichloromethane/methanol = 20/1 to 10/1) and preparative high-performance liquid chromatography (Waters Xbridge, 19*150mm, 30*150mm, 5 µm; mobile phase A: 0.5% aqueous TFA solution, mobile phase B: acetonitrile, gradient ratio: phase B 5%-95%) to obtain compound Int-A-15 (587 mg, yield 36.3%).

### Step 12:

A solution of HCl in dioxane (4 M, 5.0 mL) was added to a solution of compound Int-A-15 (280 mg, 0.303 mmol) in dichloromethane (2.0 mL) at 0°C. The reaction solution was then stirred at room temperature for 2 hours and concentrated to obtain compound Int-A-16 (0.245 g, crude product). It was directly used in the next step without further purification.

MS m/z (ESI):622.6 [M+1]⁺.

### Step 13:

Compound Int-A-16 (100 mg crude product, 0.124 mmol) was dissolved in dry N,N-dimethylformamide (6 mL). N,N-diisopropylethylamine (370 mg, 2.9 mmol) was added under an ice bath, followed by the addition of compound Int-A-7 (380 mg, 0.51 mmol). The reaction was stirred at room temperature overnight. After the reaction was completed, ethyl acetate (15 mL) was added, followed by sequential washing with water (10 mL) and saturated brine (15 mL). The organic phase was dried over anhydrous sodium sulfate, filtered, and concentrated to obtain a crude product. The crude product was purified by silica gel column chromatography (ethyl acetate:methanol = 8:1) to obtain compound Int-A-17 (140 mg, yield 45.9%).

¹H NMR (400 MHz, CDCl₃): *δ* 11.39 (s, 3H), 8.41 (d, 3H), 6.34 - 6.10 (m, 3H), 5.89 (s, 3H), 5.31 - 5.20 (m, 6H), 4.43 - 4.02 (m, 9H), 4.17 - 4.00 (m, 10H), 3.79 (s, 9H), 3.70 - 3.47 (m, 34H), 3.28-3.21 (m, 3H), 3.00 - 2.93 (m, 9H), 2.78-2.62 (m, 10H), 1.89 (s, 9H), 1.48 (d, 54H), 1.43 (s, 3H), 1.38 - 1.34 (m, 15H).

### Step 14:

Compound Int-A-17 (140 mg, 0.057 mmol) was dissolved in methanol (10 mL), and an aqueous solution (10 mL) of lithium hydroxide monohydrate (22 mg, 0.52 mmol) was added under an ice bath. The reaction was stirred at room temperature for 16 hours. After the reaction was completed, IRN 77 resin was added for neutralization to neutral pH. The neutralized reaction solution was filtered and concentrated to obtain compound Int-A-18 (110 mg, yield 80%).

MS m/z (ESI): 806.6 ([M+3]/3)⁺.

### Step 15:

Compound Int-A-18 (110 mg, 0.045 mmol) was dissolved in dichloromethane (3 mL), and trifluoroacetic acid (6 mL) was added. The reaction was stirred at room temperature for 2 hours. After the reaction was completed, the reaction solution was evaporated to dryness, and then water (3 mL) was added, and the mixture was stirred at room temperature for 1 hour. The reaction solution was concentrated, and purified by preparative high-performance liquid chromatography (Waters Xbridge, 19*150mm, 30*150mm, 5µm; mobile phase A: 0.5% aqueous TFA solution, mobile phase B: acetonitrile, gradient ratio: phase B 5%-95%) to obtain the title product Int-A (30.1 mg, yield 31%).
MS m/z (ESI): 848.7 ([M+2]/2)⁺,566.0([M+3]/3)⁺;
¹H NMR (400 MHz, CD₃OD): *δ* 5.83 (s, 3H), 4.93 (d, 3H), 4.51 (d, 3H), 4.42-4.33 (m, 3H), 4.24 - 4.14 (m, 5H), 4.09-3.76 (m, 17H), 3.7 - 3.41 (m, 37H), 3.22-3.13 (m, 4H), 2.94-2.81 (m, 9H), 1.87 (s, 9H).

### Example 2 Synthesis of Intermediate Int-B

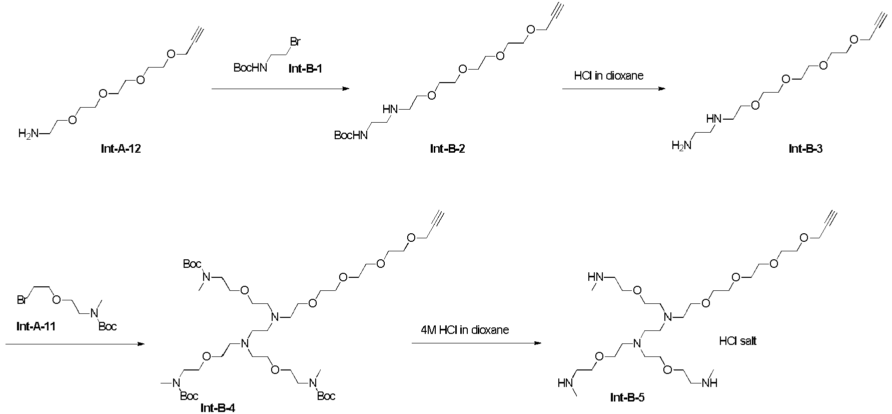

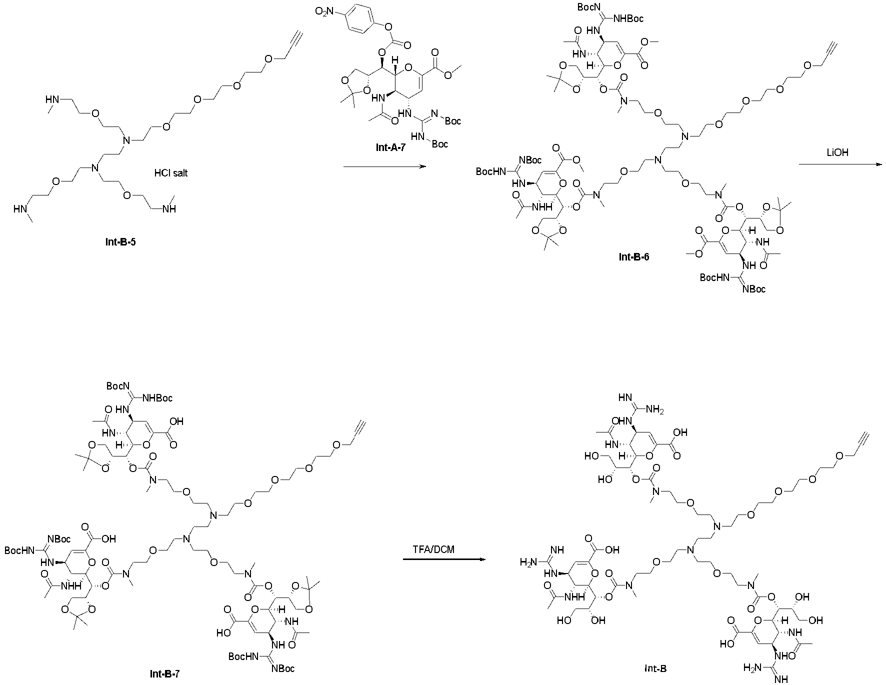

### Step 1:

Compound Int-A-12 (3.1 g, 13.4 mmol) and potassium carbonate (3.93 g, 28.5 mmol) were added to a solution of compound Int-B-1 (3.0 g, 13.4 mmol) in N,N-dimethylformamide (45 mL), and then the reaction solution was heated and stirred at 55°C overnight. The reaction solution was concentrated under reduced pressure to remove N,N-dimethylformamide, and water (60 mL) was added, followed by extraction with dichloromethane (60 mL × 3). The organic phases were combined, washed with saturated brine (60 mL), dried over anhydrous sodium sulfate, evaporated to dryness, and purified by silica gel column chromatography (dichloromethane/methanol = 20/1 to 10/1) to obtain compound Int-B-2 (0.810 g, yield 16.1%).

¹H NMR (400 MHz, CDCl₃) *δ*: 5.16 (br, 1H), 4.21 (s, 2H), 3.71 - 3.59 (m, 14H), 3.28-3.21 (m, 2H), 2.71-2.82 (m, 4H), 2.44 (s, 1H), 1.44 (s, 9H).

### Step 2:

A solution of HCl in dioxane (4 M, 10 mL) was added to a solution of compound Int-B-2 (800 mg, 2.14 mmol) in dichloromethane (5.0 mL) at 0°C. The system was stirred at room temperature for 4 hours and concentrated to obtain compound Int-B-3 (0.75 g, crude product), which was directly used in the next step.

### Step 3:

Compound Int-A-11 (2.20 g, 7.8 mmol) and potassium carbonate (1.35 g, 9.8 mmol) were added to a solution of compound Int-B-3 (0.677 g, 2.0 mmol) in N,N-dimethylformamide (10 mL), and then the mixture was heated and stirred at 65°C overnight. The reaction solution was concentrated under reduced pressure to remove N,N-dimethylformamide, and water (15 mL) was added. The solution was extracted with DCM (15 mL × 3). The organic phases were combined, washed with saturated brine (20 mL), dried over anhydrous sodium sulfate, evaporated to dryness, and purified by silica gel column chromatography (dichloromethane/methanol = 20/1 to 10/1) to obtain compound Int-B-4 (970 mg, yield 56.6%).

MS m/z (ESI): 439.9 ([M+2]/2)⁺.

### Step 4:

A solution of HCl in dioxane (4 M, 6.0 mL) was added to a solution of compound Int-B-4 (406 mg, 0.462 mmol) in dichloromethane (1.0 mL) at 0°C. The reaction solution was then stirred at room temperature for 1 hour and concentrated to obtain compound Int-B-5 (355 mg, crude product), which was directly used in the next step.

MS m/z (ESI): 578.7 [M+1]⁺.

### Step 5:

Compound Int-B-5 (215 mg, 0.28 mmol) was dissolved in dry N,N-dimethylformamide (6 mL), and N,N-diisopropylethylamine (1 mL, 6.2 mmol) was added under an ice bath, followed by the addition of compound Int-B-5 (940 mg, 1.25 mmol). The reaction was stirred at room temperature for 16 h. After the reaction was completed, ethyl acetate (30 mL) was added, followed by sequential washing with water (15 mL) and brine (15 mL). The organic phase was dried over anhydrous sodium sulfate, filtered, and concentrated to obtain a crude product. The crude product was purified by preparative chromatography (ethyl acetate:methanol = 8:1) to obtain compound Int-B-6 (170 mg, yield 25.1%).

¹H NMR (400 MHz, CDCl₃): *δ* 11.39 (s, 3H), 8.41 (d, 3H), 6.37-6.12 (m, 3H), 5.89 (s, 3H), 5.43-5.15 (m, 6H), 4.47-3.95 (m, 15H), 3.79 (s, 9H), 3.72 - 3.46 (m, 30H), 3.38-3.12 (m, 3H), 3.05-2.55 (m, 23H), 1.89 (s, 9H), 1.48 (s, 54H), 1.39 - 1.33 (m, 18H).

### Step 6:

Compound Int-B-6 (140 mg, 0.058 mmol) was dissolved in methanol (10 mL), and an aqueous solution (10 mL) of lithium hydroxide monohydrate (22 mg, 0.51 mmol) was added at 0°C. The reaction was stirred at room temperature for 16 hours. After the reaction was completed, IRN 77 resin was added for neutralization to neutral pH. The neutralized reaction solution was filtered and concentrated to obtain compound Int-B-7 (150 mg, crude product), and the product was directly used in the next reaction.

MS m/z (ESI): 792.0 ([M+3]/3)⁺.

### Step 7:

The compound Int-B-7 from the previous step (150 mg, crude product) was dissolved in dichloromethane (2 mL), and trifluoroacetic acid (4 mL) was added. The reaction was stirred at room temperature for 2 hours. After the reaction was completed, the reaction solution was evaporated to dryness, and then water (4 mL) was added, and the mixture was stirred at room temperature for 1 hour. The reaction solution was concentrated, and purified by preparative high-performance liquid chromatography (Waters Xbridge, 19*150mm, 30*150mm, 5 µm; mobile phase A: 0.5% aqueous TFA solution, mobile phase B: acetonitrile, gradient ratio: phase B 5% to 95%) to obtain the title product Int-B (61.2 mg, two-step yield 47.5%).
MS m/z (ESI): 827.1 ([M+2]/2)⁺,551.8([M+3]/3)⁺;
¹H NMR (400 MHz, CD₃OD): *δ* 5.90 (s, 3H), 5.01 (d, 3H), 4.54 (d, 3H), 4.40 (d, 3H), 4.26 - 4.16 (m, 5H), 4.04 - 3.97 (m, 3H), 3.88 - 3.55 (m, 36H), 3.53 - 3.12 (m, 14H), 3.03-2.77 (m, 10H), 1.95 (s, 9H).

### Example 3 Synthesis of Intermediate Int-C

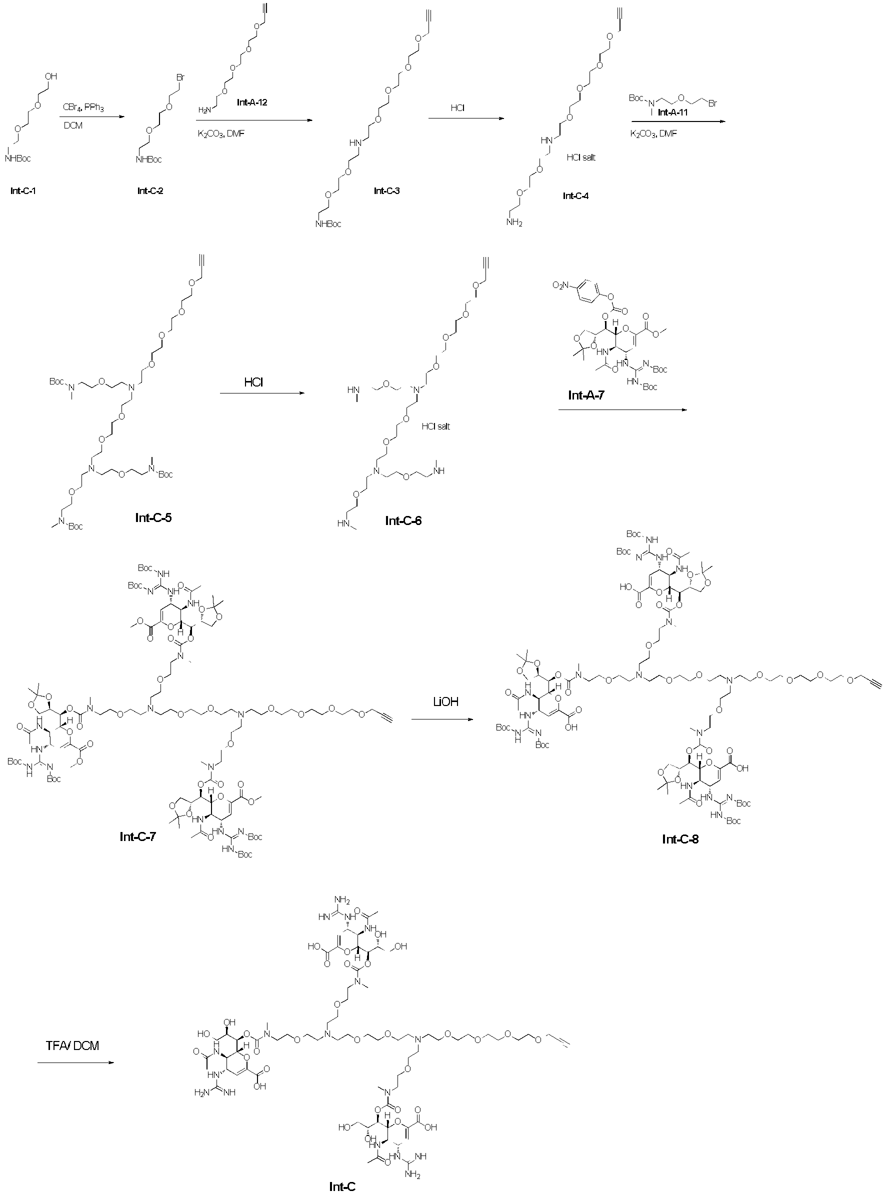

### Step 1:

Triphenylphosphine (7.89 g, 30 mmol) was added to a solution of compound Int-C-1 (5.0 g, 20 mmol) in dichloromethane (100 mL) at 0°C under stirring, and carbon tetrabromide (9.97 g, 30 mmol) was added in batches. After completion of the addition, the mixture was stirred at room temperature. After the reaction was completed, the reaction solution was concentrated under reduced pressure, and purified by silica gel column chromatography (dichloromethane/ethyl acetate = 10/1 to 4/1) to obtain compound Int-C-2 (4.70 g, yield: 75.3%).

¹H NMR (400 MHz, CDCl₃): *δ* 5.01 (br, 1H), 3.82 (t, 2H), 3.66-3.62 (m, 4H), 3.55 (t, 2H), 3.49 (t, 2H), 3.33-3.32 (m, 2H), 1.45 (s, 9H).

### Step 2:

Compound Int-A-12 (2 g, 8.64 mmol) and potassium carbonate (2.39 g, 17.30 mmol) were added to a solution of compound Int-C-2 (2.70 g, 8.64 mmol) in N,N-dimethylformamide (40 mL). The reaction system was heated and stirred at 55°C overnight. The reaction solution was concentrated to remove N,N-dimethylformamide, and water (20 mL) was added. The solution was extracted with dichloromethane (20 mL × 3). The organic phases were combined, washed with a saturated sodium chloride solution (40 mL), dried over anhydrous sodium sulfate, concentrated under reduced pressure, and purified by silica gel column chromatography (dichloromethane/methanol = 20/1 to 10/1) to obtain compound Int-C-3 (2 g, yield: 50.0%).

MS m/z (ESI): 463.3 [M+H] ⁺.

¹H NMR (400 MHz, CDCl₃): *δ* 5.24 (br, 1H), 4.21 (d, 2H), 3.69-3.60 (m, 20H), 3.54 (t, 2H), 3.49 (s, 1H), 3.32-3.31 (m, 2H), 2.84 (t, 4H), 2.44 (t, 1H), 1.44 (s, 9H).

### Step 3:

A solution of hydrogen chloride in dioxane (4 M, 20 mL) was added to a solution of compound Int-C-3 (1.50 g, 3.24 mmol) in dichloromethane (5 mL) at 0°C. The reaction system was stirred at room temperature for 2 hours and concentrated to obtain compound Int-C-4 (1.41 g, crude product), which was directly used in the next reaction.

MS m/z (ESI): 363.2 [M+H]⁺.

¹H NMR (400 MHz, DMSO-*d6*): *δ* 8.89 (br, 2H), 8.05 (br, 3H), 4.15 (d, 2H), 3.72 (t, 4H), 3.66-3.60 (m, 6H), 3.59-3.51 (m, 12H), 3.46 (t, 1H), 3.21-3.10 (m, 4H), 2.97 (dd, 2H).

### Step 4:

Compound Int-A-11 (4.09 g, 14.49 mmol) and potassium carbonate (2.69 g, 19.46 mmol) were added to a solution of compound Int-C-4 (1.41 g, crude product from the previous step) in *N,N*-dimethylformamide (15 mL), and then the system was heated and stirred at 65°C overnight. The reaction solution was concentrated to remove *N,N*-dimethylformamide, and water (15 mL) was added. The solution was extracted with dichloromethane (15 mL × 3). The organic phases were combined, washed with a saturated sodium chloride solution (20 mL), dried over anhydrous sodium sulfate, concentrated under reduced pressure, and purified by silica gel column chromatography (dichloromethane/methanol = 20/1 to 10/1) to obtain compound Int-C-5 (1.30 g, two-step yield: 41.5%).

MS m/z (ESI): 483.9 ([M+2H]/2)⁺.

¹H NMR (400 MHz, CDCl₃): *δ* 4.20 (d, 2H), 3.72-3.62 (m, 12H), 3.61-3.47 (m, 24H), 3.36 (s, 6H), 2.90 (s, 9H), 2.77 (d, 10H), 2.44 (s, 1H), 1.45 (s, 27H).

### Step 5:

A solution of hydrogen chloride in dioxane (4 M, 10 mL) was added to a solution of compound Int-C-5 (400 mg, 0.41 mmol) in dichloromethane (5.0 mL) at 0°C. Then the reaction system was stirred at room temperature for 1 hour and concentrated to obtain compound Int-C-6 (350 mg, crude product), which was directly used in the next reaction.

MS m/z (ESI): 666.5 [M+H] ⁺.

### Step 6:

Compound Int-A-7 (933 mg, 1.24 mmol) and *N,N*-diisopropylethylamine (533 mg, 4.12 mmol) were added to a solution of compound Int-C-6 (350 mg, crude product from the previous step) in *N,N*-dimethylformamide (10 mL), and then the mixture was stirred at room temperature for 12 hours. The reaction solution was concentrated at room temperature to remove *N,N*-dimethylformamide, and purified by reverse phase preparative chromatography to obtain compound Int-C-7 (302 mg, two-step yield: 29.4%).

¹H NMR (400 MHz, CDCl₃): *δ* 11.40 (s, 3H), 8.41 (d, 3H), 6.21-6.06 (m, 4H), 5.88 (s, 3H), 5.27-5.20 (m, 6H), 4.41-4.35 (m, 6H), 4.20 (d, 2H), 4.17-3.99 (m, 10H), 3.79 (s, 9H), 3.69 (d, 4H), 3.66-3.61 (m, 7H), 3.60-3.48 (m, 27H), 3.28-3.21 (m, 4H), 2.99 (s, 6H), 2.94 (s, 3H), 2.75 (br, 10H), 2.47-2.46 (m, 1H), 1.90-1.80 (m, 9H), 1.48 (d, 52H), 1.40-1.33 (m, 18H).

### Step 7:

An aqueous solution (11.2 mL) of lithium hydroxide monohydrate (13.3 mg, 0.32 mmol) was added to a solution of compound Int-C-7 (200 mg, 79.88 µmol) in methanol (11.2 mL) at 0°C, and then stirred at 20°C for 12 hours. After the reaction was completed, the reaction solution was adjusted to pH=7 using IRN77 ion exchange resin. The mother liquor after filtration was concentrated to obtain compound Int-C-8 (197 mg, crude product), which was directly used in the next reaction.

MS m/z (ESI): 821.3 ([M+3H]/3)⁺.

### Step 8:

Trifluoroacetic acid (5.0 mL) was added to a solution of compound Int-C-8 (197 mg, crude product from the previous step) in dichloromethane (5.0 mL) at 0°C, and then stirred at 20°C for 1 hour. After concentration, water (5 mL) was added, followed by stirring for 2 hours and concentration under reduced pressure. The resulting mixture was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-C trifluoroacetate (43 mg, two-step yield: 23.3%).

MS m/z (ESI): 870.9 ([M+2H]/2)⁺.

¹H NMR (400 MHz, CD₃OD): *δ* 5.92 (s, 3H), 5.02-5.04 (m, 3H), 4.56 (dd, 3H), 4.43 (d, 3H), 4.31-4.18 (m, 6H), 4.09-3.90(m, 17H), 3.74-3.68 (m, 22H), 3.62-3.42 (m, 17H), 3.26-3.14 (m, 5H), 3.01 (s, 5H), 2.98-2.92 (m, 4H), 1.97 (s, 9H).

### Example 4 Synthesis of Intermediate Int-D

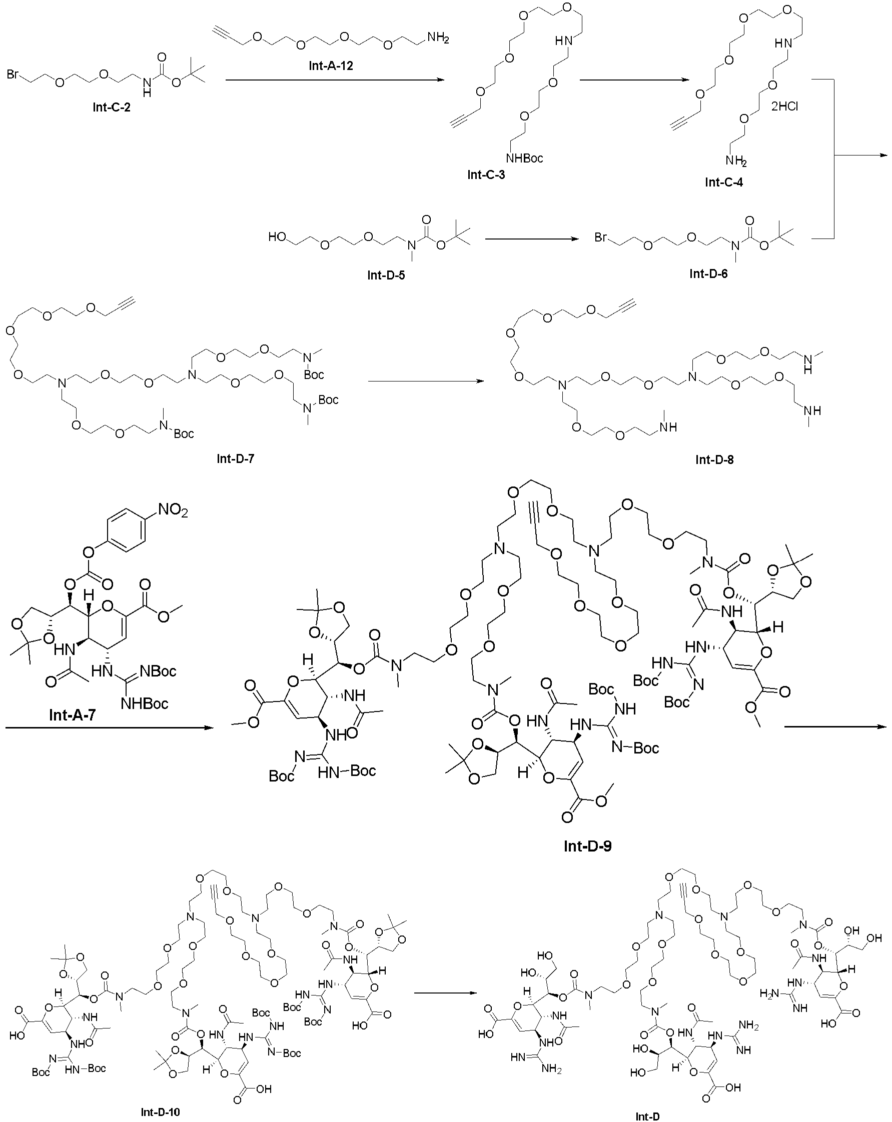

### Step 1: Synthesis of Compound Int-C-3

**Compound Int-A-12** (1 g, 4.32 mmol, purchased from Accela) and **compound Int-C-2** (1.35 g, 4.32 mmol, prepared using a literature method, Bioorganic and Medicinal Chemistry Letters, 2012, vol. 22, # 2, p. 1151 - 1155) were dissolved in DMF and stirred. After potassium carbonate (1.2 g, 8.65 mmol) was added, the mixture was heated to 55°C and reacted for 16 hours. The reaction solution was concentrated to dryness, and dichloromethane (20 mL) and water (10 mL) were added for liquid separation. The aqueous phase was extracted with dichloromethane (20 mL × 2), and the organic phases were combined, dried over anhydrous sodium sulfate, concentrated to dryness, and purified by column chromatography (DCM:MeOH = 0 to 10%) to obtain 1.12 g of **compound Int-C-3** (yield: 55.99%).

MS m/z (ESI): 463.3[M+1]⁺.

### Step 2: Synthesis of Compound Int-C-4

**Compound Int-C-3** (300 mg, 0.648 mmol) was dissolved in dichloromethane (1.5 mL) and stirred under an ice-water bath. A solution of hydrochloric acid in dioxane (6 mL) was added, and the mixture naturally warmed to room temperature to react for 2 hours. The reaction solution was concentrated to dryness to obtain 290 mg of a crude product, which was directly used in the next reaction.

MS m/z (ESI): 363.2[M+1]⁺.

### Step 3: Synthesis of Compound Int-D-6

**Compound Int-D-5** (2.51 g, 9.53 mmol, prepared using the method in WO2019/195609) was dissolved in dichloromethane (30 mL) and stirred. Triphenylphosphine (3.75 g, 14.30 mmol) was added. After stirring at room temperature for 15 minutes, carbon tetrabromide (4.74 g, 14.30 mmol) was added in batches. After completion of the addition, the mixture reacted at room temperature for 1 hour. The reaction solution was concentrated to dryness and purified by column chromatography (DCM:EA = 0 to 50%) to obtain 2.87 g of **compound Int-D-6** (yield: 92.28%).

MS m/z (ESI): 348.1[M+23]⁺.

### Step 4: Synthesis of Compound Int-D-7

**Compound Int-C-4** (917 mg, 2.11 mmol) and **compound Int-D-6** (2.4 g, 7.37 mmol) were dissolved in DMF (10 mL) and stirred. After potassium carbonate (2.91 g, 21.06 mmol) was added, the mixture was heated to 65°C and reacted for 16 hours. The reaction solution was concentrated to dryness, dichloromethane (20 mL) and water (10 mL) were added for liquid separation. The aqueous phase was extracted with dichloromethane (20 mL × 2), and the organic phases were combined, dried over anhydrous sodium sulfate, filtered, concentrated to dryness, and purified by column chromatography (DCM:MeOH = 0 to 10%) to obtain 1.47 g of **compound Int-D-7** (yield: 63.55%).

MS m/z (ESI):550.0 [M/2+1]⁺.

### Step 5: Synthesis of Compound Int-D-8

**Compound Int-D-7** (500 mg, 0.455 mmol) was dissolved in dichloromethane (5 mL) and stirred under an ice-water bath. A solution of hydrochloric acid in dioxane (12.52 mL) was added, and the mixture naturally warmed to room temperature to react for 2 hours. The reaction solution was concentrated to dryness to obtain 481.17 mg of a crude product, which was directly used in the next reaction.

MS m/z (ESI):798.3 [M+1]⁺.

### Step 6: Synthesis of Compound Int-D-9

**Compound Int-D-8** (446.27 mg, 0.456 mmol) was dissolved in DMF (5 mL) and stirred. DIPEA (589.50 mg, 4.56 mmol) was added. After stirring at room temperature for 5 minutes, **compound Int-A-7** (1.03 g, 1.37 mmol) was added. The mixture reacted at room temperature for 16 hours and was purified by column chromatography (ammonium bicarbonate:methanol = 70 to 95%) to obtain 590.9 mg of **compound Int-D-9** (yield: 49.15%).

MS m/z (ESI):1318.7 [M/2+1]⁺.

### Step 7: Synthesis of Compound Int-D-10

**Compound Int-D-9** (200 mg, 75.87 µmol) was dissolved in methanol (15 mL) and stirred. Water (15 mL) was added, followed by the addition of lithium hydroxide monohydrate (31.84 mg, 758.75 µmol). After completion of the addition, the mixture reacted at room temperature for 16 hours. IRN77 ion exchange resin was added to the reaction to adjust the pH to 7, followed by filtration. The filter cake was rinsed with methanol, and the filtrate was concentrated to dryness to obtain 0.197 g of a crude product, which was directly used in the next step.

MS m/z (ESI): 1297.6 [M/2+1]⁺.

### Step 8: Synthesis of Compound Int-D

**Compound Int-D-10** (0.197 g, 75.95 µmol) was dissolved in DCM (6 mL) and stirred at room temperature. After TFA (8.88 g, 77.85 mmol) was added, the mixture was stirred at room temperature for 5 hours, and the reaction solution was concentrated to dryness. After water (6 mL) was added, the mixture was stirred at room temperature for 3.5 hours and purified by column chromatography (TFA:acetonitrile = 70 to 85%) to obtain 117.7 mg of **compound Int-D** (yield: 63.43%).

MS m/z (ESI):937.3 [M/2+1]⁺, 625.3 [M/3+1]⁺, 469.1 [M/4+1]⁺.

¹H NMR (400 MHz, CDCl3): δ 5.89-5.80 (m, 3H), 5.05-4.95 (m, 5H), 4.58-4.49 (m, 3H), 4.46-4.38 (m, 3H), 4.29-4.13 (m, 5H), 4.09-3.96 (m, 3H), 3.96-3.75 (m, 15H) , 3.75-3.44 (m, 56H), 3.22-3.10 (m, 3H), 3.02-2.89 (m, 10H), 1.98-1.91 (m, 9H).

### Example 5 Synthesis of Intermediate Int-E

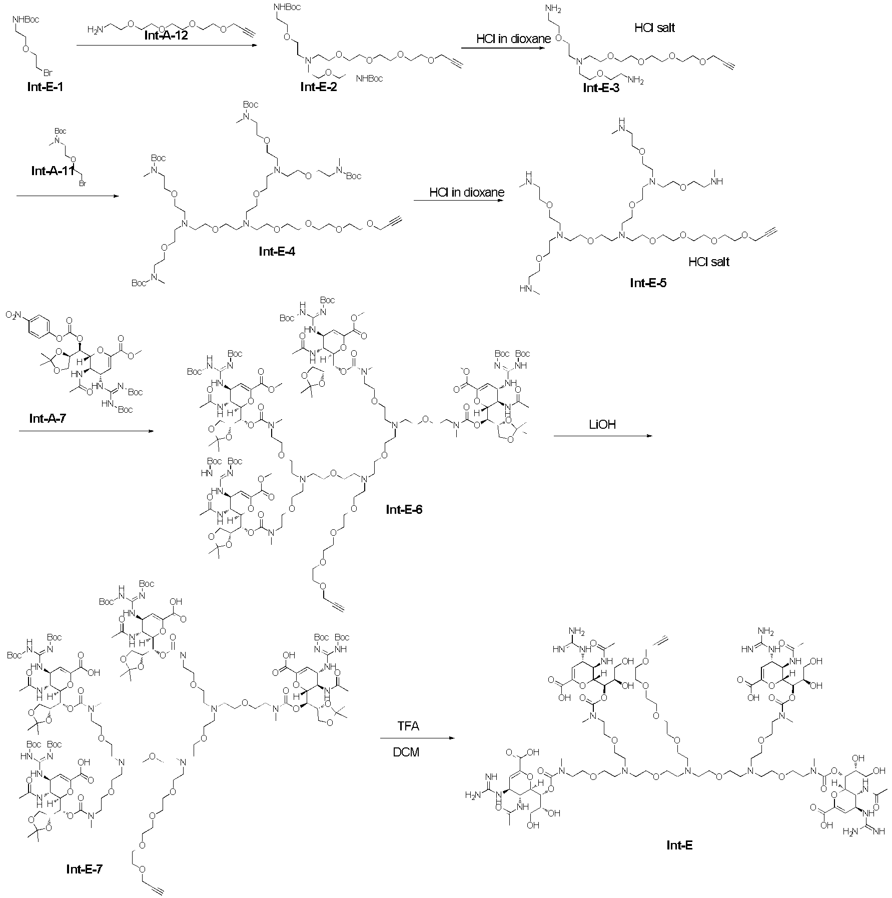

### Step 1:

Compound Int-E-1 (3.82 g, 14.25 mmol) and potassium carbonate (2.68 g, 19.39 mmol) were added to a solution of compound Int-A-12 (1.50 g, 6.49 mmol) in N,N-dimethylformamide (25 mL), and then the system was heated and stirred at 55°C for 12 hours. The reaction solution was concentrated to remove N,N-dimethylformamide, and water (20 mL) was added. The solution was extracted with dichloromethane (20 mL × 3). The organic phases were combined, washed with a saturated sodium chloride solution (30 mL), dried over anhydrous sodium sulfate, concentrated under reduced pressure, and purified by silica gel column chromatography (dichloromethane/methanol = 30/1 to 10/1) to obtain compound Int-E-2 (1.95 g, yield: 49.6%).

MS m/z (ESI): 606.4 [M+H] ⁺.

¹H NMR (400 MHz, CDCl₃): *δ* 4.21 (d, 2H), 3.74-3.58 (m, 14H), 3.58-3.46 (m, 10H), 3.30 (d, 4H), 2.78-2.75 (m, 6H), 2.44 (t, 1H), 1.44 (s, 18H)..

### Step 2:

A solution of hydrogen chloride in dioxane (4 M, 10 mL) was added to a solution of compound Int-E-2 (1.85 g, 3.05 mmol) in dichloromethane (2.0 mL) at 0°C. The reaction solution was then stirred at 0°C for 1 hour and concentrated to obtain compound Int-E-3 (1.51 g, crude product), which was directly used in the next reaction.

### Step 3:

Compound Int-A-11 (4.70 g, 16.66 mmol) and potassium carbonate (2.93 g, 21.20 mmol) were added to a solution of compound Int-E-3 (1.51 g, crude product from the previous step) in *N,N*-dimethylformamide (20 mL), and then the system was heated and stirred at 65°C overnight. The reaction solution was concentrated to remove the solvent, and water (15 mL) was added. The solution was extracted with dichloromethane (15 mL × 3). The organic phases were washed with brine (20 mL). The combined organic phases were dried over anhydrous sodium sulfate, filtered, concentrated under reduced pressure, and purified by silica gel column chromatography (dichloromethane/methanol = 20/1 to 10/1) to obtain compound Int-E-4 (1.32 g, two-step yield: 35.8%).

MS m/z (ESI): 606.0 [M+2H] /2⁺.

¹H NMR (400 MHz, CDCl₃): *δ* 4.20 (d, 2H), 3.71-3.59 (m, 16H), 3.51-3.49 (m, 29H), 3.37 (s, 8H), 2.90 (s, 12H), 2.76 (s, 12H), 1.45 (s, 36H).

### Step 4:

A solution of hydrogen chloride in dioxane (4 M, 6 mL) was added to a solution of Int-E-4 (250 mg, 0.21 mmol) in dichloromethane (3.0 mL) at 0°C. The reaction solution was then stirred at room temperature for 2 hours and concentrated to obtain compound Int-E-5 (220 mg, crude product), which was directly used in the next reaction.

### Step 5:

Compound Int-A-7 (619 mg, 0.82 mmol) and *N,N*-diisopropylethylamine (266 mg, 2.06 mmol) were added to a solution of Int-E-5 (220 mg, crude product from the previous step) in *N,N*-dimethylformamide (10 mL), and then the mixture was stirred at room temperature for 12 hours. The reaction solution was concentrated at room temperature to remove *N,N*-dimethylformamide, and purified by reverse phase preparative chromatography to obtain compound Int-E-6 (270 mg, two-step yield: 40.1%).

MS m/z (ESI): 816.0 [M+4H] /4⁺.

¹H NMR (400 MHz, CDCl₃): *δ* 11.39 (s, 4H), 8.41 (d, 4H), 6.23-6.11 (m, 5H), 5.89 (s, 4H), 5.27-5.10 (m, 8H), 4.38-4.36 (m, 8H), 4.20 (d, 2H), 4.17-3.96 (m, 12H), 3.79 (s, 12H), 3.72-3.61 (m, 12H), 3.59-3.53 (m, 14H), 3.49-3.47 (m, 20H), 3.23 (s, 4H), 2.99 (s, 6H), 2.94 (s, 4H), 2.76-2.73 (m, 16H), 2.47 (t, 1H), 1.89 (s, 12H), 1.48 (d, 72H), 1.40-1.32 (m, 23H).

### Step 6:

An aqueous solution (12.3 mL) of lithium hydroxide monohydrate (26 mg, 0.62 mmol) was added to a solution of Int-E-6 (200 mg, 61.34 µmol) in methanol (12.3 mL) at 0°C, and then stirred at 20°C for 12 hours. After the reaction was completed, the reaction solution was adjusted to pH=7 using IRN77 ion exchange resin. The mother liquor after filtration was concentrated to obtain compound Int-E-7 (196 mg, crude product), which was directly used in the next reaction.

MS m/z (ESI): 802.0 [M+4H]/4⁺.

### Step 7:

Trifluoroacetic acid (10 mL) was added to a solution of compound Int-E-7 (196 mg, crude product from the previous step) in dichloromethane (5 mL) at 0°C. The reaction solution was then stirred at 20°C for 2 hours. After concentration, water (10 mL) was added, and the mixture was stirred for 2 hours. The mixture was concentrated under reduced pressure, and purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-E trifluoroacetate (50 mg, two-step yield: 26.9%).

MS m/z (ESI): 1121.8 [M+2H]/2⁺.

¹H NMR (400 MHz, D₂O): *δ* 5.99 (s, 4H), 4.86 (t, 4H), 4.38(t, 4H), 4.26 (d, 4H), 4.15 (s, 2H), 4.05-3.92 (m, 8H), 3.69-3.42 (m, 53H), 3.18-2.94 (m, 4H), 2.79-2.54 (m, 28H), 1.75 (s, 12H).

### Example 6 Synthesis of Intermediate Int-F

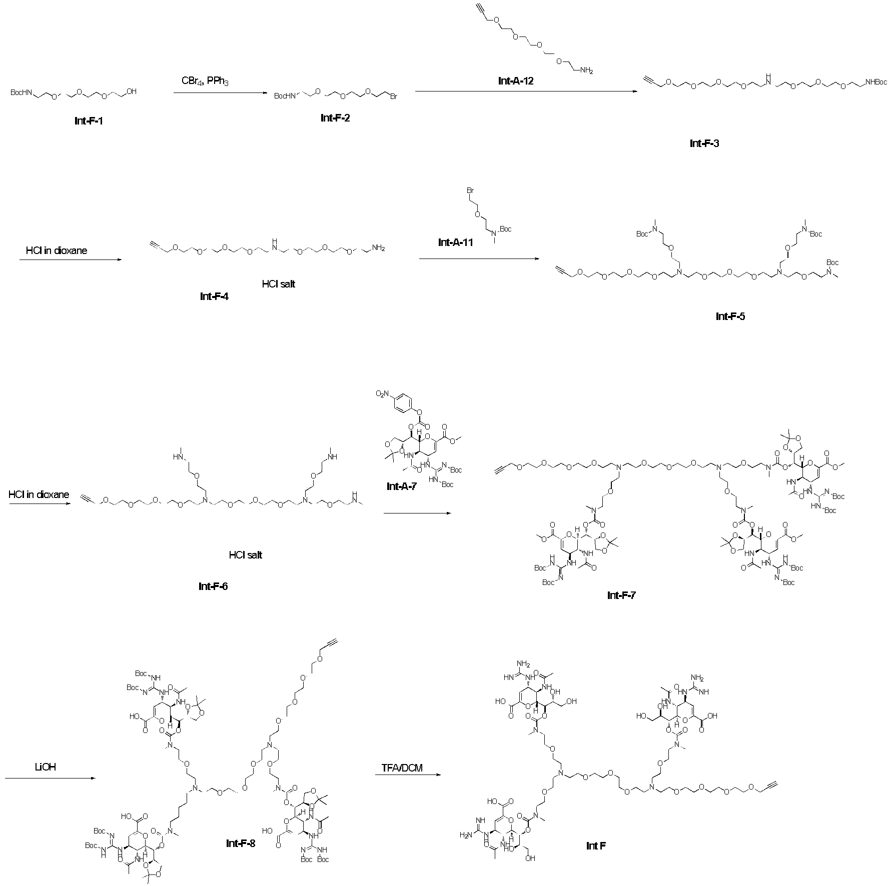

### Step 1:

Triphenylphosphine (3.35 g, 12.77 mmol) was added to a solution of compound Int-F-1 (2.50 g, 8.52 mmol) in dichloromethane (40 mL) at 0°C. After continuing stirring for 15 minutes, carbon tetrabromide (4.23 g, 12.76 mmol) was added in batches. After completion of the addition, the mixture was stirred at room temperature for 4 hours. The reaction solution was directly concentrated under reduced pressure, and purified by silica gel column chromatography (petroleum ether/ethyl acetate = 10/1 to 2/1) to obtain the product Int-F-2 (2.10 g, yield 69.2%).

¹H NMR (400 MHz, CDCl₃): *δ* 5.05 (br, 1H), 3.82 (t, 2H), 3.71-3.61 (m, 8H), 3.55 (t, 2H), 3.48 (t, 2H), 3.36-3.27 (m, 2H), 1.45 (s, 9H).

### Step 2:

Compound Int-A-12 (1.29 g, 5.58 mmol) and potassium carbonate (1.56 g, 11.29 mmol) were added to a solution of compound Int-F-2 (2.00 g, 5.61 mmol) in *N,N*-dimethylformamide (20 mL), and then heated and stirred at 55°C for 6 hours. The reaction solution was concentrated to remove *N,N*-dimethylformamide, and water (10 mL) was added. The solution was extracted with dichloromethane (10 mL × 3). The organic phases were combined, washed with a saturated sodium chloride solution (20 mL), dried over anhydrous sodium sulfate, concentrated under reduced pressure, and purified by silica gel column chromatography (dichloromethane/methanol = 30/1 to 10/1) to obtain compound Int-F-3 (1.38 g, yield: 48.6%).

¹H NMR (400 MHz, CDCl₃): *δ* 5.26 (s, 1H), 4.21 (d, 2H), 3.73-3.57 (m, 23H), 3.54 (t, 2H), 3.31 (d, 2H), 2.85 (t, 4H), 2.44 (s, 1H), 1.44 (s, 9H).

### Step 3:

A solution of hydrogen chloride in dioxane (4 M, 10 mL) was added to a solution of compound Int-F-3 (1.28 g, 2.53 mmol) in dichloromethane (4.0 mL) at 0°C. The system was then stirred at 20°C for 2 hours and concentrated to obtain compound Int-F-4 hydrochloride (1.21 g, crude product), which was directly used in the next reaction.

¹H NMR (400 MHz, DMSO): *δ* 8.74 (br, 2H), 7.93 (br, 3H), 4.15 (d, 2H), 3.70 (t, 4H), 3.55 (d, 24H), 3.46 (t, 1H), 3.15 (dd, 4H), 2.97 (dd, 2H).

### Step 4:

Compound Int-A-11 (2.81 g, 9.96 mmol) and potassium carbonate (2.43 g, 17.58 mmol) were added to a solution of compound Int-F-4 hydrochloride (1.21 g, crude product from the previous step) in *N,N*-dimethylformamide (15 mL), and then heated and stirred at 65°C overnight. The mixture was concentrated to remove *N,N*-dimethylformamide, and water (15 mL) was added. The mixture was extracted with dichloromethane (15 mL × 3). The organic phases were combined, washed with a saturated sodium chloride solution (20 mL), dried over anhydrous sodium sulfate, concentrated under reduced pressure, and purified by silica gel column chromatography (dichloromethane/methanol = 20/1 to 10/1) to obtain compound Int-F-5 (1.06 g, yield: 41.5%).

¹H NMR (400 MHz, CDCl₃): *δ* 4.21 (d, 2H), 3.72-3.58 (m, 24H), 3.53-3.37 (m, 22H), 2.90 (s, 9H), 2.82-2.73 (m, 10H), 2.44 (s, 1H), 1.45 (s, 27H).

### Step 5:

Compound Int-F-5 (250 mg, 0.25 mmol) was dissolved in dichloromethane (4 mL), and a solution of hydrogen chloride in dioxane (4 M, 4 mL) was added at 0°C. The reaction system was stirred at 0°C for 2 hours. After the reaction was completed, the reaction solution was concentrated to obtain compound Int-F-6 (220 mg, crude product), which was directly used in the next reaction.

MS m/z (ESI): 710.5 [M+H] ⁺.

### Step 6:

The crude compound Int-F-6 hydrochloride (220 mg, crude product from the previous step) was dissolved in dry N,N-dimethylformamide (4 mL), and *N,N*-diisopropylethylamine (682 mg, 5.28 mmol) was added at 0°C, followed by the addition of compound Int-A-7 (660 mg, 0.88 mmol). The reaction was stirred at room temperature for 16 hours. After the reaction was completed, ethyl acetate (30 mL) was added, followed by sequential washing with water (15 mL) and a saturated sodium chloride solution (15 mL). The organic phase was dried over anhydrous sodium sulfate, filtered, and concentrated to obtain a crude product. The crude product was purified by a rapid preparative instrument with an elution system (dichloromethane/methanol = 0 to 10%) to obtain compound Int-F-7 (220 mg, yield: 34.4%).

¹H NMR (400 MHz, CD₃OD): *δ* 5.97 (s, 3H), 5.37-5.33 (m, 3H), 4.99 (d, 3H), 4.49-4.42 (m, 3H), 4.37 (d, 3H), 4.21 (d, 8H), 4.08 (d, 3H), 3.83 (s, 9H), 3.73-3.57 (m, 40H), 3.39-3.23 (m, 5H), 3.19-3.12 (m, 3H), 3.08-2.91 (m, 17H), 1.95-1.91 (m, 10H), 1.53 (s, 27H), 1.48 (s, 27H), 1.39-1.34 (m, 18H).

### Step 7:

Compound Int-F-7 (220 mg, 86.35 µmol) was dissolved in methanol (10 mL), and an aqueous solution (10 mL) of lithium hydroxide monohydrate (22 mg, 0.52 mmol) was added at 0°C. The reaction was stirred at room temperature for 16 hours. After the reaction was completed, IRN 77 resin was added for neutralization. The neutralized reaction solution was filtered and concentrated to obtain compound Int-F-8 (190 mg, crude product), which was directly used in the next reaction.

MS m/z (ESI): 835.3 ([M+3H]/3)⁺.

### Step 8:

Compound Int-F-8 (190 mg, crude product from the previous step) was dissolved in dichloromethane (2 mL), and trifluoroacetic acid (4 mL) was added. The reaction was stirred at room temperature for 2 hours. After the reaction was completed, the reaction solution was concentrated under reduced pressure, and then water (4 mL) was added, followed by stirring at room temperature for 1 hour. After the reaction solution was concentrated, the resulting crude product was purified by high-performance liquid chromatography (chromatographic column: SharpSil-T, 30*150 mm, 5 µm; mobile phase: aqueous phase (10 mM ammonium bicarbonate) and acetonitrile, gradient ratio: aqueous phase 25% to 42%) to obtain the title product Int-F (53 mg, yield: 29.6%).

MS m/z (ESI): 893.2 ([M+2H]/2)⁺.

¹H NMR (400 MHz, CD₃OD): *δ* 5.92 (d, 3H), 5.03 (dd, 3H), 4.57 (dd, 3H), 4.44 (d, 3H), 4.31-4.19 (m, 5H), 4.09-3.99 (m, 4H), 3.95-3.82 (m, 12H), 3.82-3.46 (m, 46H), 3.30-3.21 (m, 3H), 2.98-2.93 (m, 10H), 1.97 (s, 9H).

### Example 7 Synthesis of Intermediate Int-G

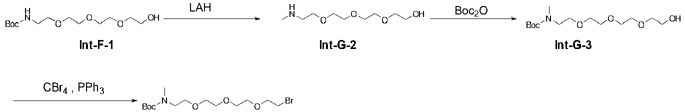

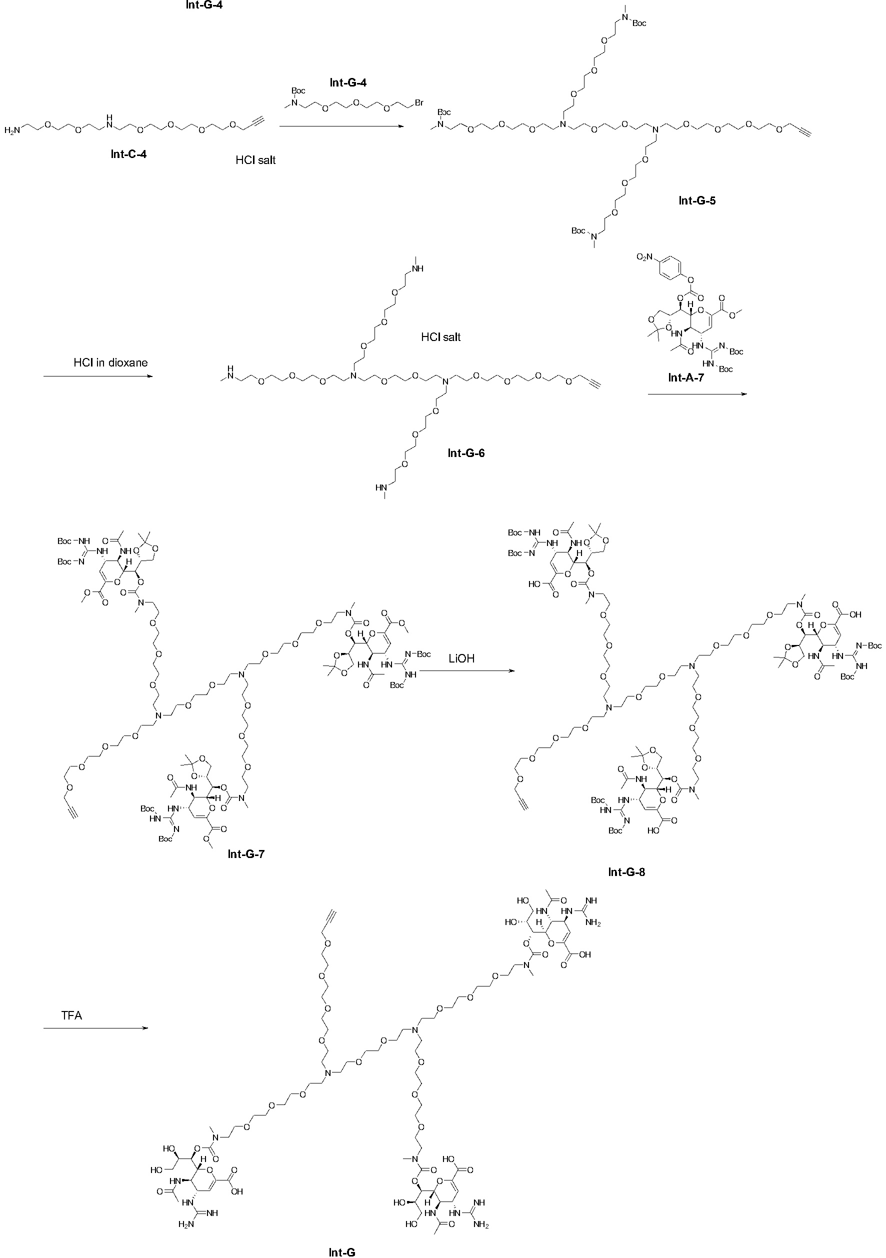

### Step 1:

A solution of lithium aluminum hydride in tetrahydrofuran (2.5 M, 28.6 mL, 71.5 mmol) was added dropwise to a solution of compound Int-F-1 (7.0 g, 23.86 mmol) in tetrahydrofuran (70 mL) at 0°C. After completion of the addition, the mixture was heated to 70°C and stirred for 4 hours. After the reaction was completed, the cooled reaction solution was slowly poured into a system of tetrahydrofuran (100 mL) containing sodium sulfate decahydrate (40 g) at 0°C, and stirred at room temperature for 1 hour. The solid was removed by filtration, and the mother liquor was directly concentrated under reduced pressure to obtain compound Int-G-2 (4.94 g, crude product), which was directly used in the next reaction.

MS m/z (ESI): 208.1 [M+H] ⁺.

¹H NMR (400 MHz, CDCl₃): *δ* 3.73-3.71 (m, 2H), 3.66-3.64 (m, 8H), 3.62-3.58 (m, 4H), 2.77-2.73 (t, 2H), 2.42 (s, 3H).

### Step 2:

Di-tert-butyl dicarbonate (6.31 g, 28.9 mmol) was added to a solution of Int-G-2 (4.94 g, crude product from the previous step) in dichloromethane (60 mL) at 0°C, and the mixture was stirred at room temperature overnight. The reaction solution was concentrated, and purified by silica gel column chromatography (dichloromethane/ethyl acetate = 10/1 to 2/1) to obtain compound Int-G-3 (4.17 g, two-step yield: 56.9%).

MS m/z (ESI): 330.2 [M+Na] ⁺.

¹H NMR (400 MHz, CDCl₃): *δ* 3.76-3.70 (m, 2H), 3.70-3.54 (m, 12H), 3.42-3.35 (m, 2H), 2.91 (s, 3H), 1.45 (s, 9H).

### Step 3:

Triphenylphosphine (5.11 g, 19.48 mmol) was added to a solution of Int-G-3 (4.0 g, 13.01 mmol) in dichloromethane (60 mL) at 0°C. After continuing stirring for 10 minutes, carbon tetrabromide (6.47 g, 19.51 mmol) was added in batches. After completion of the addition, the mixture was stirred at room temperature for 1 hour. The reaction solution was directly concentrated under reduced pressure, and purified by silica gel column chromatography (petroleum ether/ethyl acetate = 10/1 to 2/1) to obtain compound Int-G-4 (3.9 g, yield 81.0%).

¹H NMR (400 MHz, CDCl₃): *δ* 3.81 (t, 2H), 3.71-3.55 (m, 10H), 3.47 (t, 2H), 3.39 (s, 2H), 3.44-3.37 (m, 3H), 1.45 (s, 9H).

### Step 4:

Compound Int-G-4 (3.22 g, 8.70 mmol) and potassium carbonate (1.71 g, 12.37 mmol) were added to a solution of compound Int-C-4 (1.08 g, 2.48 mmol) in *N,*N-dimethylformamide (15 mL), and then heated and stirred at 65°C overnight. The reaction solution was concentrated to remove *N,N*-dimethylformamide, and water (15 mL) was added. The solution was extracted with dichloromethane (15 mL × 3). The organic phase was washed with a saturated sodium chloride solution (20 mL), dried over anhydrous sodium sulfate, concentrated under reduced pressure, and purified by silica gel column chromatography (dichloromethane/methanol = 20/1 to 10/1) to obtain compound Int-G-5 (1.29 g, yield: 42.3%).

MS m/z (ESI): 616.0 ([M+2H]/2)⁺.

¹H NMR (400 MHz, CDCl₃): *δ* 4.21 (d, 2H), 3.72-3.52 (m, 60H), 3.43-3.35 (m, 8H), 2.95-2.72 (m, 18H), 1.45 (s, 27H).

### Step 5:

A solution of hydrogen chloride in dioxane (4 M, 10 mL) was added to a solution of compound Int-G-5 (400 mg, 0.33 mmol) in dichloromethane (5.0 mL) at 0°C. The reaction solution was then stirred at room temperature for 2 hours and concentrated to obtain compound Int-G-6 (360 mg, crude product), which was directly used in the next reaction.

MS m/z (ESI): 465.9 ([M+2H]/2)⁺.

### Step 6:

Compound Int-A-7 (780 mg, 1.04 mmol) and *N,N*-diisopropylethylamine (418 mg, 3.23 mmol) were added to a solution of compound Int-G-6 (360 mg, crude product from the previous step) in *N,N*-dimethylformamide (10 mL), and then the mixture was stirred at room temperature for 12 hours. The reaction solution was concentrated at room temperature to remove *N,N*-dimethylformamide, and purified by reverse phase preparative chromatography to obtain compound Int-G-7 (500 mg, two-step yield: 55.6%).

MS m/z (ESI): 692.8 ([M+4H]/4)⁺.

¹H NMR (400 MHz, CDCl₃): *δ* 11.40 (s, 3H), 8.41 (d, 3H), 6.15-6.09 (m, 3H), 5.88 (s, 3H), 5.26-5.21 (m, 6H), 4.43-4.34 (m, 6H), 4.20 (d, 2H), 4.17-3.99 (m, 10H), 3.79 (s, 8H), 3.70-3.68 (m, 4H), 3.70-3.68 (m, 46H), 3.55-3.48 (m, 14H), 3.41-3.28 (m, 4H), 3.00 (s, 4H), 2.94 (s, 4H), 2.76 (s, 10H), 1.89 (d, 8H), 1.48 (d, 54H), 1.38-1.33 (m, 19H).

### Step 7:

An aqueous solution (14.4 mL) of lithium hydroxide monohydrate (30 mg, 0.71 mmol) was added to a solution of compound Int-G-7 (200 mg, 72.25 µmol) in methanol (14.4 mL) at 0°C, and then stirred at 20°C for 12 hours. After the reaction was completed, the reaction solution was adjusted to pH=7 using IRN77 ion exchange resin. The mother liquor after filtration was concentrated to obtain compound Int-G-8 (197 mg, crude product), which was directly used in the next reaction.

MS m/z (ESI): 682.3 ([M+4H]/4)⁺.

### Step 8:

Trifluoroacetic acid (10 mL) was added to a solution of compound Int-G-8 (197 mg, crude product from the previous step) in dichloromethane (5.0 mL) at 0°C. The reaction was stirred at room temperature for 2 hours. After the reaction was completed, the reaction solution was concentrated under reduced pressure, and then water (4 mL) was added, followed by stirring at room temperature for 1 hour. The reaction solution was concentrated, the resulting crude product was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-G trifluoroacetate (25.0 mg, two-step yield: 13.4%).

¹H NMR (400 MHz, D₂O): *δ* 5.59-5.57 (m, 3H), 4.93-4.84 (m, 3H), 4.43 (dd, 3H), 4.32 (dd, 3H), 4.16 (s, 2H), 4.09-4.00 (m, 6H), 3.61-3.58 (m, 64H), 3.41 (dd, 4H), 3.31-3.28 (m, 2H), 3.17-3.12 (m, 2H), 2.89 (s, 4H), 2.81-2.77 (d, 16H), 1.88 (s, 9H).

### Example 8 Synthesis of Intermediate Int-H

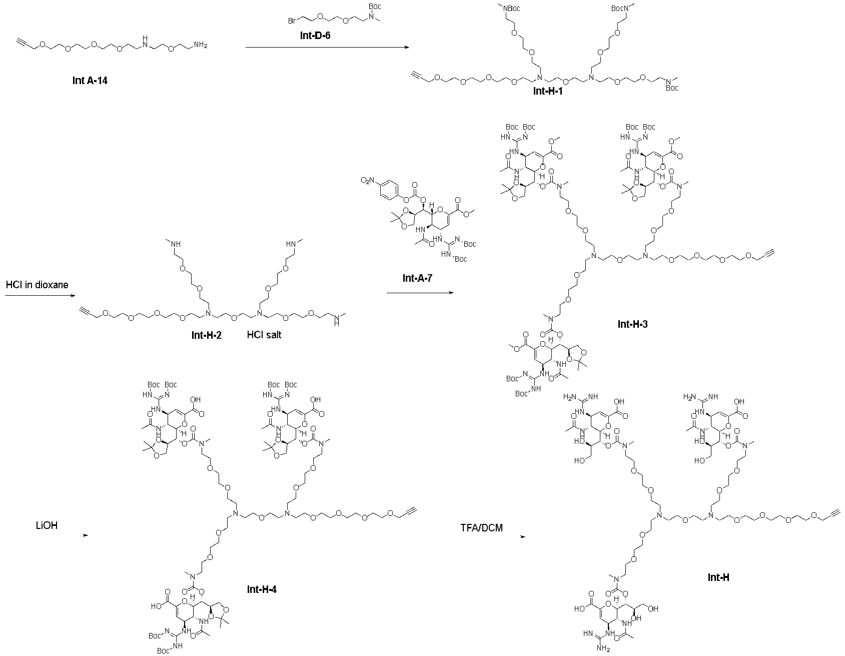

### Step 1:

Compound Int-A-14 hydrochloride (572 mg, 1.46 mmol) was dissolved in dry N,N-dimethylformamide (10 mL), and potassium carbonate (2.50 g, 18.09 mmol) and compound Int-D-6 (2.30 g, 6.76 mmol) were added sequentially. The reaction was stirred at 65°C for 16 hours. After the reaction was completed, the reaction solution was concentrated. Water (30 mL) and dichloromethane (30 mL) were added, and the aqueous phase was extracted with dichloromethane (30 mL × 2). The combined organic phases were dried over anhydrous sodium sulfate, filtered, and concentrated to obtain a crude product. The crude product was purified by a rapid preparative instrument with an elution system (dichloromethane/methanol = 0 to 10%) to obtain compound Int-H-1 (350 mg, yield: 22.7%).

MS m/z (ESI): 1054.7 [M+H]⁺.

¹H NMR (400 MHz, CDCl₃): *δ* 4.21 (d, 3H), 3.72-3.49 (m, 45H), 3.42-3.32 (m, 7H), 2.91 (s, 9H), 2.81-2.73 (m, 7H), 2.45 (s, 1H), 1.45 (s, 27H).

### Step 2:

Compound Int-H-1 (250 mg, 0.24 mmol) was dissolved in dichloromethane (8 mL), and a solution of hydrogen chloride in dioxane (4 M, 4 mL) was added at 0°C. The reaction was stirred at 0°C for 2 hours. After the reaction was completed, the reaction solution was concentrated to obtain compound Int-H-2 (222 mg, crude product), which was directly used in the next reaction.

MS m/z (ESI): 754.5 [M+H] ⁺.

### Step 3:

Compound Int-H-2 (222 mg, crude product) was dissolved in dry *N,N*-dimethylformamide (5 mL). *N,N*-diisopropylethylamine (464 mg, 3.59 mmol) was added at 0°C, followed by the addition of compound Int-A-7 (600 mg, 0.80 mmol). The reaction was stirred at room temperature for 16 hours. After the reaction was completed, ethyl acetate (30 mL) was added, followed by sequential washing with water (15 mL) and a saturated sodium chloride solution (15 mL). The organic phase was dried over anhydrous sodium sulfate, filtered, and concentrated to obtain a crude product. The crude product was purified by a rapid preparative instrument with an elution system (dichloromethane/methanol = 20:1 to 10:1) to obtain compound Int-H-3 (230 mg, two-step yield: 37.0%).

MS m/z (ESI): 648.9 ([M+4H]/4)⁺.

### Step 4:

Compound Int-H-3 (230 mg, 88.74 µmol) was dissolved in methanol (14 mL), and an aqueous solution (14 mL) of lithium hydroxide monohydrate (30 mg, 0.71 mmol) was added at 0°C. The reaction was stirred at room temperature for 16 hours. After the reaction was completed, IRN 77 resin was added for neutralization. The neutralized reaction solution was filtered and concentrated to obtain compound Int-H-4 (226 mg, crude product), which was directly used in the next reaction.

MS m/z (ESI): 850.7 ([M+3H]/3)⁺.

### Step 5:

The crude compound Int-H-4 (226 mg, crude product from the previous step) was dissolved in dichloromethane (2 mL), and trifluoroacetic acid (4 mL) was added. The reaction was stirred at room temperature for 2 hours. After the reaction was completed, it was concentrated under reduced pressure, and then water (4 mL) was added, followed by stirring at room temperature for 1 hour. After the reaction solution was concentrated, the resulting crude product was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-H trifluoroacetate (86.8 mg, two-step yield: 40.7%).

MS m/z (ESI): 610.5 ([M+3H]/3)⁺.

¹H NMR (400 MHz, CD₃OD): *δ* 5.91 (dd, 3H), 5.06-4.99 (m, 3H), 4.57 (dd, 3H), 4.44 (d, 3H), 4.28-4.18 (m, 5H), 4.12-3.99 (m, 4H), 3.99-3.79 (m, 14H), 3.79-3.47 (m, 48H), 3.23-3.14 (m, 3H), 2.97 (d, 10H), 1.97 (d, 9H).

### Example 9 Synthesis of Intermediate Int-I

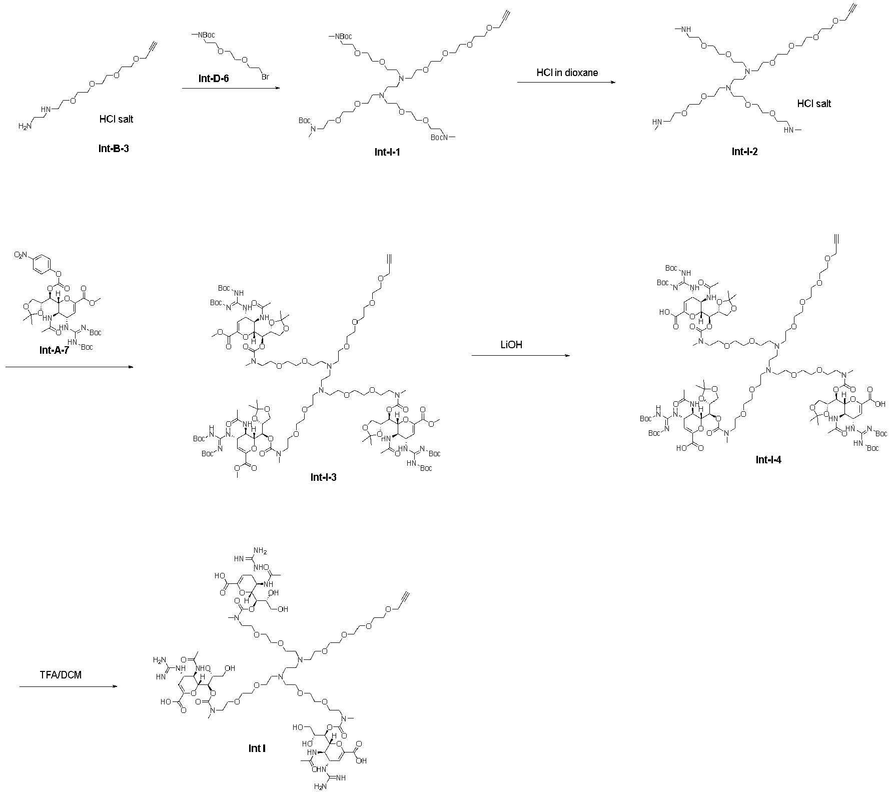

### Step 1:

Compound Int-B-3 (900 mg, 2.59 mmol) was dissolved in dry *N,N*-dimethylformamide (10 mL), and potassium carbonate (2.6 g, 18.81 mmol) and compound Int-D-6 (2.50 g, 7.66 mmol) were added sequentially. The reaction was stirred at 65°C for 16 hours. After the reaction was completed, the reaction solution was concentrated. Water (30 mL) and dichloromethane (30 mL) were added, and the aqueous phase was extracted with dichloromethane (30 mL × 2). The combined organic phases were dried over anhydrous sodium sulfate, filtered, and concentrated to obtain a crude product. The crude product was purified by a rapid preparative instrument with an elution system (dichloromethane/methanol = 20/1 to 10/1) to obtain compound Int-I-1 (300 mg, yield: 11.5%).

MS m/z (ESI): 1032.7 [M+Na] ⁺.

¹H NMR (400 MHz, CDCl₃): *δ* 4.20 (d, 2H), 3.86-3.51 (m, 40H), 3.42-3.16 (m, 16H), 2.89 (d, 9H), 2.47 (t, 1H), 1.45 (s, 27H).

### Step 2:

Compound Int-I-1 (300 mg, 0.30 mmol) was dissolved in dichloromethane (8 mL), and a solution of hydrogen chloride in dioxane (4 M, 4 mL) was added at 0°C. The reaction was stirred at 0°C for 2 hours. After the reaction was completed, it was concentrated to obtain compound Int-I-2 hydrochloride (265 mg, crude product), which was directly used in the next reaction.

MS m/z (ESI): 710.5 [M+H]⁺.

### Step 3:

Compound Int-I-2 (265 mg, crude product) was dissolved in dry *N,N*-dimethylformamide (8 mL), *N,N*-diisopropylethylamine (374 mg, 2.89 mmol) was added at 0°C, followed by the addition of compound Int-A-7 (750 mg, 1 mmol). The reaction was stirred at room temperature for 16 hours. After the reaction was completed, ethyl acetate (50 mL) was added, followed by sequential washing with water (30 mL) and a saturated sodium chloride solution (30 mL). The organic phase was dried over anhydrous sodium sulfate, filtered, and concentrated to obtain a crude product. The crude product was purified by a rapid preparative instrument with an elution system (dichloromethane/methanol = 0 to 10%) to obtain compound Int-I-3 (330 mg, two-step yield: 43.2%).

¹H NMR (400 MHz, CDCl3): *δ* 11.41 (s, 3H), 8.41 (d, 3H), 5.95-5.81 (m, 6H), 5.30-5.21 (m, 6H), 4.43-4.34 (m, 6H), 4.20 (d, 2H), 4.10-3.95 (m, 6H), 3.79 (s, 9H), 3.72-3.49 (m, 42H), 3.40-3.10 (m, 8H), 2.97 (d, 9H), 2.78-2.60 (m, 9H), 2.46 (s, 1H), 1.90 (d, 9H), 1.48 (d, 54H), 1.39-1.33 (m, 18H).

### Step 4:

Compound Int-I-3 (330 mg, 0.13 mmol) was dissolved in methanol (20 mL), and an aqueous solution (20 mL) of lithium hydroxide monohydrate (42 mg, 1.00 mmol) was added at 0°C. The reaction was stirred at room temperature for 16 hours. After the reaction was completed, IRN 77 resin was added for neutralization. The neutralized reaction solution was filtered and concentrated to obtain compound Int-I-4 (285 mg, crude product), which was directly used in the next reaction.

MS m/z (ESI): 836.1 ([M+3H]/3) ⁺.

### Step 5:

Compound Int-I-4 (285 mg, crude product) was dissolved in dichloromethane (3 mL), and trifluoroacetic acid (6 mL) was added. The reaction was stirred at room temperature for 2 hours. After the reaction was completed, the reaction solution was concentrated under reduced pressure, and then water (4 mL) was added, followed by stirring at room temperature for 1 hour. After the reaction solution was concentrated, the resulting crude product was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-I trifluoroacetate (154.0 mg, two-step yield: 50.3%).

¹H NMR (400 MHz, CD₃OD): *δ* 5.94 (d, 3H), 5.08-4.99 (m, 3H), 4.57 (d, 3H), 4.44 (d, 3H), 4.30-4.19 (m, 5H), 4.08-4.00 (m, 4H), 3.92-3.77 (m, 10H), 3.73-3.46 (m, 48H), 3.24-3.14 (m, 3H), 2.98 (d, 10H), 1.97 (d, 9H).

### Example 10 Synthesis of Intermediate Int-J

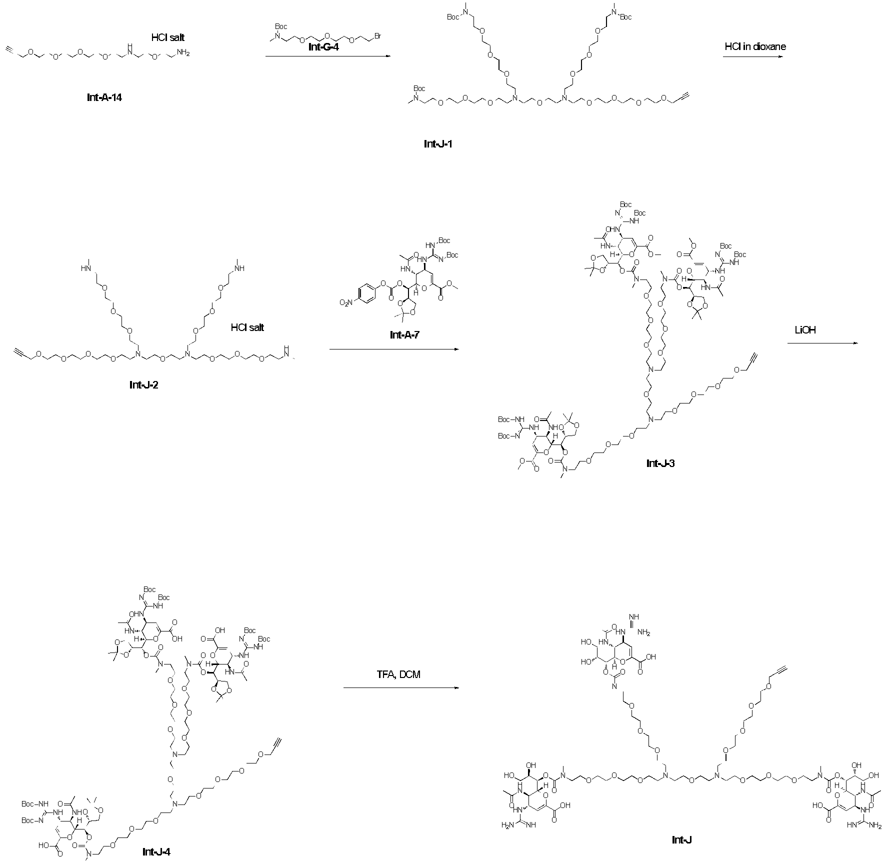

### Step 1:

Compound Int-G-4 (3.35 g, 9.05 mmol) and potassium carbonate (3.89 g, 28.15 mmol) were added to a solution of compound Int-A-14 (900 mg, 2.30 mmol) in *N,N*-dimethylformamide (15 mL), and then heated and stirred at 65°C overnight. The reaction solution was concentrated to remove *N,N*-dimethylformamide, and water (15 mL) was added. The solution was extracted with dichloromethane (15 mL × 3). The organic phases were combined, washed with a saturated sodium chloride solution (20 mL), dried over anhydrous sodium sulfate, concentrated under reduced pressure, and purified by silica gel column chromatography (dichloromethane/methanol = 20/1 to 10/1) to obtain compound Int-J-1 (1.20 g, yield: 44.0%).

MS m/z (ESI): 594.0 ([M+2H]/2)⁺.

¹H NMR (400 MHz, CDCl₃): *δ* 4.20 (d, 2H), 3.76-3.51 (m, 54H), 3.43-3.32 (m, 6H), 2.91 (s, 9H), 2.90-2.78 (m, 9H), 2.29 (s, 4H), 1.45 (s, 27H).

### Step 2:

A solution of hydrogen chloride in dioxane (4 M, 10 mL) was added to a solution of compound Int-J-1 (400 mg, 0.34 mmol) in dichloromethane (10 mL) at 0°C. The reaction solution was then stirred at room temperature for 2 hours and concentrated to obtain compound Int-J-2 (360 mg, crude product), which was directly used in the next reaction.

MS m/z (ESI): 908.8[M+Na] ⁺.

### Step 3:

Int-A-7 (809 mg, 1.08 mmol) and *N,N*-diisopropylethylamine (433 mg, 3.35 mmol) were added to a solution of compound Int-J-2 (360 mg, crude product from the previous step) in *N,N*-dimethylformamide (10 mL), and then the mixture was stirred at room temperature for 12 hours. The reaction solution was concentrated at room temperature to remove *N,N*-dimethylformamide, and purified by reverse phase preparative chromatography to obtain compound Int-J-3 (640 mg, two-step yield: 69.1%).

MS m/z (ESI): 681.9 ([M+4H] /4)⁺.

### Step 4:

A solution (44 mL) of lithium hydroxide (92.4 mg, 2.20 mmol) was added to a solution of compound Int-J-3 (600 mg, 0.22 mmol) in methanol (44 mL) at 0°C, and then stirred at 20°C for 12 hours. After the reaction was completed, the reaction solution was adjusted to pH=7 using IRN77 ion exchange resin. The mother liquor after filtration was concentrated to obtain compound Int-J-4 (590 mg, crude product), which was directly used in the next reaction.

MS m/z (ESI): 671.0 ([M+4H] /4)⁺.

### Step 5:

Trifluoroacetic acid (5 mL) was added to a solution of compound Int-J-4 (590 mg, crude product from the previous step) in dichloromethane (5 mL) at 0°C. The reaction solution was then stirred at 20°C for 2 hours. After concentration, water (10 mL) was added, and the solution was stirred for 2 hours. The solution was concentrated under reduced pressure, the resulting crude product was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-J trifluoroacetate (140 mg, two-step yield: 25.1%).

¹H NMR (400 MHz, D₂O): *δ* 5.52(d, 3H), 4.85-4.75 (m, 3H), 4.37 (dd, 3H), 4.26 (dd, 3H), 4.09 (s,2H), 4.02-3.94 (m, 6H), 3.52 (br, 62H), 3.35 (dd, 3H), 3.31-2.97 (m, 4H), 2.88 (s, 4H), 2.75-2.71 (m, 15H), 1.81 (s, 9H).

### Example 11 Synthesis of Intermediate Int-K

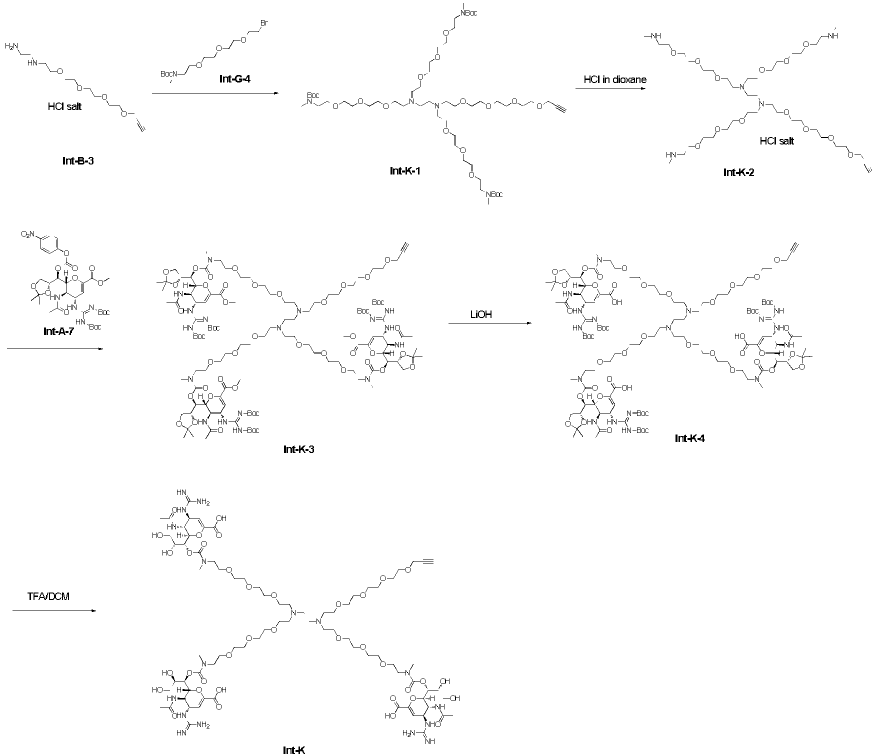

### Step 1:

Compound Int-B-3 (586 mg, 1.69 mmol) was dissolved in dry *N,N*-dimethylformamide (10 mL), and potassium carbonate (3.0 g, 21.71 mmol) and compound Int-G-4 (3.20 g, 8.64 mmol) were added sequentially. The reaction was stirred at 65°C for 16 hours. After the reaction was completed, the reaction solution was concentrated. Water (30 mL) and dichloromethane (30 mL) were added, and the aqueous phase was extracted with dichloromethane (30 mL × 2). The combined organic phases were dried over anhydrous sodium sulfate, filtered, and concentrated to obtain a crude product. The crude product was purified by a rapid preparative instrument with an elution system (dichloromethane/methanol = 0 to 10%) to obtain compound Int-K-1 (800 mg, yield: 41.4%).

MS m/z (ESI): 1165.7 [M+Na]⁺.

### Step 2:

Compound Int-K-1 (300 mg, 0.26 mmol) was dissolved in dichloromethane (8 mL), and a solution of hydrogen chloride in dioxane (4 M, 4 mL) was added at 0°C. The reaction was stirred at 0°C for 2 hours. After the reaction was completed, the reaction solution was concentrated to obtain compound Int-K-2 (269 mg, crude product), which was directly used in the next reaction.

MS m/z (ESI): 421.9 ([M+2H]/2) ⁺.

### Step 3:

Compound Int-K-2 (269 mg, crude product from the previous step) was dissolved in dry *N,N*-dimethylformamide (6 mL). *N,N*-diisopropylethylamine (373 mg, 2.89 mmol) was added under an ice bath, followed by the addition of compound Int-A-7 (650 mg, 0.86 mmol). The reaction was stirred at room temperature overnight. After the reaction was completed, ethyl acetate (30 mL) was added, followed by sequential washing with water (15 mL) and a saturated sodium chloride solution (15 mL). The organic phase was dried over anhydrous sodium sulfate, filtered, and concentrated to obtain a crude product. The crude product was purified by a rapid preparative instrument with an elution system (dichloromethane/methanol = 0 to 10%) to obtain compound Int-K-3 (350 mg, two-step yield: 50.2%).

MS m/z (ESI): 894.1 ([M+3H]/3)⁺.

¹H NMR (400 MHz, CDCl3): *δ* 11.39 (s, 3H), 8.41 (d, 3H), 6.36-6.12 (m, 3H), 5.90 (dd, 3H), 5.36-5.16 (m, 6H), 4.43-4.35 (m, 6H), 4.23-3.95 (m, 14H), 3.80-3.58 (m, 62H), 3.41-3.17 (m, 12H), 3.01-2.98 (m, 9H), 2.50 (t, 1H), 1.89 (s, 9H), 1.48 (d, 54H), 1.38-1.33 (m, 18H).

### Step 4:

Compound Int-K-3 (350 mg, 0.13 mmol) was dissolved in methanol (22 mL), and an aqueous solution (22 mL) of lithium hydroxide monohydrate (45 mg, 1.07 mmol) was added under an ice bath. The reaction was stirred at room temperature for 16 hours. After the reaction was completed, IRN 77 resin was added for neutralization. The neutralized reaction solution was filtered and concentrated to obtain compound Int-K-4 (310 mg, crude product), which was directly used in the next reaction.

MS m/z (ESI): 880.2 ([M+3H]/3)⁺.

### Step 5:

Compound Int-K-4 (310 mg, crude product from the previous step) was dissolved in dichloromethane (3 mL), and trifluoroacetic acid (6 mL) was added. The reaction was stirred at room temperature for 2 hours. After the reaction was completed, the reaction solution was concentrated under reduced pressure, and then water (4 mL) was added, followed by stirring at room temperature for 1 hour. The reaction solution was concentrated, the resulting crude product was purified by preparative high-performance liquid chromatography (Waters XBridge, 19*150 mm, 5 µm; mobile phase A: 0.5% aqueous TFA solution, mobile phase B: acetonitrile, gradient ratio: phase B 5% to 95%) to obtain the title product Int-K trifluoroacetate (203 mg, two-step yield: 62.9%).

MS m/z (ESI): 639.8 ([M+3H]/3)⁺.

¹H NMR (400 MHz, CD₃OD): *δ* 5.94 (s, 3H), 5.03 (t, 3H), 4.58 (dd, 3H), 4.42 (dd, 3H), 4.29-4.19 (m, 5H), 4.13-4.01 (m, 3H), 3.90-3.76 (m, 10H), 3.74-3.58 (m, 51H), 3.55-3.44 (m, 10H), 3.26-3.17 (m, 3H), 3.03-2.94 (m, 10H), 1.97 (s, 9H).

### Example 12 Synthesis of Intermediate Int-L

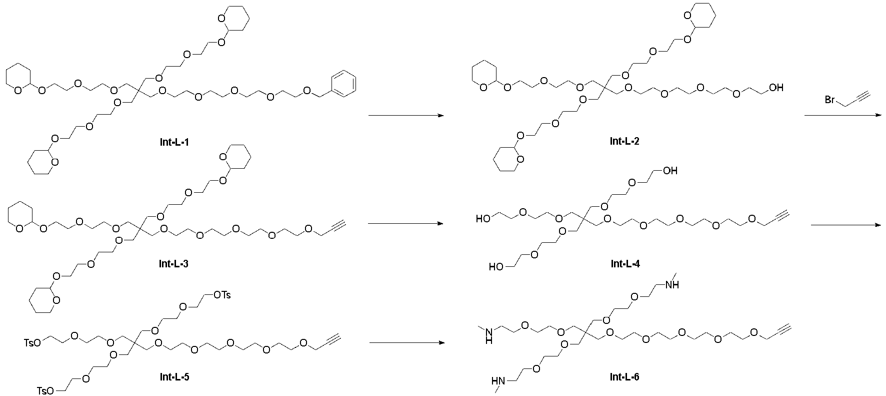

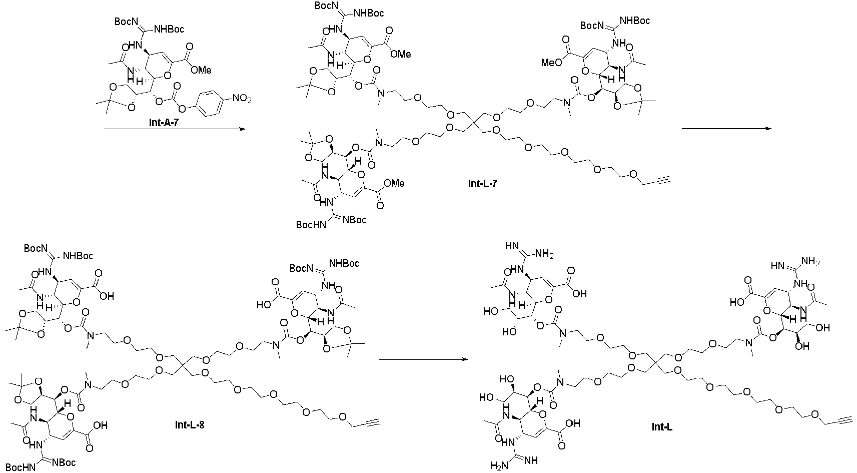

### Step 1: Synthesis of Int-L-2

**Compound Int-L-1** (0.176 g, 0.19 mmol, prepared using a known method, Journal of Organic Chemistry, 2012, vol. 77, # 20, p. 8879 - 8887) was dissolved in THF (10 mL), and 10% Pd (0.124 g) was added. The air in the system was replaced with hydrogen four times, and then the reaction was carried out at room temperature under a hydrogen atmosphere for four hours. After the reaction was completed, the reaction solution was filtered and concentrated to obtain 0.16 g of crude product, which was directly used in the next step.

MS m/z (ESI):830.5 [M+H]⁺.

### Step 2: Synthesis of Compound Int-L-3

**Compound Int-L-2** (0.16 g, 0.19 mmol) was dissolved in dry tetrahydrofuran (4 mL), and a solution of potassium *tert*-butoxide in THF (0.39 mL, 0.394 mmol) was added under cooling in an ice-water bath. After completion of the addition, the reaction was stirred at room temperature for half an hour. The reaction solution was cooled again with an ice-water bath, and propargyl bromide (0.0703 g, 0.591 mmol) was added thereto. After completion of the addition, the reaction naturally warmed to room temperature and was stirred overnight. The reaction solution was filtered, and the filter cake was washed with DCM. The filtrate was concentrated and purified by column chromatography (petroleum ether:ethyl acetate = 1:5) to obtain 0.134 g of **compound Int-L-3** (yield: 81%).

¹H NMR (400 MHz, CDCl₃): *δ* 4.60 (t, 3H), 4.17(d, 2 H), 3.87 ~ 3.44 (m, 46H), 3.40 (s, 8H), 2.42 (t, 1H), 1.86 ~ 1.44 (m, 18H).

### Step 3: Synthesis of Compound Int-L-4

**Compound Int-L-3** (134 mg, 0.154 mmol) was dissolved in anhydrous methanol (5 mL), and *p*-toluenesulfonic acid monohydrate (14 mg, 0.072 mmol) was added. The reaction was stirred at room temperature for 1 hour. After the reaction was completed, sodium bicarbonate (20 mg, 0.238 mmol) was added for neutralization, followed by concentration to obtain 95 mg of crude product, which was directly used in the next step.

MS m/z (ESI):615.4 [M+H]⁺.

### Step 4: Synthesis of Compound Int-L-5

**Compound Int-L-4** (95 mg, 0.154 mmol) was dissolved in DCM (5 mL), and triethylamine (156 mg, 1.542 mmol), *p*-toluenesulfonyl chloride (147 mg, 0.771 mmol), and DMAP (0.9 mg, 0.008 mmol) were added sequentially. The reaction was stirred at room temperature overnight. After the reaction was completed, the reaction was quenched by adding a saturated aqueous sodium bicarbonate solution, and then extracted with DCM. The resulting solution was dried, filtered, and concentrated to obtain 166 mg of crude product, which was directly used in the next step.

MS m/z (ESI):1077.3 [M+H]⁺.

### Step 5: Synthesis of Compound Int-L-6

**Compound Int-L-5** (166 mg, 0.154 mmol) was dissolved in a solution of methylamine in ethanol (5 mL, about 30%), and stirred at room temperature overnight. After the reaction was completed, the mixture was concentrated. The concentrated solution was dissolved with water and DCM, and the organic layer was separated. The aqueous layer was extracted with DCM again. The aqueous phase was concentrated under reduced pressure to obtain 70 mg of **compound Int-L-6** (yield: 70%).

MS m/z (ESI):654.4 [M+H]⁺.

### Step 6: Synthesis of Compound Int-L-7

**Compound Int-A-7** (765 mg, 1.02 mmol) and *N,N*-diisopropylethylamine (571 mg, 4.43 mmol) were added to a solution of **compound Int-L-6** (193 mg, 0.295 mmol) in *N,N-*dimethylformamide (8 mL) and dichloromethane (8 mL), and then heated to 60°C and stirred for 5 hours. The reaction solution was concentrated at room temperature to remove dichloromethane and N,N-dimethylformamide, and purified by column chromatography (acetonitrile:water = 0 to 100%) to obtain 322 mg of **compound Int-L-7** (yield: 44%).

MS m/z (ESI): 831.4 ([M/3+H]⁺.

¹H NMR (400 MHz, CDCl₃): *δ* 11.41 (bs, 2H), 8.75 (bs, 2H), 6.76 (bs, 2H), 5.86 (s, 3H), 5.50-5.22 (m, 6H), 4.43-4.38 (m, 6H), 4.21-3.98 (m, 11H), 3.80 (s, 9H), 3.69-3.23 (m, 48H), 2.99-2.94 (m, 9H), 2.48 (s, 1H), 1.85 (s, 9H), 1.50 (m, 52H), 1.40-1.30 (m, 23H).

### Step 7: Synthesis of Compound Int-L-8

An aqueous solution (4.2 mL) of lithium hydroxide monohydrate (26 mg, 0.627 mmol) was added to a solution of **compound Int-L-7** (260 mg, 0.104 mmol) in methanol (4.2 mL) at 0°C, and then stirred at 20°C for 10 minutes. After the reaction was completed, the reaction solution was adjusted to pH=5 to 6 using IRN77 ion exchange resin. The mother liquor after filtration was concentrated to obtain 250 mg of **compound Int-L-8** (yield: 98%).

MS m/z (ESI): 1225.5 ([M/2+H]⁺.

### Step 8: Synthesis of Compound Int-L

Trifluoroacetic acid (2.1 mL) was added to a solution of **compound Int-L-8** (250 mg, 0.102 mmol) in dichloromethane (2.1 mL) at 0°C. The reaction was stirred at room temperature for 2 hours. After the reaction was completed, the reaction solution was concentrated under reduced pressure, and then acetonitrile (2.1 mL) and water (2.1 mL) were added, followed by stirring at room temperature for 3 hours. The reaction solution was concentrated, and purified by column chromatography (TFA:acetonitrile = 5% to 95%) to obtain 65 mg of **compound Int-L** (TFA salt, yield: 37%).

¹H NMR (400 MHz, DMSO-*d₆*): *δ* 12.87 (bs, 2H), 8.19-8.03 (m, 3H), 7.98-7.00 (m, 14H), 5.71 (s, 3H), 5.22-4.82 (m, 6H), 4.62-4.29 (m, 6H), 4.20 (d, 2H), 4.14-3.81 (m, 6H), 3.70-3.68 (m, 48H), 3.55-3.48 (m, 9H), 2.94-2.85 (m, 9H), 1.75 (s, 9H).

MS m/z (ESI): 865.1 ([M/2+H]⁺.

### Example 13 Synthesis of Conjugate 1-D-E1 Based on Lysine Conjugation

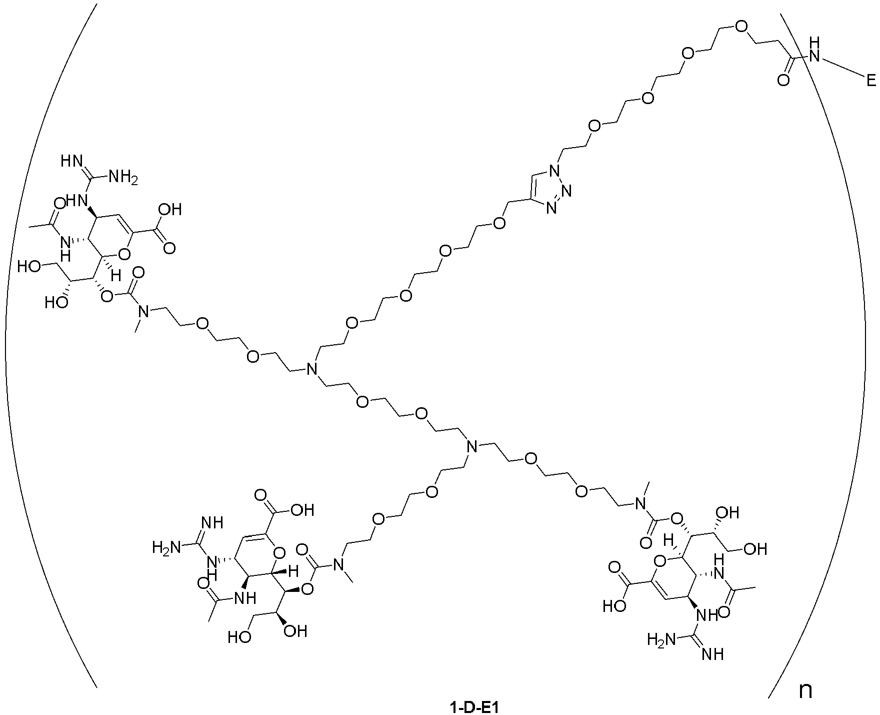

Preparation of Azido-PEG4-NHS ester solution: 16.75 mg of PEG4-azido NHS (CAS: 944251-24-5, purchased from Shaanxi Xinyan Bomei Biotechnology) was dissolved in 100 µl of DMF, and then the volume was adjusted with a 1×PBS pH 7.2 buffer solution at 0°C to a final concentration of 50 mM.

Preparation of click reagent: 10.0 mg of CuSO₄ (5 mM) was dissolved in 12.53 ml of 1×PBS. 5 ml was taken and added to 43.1 mg of BTTAA (20 mM, CAS: 1334179-85-9, purchased from Energy Chemical) and 247.5 mg of sodium ascorbate (250 mM) for later use.

**Step 1:** Protein Fc (963.4 µL, 10.38 mg/mL, its sequence is as set forth in SEQ ID NO: 1), PBS pH 7.2 (36.6 µL), and PEG4-azido solution (25.0 µL) were added into a 15 mL reaction tube, and shaken at room temperature for 3 hours. The reaction solution was purified with a centrifugal desalting column under a PBS pH 6.3 buffer solution to obtain Fc-azido (8.95 mg, yield 89.5%, purity 97.8%). By MALDI-TOF analysis, the DAR value was 5.8.

**Step 2:** Fc-azido (8.95 mg), a solution of intermediate compound **Int-D** (4.49 mg) in 1×PBS pH=6.3 (76 µL), and the click reagent (499 µL) were added into a 15 mL reaction tube, and shaken at room temperature for 20 hours. The reaction solution was desalted with PBS (pH=5.0), and purified by column chromatography to obtain **conjugate 1-D-E1** (6.9 mg, yield 86.4%, purity 98.2%). By Maldi tof analysis, the DAR value was 3.86.

### Example 14 Synthesis of Conjugate 1-L-E1 Based on Lysine Conjugation

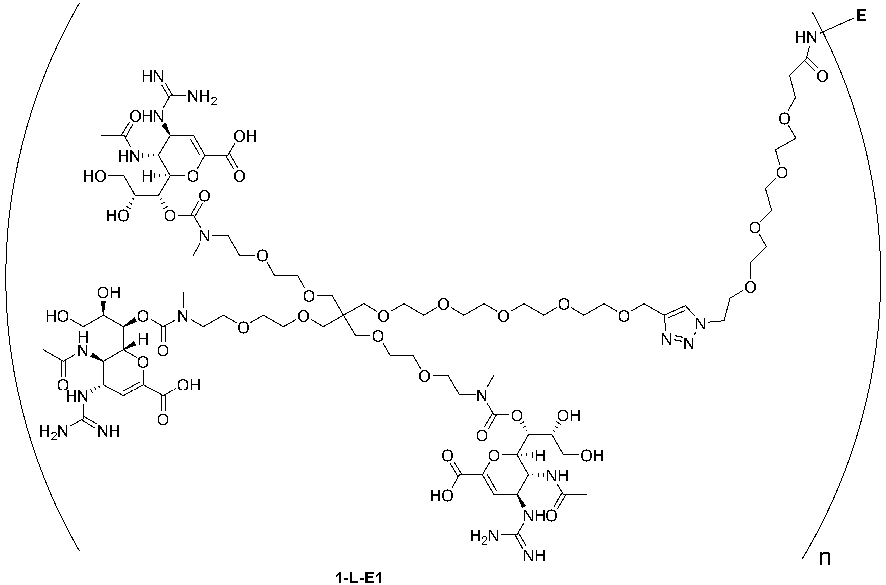

**Step 1:** Fc-azido was prepared using the same method as in Example 13 (8.81 mg, yield 88.1%, purity 97.7%). By Maldi tof analysis, the DAR value was 5.57.

**Step 2:** Fc-azido (8.81 mg), a solution of intermediate compound **Int-L** (4.07 mg) in 1×PBS pH=6.3 (313 µL), and the click reagent (490 µL) were added into a 15 mL reaction tube, and shaken at room temperature for 20 hours. The reaction solution was desalted with PBS (pH=5.0), and purified by column chromatography to obtain conjugate 1-L-E1 (6.6 mg, yield 74.9%, purity 98.8%). By Maldi tof analysis, the DAR value was 3.98.

### Example 15 Synthesis of Conjugate Molecules Based on Interchain Disulfide Bond Cysteine Conjugation

The purified Fc was exchanged into a 0.1 M phosphate buffer solution (pH 8.0) containing 2 mM EDTA using a centrifugal concentrator tube with a molecular weight cutoff of 30,000 Daltons, to a final concentration of 2.5 mg/ml. DTT (Beyotime) was added to 2.5 mg of Fc to a final concentration of 3 mM. After mixing uniformly, the mixture was incubated at 37°C for 2 to 3 hours. After the reaction was completed, excess DTT was removed by Sephadex G-25 resin (Cytiva). The content of reduced cysteine in the solution was determined with DTNB (Solarbio).

10 equivalents of Azido-PEG3-Maleimide (purchased from Alfa Aesar, 0.01 M stock solution dissolved in DMSO/PBS) were added to 2.5 mg of reduced Fc. After mixing uniformly, the mixture was incubated with oscillation at room temperature for 2 to 3 hours to obtain Fc-azido. Immediately thereafter, 20 equivalents of free cysteine were added to quench unreacted Azido-PEG3-Maleimide, and quenching was performed at 10°C for 30 minutes. After the reaction was completed, the buffer solution was exchanged into PBS by Sephadex G-25 resin. 10 equivalents of the aforementioned intermediate were added to Fc-azido. After mixing uniformly, 10 equivalents of copper ion catalyst reagent (0.005 M copper sulfate, 0.02 M BTTAA, and 0.25 M sodium ascorbate) were added to initiate a click reaction. The reaction was carried out at room temperature for 12 to 20 hours. After the reaction was completed, the reaction solution was purified with a MabPurix prepacked column and HiLoad Superdex 200 16/600 PG to obtain a conjugate molecule, and its DAR value was analyzed.

### Example 16 Synthesis of Conjugate Molecules Based on Single-Point Cysteine Mutation Conjugation

The purified Fc single-point cysteine mutant was exchanged into a 0.1 M phosphate buffer solution (pH 8.0) containing 2 mM EDTA using a centrifugal concentrator tube with a molecular weight cutoff of 30,000 Daltons, to a final concentration of 2.5 mg/ml. DTT was added to 2.5 mg of Fc mutant to a final concentration of 3 mM. After mixing uniformly, the mixture was incubated at 37°C for 2 to 3 hours. After the reaction was completed, excess DTT was removed by Sephadex G-25 resin. The content of reduced cysteine in the solution was determined with DTNB. The reduced Fc mutant was exchanged into a 0.1 M phosphate buffer solution (pH 6.5) containing 2 mM EDTA and 1 mM dehydroascorbic acid using Sephadex G-25 resin. After incubation at room temperature for 2 to 3 hours, the Fc mutant was exchanged into a 0.1 M phosphate buffer solution (pH 8.0) containing 2 mM EDTA using Sephadex G-25 resin. The content of reduced cysteine in the solution was determined with DTNB.

5 equivalents of Azido-PEG3-Maleimide (0.01 M stock solution dissolved in DMSO/PBS) were added to 2.5 mg of re-oxidized Fc mutant. After mixing uniformly, the mixture was incubated with oscillation at room temperature for 2-3 hours to obtain Fc mutant-azido. Immediately thereafter, 20 equivalents of free cysteine were added to quench unreacted Azido-PEG3-Maleimide, and quenching was performed at 10°C for 30 minutes. After the reaction was completed, the buffer solution was exchanged into PBS by Sephadex G-25 resin. 5 equivalents of the aforementioned intermediate were added to Fc mutant-azido. After mixing uniformly, 10 equivalents of copper ion catalyst reagent (0.005 M copper sulfate, 0.02 M BTTAA, and 0.25 M sodium ascorbate) were added to initiate a click reaction. The reaction was carried out at room temperature for 12 to 20 hours. After the reaction was completed, the reaction solution was purified with a MabPurix prepacked column and HiLoad Superdex 200 16/600 PG to obtain a conjugate molecule, and its DAR value was analyzed.

### Example 17 Synthesis of Conjugate Molecules Based on Double-Point Cysteine Mutation Conjugation

The purified Fc double-point cysteine mutant was exchanged into a 0.1 M phosphate buffer solution (pH 8.0) containing 2 mM EDTA using a centrifugal concentrator tube with a molecular weight cutoff of 30,000 Daltons, to a final concentration of 2.5 mg/ml. DTT was added to 2.5 mg of Fc mutant to a final concentration of 3 mM. After mixing uniformly, the mixture was incubated at 37°C for 2 to 3 hours. After the reaction was completed, excess DTT was removed by Sephadex G-25 resin. The content of reduced cysteine in the solution was determined with DTNB. The reduced Fc mutant was exchanged into a 0.1 M phosphate buffer solution (pH 6.5) containing 2 mM EDTA and 1 mM dehydroascorbic acid using Sephadex G-25 resin. After incubation at room temperature for 2 to 3 hours, the Fc mutant was exchanged into a 0.1 M phosphate buffer solution (pH 8.0) containing 2 mM EDTA using Sephadex G-25 resin. The content of reduced cysteine in the solution was determined with DTNB.

10 equivalents of Azido-PEG3-Maleimide (0.01 M stock solution dissolved in DMSO/PBS) were added to 2.5 mg of re-oxidized Fc mutant. After mixing uniformly, the mixture was incubated with oscillation at room temperature for 2 to 3 hours to obtain Fc mutant-azido. Immediately thereafter, 20 equivalents of free cysteine were added to quench unreacted Azido-PEG3-Maleimide, and quenching was performed at 10°C for 30 minutes. After the reaction was completed, the buffer solution was exchanged into PBS by Sephadex G-25 resin. 10 equivalents of the aforementioned intermediate were added to Fc mutant-azido. After mixing uniformly, 10 equivalents of copper ion catalyst reagent (0.005 M copper sulfate, 0.02 M BTTAA, and 0.25 M sodium ascorbate) were added to initiate a click reaction. The reaction was carried out at room temperature for 12 to 20 hours. After the reaction was completed, the reaction solution was purified with a MabPurix prepacked column and HiLoad Superdex 200 16/600 PG to obtain a conjugate molecule, and its DAR value was analyzed.

### Example 18 Synthesis of Conjugate 2-D-El Based on Site-Specific Glycan Conjugation

Preparation of click reagent: 10.0 mg of CuSO₄ (5 mM) was dissolved in 12.53 ml of 1×PBS. 5 ml was taken and added to 43.1 mg of BTTAA (20 mM, CAS: 1334179-85-9, purchased from Energy Chemical) and 247.5 mg of sodium ascorbate (250 mM) for later use.

**Step 1:** Fc (963.4 µL, 10.38 mg/mL), 1×PBS pH 7.2 (36.6 µL), and Endos (WT) (0.1 mg, 122 µL, 0.82 mg/ml) were added into a 15 mL reaction tube, and shaken at 37°C for 3 hours. A 1×PBS pH=7.2 (30 µL) buffer solution of N₃-PEG3-Oxa (3.11 mg) was added, and shaken at 37°C for 1 hour. After the reaction was completed, the reaction solution was purified with a MabPurix prepacked column and HiLoad Superdex 200 16/600 PG to obtain 9.5 mg of conjugate molecule Fc-1 (yield 95.0%, purity 98.5%), and the DAR value was 1.82.

**Step 2:** Fc-1 (9.5 mg), a solution of intermediate compound **Int-D** (3.39 mg) in 1×PBS pH=6.3 (76 µL), and a click reagent (529 µL) were added into a 15 mL reaction tube, and shaken at room temperature for 20 hours. The reaction solution was desalted with PBS (pH=5.0), and purified by column chromatography to obtain conjugate **2-D-El** (7.5 mg, yield 78.9%, purity 98.0%). By Maldi tof analysis, the DAR value was 3.80.

### Example 19 Synthesis of Intermediate Int-L1

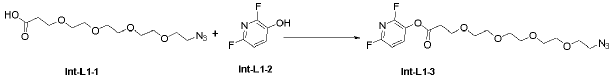

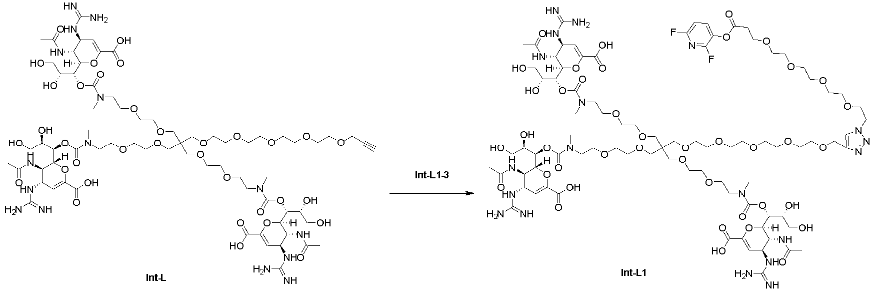

### Step 1: Synthesis of Int-L1-3

Under a nitrogen atmosphere, compound **Int-L1-1** (0.35 g, 1.20 mmol) and 2,6-difluoro-3-hydroxypyridine (0.2 g, 1.56 mmol) were dissolved in DCM (5 mL). EDCI (0.30 g, 1.56 mmol) was added under cooling in an ice-water bath. Subsequently, the reaction was warmed to room temperature and stirred for 2 h. The reaction solution was directly concentrated, and purified by reverse phase column purification (0.1% TFA in H₂O:ACN=60:40) to obtain 0.48 g of **Int-L1-3,** yield: 98%.

MS m/z (ESI): 427.1 [M+Na].

### Step 2: Synthesis of Int-L1

Under a nitrogen atmosphere, compound **Int-L** (TFA salt, 0.32 g, 0.139 mmol) and **Int-L1-3** (0.23 g, 0.556 mmol) were dissolved in NMP (1 mL), and tetrakis(acetonitrile)copper(I) hexafluorophosphate (0.16 g, 0.418 mmol) was added at room temperature. The mixture was reacted at room temperature for 20 minutes, then directly purified by preparative HPLC (Waters XBridge, 19*150 mm, 5 µm; mobile phase A: 0.05% aqueous TFA solution, mobile phase B: acetonitrile, gradient ratio: phase B 5% to 95%) to obtain 0.2 g of compound **Int-L1** TFA salt, yield: 53.7%, purity: 93.9%.

¹H NMR (400 MHz, D₂O): *δ* 8.02 (s, 1H), 7.90-7.82 (m, 1H), 7.01-6.98 (m, 1H), 5.87 (s, 3H), 4.92-4.89 (m, 5H), 4.59-4.48 (m, 7H), 4.36 (d, 3H), 4.13-3.98 (m, 6H), 3.89-3.81 (m, 5H), 3.75-3.51 (m, 51H), 3.46-3.38 (m, 11H), 3.32-3.13 (m, 3H), 2.93-2.74 (m, 9H), 1.89 (s, 9H).

MS m/z (ESI): 1067.3 [M/2+H]⁺.

### Example 20 Synthesis of Intermediate Int-M

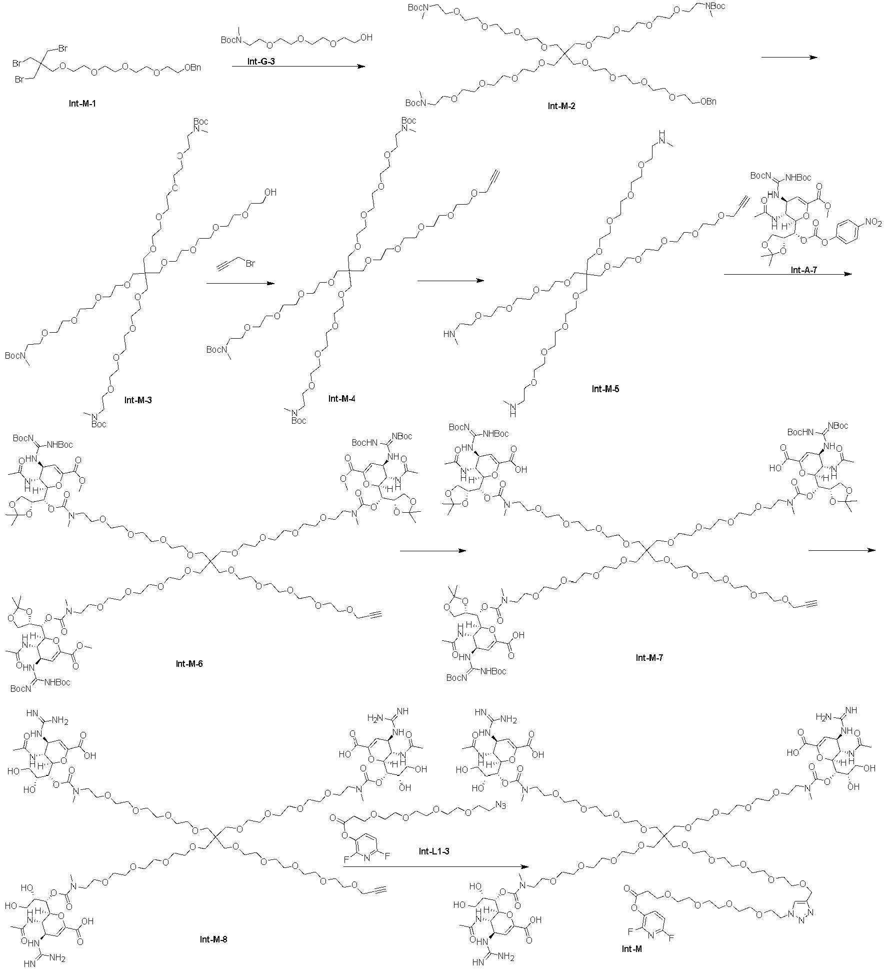

### Step 1: Synthesis of Int-M-2

KOH (756 mg, 13.5 mmol) was added to a 25 mL single-neck flask at room temperature, and DMSO (anhydrous, 5 ml) was added and stirred uniformly. **Compound Int-G-3** (1.38 g, 4.5 mmol) was added, and stirred at room temperature for 15 minutes. **Int-M-1** (591 mg, 1.0 mmol, prepared using a known method, Journal of Organic Chemistry, 2012, vol. 77, # 20, p. 8879 - 8887) and KI (83 mg, 0.5 mmol) were added, and stirred for 10 minutes. The temperature was raised to an external temperature of 75°C to react for 6 hours. The reaction solution was cooled to room temperature, and diluted with ethyl acetate (20 mL). Deionized water (30 ml) was added slowly under an ice-water bath. The aqueous phase was extracted twice with ethyl acetate (2 x 20 ml). The organic phases were combined, washed with saturated brine (20 ml), dried over anhydrous sodium sulfate, filtered, and concentrated to obtain a crude product. The crude product was purified by reverse phase column chromatography (ACN:H₂O = 90%) to obtain 1.0 g of **compound Int-M-2,** yield: 78.7%, purity: 95%.

MS m/z (ESI): 1293.7 [M+Na].

### Step 2: Synthesis of Compound Int-M-3

**Compound Int-M-2** (1.0 g, 0.787 mmol) and 20% Pd(OH)₂/C (0.8 g) were added to a 50 mL single-neck flask, followed by adding MeOH (30 mL) and stirring at room temperature for 5 minutes. Subsequently, replacement of hydrogen of one atmosphere was performed three times. The mixture was heated to 30°C and reacted for 18 hours, and then filter with diatomaceous earth. The filter cake was washed three times with methanol (10 mL × 3). The resulting filtrate was directly concentrated to obtain 0.9 g of crude **compound Int-M-3,** yield 106.06%, which was directly used in the next step.

MS m/z (ESI): 1198.7 [M+H₂O].

### Step 3: Synthesis of Compound Int-M-4

**Compound Int-M-3** (0.9 g, 0.762 mmol) was dissolved in anhydrous THF (10 mL), cooled to 0°C under an ice-water bath, and stirred. Potassium *tert*-butoxide (1.54 mL, 1.54 mmol, 1 M in THF) was added, then warmed to room temperature and stirred for one hour. The reaction solution was cooled again with an ice bath, and 3-bromopropyne (272 mg, 2.29 mmol) was added dropwise. Subsequently, the reaction was slowly warmed to room temperature and reacted for two hours. The reaction solution was cooled with an ice-water bath, quenched with a saturated aqueous ammonium chloride solution (30 mL), and extracted with EtOAc (30 mL × 3). The organic phases were combined, washed sequentially with water (20 mL) and saturated brine (20 mL), dried over anhydrous sodium sulfate (30 g), filtered, and concentrated under reduced pressure. The resulting residue was purified by reverse phase column chromatography (ACN:0.1% TFA in H₂O = 50% to 75%) to obtain 0.8 g of **compound Int-M-4,** purity: 80%, yield: 86%.

MS m/z (ESI): 1118.4 [M-100].

### Step 4: Synthesis of Compound Int-M-5

**Compound Int-M-4** (0.9 g, 0.75 mmol) was dissolved in DCM (11 mL), cooled with an ice-water bath, and stirred. Subsequently, TFA (11 mL) was added dropwise. The reaction was warmed to room temperature and reacted for two hours. The reaction was stopped, directly concentrated, and the residue was co-evaporated twice with DCM (10 mL × 2). Subsequently, the mixture was dried with an oil pump to obtain crude **compound Int-M-5,** which was directly used in the next step.

MS m/z (ESI): 459.8 [M/2+H]⁺.

### Step 5: Synthesis of Compound Int-M-6

**Compound Int-M-5** (350 mg, 0.382 mmol) was added into a single-neck flask. DCM (6 mL), DMF (6 mL) and DIEA (0.99 g, 7.65 mmol) were added at room temperature, and stirred at room temperature for 5 minutes. **Compound Int-A-7** (0.862 g, 1.15 mmol) was added in batches. Replacement with nitrogen was performed three times. Subsequently, the mixture was heated to 49°C to react for 10 hours. The reaction was stopped, directly concentrated, and the residue was dissolved in H₂O (20 mL) and extracted with EA (20 mL × 2). The organic phases were combined, concentrated under reduced pressure, and then purified by reverse phase column chromatography (ACN/H₂O, 90 to 93% eluting the product) to obtain 0.8 g of **compound Int-M-6,** yield: 75.9%, purity: 84%.

MS m/z (ESI): 919.5 [M/3+H]⁺.

### Step 6: Synthesis of Compound Int-M-7

**Compound Int-M-6** (0.8 g, 0.29 mmol) was dissolved in MeOH (7 mL) and THF (7 mL). The solution was cooled with an ice-water bath, and H₂O (7 mL) was added. Subsequently, lithium hydroxide monohydrate (88 mg, 2.32 mmol) was added. The reaction was warmed to room temperature and stirred for 3 hours. A cation exchange resin was added until the pH was 5 to 6. Subsequently, the mixture was filtered, and the filter cake was washed with MeOH (10 mL × 2). The filtrate was concentrated to obtain 0.6 g of **compound Int-M-7** (yield: 76.2%), which was directly used in the next step.

MS m/z (ESI): 905.3 [M/3+H]⁺.

### Step 7: Synthesis of Compound Int-M-8

**Compound Int-M-7** (0.6 g, 0.224 mmol) was dissolved in DCM (4 mL), and TFA (4 mL) was added under cooling in an ice-water bath. Subsequently, the reaction was warmed to room temperature and stirred for 2 hours. The reaction solution was directly concentrated, and the residue was co-evaporated twice with dichloromethane (4 mL). Subsequently, the resulting residue was dissolved in ACN (5 mL) and H₂O (5 mL), and stirred at room temperature for 3 hours. The mixture was directly concentrated, and purified by reverse phase column chromatography (ACN/0.1% TFA in H₂O, 40% eluting the product) to obtain 0.42 g of **compound Int-M-8** TFA salt, yield: 73%, purity: 87.6%.

MS m/z (ESI): 997.3 [M/2+H]⁺.

### Step 8: Synthesis of Compound Int-M

Under a nitrogen atmosphere, **compound Int-M-8** (TFA salt, 50 mg, 0.025 mmol) and **compound Int-L1-3** (55 mg, 0.15 mmol) were dissolved in NMP (1 mL). Tetrakis(acetonitrile)copper(I) hexafluorophosphate (27 mg, 0.075 mmol) was added at room temperature, and then reacted at room temperature for 20 minutes. The reaction solution was directly purified by preparative HPLC (Waters XBridge, 19*150 mm, 5 µm; mobile phase A: 0.05% aqueous TFA solution, mobile phase B: acetonitrile, gradient ratio: phase B 5% to 95%) to obtain 35 mg of **compound Int-M** TFA salt, yield: 58%, purity: 92.8%.

¹H NMR (400 MHz, D₂O): *δ* 7.99 (s, 1H), 7.90-7.82 (m, 1H), 7.02-6.98 (m, 1H), 5.77 (s, 3H), 4.92-4.88 (m, 5H), 4.59-4.46 (m, 13H), 4.35 (d, 5H), 4.13-3.98 (m, 10H), 3.89-3.81 (m, 6H), 3.68-3.50 (m, 47H), 3.46-3.39 (m, 11H), 3.30-3.15 (m, 11H), 2.93-2.88 (m, 7H), 2.81 (s, 6H), 1.89 (s, 9H).

MS m/z (ESI): 1199.4 [M/2+H]⁺.

### Example 21 Synthesis of Intermediate Int-N

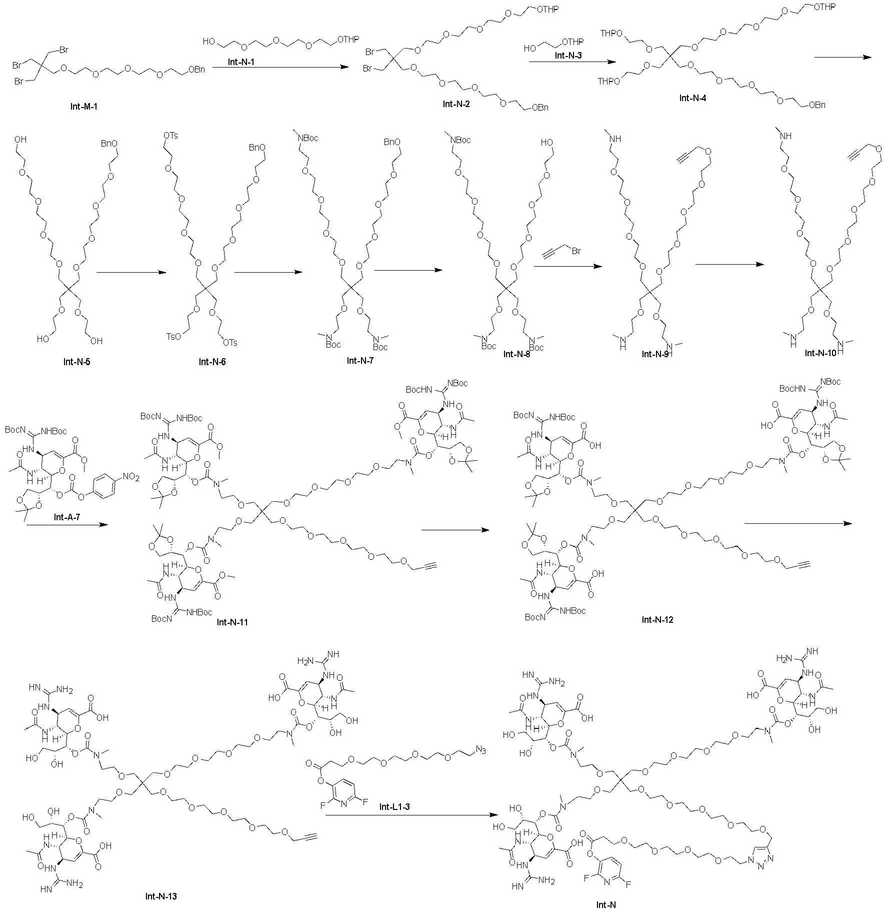

### Step 1: Synthesis of Compound Int-N-2

Under an ice-water bath, **compound Int-M-1** (2.2 g, 3.72 mmol, prepared using a known method, Journal of Organic Chemistry, 2012, vol. 77, # 20, p. 8879 - 8887) was added to a 50 mL single-neck flask. DMAc (anhydrous, 10 ml) was added and stirred uniformly. tBuOK (477 mg, 5.0 mmol) was added, and stirred for 10 minutes. **Compound Int-N-1** (purchased from Leyan, 0.69 g, 2.47 mmol) was added, and the temperature was raised to an external temperature of 60°C to react for 16 hours. The reaction solution was cooled to room temperature, and diluted with ethyl acetate (20 mL). Deionized water (30 ml) was slowly added under an ice-water bath. The aqueous phase was extracted twice with ethyl acetate (220 ml). The organic phases were combined, washed with saturated brine (20 ml), dried over anhydrous sodium sulfate, filtered, and concentrated to obtain a crude product. The crude product was purified by reverse phase column chromatography (ACN:H₂O = 70%) to obtain 1.5 g of **compound Int-N-2,** yield: 76%, purity: 92%.

MS m/z (ESI): 806.2 [M+H₂O].

### Step 2: Synthesis of Compound Int-N-4

KOH (1.92 g, 34.24 mmol) was added to a 25 mL single-neck flask at room temperature. DMSO (anhydrous, 5 ml) was added and stirred uniformly. **Compound Int-N-3** (1.67 g, 11.41 mmol) was added, and stirred at room temperature for 15 minutes. **Compound Int-N-2** (1.5 g, 1.9 mmol) and KI (157 mg, 0.95 mmol) were added, and stirred for 10 minutes. The temperature was raised to an external temperature of 75°C to react for 6 hours. The reaction solution was cooled to room temperature, and diluted with ethyl acetate (20 mL). Deionized water (30 ml) was slowly added under an ice-water bath. The aqueous phase was extracted twice with ethyl acetate (220 ml). The organic phases were combined, washed with saturated brine (20 ml), dried over anhydrous sodium sulfate, filtered, and concentrated to obtain a crude product. The crude product was purified by reverse phase column chromatography (ACN:H₂O = 90%) to obtain 1.5 g of **compound Int-N-4,** yield: 85%, purity: 90%.

MS m/z (ESI): 936.6 [M+ H₂O].

### Step 3: Synthesis of Compound Int-N-5

**Compound Int-N-4** (1.5 g, 1.63 mmol) was dissolved in anhydrous methanol (15 mL), and hydrochloric acid (0.4 mL, 5.0 mmol, 12 M) was added. The reaction was stirred at room temperature for 1 hour. After the reaction was completed, the reaction solution was concentrated to obtain 1.1 g of crude **compound Int-N-5,** which was directly used in the next step.

MS m/z (ESI):667.4 [M+H]⁺.

### Step 4: Synthesis of Compound Int-N-6

Under an ice-water bath, crude **compound Int-N-5** (1.1 g, 1.63 mmol) was dissolved in pyridine (20 mL), and *p*-toluenesulfonyl chloride (6.29 g, 33.0 mmol) was added. The reaction was stirred at room temperature overnight. After the reaction was completed, the reaction solution was concentrated, and the resulting crude product was purified by reverse phase column chromatography (ACN:H₂O = 90%) to obtain 0.75 g of **compound Int-N-6,** yield: 40%, purity: 95%.

MS m/z (ESI):1129.5 [M+H]⁺.

### Step 5: Synthesis of Compound Int-N-7

**Compound Int-N-6** (0.75 g, 0.67 mmol) was dissolved in a solution of methylamine in ethanol (5 mL, about 30%), and stirred at room temperature overnight. After the reaction was completed, the reaction solution was concentrated. The concentrated solution was dissolved with DCM (10 mL), and Boc₂O (2.47 g, 11.33 mmol) and triethylamine (2.29 g, 22.66 mmol) were added. The reaction solution was stirred overnight, and then directly concentrated. The crude product was purified by reverse phase column chromatography (ACN:H₂O = 95%) to obtain 0.2 g of **compound Int-N-7,** yield: 35%, purity: 94%.

MS m/z (ESI):1023.6 [M+ H₂O].

### Step 6: Synthesis of Compound Int-N-8

**Compound Int-N-7** (0.2 g, 0.198 mmol) and 20% Pd(OH)₂/C (0.1 g) were added to a 50 mL single-neck flask. Subsequently, MeOH (10 mL) was added, and stirred at room temperature for 5 minutes. Subsequently, replacement with hydrogen of one atmosphere was performed three times. The reaction solution was heated to 30°C, reacted for 18 hours, and then filtered with diatomaceous earth. The filter cake was washed three times with methanol (5 mL × 3). The resulting filtrate was directly concentrated to obtain 0.182 g of crude **compound Int-N-8,** which was directly used in the next step.

MS m/z (ESI): 933.6 [M+18].

### Step 7: Synthesis of Compound Int-N-9

**Compound Int-N-8** (0.182 g, 0.198 mmol) was dissolved in anhydrous THF (5 mL), cooled to 0°C under an ice-water bath, and stirred. Potassium tert-butoxide (0.5 mL, 0.5 mmol, 1 M in THF) was added, warmed to room temperature and stirred for one hour. The reaction solution was cooled again with an ice bath, and 3-bromopropyne (70.9 mg, 0.595 mmol) was added dropwise. Subsequently, the reaction was slowly warmed to room temperature and reacted for two hours. The reaction solution was cooled with an ice-water bath, and quenched with a saturated aqueous ammonium chloride solution (10 mL). Subsequently, the solution was extracted with EtOAc (10 mL × 3). The organic phases were combined, washed sequentially with water (10 mL) and saturated brine (10 mL), dried over anhydrous sodium sulfate (5 g), filtered, and concentrated under reduced pressure. The resulting residue was purified by reverse phase column chromatography (ACN:0.1% TFA in H₂O = 65% to 75%) to obtain 0.15 g of **compound Int-N-9,** yield: 79%.

MS m/z (ESI): 971.6 [M+ H₂O].

### Step 8: Synthesis of Compound Int-N-10

**Compound Int-N-9** (0.15 g, 0.157 mmol) was dissolved in DCM (1.5 mL), cooled with an ice-water bath, and stirred. Subsequently, TFA (1.5 mL) was added dropwise. The reaction was warmed to room temperature and reacted for two hours. The reaction was stopped, and directly concentrated. The resulting residue was co-evaporated twice with DCM (5 mL × 2), and dried with an oil pump to obtain crude **compound Int-N-10,** which was directly used in the next step.

MS m/z (ESI): 654.3 [M+H]⁺.

### Step 9: Synthesis of Compound Int-N-11

**Compound Int-N-10** (103 mg, 0.157 mmol) was added to a single-neck flask. DCM (2 mL) and DMF (2 mL) were added at room temperature. DIEA (0.41 g, 3.15 mmol) was added, and stirred at room temperature for 5 minutes. **Compound Int-A-7** (0.355 g, 0.472 mmol) was added in batches. Replacement with nitrogen was performed three times. Subsequently, the reaction solution was heated to 49°C to react for 10 hours. The reaction was stopped, and directly concentrated. The resulting residue was dissolved in H₂O (10 mL) and extracted with EA (10 mL × 2). The organic phases were combined, concentrated under reduced pressure, and then purified by reverse phase column chromatography (ACN/H₂O, 90 to 93% eluting the product) to obtain 0.2 g of **compound Int-N-11,** yield: 50.95%.

MS m/z (ESI): 831.4 [M/3+H]⁺.

### Step 10: Synthesis of Compound Int-N-12

**Compound Int-N-11** (0.2 g, 0.083 mmol) was dissolved in MeOH (1.5 mL) and THF (1.5 mL), cooled with an ice-water bath, and H₂O (1.5 mL) was added. Subsequently, lithium hydroxide monohydrate (25 mg, 0.66 mmol) was added. The reaction was warmed to room temperature and stirred for 3 hours. A cation exchange resin was added until the pH was 5 to 6. Subsequently, the mixture was filtered, and the filter cake was washed with MeOH (3 mL × 2). The filtrate was concentrated to obtain 0.19 g of crude **compound Int-N-12,** which was directly used in the next step.

MS m/z (ESI): 817.3 [M/3+H]⁺.

### Step 11: Synthesis of Compound Int-N-13

**Compound Int-N-12** (0.19 g, 0.08 mmol) was dissolved in DCM (1.8 mL). TFA (1.8 mL) was added under cooling in an ice-water bath. Subsequently, the reaction was warmed to room temperature and stirred for 2 hours. The reaction solution was directly concentrated, and the resulting residue was co-evaporated twice with dichloromethane (3 mL). Subsequently, the resulting residue was dissolved in ACN (3 mL) and H₂O (3 mL), and stirred at room temperature for 3 hours. The solution was directly concentrated, and purified by reverse phase column chromatography (ACN/0.1% TFA in H₂O, 40% eluting the product) to obtain 90 mg of **compound Int-N-13** TFA salt, yield: 46%.

MS m/z (ESI): 577.1 [M/3+H]⁺.

### Step 12: Synthesis of Compound Int-N

Under a nitrogen atmosphere, **compound Int-N-13** (TFA salt, 80 mg, 0.035 mmol) and **compound Int-L1-3** (140 mg, 0.35 mmol) were dissolved in NMP (1 mL). Tetrakis(acetonitrile)copper(I) hexafluorophosphate (65 mg, 0.174 mmol) was added at room temperature, and reacted at room temperature for 20 minutes. The reaction solution was directly purified by preparative HPLC (Waters XBridge, 19*150 mm, 5 µm; mobile phase A: 0.05% aqueous TFA solution, mobile phase B: acetonitrile, gradient ratio: phase B 5% to 95%) to obtain 12.5 mg of **compound Int-N** TFA salt, yield: 10%, purity: 90.8%.

¹H NMR (400 MHz, D₂O): *δ* 7.98 (s, 1H), 7.89-7.83 (m, 1H), 7.01-6.97 (m, 1H), 5.80 (s, 3H), 4.91-4.85 (m, 5H), 4.60-4.46 (m, 10H), 4.34 (d, 5H), 4.12-3.98 (m, 8H), 3.88-3.81 (m, 6H), 3.62-3.50 (m, 44H), 3.45-3.29 (m, 10H), 2.93-2.88 (m, 6H), 2.81 (s, 6H), 1.88 (s, 9H).

MS m/z (ESI): 711.9 [M/3+H]⁺.

### Example 22 Synthesis of Intermediate Int-O

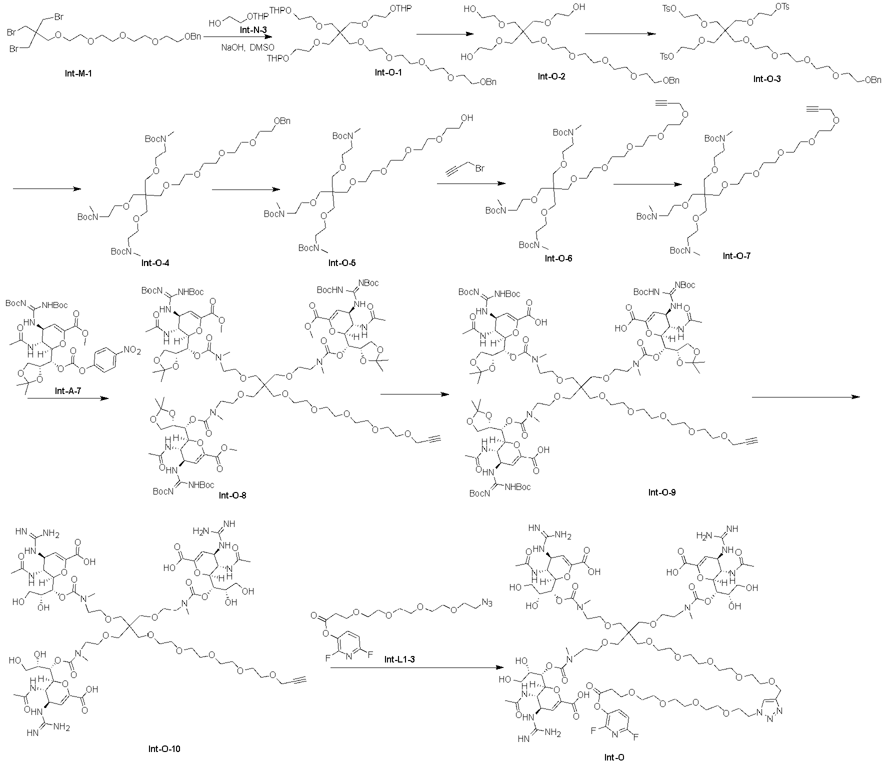

### Step 1: Synthesis of Compound Int-O-1

KOH (0.96 g, 17.12 mmol) was added to a 25 mL single-neck flask at room temperature. DMSO (anhydrous, 5 ml) was added and stirred uniformly. **Compound Int-N-3** (purchased from Leyan, 1.32 g, 9.0 mmol) was added, and stirred at room temperature for 15 minutes. **Compound Int-M-1** (1.18 g, 2.0 mmol, prepared using a known method, Journal of Organic Chemistry, 2012, vol. 77, # 20, p. 8879 - 8887) and KI (166 mg, 0.5 mmol) were added, and stirred for 10 minutes. The temperature was raised to an external temperature of 75°C to react for 6 hours. The reaction solution was cooled to room temperature, and diluted with ethyl acetate (20 mL). Deionized water (20 ml) was slowly added under an ice-water bath. The aqueous phase was extracted twice with ethyl acetate (220 ml). The organic phases were combined, washed with saturated brine (20 ml), dried over anhydrous sodium sulfate, filtered, and concentrated to obtain a crude product. The crude product was purified by reverse phase column chromatography (ACN:H₂O = 90%) to obtain 1.0 g of **compound Int-O-1,** yield: 67%, purity: 95%.

MS m/z (ESI): 804.5 [M+ H₂O].

### Step 2: Synthesis of Compound Int-O-2

**Compound Int-O-1** (1.0 g, 1.27 mmol) was dissolved in anhydrous methanol (10 mL), and hydrochloric acid (0.53 mL, 6.4 mmol, 12 M) was added. The reaction was stirred at room temperature for 1 hour. After the reaction was completed, the reaction solution was concentrated to obtain 0.9 g of crude **compound Int-O-2,** which was directly used in the next step.

MS m/z (ESI):535.3 [M+H]⁺.

### Step 3: Synthesis of Compound Int-O-3

Under an ice-water bath, crude **compound Int-O-2** (0.9 g, 1.27 mmol) was dissolved in pyridine (15 mL), and p-toluenesulfonyl chloride (4.2 g, 22.0 mmol) was added. The reaction was stirred at room temperature overnight. After the reaction was completed, the reaction solution was concentrated. The crude product was purified by reverse phase column chromatography (ACN:H₂O = 90%) to obtain 0.8 g of **compound Int-O-3,** yield: 63%, purity: 95%.

MS m/z (ESI):997.5 [M+H]⁺.

### Step 4: Synthesis of Compound Int-O-4

**Compound Int-O-3** (0.8 g, 0.8 mmol) was dissolved in a solution of methylamine in ethanol (8 mL, about 30%), and stirred at room temperature overnight. After the reaction was completed, the reaction solution was concentrated. The concentrated solution was dissolved with DCM (10 mL), and Boc₂O (2.18 g, 10.0 mmol) and triethylamine (2.02 g, 20.0 mmol) were added. The reaction solution was stirred overnight, and then directly concentrated. The crude product was purified by reverse phase column chromatography (ACN:H₂O = 95%) to obtain 0.5 g of **compound Int-O-4,** yield: 72%, purity: 95%.

MS m/z (ESI):891.6 [M+ H₂O].

### Step 5: Synthesis of Compound Int-O-5

**Compound Int-O-4** (0.255 g, 0.291 mmol) was dissolved in 10 mL of MeOH, and 20% palladium hydroxide on carbon (0.255 g) was added. The air in the system was replaced with hydrogen four times, and then the reaction was carried out at room temperature under a hydrogen atmosphere for ten hours. After the reaction was completed, the reaction solution was filtered and concentrated to obtain 0.22 g of crude **compound Int-O-5,** which was directly used in the next step. Yield: 96.1%.

MS m/z (ESI):806.48 [M+Na].

### Step 6: Synthesis of Compound Int-O-6

**Compound Int-O-5** (0.2 g, 0.28 mmol) was dissolved in dry tetrahydrofuran (4 mL), and a solution of potassium tert-butoxide in THF (0.56 mL, 0.56 mmol) was added under cooling in an ice-water bath. After completion of the addition, the reaction was stirred at room temperature for one hour. The reaction solution was cooled with an ice-water bath, and propargyl bromide (0.1 g, 0.84 mmol) was added thereto. After completion of the addition, the reaction naturally warmed to room temperature and was stirred for 2 h. The reaction solution was cooled with an ice-water bath, quenched with a saturated aqueous ammonium chloride solution, extracted with ethyl acetate, dried, concentrated, and purified by column chromatography (water:acetonitrile = 35%) to obtain 0.174 g of **compound Int-O-6,** yield: 75%.

MS m/z (ESI):844.53 [M+Na].

### Step 7: Synthesis of Compound Int-O-7

**Compound Int-O-6** (174 mg, 0.211 mmol) was dissolved in DCM (2 mL), and trifluoroacetic acid (2 mL) was added under cooling in an ice-water bath. The reaction was naturally warmed and stirred for 1 hour. After the reaction was completed, the reaction solution was concentrated to obtain 147 mg of crude **compound Int-O-7,** which was directly used in the next step.

MS m/z (ESI): 522.4 [M+H]⁺.

### Step 8: Synthesis of Compound Int-O-8

*N,N*-diisopropylethylamine (728 mg, 5.64 mmol) was added to a solution of **compound Int-O-7** (147 mg, 0.295 mmol) in N,N-dimethylformamide (4 mL) and dichloromethane (4 mL), and stirred at room temperature for 5 min. **Compound Int-A-7** (635 mg, 0.845 mmol) was added in batches. Then the reaction solution was heated to 49°C and stirred overnight. The reaction solution was concentrated at room temperature to remove dichloromethane and N,N-dimethylformamide, and purified by column chromatography (acetonitrile:water = 95%) to obtain 274 mg of **compound Int-O-8,** yield: 41%.

MS m/z (ESI): 787.2 [M/3+H]⁺.

### Step 9: Synthesis of Compound Int-O-9

Under an ice-water bath, an aqueous solution (3 mL) of lithium hydroxide monohydrate (38 mg, 0.928 mmol) was added to a solution of **compound Int-O-8** (274 mg, 0.116 mmol) in methanol (3 mL) and tetrahydrofuran (3 mL), and naturally warmed. After the reaction was completed, the reaction solution was adjusted to pH = 5 to 6 using IRN77 ion exchange resin. The mother liquor after filtration was concentrated to obtain 243 mg of **compound Int-O-9,** yield: 90%.

MS m/z (ESI): 773.3 [M/3+H]⁺.

### Step 10: Synthesis of Compound Int-O-10

Under an ice-water bath, trifluoroacetic acid (2 mL) was added to a solution of **compound Int-O-9** (243 mg, 0.104 mmol) in dichloromethane (2 mL). The reaction was stirred at room temperature for 1 hour. After the reaction was completed, the reaction solution was concentrated under reduced pressure, and then acetonitrile (2 mL) and water (1 mL) were added, followed by stirring at room temperature for 3 hours. The reaction solution was concentrated, and then purified by column chromatography (0.1% TFA in H₂O:acetonitrile = 75%) to obtain 213 mg of **compound Int-O-10** TFA salt, yield: 93.75%.

MS m/z (ESI): 573.1 [M/3+H]⁺.

### Step 11: Synthesis of Compound Int-O

Tetrakis(acetonitrile)copper(I) hexafluorophosphate (103 mg, 0.276 mmol) and **compound Int-L1-3** (149 mg, 0.369 mmol) were sequentially added to a solution of **compound Int-O-10** (TFA salt, 200 mg, 0.092 mmol) in N-methylpyrrolidone (0.5 mL). The reaction was stirred at room temperature for 20 minutes. The reaction solution was purified by preparative high-performance liquid chromatography (Waters XBridge, 19*150 mm, 5 µm; mobile phase A: 0.05% aqueous TFA solution, mobile phase B: acetonitrile, gradient ratio: phase B 5% o 95%) to obtain 38 mg of **compound Int-O** TFA salt, yield: 20.57%.

¹H NMR (400 MHz, D₂O): *δ* 7.99 (s, 1H), 7.77 (t, 1H), 7.01-6.97 (m, 1H), 5.66 (s, 3H), 4.89-4.83 (m, 5H), 4.59-4.31 (m, 13H), 4.06 (d, 9H), 3.88-3.81 (m, 6H), 3.62-3.42 (m, 42H), 3.41-3.27 (m, 12H), 3.22-2.97 (m, 3H), 2.93-2.88 (m, 8H), 2.81 (s, 6H), 2.62 (s, 2H), 1.87 (s, 9H).

MS m/z (ESI): 667.8 [M/3+H]⁺.

### Example 23 Synthesis of Intermediate Int-P

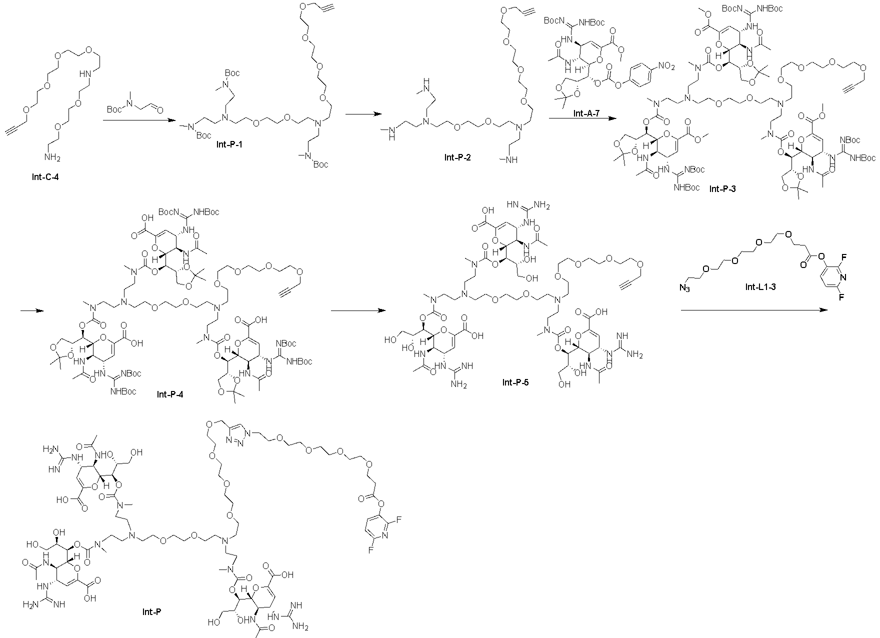

### Step 1: Synthesis of Compound Int-P-1

**Compound Int-C-4** (1 g, 2.76 mmol) was dissolved in methanol (10 mL), and N-tert-butoxycarbonyl-(methylamino)acetaldehyde (2.87 g, 16.55 mmol, purchased from Leyan) and 1 drop of acetic acid were added, and stirred at 20°C for 1 hour. Sodium triacetoxyborohydride (2.92 g, 13.79 mmol) was added under ice-water cooling, and then naturally warmed to 20°C and reacted for 16 hours. The reaction solution was concentrated to remove the solvent. Water and dichloromethane were added to the residue, stirred, and separated. The organic phase was concentrated to dryness, and purified by column chromatography (methanol:dichloromethane = 7%) to elute the product, which was collected and concentrated to obtain 1.9 g of **compound Int-P-1,** yield 82.57%.

MS (ESI): m/z 835.6 [M+1]⁺.

### Step 2: Synthesis of Compound Int-P-2

**Compound Int-P-1** (0.52 g, 623.43 µmol) was dissolved in dichloromethane (2.5 mL) and stirred. A 4.0 M solution of hydrogen chloride in dioxane (5 mL) was added under ice-water cooling and stirred for 1 to 2 hours. The reaction solution was concentrated to obtain 330 mg of **compound Int-P-2,** yield 100%.

MS (ESI): m/z 535.4 [M+1]⁺.

### Step 3: Synthesis of Compound Int-P-3

**Compound Int-P-2** (0.33 g, 623.43 µmol) and Int-A-7 (1.82 g, 1.37 mmol) were dissolved in N,N-dimethylformamide (4 mL). Diisopropylethylamine (1.21 g, 9.35 mmol) was added under ice-water cooling, and naturally warmed to room temperature and reacted for 16 hours. The reaction solution was directly purified by reverse phase column chromatography (methanol:water = 100%) to elute the product, which was collected and concentrated to obtain 0.76 g of **compound Int-P-3** TFA salt (yield 51.4%).

MS (ESI): m/z 1186.3 ([M+1]/2)⁺.

### Step 4: Synthesis of Compound Int-P-4

**Compound Int-P-3** (0.76 g, 320.46 µmol) was dissolved in methanol (6 mL) and water (6 mL). Lithium hydroxide monohydrate (80.69 mg, 1.92 mmol) was added under ice-water cooling, and naturally warmed to room temperature and reacted for 3 hours. The reaction solution was adjusted to a pH value of 5 to 6 with a strongly acidic resin under ice-water cooling, filtered, and concentrated to obtain 0.74 g of **compound Int-P-4,** yield 99%.

MS (ESI): m/z 1165.1 ([M+1]/2)⁺.

### Step 5: Synthesis of Compound Int-P-5

**Compound Int-P-4** (0.74 g, 317.66 µmol) was dissolved in dichloromethane (4 mL). Trifluoroacetic acid (3.5 mL) was added under ice-water cooling, and naturally warmed to room temperature and reacted for 3 hours. The reaction solution was concentrated to remove the solvent, dissolved in water (4 mL), and purified by preparative reverse phase chromatography (acetonitrile:0.1% TFA in H₂O = 25%) to obtain 0.15 g of **compound Int-P-5** TFA salt, yield 29.35%.

MS (ESI): m/z 804.9 ([M+1]/2)⁺.

### Step 6: Synthesis of Compound Int-P

Tetrakis(acetonitrile)copper(I) hexafluorophosphate (71.8 mg, 0.193 mmol) and **compound Int-L1-3** (155 mg, 0.385 mmol) were sequentially added to a solution of **compound Int-P-5** (TFA salt, 143 mg, 0.064 mmol) in N-methylpyrrolidone (1.0 mL). The reaction was stirred at room temperature for 20 minutes. The reaction solution was purified by preparative high-performance liquid chromatography (Waters XBridge, 19*150 mm, 5 µm; mobile phase A: 0.05% aqueous TFA solution, mobile phase B: acetonitrile, gradient ratio: phase B 5% to 95%) to obtain 80 mg of **compound Int-P** TFA salt, yield: 48.2%.

¹H NMR (400 MHz, D₂O): *δ* 7.98 (s, 1H), 7.89-7.83 (m, 1H), 7.01-6.97 (m, 1H), 5.88 (d, 3H), 4.99-4.84 (m, 3H), 4.62-4.32 (m, 14H), 4.15-3.99 (m, 7H), 3.89-3.74 (m, 10H), 3.65-3.40(m, 50H), 2.93-2.82 (m, 9H), 1.89 (s, 9H).

MS m/z (ESI): 671.8 [M/3+H]⁺.

### Example 24 Synthesis of Intermediate Int-I1

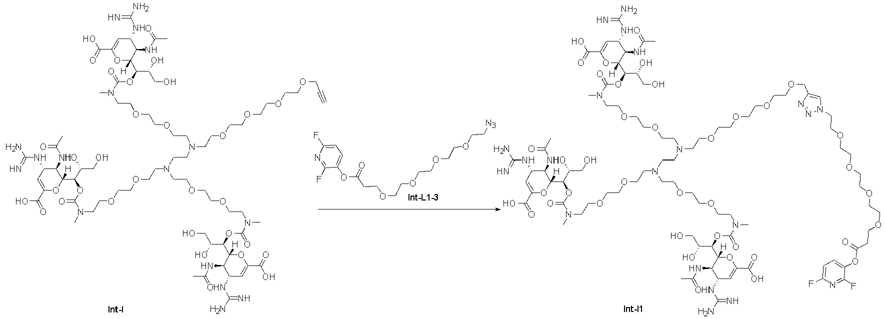

Tetrakis(acetonitrile)copper(I) hexafluorophosphate (83 mg, 0.222 mmol) and **compound Int-L1-3** (179 mg, 0.444 mmol) were sequentially added to a solution of **compound Int-I** (TFA salt, 182 mg, 0.0741 mmol) in N-methylpyrrolidone (1.5 mL). The reaction was stirred at room temperature for 20 minutes. The reaction solution was purified by preparative high-performance liquid chromatography (Waters XBridge, 19*150 mm, 5 µm; mobile phase A: 0.05% aqueous TFA solution, mobile phase B: acetonitrile, gradient ratio: phase B 5% to 95%) to obtain 70 mg of **compound Int-I1** TFA salt, yield: 34.2%.

¹H NMR (400 MHz, D₂O): *δ* 7.99 (s, 1H), 7.89-7.83 (m, 1H), 7.01-6.98 (m, 1H), 5.80 (d, 3H), 4.90-4.86 (m, 3H), 4.59-4.34 (m, 10H), 4.14-4.05 (m, 6H), 3.88-3.74 (m, 10H), 3.63-3.41 (m, 64H), 3.21-3.09 (m, 3H), 2.93-2.85 (m, 6H), 2.81 (s, 6H), 1.89 (s, 9H).

MS m/z (ESI): 730.9 [M/3+H]⁺.

### Example 25 Synthesis of Intermediate Int-G1

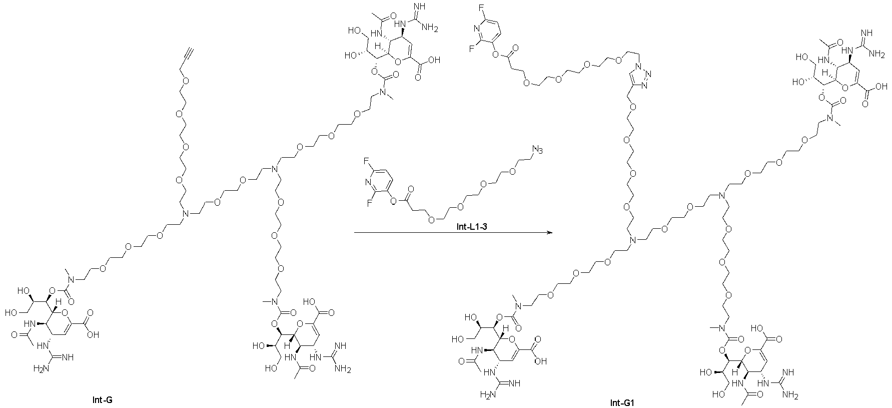

Tetrakis(acetonitrile)copper(I) hexafluorophosphate (109 mg, 0.293 mmol) and **compound 1-3** (237 mg, 0.586 mmol) were sequentially added to a solution of **compound Int-G** (TFA salt, 240 mg, 0.089 mmol) in N-methylpyrrolidone (1.0 mL). The reaction was stirred at room temperature for 20 minutes. The reaction solution was purified by preparative high-performance liquid chromatography (Waters XBridge, 19*150 mm, 5 µm; mobile phase A: 0.05% aqueous TFA solution, mobile phase B: acetonitrile, gradient ratio: phase B 5% to 95%) to obtain 100 mg of **compound Int-G1** TFA salt, yield: 37.1%.

¹H NMR (400 MHz, D₂O): *δ* 7.99 (s, 1H), 7.89-7.83 (m, 1H), 7.01-6.99 (m, 1H), 5.84 (d, 3H), 4.91-4.87 (m, 3H), 4.61-4.47 (m, 7H), 4.38-4.35 (m, 3H), 4.14-3.98 (m, 7H), 3.88-3.78 (m, 16H), 3.64-3.41 (m, 76H), 3.21-3.11 (m, 4H), 2.93-2.83 (m, 6H), 2.81 (s, 6H), 1.89 (s, 9H).

MS m/z (ESI): 804.0 [M/3+H]⁺.

### Example 26 Synthesis of Intermediate Int-D1

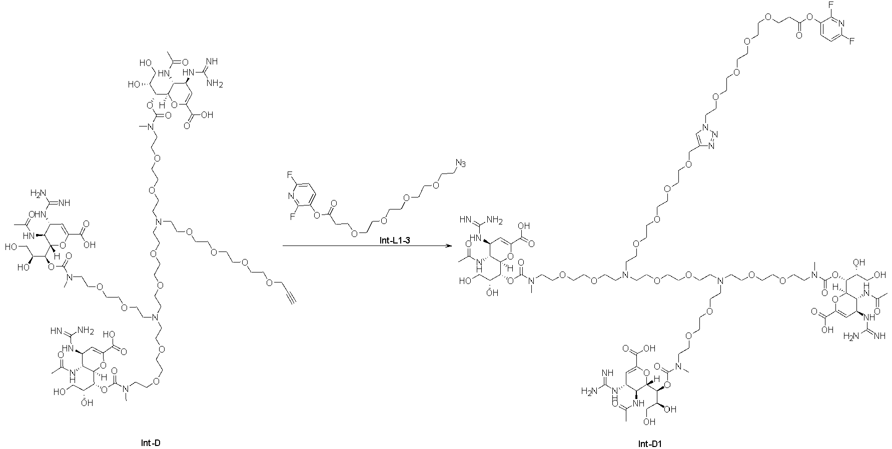

Tetrakis(acetonitrile)copper(I) hexafluorophosphate (156 mg, 0.419 mmol) and **compound Int-L1-3** (339 mg, 0.838 mmol) were sequentially added to a solution of **compound Int-D** (TFA salt, 260 mg, 0.139 mmol) in N-methylpyrrolidone (2.5 mL). The reaction was stirred at room temperature for 20 minutes. The reaction solution was purified by preparative high-performance liquid chromatography (Waters XBridge, 19*150 mm, 5 µm; mobile phase A: 0.05% aqueous TFA solution, mobile phase B: acetonitrile, gradient ratio: phase B 5% to 95%) to obtain 210 mg of **compound Int-D1** TFA salt, yield: 53.1%.

¹H NMR (400 MHz, D₂O): *δ* 7.99 (s, 1H), 7.89-7.83 (m, 1H), 7.01-6.98 (m, 1H), 5.91 (d, 3H), 4.91-4.87 (m, 3H), 4.59-4.48 (m, 6H), 4.37-4.36 (m, 3H), 4.13-3.97 (m, 6H), 3.88-3.78 (m, 12H), 3.64-3.41 (m, 70H), 3.20-3.09 (m, 4H), 2.93-2.87 (m, 6H), 2.80 (s, 6H), 1.88 (s, 9H).

MS m/z (ESI): 759.9 [M/3+H]⁺.

### Example 27 Preparation of Conjugate 1-I-E2

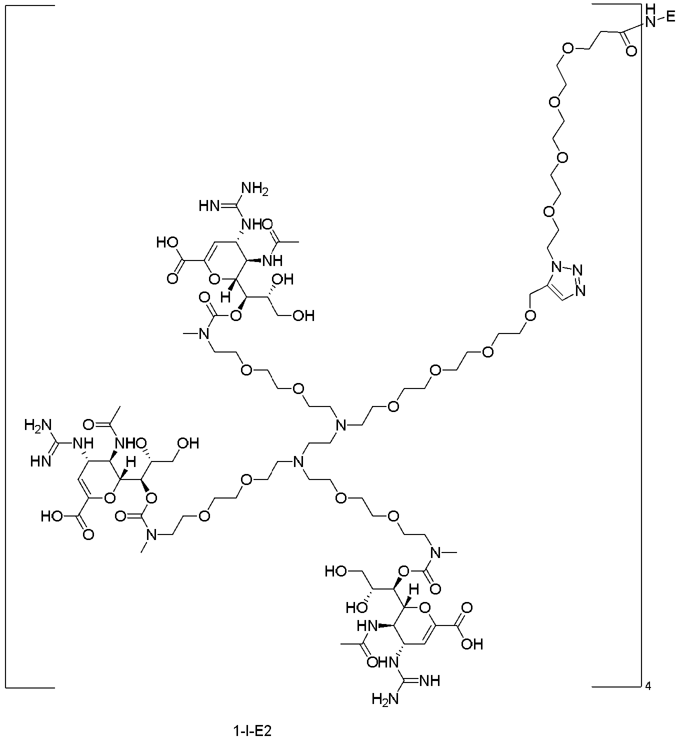

Preparation of 20 mM NaOAc/HOAc pH=5.0: NaOAc (3.28 g) was dissolved in water for injection (1.8 L). The pH was adjusted to 5.0 with concentrated HOAc. The volume was adjusted to 2.0 L. The solution was filtered using a 20 µm membrane filtration system.

Preparation of 1.0 M NaHCO₃/Na₂CO₃ pH 9.5: Phase A: 50 mL of 1.0 M NaHCO₃ (4.2 g); Phase B: 50 mL of 1.0 M Na₂CO₃ (5.3 g). Phase B was added to Phase A to adjust the pH to 9.5 for later use.

Steps: Protein E2 (3.0 mg, 0.05 µmol, 103.5 µL, whose sequence is as set forth in SEQ ID NO: 8) and 20 mM NaOAc/HOAc pH=5.0 buffer (46.5 µL) were added into a 2 mL reaction tube. The pH was adjusted to between 9.0 and 9.5 (approximately pH=9.23) using 1.0 M NaHCO₃/Na₂CO₃ pH=9.5 (6 µL). A solution of **Int-I1** (593.03 µg, 0.27 µmol, 5 eq) in DMSO (9 µL) was added (pH=8.83), and the pH was adjusted to between 9.0 and 9.5 (approximately pH=9.21) using 1.0 M NaHCO₃/Na₂CO₃ pH=9.5 (0.6 µL). The solution was shaken at 25°C for 1 hour. At intervals of 1 h, additional **Int-I1** was added in amounts of 2, 2, and 1 eq, respectively, and the pH was adjusted to between 9.0 and 9.5 (pH=9.35) using 1.0 M NaHCO₃/Na₂CO₃ pH=9.5 (0.5 µL), followed by shaking at 25°C for 1 hour; a total of 3 supplementary additions were made. After the total amount of small molecules reached 10 eq, ultrafiltration was performed 4 times with a PBS 5.0 buffer to obtain conjugate product 1-I-E2 (2.92 mg, yield 97.4%, purity 98.3%). By mass spectrometry analysis, the DAR was 4.36.

### Example 28 Preparation of Conjugate 1-M-E2

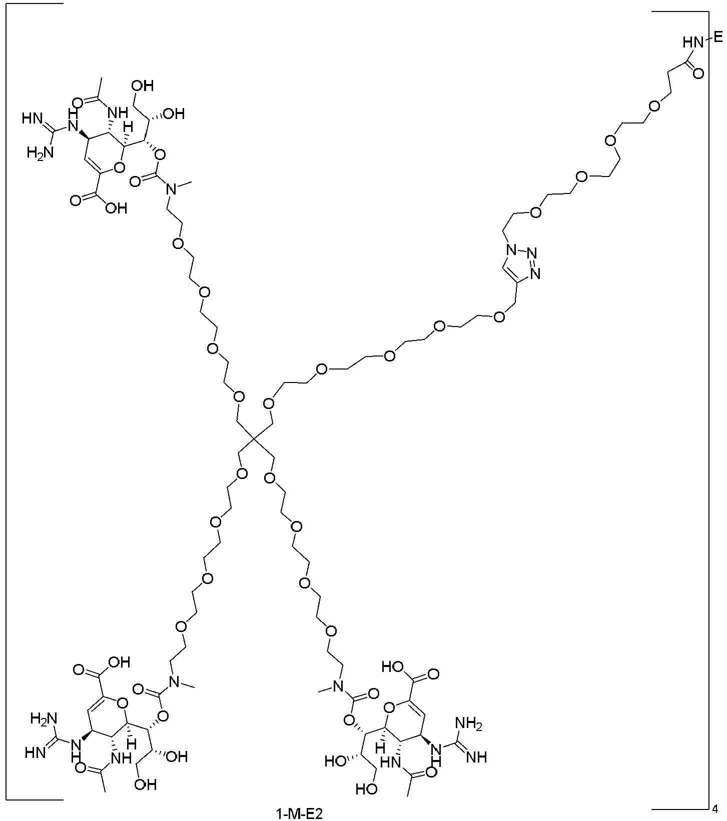

Steps: It was prepared using the same method as in Example 27. At intervals of 1 h, additional Int-M was added in amounts of 1, 1, and 1 eq, respectively. A total of 3 supplementary additions were made, and the total amount of small molecules was 8 eq. After ultrafiltration, conjugate product **1-M-E2** (2.51 mg, yield 83.7%, purity 98.4%) was obtained. By mass spectrometry analysis, the DAR was 4.48.

### Example 29 Preparation of Conjugate 1-G-E2

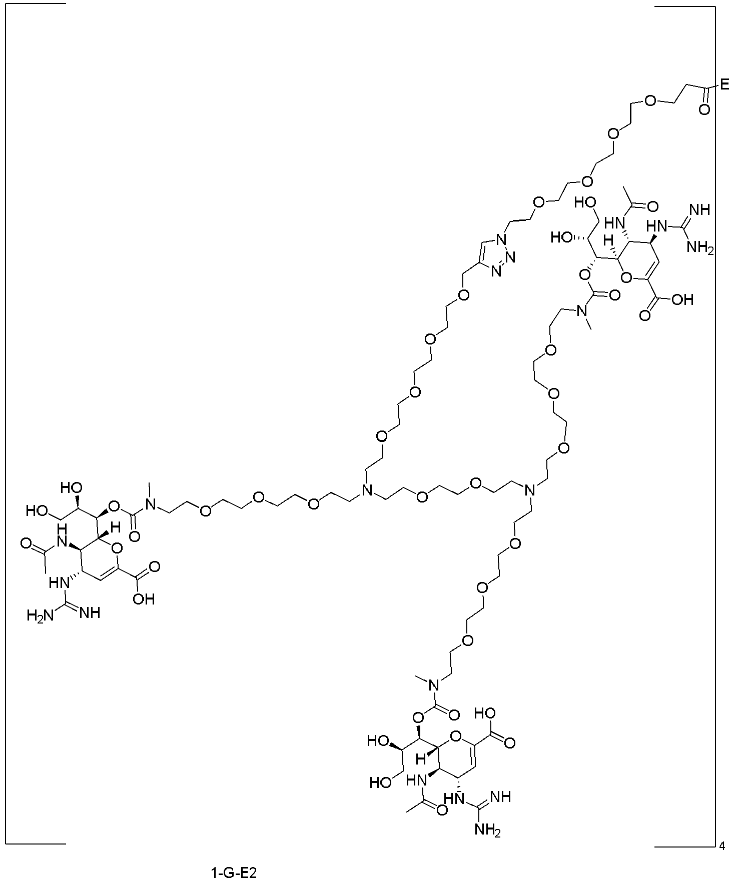

Steps: It was prepared using the same method as in Example 27. At intervals of 1 h, additional **Int-G1** was added in amounts of 2 and 1 eq, respectively. A total of 2 supplementary additions were made, and the total amount of small molecules was 8 eq. After ultrafiltration, conjugate product **1-G-E2** (2.98 mg, yield 99.5%, purity 98.6%) was obtained. By mass spectrometry analysis, the DAR was 4.14.

### Example 30 Preparation of Conjugate 1-P-E2

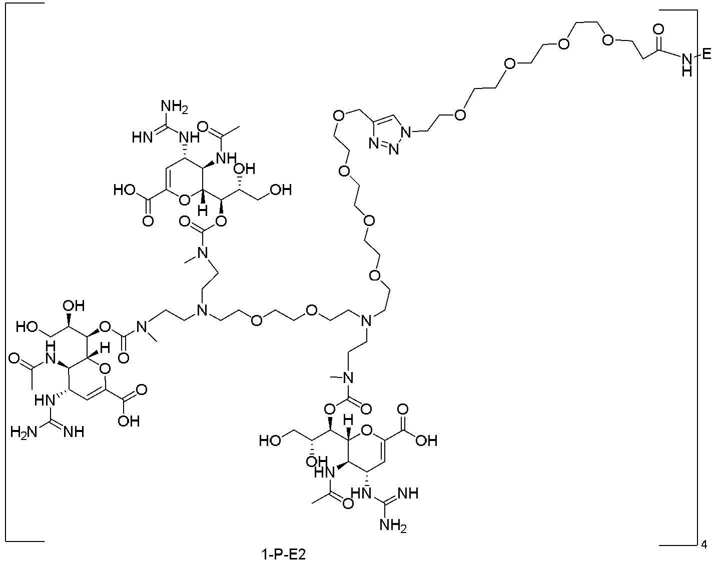

Steps: It was prepared using the same method as in Example 27. At intervals of 1 h, additional **Int-P** was added in amounts of 2, 2, and 1 eq, respectively. A total of 3 supplementary additions were made, and the total amount of small molecules was 10 eq. After ultrafiltration, conjugate product **1-P-E2** (2.95 mg, yield 98.4%, purity 98.4%) was obtained. By mass spectrometry analysis, the DAR was 4.54.

### Example 31 Preparation of Conjugate 1-L-E1

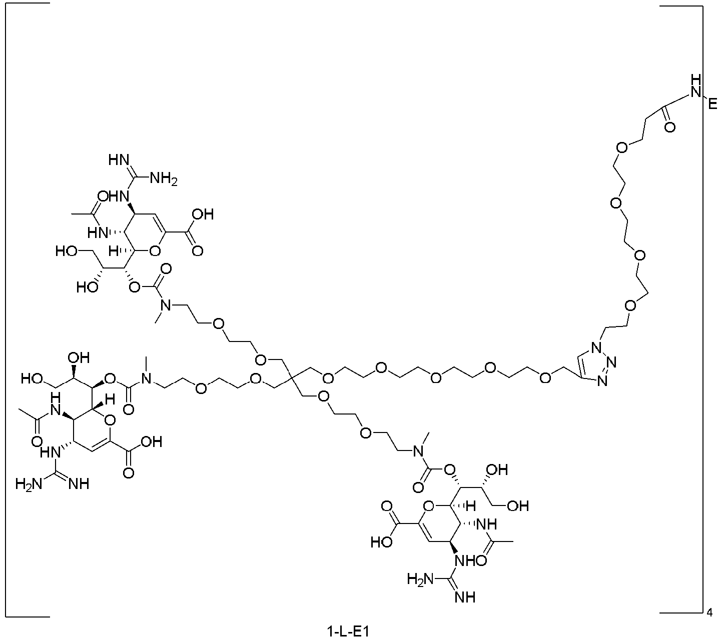

Steps: It was prepared using the same method as in Example 27, and the protein Fc sequence is as set forth in SEQ ID NO: 1. At intervals of 1 h, additional **Int-L1** was added in amounts of 1, 1, and 1 eq, respectively. A total of 3 supplementary additions were made, and the total amount of small molecules was 8 eq. After ultrafiltration, conjugate product **1-L-EI** (2.88 mg, yield 96.1%, purity 99.3%) was obtained. By mass spectrometry analysis, the DAR was 4.46.

### Example 32 Preparation of Conjugate 1-L-E3

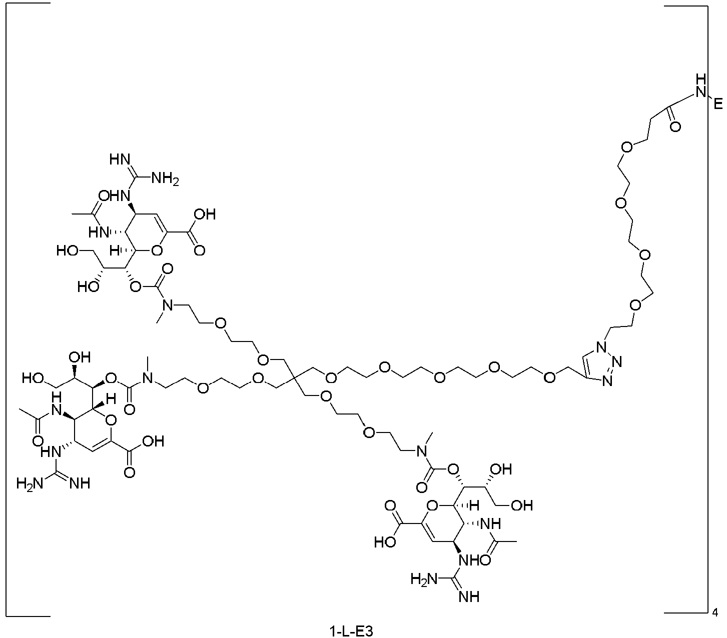

Steps: It was prepared using the same method as in Example 27, and the protein used was hIgG1 (its heavy chain sequence is as set forth in SEQ ID NO: 9, and the light chain sequence is as set forth in SEQ ID NO: 10). At intervals of 1 h, additional **Int-L1** was added in amounts of 2, 2, and 1 eq, respectively. A total of 3 supplementary additions were made, and the total amount of small molecules was 10 eq. After ultrafiltration, conjugate product **1-L-E3** (2.86 mg, yield 95.6%, purity 98.7%) was obtained. By mass spectrometry analysis, the DAR was 4.59.

### Example 33 Preparation of Conjugate 1-L-E4

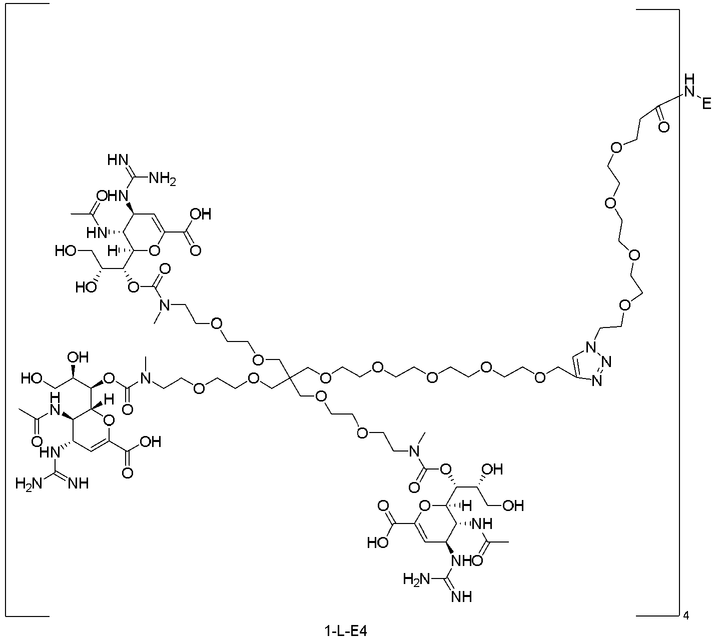

Steps: It was prepared using the same method as in Example 27, and the protein used was human serum albumin (MCE, Cat. No. HY-P1956-100mg). At intervals of 1 h, additional **Int-L1** was added in amounts of 2 and 2 eq, respectively. A total of 2 supplementary additions were made, and the total amount of small molecules was 9 eq. After ultrafiltration, the purity was 80.1%. The solution was purified by a HiLoad Superdex 200 pg preparative SEC chromatography column (mobile phase: 1×PBS 5.0) to obtain conjugate product **1-L-E4** (0.97 mg, yield 32.3%, purity 95.6%). By mass spectrometry analysis, the DAR was 4.53.

### Example 34 Preparation of Conjugate 1-O-E2

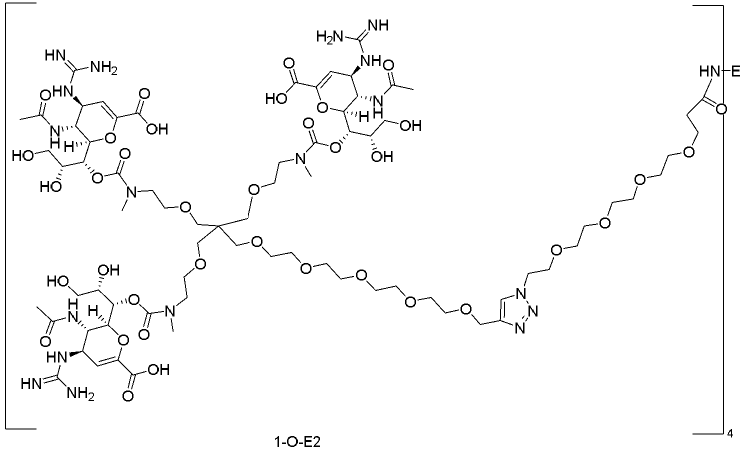

Steps: It was prepared using the same method as in Example 27. At intervals of 1 h, additional Int-O was added in amounts of 2 and 2 eq, respectively. A total of 2 supplementary additions were made, and the total amount of small molecules was 9 eq. After ultrafiltration, conjugate product **1-O-E2** (2.65 mg, yield 88.6%, purity 98.3%) was obtained. By mass spectrometry analysis, the DAR was 4.46.

### Example 35 Preparation of Conjugate 1-N-E2

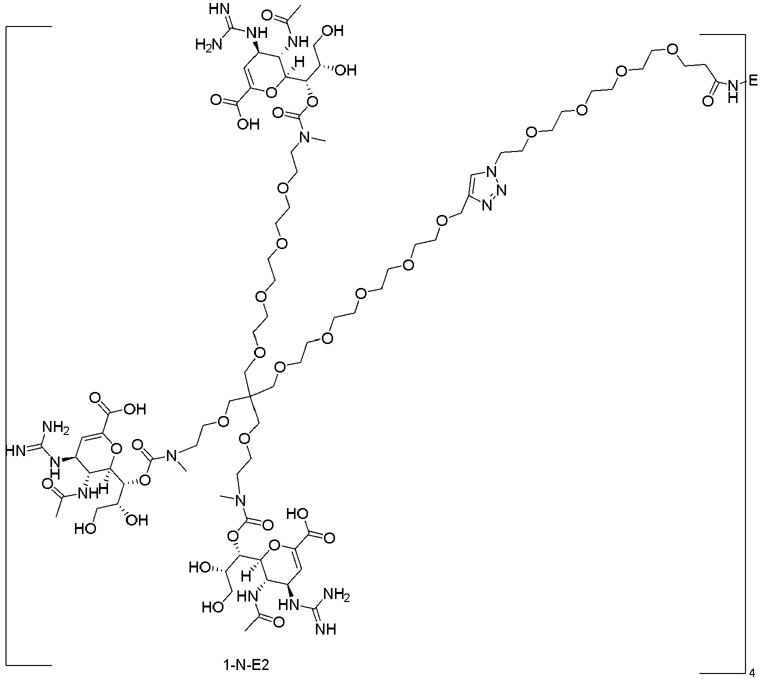

Steps: It was prepared using the same method as in Example 27. At intervals of 1 h, additional **Int-N** was added in an amount of 2 eq. A total of 1 supplementary addition was made, and the total amount of small molecules was 7 eq. After ultrafiltration, conjugate product **1-N-E2** (2.94 mg, yield 98.2%, purity 98.6%) was obtained. By mass spectrometry analysis, the DAR was 4.28.

### Example 36 Preparation of Conjugate 1-L-E2

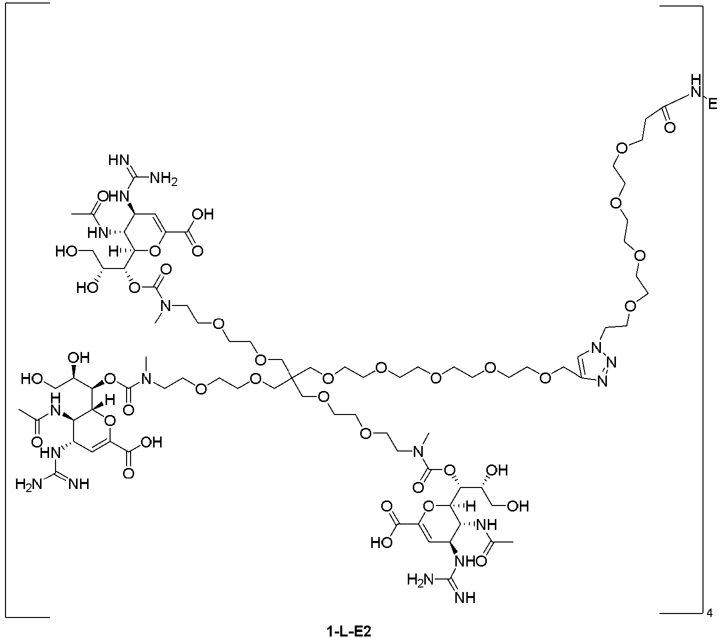

Steps: It was prepared using the same method as in Example 27. At intervals of 1 h, additional **Int-L1** was added in amounts of 1 and 1 eq, respectively. A total of 2 supplementary additions were made, and the total amount of small molecules was 7 eq. After ultrafiltration, conjugate product **1-L-E2** (2.40 mg, yield 80.6%, purity 98.8%) was obtained. By mass spectrometry analysis, the DAR was 4.39.

### Example 37 Preparation of Conjugate 1-D-E2

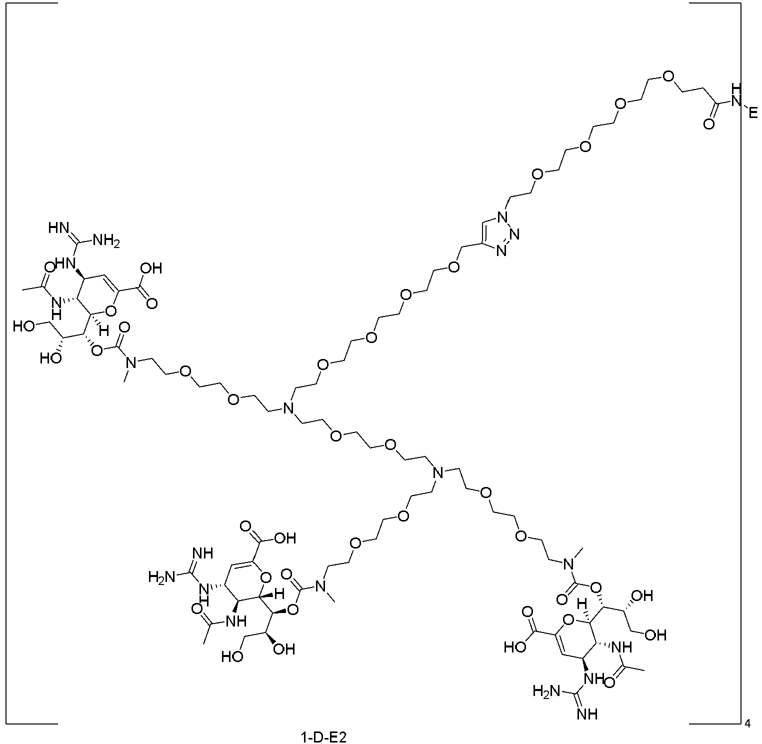

Steps: It was prepared using the same method as in Example 27. At intervals of 1 h, additional **Int-D1** was added in amounts of 1 and 1 eq, respectively. A total of 2 supplementary additions were made, and the total amount of small molecules was 7 eq. After ultrafiltration, conjugate product **1-D-E2** (2.77 mg, yield 92.3%, purity 98.8%) was obtained. By mass spectrometry analysis, the DAR was 4.28.

### Example 38 Preparation of Conjugate 1-L-E2

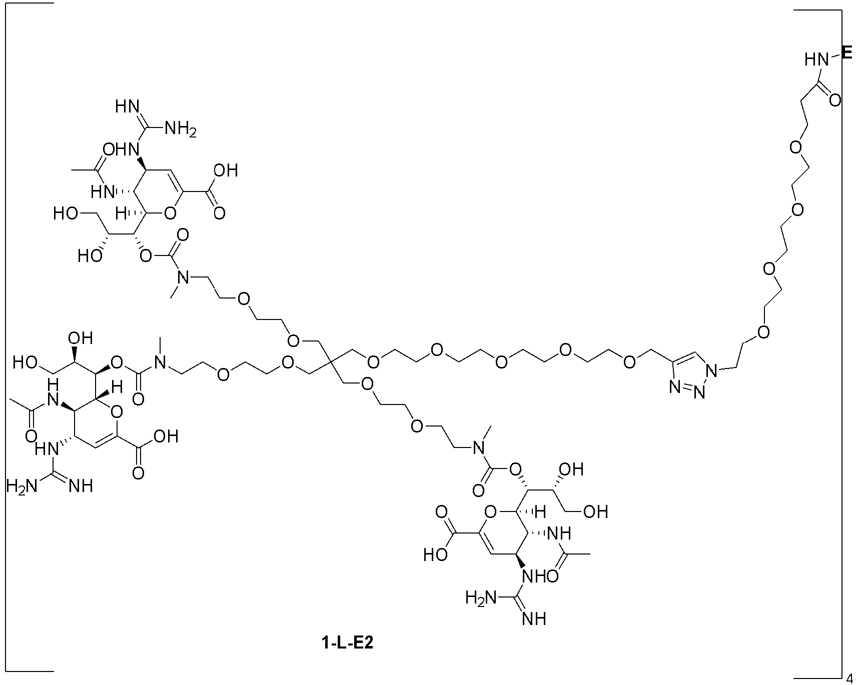

Step 1: Fc-azido was prepared using the same method as in Example 13 (7.66 mg, yield 76.6%, purity 99.3%, and the Fc sequence is as set forth in SEQ ID NO: 8).

Step 2: Fc-azido (7.66 mg), a solution of compound **Int-L** (3.61 mg, 15.0 eq) in 1×PBS pH=6.3 (277.7 µL), and a click reagent (428 µL) were added into a 15 mL reaction tube, and shaken at room temperature for 20 hours. After desalting and purification with PBS (pH=5.0), the solution was purified by a HiLoad Superdex 200 pg preparative SEC chromatography column (mobile phase: 1×PBS 5.0), and the collected liquid was concentrated to obtain conjugate **1-L-E2** (5.26 mg, yield 68.7%, purity 98.0%). By Maldi tof analysis, the DAR value was 4.25.

### Biological Evaluation

The present disclosure is further described and explained below in conjunction with test examples, but these examples are not intended to limit the scope of the present disclosure.

### Test Example 1: Cell-Level Assay for Cytotoxicity and Anti-Influenza Virus Activity of Conjugate Molecules

### 1. Test Articles

Test compounds (Conjugate 1-D-E1, Conjugate 1-L-E1): The initial test concentration was 1 µM for both, and 9 concentrations were obtained by diluting with a 3.16-fold gradient.

Reference drug 1: Zanamivir (purchased from Shanghai Bide, lot number 139110-90-8). The initial test concentration was 100 µM, and 9 concentrations were obtained by diluting with a 3.16-fold gradient.

Reference drug 2: Conjugate C3-E1 (prepared according to the method of Example 13, wherein the intermediate control compound used had a structure shown as below, prepared with reference to the synthesis method for Int-83 in Example 145 in WO2021046549A1). The initial test concentration was 1 µM, and 9 concentrations were obtained by diluting with a 3.16-fold gradient.

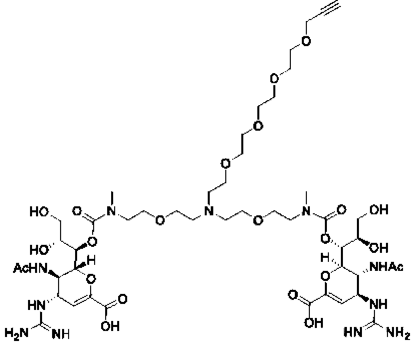

### 2. Experimental Instruments and Materials

| **Reagent name** | **Manufacturer** | **Item number** |
|---|---|---|
| MDCK cells | ATCC | CCL-34 |
| Influenza H1N1 A/PR/8/34 | ATCC | VR-95 |
| FBS | Gibco | 10091-141C |
| Penicillin-Streptomycin | Gibco | 15140-122 |
| DMEM | Gibco | 11995-065 |
| Trypsin | Sigma | T8802-100MG |
| OptiPRO^{™} SFM | Gibco | 12309-019 |
| PBS | BasalMedia | B310KJ |
| 0.25%Trypsin-EDTA (1x) | Gibco | 25200-056 |
| Cell Counting Kit-8 (CCK-8) | Dojindo | CK04 |
| T75 flask | Greiner | 658175 |
| 50mL conical tubes | Greiner | 227261 |
| 5mL pipette | Sanifico | SEP05005 |
| 10mL pipette | Sanifico | SEP05010 |
| 25mL pipette | Sanifico | SEP02525 |
| 96 well cell culture plate, Flat | Corning | 3599 |
| 1000ul pipette tips | Rainin | 30389211 |
| 250ul pipette tips | Rainin | 30859285 |
| 20ul pipette tips | Rainin | 30389200 |

### 3. Test Methods

### 1) Preparation of test reagents:

MDCK cell culture medium: DMEM + 10% FBS + 1% Penicillin-Streptomycin
2 g/ml Trypsin: 10 g of Trypsin powder was dissolved in 5 ml of DMEM culture medium
Pre-infection cell culture medium: OptiPRO^{™} SFM + 1% Penicillin-Streptomycin
Infection cell culture medium: OptiPRO^{™} SFM + 2 µg/ml Trypsin

### 2) Determination method:

The cultured MDCK cells were resuspended in an OptiPRO^{™} SFM culture medium, seeded into a 96-well plate at 5×10³ cells per 100 µl of OptiPRO^{™} SFM + 1% Penicillin-Streptomycin per well, and cultured overnight in a 37°C incubator (cell incubator model: ESCO CCL-170B-8). On the second day, the test compounds and reference drugs were diluted to specified concentrations in OptiPRO^{™} SFM + 2 µg/ml Trypsin, and added to the wells pre-seeded with cells at a volume of 50 µl per well. After incubation at 37°C for 2 hours, the influenza virus (Influenza H1N1 A/PR/8/34) was diluted in OptiPRO^{™} SFM + 2 µg/ml Trypsin and added to the wells containing the cells and compounds at a volume of 50 µl per well. The prepared 96-well plate was incubated at 37°C for 5 days. After 5 days, 20 µl of a CCK-8 solution was added to each well. After incubating in a 37°C incubator for 2 hours, the absorbance was measured at 450 nm.

### 4. Test Results

**Table 1 EC₅₀ and CC₅₀ of conjugate/compound at the MDCK cell level**

| Conjugate/compound | EC₅₀/µM | CC₅₀/µM |
|---|---|---|
| Zanamivir | 3.528 | >100 |
| Conjugate C3-E1 | 0.01709 | >1 |
| Conjugate 1-L-E1 | 0.001585 | >1 |
| Conjugate 1-D-E1 | 0.002081 | >1 |

### Test Example 2: Cell-Level Assay for Anti-Influenza H1N1 A/PR/8/34 Virus Activity of Conjugate Molecules

### 1. Test Articles

Test compounds (Conjugate 1-D-E2, Conjugate 1-L-E2): The initial test concentration was 1 µM for both, and 9 concentrations were obtained by diluting with a 3.16-fold gradient.

Reference drug 1: Zanamivir (purchased from Shanghai Bide, lot number 139110-90-8). The initial test concentration was 100 µM, and 9 concentrations were obtained by diluting with a 3.16-fold gradient.

Reference drug 2: Conjugate C3-E1. The initial test concentration was 1 µM, and 9 concentrations were obtained by diluting with a 3.16-fold gradient.

### 2. Experimental Instruments and Materials

| **Reagent name** | **Manufacturer** | **Item number** |
|---|---|---|
| MDCK cells | ATCC | CCL-34 |
| FBS | Gibco | 10091-141C |
| Penicillin-Streptomycin | Gibco | 15140-122 |
| DMEM | Gibco | 11995-065 |
| Trypsin | Sigma | T8802-100MG |
| OptiPRO^{™} SFM | Gibco | 12309-019 |
| PBS | BasalMedia | B310KJ |
| 0.25%Trypsin-EDTA (1x) | Gibco | 25200-056 |
| Cell Counting Kit-8 (CCK-8) | Dojindo | CK04 |
| T75 flask | Greiner | 658175 |
| 50mL conical tubes | Greiner | 227261 |
| 5mL pipette | Sanifico | SEP05005 |
| 10mL pipette | Sanifico | SEP05010 |
| 25mL pipette | Sanifico | SEP02525 |
| 96 well cell culture plate, Flat | Corning | 3599 |
| 1000ul pipette tips | Rainin | 30389211 |
| 250ul pipette tips | Rainin | 30859285 |
| 20ul pipette tips | Rainin | 30389200 |

### 3. Test Methods

### 1) Preparation of test reagents:

MDCK cell culture medium: DMEM + 10% FBS + 1% Penicillin-Streptomycin
2 g/ml Trypsin: 10 g of Trypsin powder was dissolved in 5 ml of DMEM culture medium
Pre-infection cell culture medium: OptiPRO^{™} SFM + 1% Penicillin-Streptomycin
Infection cell culture medium: OptiPRO^{™} SFM + 2 µg/ml Trypsin

### 2) Determination method:

The cultured MDCK cells were resuspended in an OptiPRO^{™} SFM culture medium, seeded into a 96-well plate at 5×10³ cells per 100 µl of OptiPRO^{™} SFM + 1% Penicillin-Streptomycin per well, and cultured overnight in a 37°C incubator (cell incubator model: ESCO CCL-170B-8). On the second day, the test compounds and reference drugs were diluted to specified concentrations in OptiPRO^{™} SFM + 2 µg/ml Trypsin, and added to the wells pre-seeded with cells at a volume of 50 µl per well. After incubation at 37°C for 2 hours, the influenza virus (Influenza H1N1 A/PR/8/34) was diluted in OptiPRO^{™} SFM + 2 µg/ml Trypsin and added to the wells containing the cells and compounds at a volume of 50 µl per well. The prepared 96-well plate was incubated at 37°C for 5 days. After 5 days, 20 µl of a CCK-8 solution was added to each well. After incubating in a 37°C incubator for 2 hours, the absorbance was measured at 450 nm.

### 4. Test Results

**Table 2 EC₅₀ of conjugate/compound against Influenza HIN1 A/PR/8/34 virus**

| Conjugate/compound | EC₅₀/nM |
|---|---|
| Zanamivir | 12140 |
| Conjugate C3-E1 | 8.112 |
| Conjugate 1-L-E2 | 1.063 |
| Conjugate 1-D-E2 | 1.191 |

### Test Example 3: Cell-Level Assay for Anti-Influenza B/Lee Virus Activity of Conjugate Molecules

### 1. Test Articles

Test compounds (Conjugate 1-D-E2, Conjugate 1-L-E2): The initial test concentration was 1 µM for both, and 9 concentrations were obtained by diluting with a 3.16-fold gradient.

Reference drug: Zanamivir (purchased from Shanghai Bide, lot number 139110-90-8). The initial test concentration was 100 µM, and 9 concentrations were obtained by diluting with a 3.16-fold gradient.

### 2. Experimental Instruments and Materials

| **Reagent name** | **Manufacturer** | **Item number** |
|---|---|---|
| MDCK cells | ATCC | CCL-34 |
| FBS | Gibco | 10091-141C |
| Penicillin-Streptomycin | Gibco | 15140-122 |
| DMEM | Gibco | 11995-065 |
| Trypsin | Sigma | T8802-100MG |
| OptiPRO^{™} SFM | Gibco | 12309-019 |
| PBS | BasalMedia | B310KJ |
| 0.25%Trypsin-EDTA (1x) | Gibco | 25200-056 |
| Cell Counting Kit-8 (CCK-8) | Dojindo | CK04 |
| T75 flask | Greiner | 658175 |
| 50mL conical tubes | Greiner | 227261 |
| 5mL pipette | Sanifico | SEP05005 |
| 10mL pipette | Sanifico | SEP05010 |
| 25mL pipette | Sanifico | SEP02525 |
| 96 well cell culture plate, Flat | Corning | 3599 |
| 1000ul pipette tips | Rainin | 30389211 |
| 250ul pipette tips | Rainin | 30859285 |
| 20ul pipette tips | Rainin | 30389200 |

### 3. Test Methods

### 1) Preparation of test reagents:

MDCK cell culture medium: DMEM + 10% FBS + 1% Penicillin-Streptomycin
2 g/ml Trypsin: 10 g of Trypsin powder was dissolved in 5 ml of DMEM culture medium
Pre-infection cell culture medium: OptiPRO^{™} SFM + 1% Penicillin-Streptomycin
Infection cell culture medium: OptiPRO^{™} SFM + 2 µg/ml Trypsin

### 2) Determination method:

The cultured MDCK cells were resuspended in an OptiPRO^{™} SFM culture medium, seeded into a 96-well plate at 5×10³ cells per 100 µl of OptiPRO^{™} SFM + 1% Penicillin-Streptomycin per well, and cultured overnight in a 37°C incubator (cell incubator model: ESCO CCL-170B-8). On the second day, the test compounds and reference drugs were diluted to specified concentrations in OptiPRO^{™} SFM + 2 µg/ml Trypsin, and added to the wells pre-seeded with cells at a volume of 50 µl per well. After incubation at 37°C for 2 hours, the influenza virus (Influenza B/Lee) was diluted in OptiPRO^{™} SFM + 2 µg/ml Trypsin and added to the wells containing the cells and compounds at a volume of 50 µl per well. The prepared 96-well plate was incubated at 37°C for 5 days. After 5 days, 20 µl of a CCK-8 solution was added to each well. After incubating in a 37°C incubator for 2 hours, the absorbance was measured at 450 nm.

### 4. Test Results

**Table 3 EC₅₀ of conjugate/compound against Influenza B/Lee virus**

| Conjugate/compound | EC₅₀/nM |
|---|---|
| Zanamivir | 2383 |
| Conjugate 1-L-E2 | 0.225 |
| Conjugate 1-D-E2 | 0.170 |

### Test Example 4: Cell-Level Assay for Anti-Influenza H1N1 A/PR/8/34 Virus Activity of Conjugate Molecules

### 1. Test Articles

Test compounds (Conjugate 1-L-E2, Conjugate 1-L-E3, Conjugate 1-L-E4): The initial test concentration was 10 µM for all, and 12 concentrations were obtained by diluting with a 3- or 2-fold gradient.

Reference drug 1: Zanamivir (purchased from Shanghai Bide, lot number 139110-90-8). The initial test concentration was 300 µM, and 12 concentrations were obtained by diluting with a 3- or 2-fold gradient.

### 2. Experimental Instruments and Materials

| **Reagent name** | **Manufacturer** | **Item number** |
|---|---|---|
| MDCK cells | ATCC | CCL-34 |
| FBS | Gibco | 10091-141C |
| Penicillin-Streptomycin | Gibco | 15140-122 |
| DMEM | Gibco | 11995-065 |
| Trypsin | Sigma | T8802-100MG |
| OptiPRO^{™} SFM | Gibco | 12309-019 |
| PBS | BasalMedia | B310KJ |
| 0.25%Trypsin-EDTA (1x) | Gibco | 25200-056 |
| CellCounting-Lite 2.0 Luminescent Cell Viability Assay (CCL) | Vazyme | DD1101-01 |
| T75 flask | Greiner | 658175 |
| 50mL conical tubes | Greiner | 227261 |
| 5mL pipette | Sanifico | SEP05005 |
| 10mL pipette | Sanifico | SEP05010 |
| 25mL pipette | Sanifico | SEP02525 |
| 96 well cell culture plate, Flat | Corning | 3599 |
| 1000ul pipette tips | Rainin | 30389211 |
| 250ul pipette tips | Rainin | 30859285 |
| 20ul pipette tips | Rainin | 30389200 |

### 3. Test Methods

### 1) Preparation of test reagents:

MDCK cell culture medium: DMEM + 10% FBS + 1% Penicillin-Streptomycin
2 g/ml Trypsin: 10 g of Trypsin powder was dissolved in 5 ml of DMEM culture medium
Pre-infection cell culture medium: OptiPRO^{™} SFM + 1% Penicillin-Streptomycin
Infection cell culture medium: OptiPRO^{™} SFM + 2 µg/ml Trypsin

### 2) Determination method:

The cultured MDCK cells were resuspended in an OptiPRO^{™} SFM culture medium, seeded into a 96-well plate at 5×10³ cells per 100 µl of OptiPRO^{™} SFM + 1% Penicillin-Streptomycin per well, and cultured overnight in a 37°C incubator (cell incubator model: Haier, HCB-168). On the second day, the test compounds and reference drugs were diluted to specified concentrations in OptiPRO^{™} SFM + 2 µg/ml Trypsin, and added to the wells pre-seeded with cells at a volume of 50 µl per well. After incubation at 37°C for 2 hours, the influenza virus (Influenza H1N1 A/PR/8/34) was diluted in OptiPRO^{™} SFM + 2 µg/ml Trypsin and added to the wells containing the cells and compounds at a volume of 50 µl per well. The prepared 96-well plate was incubated at 37°C, and the cell status was observed every day. When the lesion reached 80% or more, cell viability was determined using a CellCounting-Lite 2.0 Luminescent Cell Viability Assay kit in a microplate reader, and the inhibition rate was calculated.

### 4. Test Results

**Table 4 EC₅₀ of conjugate/compound against Influenza H1N1 A/PR/8/34 virus**

| Conjugate/compound | EC₅₀/nM |
|---|---|
| Zanamivir | 1836 |
| Conjugate 1-L-E2 | 1.76 |
| Conjugate 1-L-E3 | 1.20 |
| Conjugate 1-L-E4 | 1.76 |

### Test Example 5: Cell-Level Assay for Anti-Influenza H1N1 A/PR/8/34 Virus Activity of Conjugate Molecules

### 1. Test Articles

Test compounds (Conjugate 1-M-E2, Conjugate 1-N-E2, Conjugate 1-O-E2): The initial test concentration was 10 µM for all, and 12 concentrations were obtained by diluting with a 3- or 2-fold gradient.

Reference drug 1: Zanamivir (purchased from Shanghai Bide, lot number 139110-90-8). The initial test concentration was 300 µM, and 12 concentrations were obtained by diluting with a 3- or 2-fold gradient.

### 2. Experimental Instruments and Materials

| **Reagent name** | **Manufacturer** | **Item number** |
|---|---|---|
| MDCK cells | ATCC | CCL-34 |
| FBS | Gibco | 10091-141C |
| Penicillin-Streptomycin | Gibco | 15140-122 |
| DMEM | Gibco | 11995-065 |
| Trypsin | Sigma | T8802-100MG |
| OptiPRO^{™} SFM | Gibco | 12309-019 |
| PBS | BasalMedia | B310KJ |
| 0.25%Trypsin-EDTA (1x) | Gibco | 25200-056 |
| CellCounting-Lite 2.0 Luminescent Cell Viability Assay (CCL) | Vazyme | DD1101-01 |
| T75 flask | Greiner | 658175 |
| 50mL conical tubes | Greiner | 227261 |
| 5mL pipette | Sanifico | SEP05005 |
| 10mL pipette | Sanifico | SEP05010 |
| 25mL pipette | Sanifico | SEP02525 |
| 96 well cell culture plate, Flat | Corning | 3599 |
| 1000ul pipette tips | Rainin | 30389211 |
| 250ul pipette tips | Rainin | 30859285 |
| 20ul pipette tips | Rainin | 30389200 |

### 3. Test Methods

### 1) Preparation of test reagents:

MDCK cell culture medium: DMEM + 10% FBS + 1% Penicillin-Streptomycin
2 g/ml Trypsin: 10 g of Trypsin powder was dissolved in 5 ml of DMEM culture medium
Pre-infection cell culture medium: OptiPRO^{™} SFM + 1% Penicillin-Streptomycin
Infection cell culture medium: OptiPRO^{™} SFM + 2 µg/ml Trypsin

### 2) Determination method:

The cultured MDCK cells were resuspended in an OptiPRO^{™} SFM culture medium, seeded into a 96-well plate at 5×10³ cells per 100 µl of OptiPRO^{™} SFM + 1% Penicillin-Streptomycin per well, and cultured overnight in a 37°C incubator (cell incubator model: Haier, HCB-168). On the second day, the test compounds and reference drugs were diluted to specified concentrations in OptiPRO^{™} SFM + 2 µg/ml Trypsin, and added to the wells pre-seeded with cells at a volume of 50 µl per well. After incubation at 37°C for 2 hours, the influenza virus (Influenza H1N1 A/PR/8/34) was diluted in OptiPRO^{™} SFM + 2 µg/ml Trypsin and added to the wells containing the cells and compounds at a volume of 50 µl per well. The prepared 96-well plate was incubated at 37°C, and the cell status was observed every day. When the lesion reached 80% or more, cell viability was determined using a CellCounting-Lite 2.0 Luminescent Cell Viability Assay kit in a microplate reader, and the inhibition rate was calculated.

### 4. Test Results

**Table 5 EC₅₀ of conjugate/compound against Influenza H1N1 A/PR/8/34 virus**

| Conjugate/compound | EC₅₀/nM |
|---|---|
| Zanamivir | 1688 |
| Conjugate 1-L-E2 | 1.88 |
| Conjugate 1-M-E2 | 3.86 |
| Conjugate 1-N-E2 | 4.55 |
| Conjugate 1-O-E2 | 4.29 |

### Test Example 6: Pharmacokinetic Study of Different Test Articles Administered to Cynomolgus Monkeys via Single Intravenous Injection

### 1. Test Articles

Reference (Conjugate C3-E1) and test article (Conjugate 1-L-E2). Specific information for each test article is shown in Table 6.

**Table 6. Sample information**

| Name | **Concentration** | Storage condition |
|---|---|---|
| Conjugate C3-E1 | 5 mg/mL | Dry, protected from light, at -80°C |
| Conjugate 1-L-E2 | 5 mg/mL | |

### Preparation method of administration formulations:

1) Vehicle: PBS for injection (Gibco^{®}, lot number: 2436336).
2) Preparation of test articles: Aseptic operations were performed in a biosafety cabinet and/or a clean bench, and the containers used were sterilized. The test articles were taken out from a -80°C freezer and allowed to thaw completely. They were then directly aliquoted according to the concentration of the test articles or diluted with the vehicle to the required administration concentration. The actual administration dose, administration formulation concentration, and administration volume for each group are shown in **Table 7.** The administration formulations for each group of test articles were prepared immediately before use, and administered via intravenous injection in the limbs.

**Table 7. Administration dose, concentration, and volume for each group**

| **Group** | **Administration dose (mg/kg)** | **Administration formulation concentration (mg/mL)** | **Administration volume (mL/kg)** |
|---|---|---|---|
| Conjugate C3-El | 2 | 5 | 0.4 |
| Conjugate 1-L-E2 | 2 | 5 | 0.4 |

### 2. Test Animals

Cynomolgus monkeys, standard grade, two animals per group, half male and half female, aged 5.0 to 6.0 years; animals were from Guangxi Fangchenggang Changchun Biotechnology Development Co., Ltd. and Hainan Jingang Biotechnology Co., Ltd., certificate numbers: 0003029, 460012000000545, 44818300000229. All test animals underwent quarantine and each animal was individually in a cage The breeding temperature was 18°C to 26°C, humidity was 40% to 70%, air exchange rate was ≥8 times per hour, and 100% fresh air was used. The animals underwent a 2-5 day acclimatization period before the test and were observed at the cage once 2-3 days before drug administration. On the day of the test, random grouping was performed according to body weight.

### 3. Test Methods

Before administration, animals were weighed. The administration volume was calculated based on the body weight, and both groups were administered by intravenous injection at the same dose. Before administration and at 0.25 h, 2 h, 6 h, 24 h (D1), 72 h (D3), 120 h (D5), 168 h (D7), 240 h (D10), 336 h (D14), and 672 h (D28) after administration, 1 mL/animal/time point of blood was collected via intravenous veins of the limbs. Whole blood was collected into test tubes containing coagulant and separation gel coagulant, and allowed to stand at room temperature for about 30 min before centrifugation. After blood collection, the samples were centrifuged at 4° C (2000 g, 10 min) within 1 hour of agglutination. The collected serum was stored at -80°C. The drug concentration was detected by ELISA: Goat anti-human IgG Monkey ads UNLB antibody (Southern Biotech, Cat No. 2049-01) at a concentration of 1 µg/ml and a volume of 100 µL/well was added to a 96-well microplate (Corning, Cat No. 9018), and incubated at 4°C overnight to coat the microplate. After discarding the liquid, the plate was washed 5 times with a PBST (pH 7.4, 0.05% Tween-20) buffer. 200 µL of 4% bovine serum albumin solution was added to each well and incubated at room temperature for 2 hours for blocking. The blocking solution was discarded, and the plate was washed 4 times with the PBST buffer. 100 µL of the diluted sample was added to each well and incubated at room temperature for 1.5 hours. After incubation, the reaction solution was discarded, and the plate was washed 4 times with PBST. 100 µL of Goat Anti-Human IgG Monkey ads HRP detection antibody (Southern Biotech, Cat No. 2049-05) diluted at 1:25k was added to each well and incubated at room temperature for 1 hour. After the plate was washed 4 times with PBST, 100 µL of TMB (Sigma, Cat No. T0440) chromogenic substrate was added, and incubated at room temperature in the dark for 10 minutes for color development. The reaction was terminated with 1 M sulfuric acid, and the absorbance was read at 450 nm using a SpectraMax M5 microplate reader.

### 4. Test Results

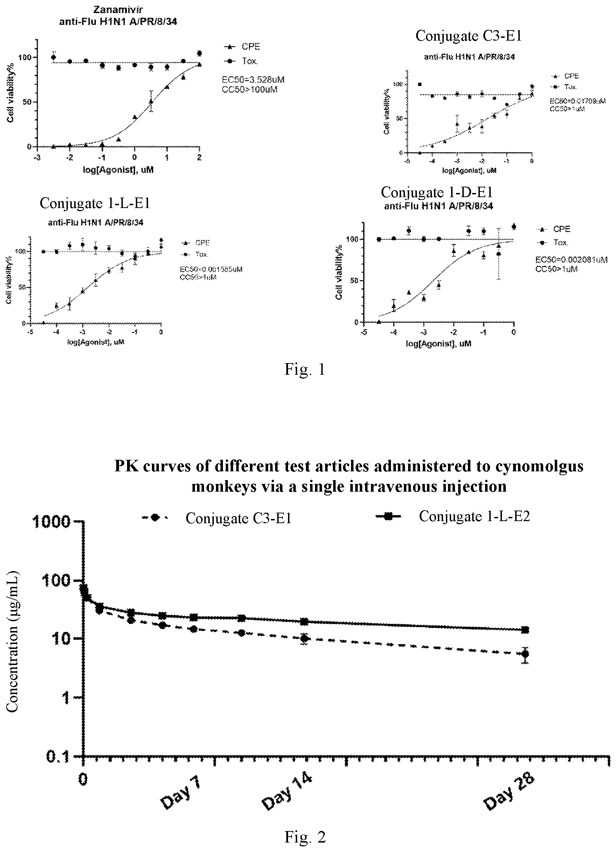

The pharmacokinetic curves and parameters of the animals in each group are shown in **Figure 2** and **Table 8** (data are expressed as Mean±SEM). The test results showed that after intravenous injection administration at the same dose, the AUCₗₐₛₜ of the test article Conjugate 1-L-E2 group was 14466+123 h*µg/mL, which was 1.7 times higher than that of the reference Conjugate C3-E1 group (8691+1713 h*µg/mL). The T₁/₂ (h) of the test article Conjugate 1-L-E2 group was 687+79 h, which was 2 times higher than that of the reference Conjugate C3-E1 group (342+85 h). The above results indicated that compared with Conjugate C3-E1, Conjugate 1-L-E2 had significantly increased drug exposure and half-life in cynomolgus monkeys.

**Table 8 PK parameters of different test articles administered to cynomolgus monkeys via single intravenous injection**

| Test article | Conjugate C3-E1 | Conjugate 1-L-E2 |
|---|---|---|
| T_{1/2} (h) | 342±85 | 687±79 |
| AUCₗₐₛₜ (h*µg/mL) | 8691±1713 | 14466±123 |
| AUC_{INF_obs} (h*µg/mL) | 11536±3489 | 28631±3512 |
| Cl__{obs} (mL/h/kg) | 0.182±0.055 | 0.0704±0.0086 |
| MRTₗₐₛₜ (h) | 222±19 | 276±5 |
| Cₘₐₓ (µg/ml) | 71.74±9.18 | 74.90±8.95 |
| Tₘₐₓ (h) | 0.25±0.0 | 0.25±0.0 |

## Claims

1. A conjugate represented by Formula (I), 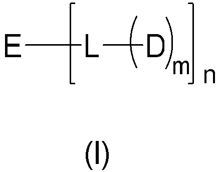
wherein, E is a protein or a polypeptide;
L is a linker covalently connecting E to D;
m is selected from the group consisting of 3, 4, 5, 6, 7, 8, 9, and 10;
n is 1 to 20 (including 1, 2, 3, 4, 5, 6, 7, 8, 9, 10, 11, 12, 13, 14, 15, 16, 17, 18, 19, 20 or any value between any two values);
each D is independently selected from the structure represented by Formula (A), 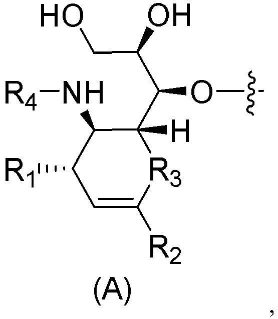
R₁ is selected from the group consisting of -OH, -NH₂, and -NHC(=NH)NHR₅;
R₂ is selected from the group consisting of -CO₂H, -P(=O)(OH)₂, and -SO₃H;
R₃ is -O- or -S-;
R₄ is selected from the group consisting of -COCH₃, -COCF₃, and -SO₂CH₃;
R₅ is selected from the group consisting of hydrogen, hydroxy, sulfhydryl, nitro, cyano, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, C₁-C₁₀ alkyl, C₁-C₁₀ alkoxy, C₂-C₁₀ alkenyl, and C₂-C₁₀ alkynyl, wherein the alkyl, alkoxy, alkenyl, and alkynyl are optionally substituted with one or more substituents selected from the group consisting of C₁-C₆ alkyl, halogen, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
Rᵢ and Rⱼ are each independently selected from the group consisting of hydrogen atom, hydroxy, C₁-C₆ alkyl, and C₁-C₆ alkoxy;
Rₖ is independently selected from the group consisting of hydrogen atom, C₁-C₆ alkyl, C₁-C₆ haloalkyl, C₁-C₆ alkoxy, hydroxy, and -NRᵢRⱼ, wherein the alkyl, alkoxy, and haloalkyl are optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3-to 10-membered cycloalkyl, 3- to 10-membered heterocyclyl, 6- to 10-membered aryl, and 5- to 10-membered heteroaryl.

2. The conjugate according to claim 1, wherein,
R₅ is selected from the group consisting of hydrogen, hydroxy, amino, and -C(O)Rₖ;
Rₖ is selected from the group consisting of hydrogen atom, C₁-C₆ alkyl, C₁-C₆ alkoxy, and hydroxy, wherein the alkyl and alkoxy are optionally substituted with one or more substituents selected from the group consisting of halogen, hydroxy, oxo, thio, carboxyl, C₁-C₆ alkoxy, C₁-C₆ alkylthio, 6- to 10-membered aryl, and 5- to 10-membered heteroaryl.

3. The conjugate according to claim 1, wherein D is selected from the structure represented by Formula (A-1),

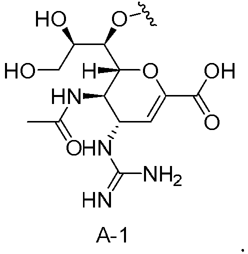

4. The conjugate according to any one of claims 1 to 3, wherein m is 3 or 4.

5. The conjugate according to any one of claims 1 to 4, wherein the linker comprises a core moiety L₂ and branch moieties L₁ and L₃, the branch moiety L₁ is used for connecting E to the core moiety, and the branch moiety L₃ is used for connecting D to the core moiety.

6. The conjugate according to claim 5, wherein the core moiety structure L₂ is selected from 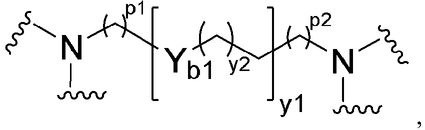
wherein, y1 is an integer from 0 to 100, preferably an integer from 0 to 30; y2 is an integer from 0 to 10, preferably an integer from 0 to 6, more preferably 1 or 2; Y_{b1} is selected from the group consisting of O and CH₂; p1 and p2 are each independently selected from the group consisting of integers from 0 to 10, preferably integers from 0 to 6, more preferably 1 or 2; or 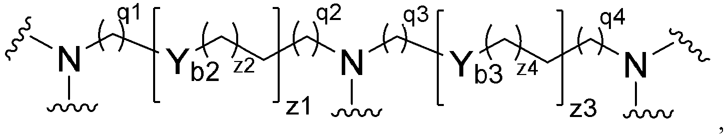
wherein, z1 is an integer from 0 to 100, preferably integers from 0 to 30; z2 is an integer from 0 to 10, preferably an integer from 0 to 6, more preferably 1 or 2; Y_{b2} is selected from the group consisting of O and CH₂; z3 is an integer from 0 to 100, preferably an integer from 0 to 30; z4 is an integer from 0 to 10, preferably an integer from 0 to 6, more preferably 1 or 2; Y_{b3} is selected from the group consisting of O and CH₂; q1, q2, q3, and q4 are each independently selected from the group consisting of integers from 0 to 10, preferably integers from 0 to 6; or

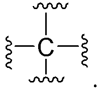

7. The conjugate according to claim 5 or 6, wherein L₁ is selected from the group consisting of -Gₐ₁-(CH₂)_{wa1}-[Yₐ₁-CH₂(CH₂)ₓₐ₂ ]xa1-(CH₂)_{wa2}-Gₐ₂-(CH₂)_{wa3}-[Yₐ₂-CH₂(CH₂)ₓₐ₄]ₓₐ₃-(CH₂)_{wa4}-Gₐ₃-, wherein xa1 and xa3 are each independently selected from the group consisting of integers from 0 to 100, preferably integers from 0 to 30; xa2 and xa4 are each independently selected from the group consisting of integers from 0 to 10, preferably integers from 0 to 6, more preferably 1 or 2; wa1, wa2, wa3, and wa4 are each independently selected from the group consisting of integers from 0 to 10, preferably integers from 0 to 6; Yₐ₁ and Yₐ₂ are each independently selected from the group consisting of O and CH₂; Gₐ₁ is the group Gₓ; Gₐ₂ and Gₐ₃ are each independently selected from the group consisting of the group Gₓ and being absent,
Gₓ is selected from the group consisting of 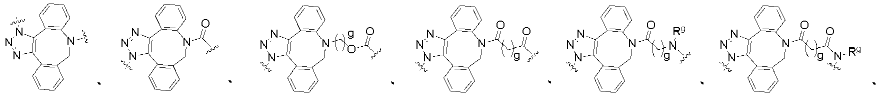 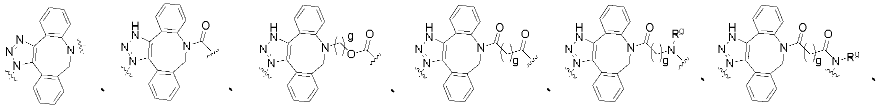 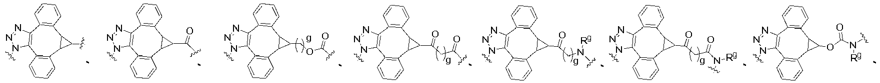 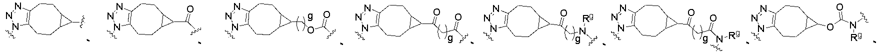 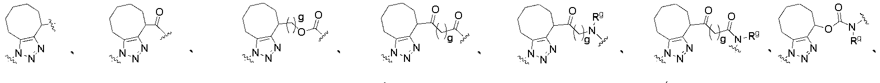 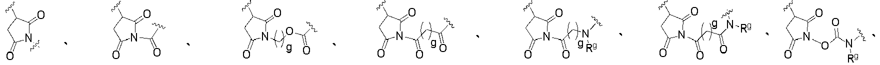 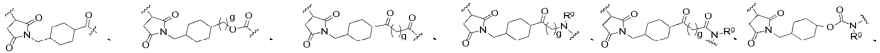 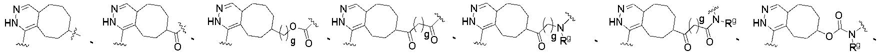 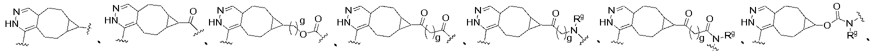 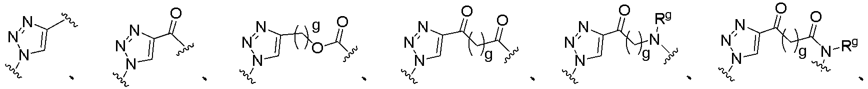 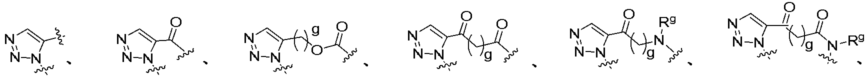 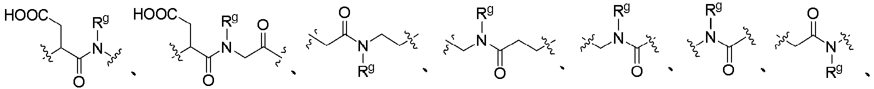 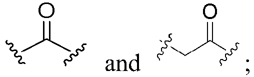 g is each independently selected from the group consisting of 0, 1, 2, 3, and 4; R^{g} is each independently selected from the group consisting of hydrogen and methyl.

8. The conjugate according to any one of claims 5 to 7, wherein L₃ comprises an end group G_{c1} connected to D, and the end group G_{c1} is selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, preferably -NR₆(C=O)-, -NR₆(C=S)-, -O(C=O)-, -(C=O)-, and -NR₆(C=O)CH₂-;
preferably, L₃ is -(CH₂)_{wc1}-[Y_{c1}-CH₂(CH₂)_{xc2}]_{xc1}-(CH₂)_{wc2}-G_{c1}-,
wherein, each xc1 is independently an integer from 0 to 100, preferably an integer from 0 to 30, more preferably an integer from 2 to 30;
each xc2 is independently an integer from 0 to 10, preferably an integer from 0 to 6, more preferably 1 or 2;
each Y_{c1} is independently selected from the group consisting of O and CH₂, preferably O;
wc1 and wc2 are each independently selected from the group consisting of integers from 0 to 10, preferably integers from 0 to 6, more preferably 0, 1, 2, or 3;
each G_{c1} is independently selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl, preferably -NR₆(C=O)-, -NR₆(C=S)-, -O(C=O)-, -(C=O)-, and -NR₆(C=O)CH₂;
m is 3 or 4.

9. The conjugate according to any one of claims 1 to 8, wherein -L(-D)ₘ is -L₁-L₂(-L₃-D)ₘ, wherein,
L₁ is a moiety connected to E, and L₁ is selected from -Gₐ₁-(CH₂)_{wa1}-[Yₐ₁-CH₂(CH₂)ₓₐ₂]ₓₐ₁-(CH₂)_{wa2}-Gₐ₂-(CH₂)_{wa3}-[Yₐ₂-CH₂(CH₂)ₓₐ₄]ₓₐ₃-(CH₂) _{wa4}-Gₐ₃-, wherein,
xa1 and xa3 are each independently selected from the group consisting of integers from 0 to 100, preferably integers from 0 to 30;
xa2 and xa4 are each independently selected from the group consisting of integers from 0 to 10, preferably integers from 0 to 6, more preferably 1 or 2;
wa1, wa2, wa3, and wa4 are each independently selected from the group consisting of integers from 0 to 10, preferably integers from 0 to 6;
Yₐ₁ and Yₐ₂ are each independently selected from the group consisting of O and CH₂;
Gₐ₁ is the group Gₓ; Gₐ₂ and Gₐ₃ are each independently selected from the group consisting of the group Gₓ and being absent;
L₂ is selected from 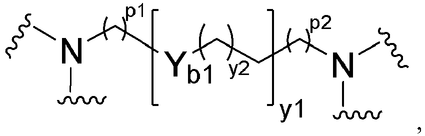
wherein, y1 is an integer from 0 to 100, preferably an integer from 0 to 30;
y2 is an integer from 0 to 10, preferably an integer from 0 to 6, more preferably 1 or 2;
Y_{b1} is selected from the group consisting of O and CH₂;
p1 and p2 are each independently selected from the group consisting of integers from 0 to 10, preferably integers from 0 to 6, more preferably 1 or 2; or 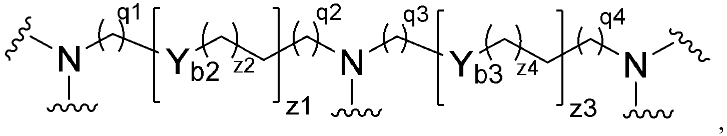
wherein, z1 is an integer from 0 to 100, preferably an integer from 0 to 30;
z2 is an integer from 0 to 10, preferably an integer from 0 to 6, more preferably 1 or 2;
Y_{b2} is selected from the group consisting of O and CH₂;
z3 is an integer from 0 to 100, preferably an integer from 0 to 30;
z4 is an integer from 0 to 10, preferably an integer from 0 to 6, more preferably 1 or 2;
Y_{b3} is selected from the group consisting of O and CH₂;
q1, q2, q3, and q4 are each independently selected from the group consisting of integers from 0 to 10, preferably integers from 0 to 6; or 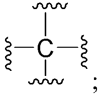
L₃ is -(CH₂)_{wc1}-[Y_{c1}-CH₂(CH₂)_{xc2}]_{xc1}-(CH₂)_{wc2}-G_{c1}-, wherein,
each xc1 is independently an integer from 0 to 100, preferably an integer from 0 to 30, more preferably an integer from 2 to 30;
each xc2 is independently an integer from 0 to 10, preferably an integer from 0 to 6, more preferably 1 or 2;
each Y_{c1} is independently selected from the group consisting of O and CH₂;
wc1 and wc2 are each independently selected from the group consisting of integers from 0 to 10, preferably integers from 0 to 6;
each G_{c1} is independently selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl;
Gₓ is selected from the group consisting of 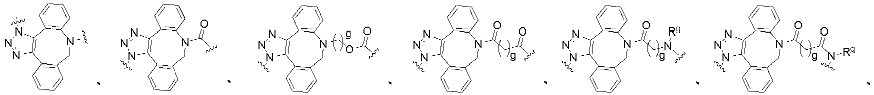 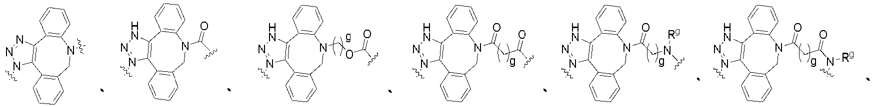 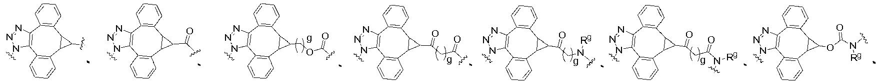 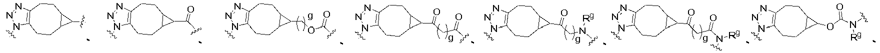 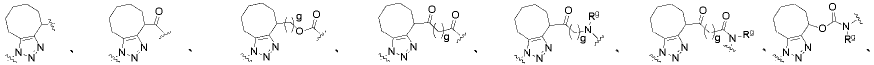 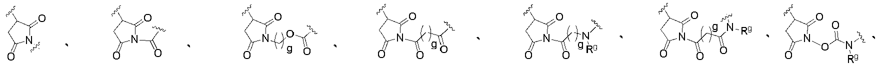 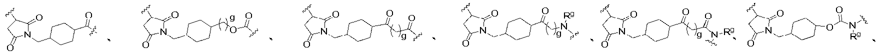 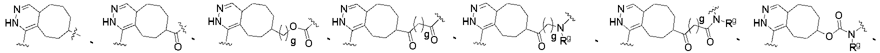 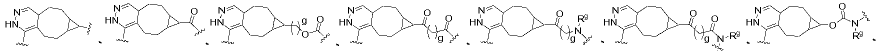 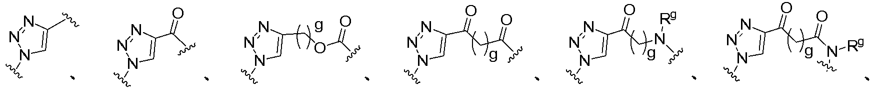 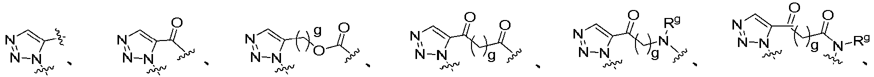 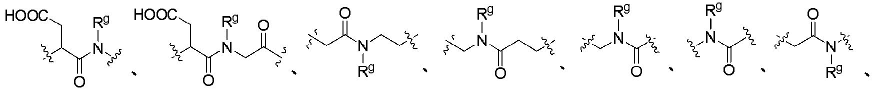 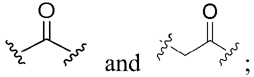 g is each independently selected from the group consisting of 0, 1, 2, 3, and 4; R^{g} is each independently selected from the group consisting of hydrogen and methyl;
m is 3 or 4.

10. The conjugate according to claim 8 or 9, wherein the number of atoms in the L₃ backbone is greater than 7, preferably greater than 8, more preferably greater than 9.

11. The conjugate according to any one of claims 1 to 10, wherein the protein or polypeptide is selected from the group consisting of an Fc domain, albumin, and an albumin-binding domain.

12. The conjugate according to any one of claims 1 to 11, wherein the Fc domain has an enhanced effector function, preferably, the effector function is selected from the group consisting of C1q binding and complement-dependent cytotoxicity, Fc receptor binding, antibody-dependent cellular cytotoxicity, phagocytosis, downregulation of cell surface receptors, and B cell activation.

13. The conjugate according to any one of claims 1 to 12, wherein the Fc domain comprises an Fc region derived from IgG, preferably, the Fc region is selected from the group consisting of Fc regions of human IgG1, IgG2, IgG3, and IgG4; more preferably, the Fc region is human IgG1.

14. The conjugate according to any one of claims 1 to 13, selected from the group consisting of: 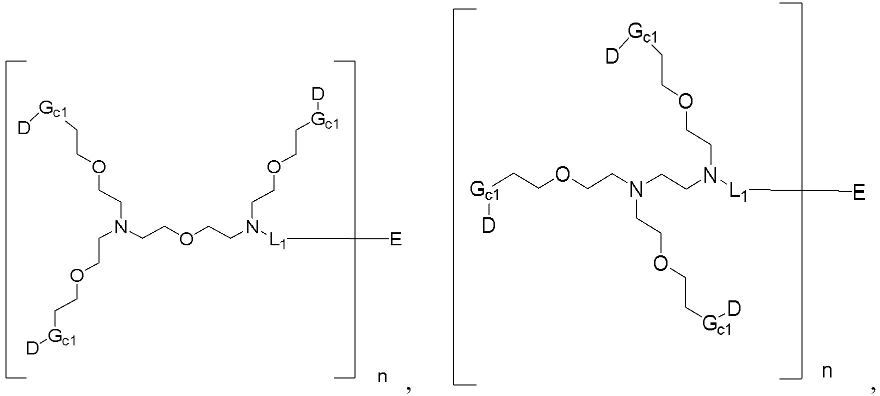 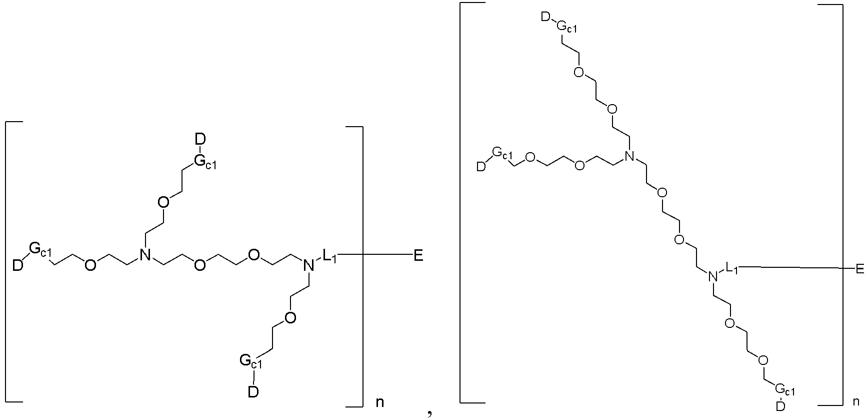 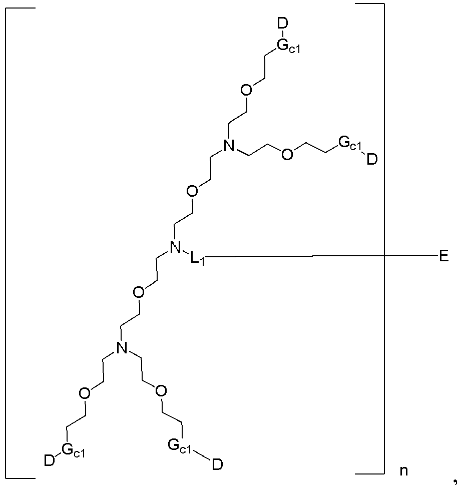 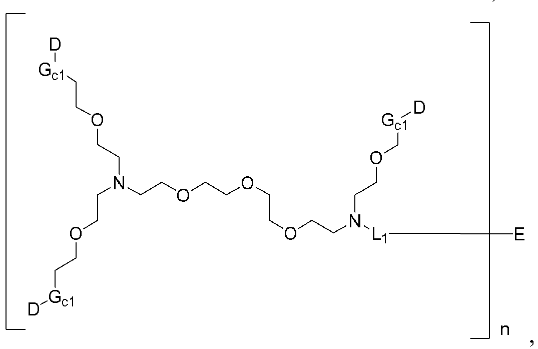 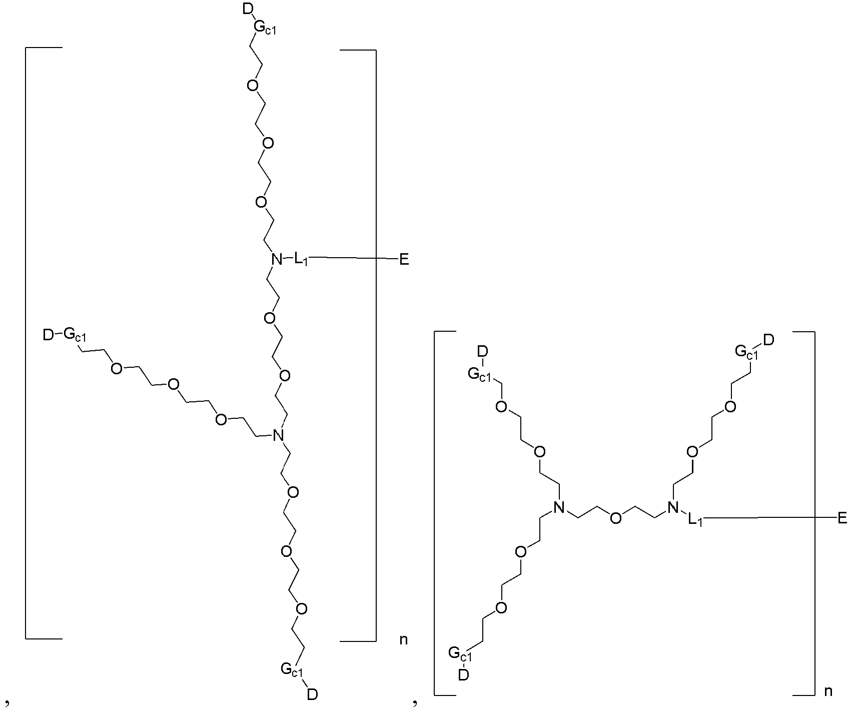 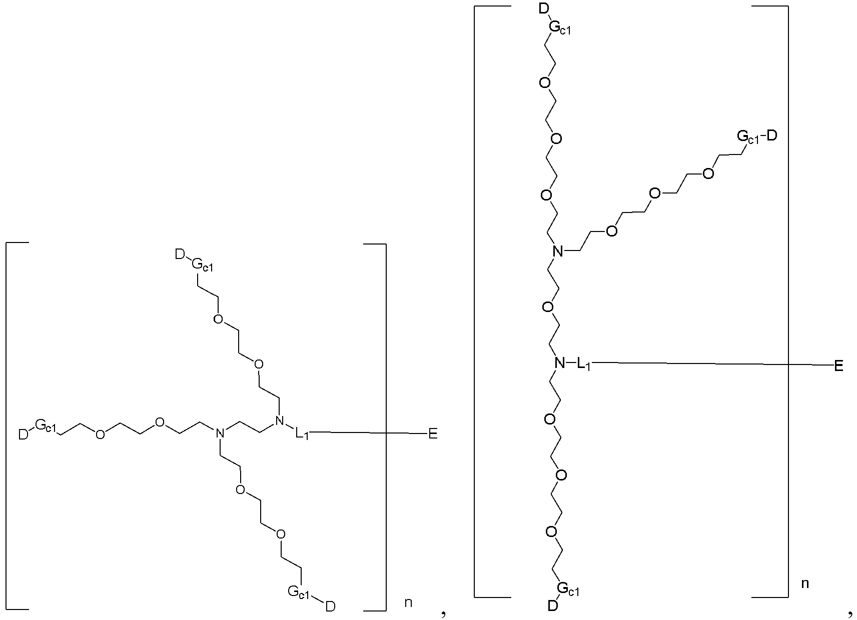 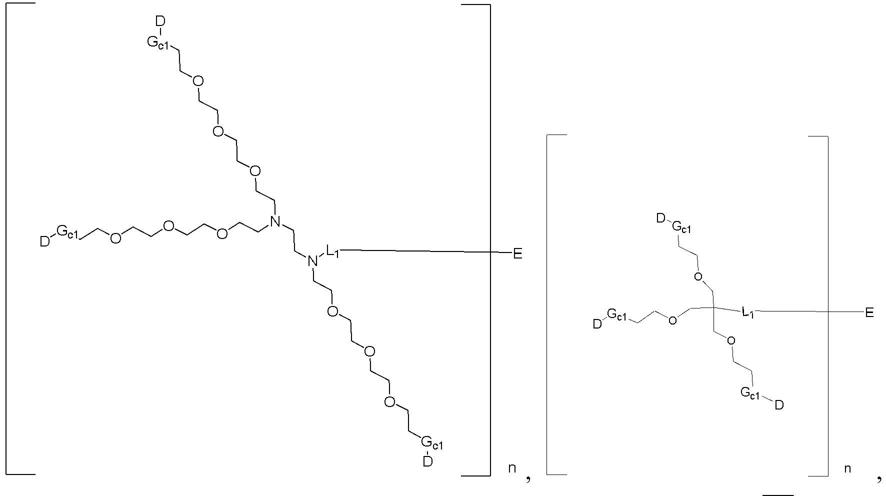 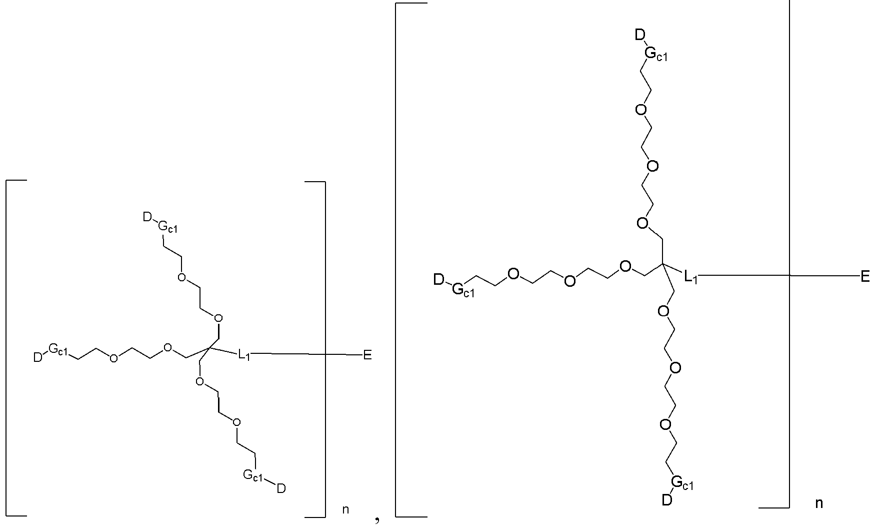 and 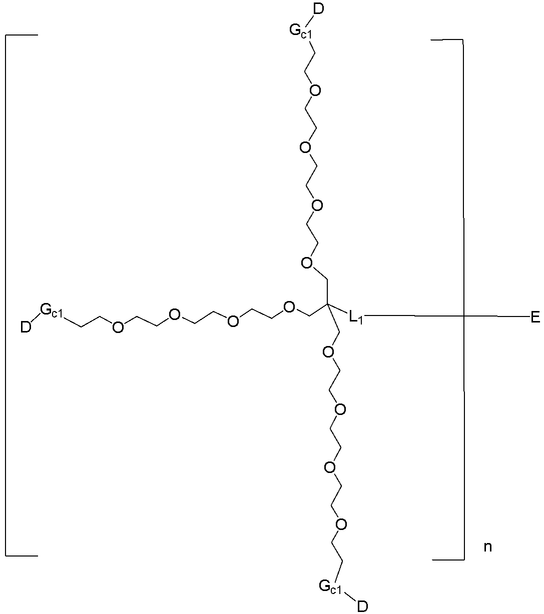 wherein,
L₁ is selected from -Gₐ₁-(CH₂)_{wa1}-[Yₐ₁-CH₂(CH₂)ₓₐ₂]ₓₐ₁-(CH₂)_{wa2}-Gₐ₂-(CH₂)_{wa3}-[Yₐ₂-CH₂(CH₂)ₓₐ₄]ₓₐ₃-(CH₂) _{wa4}-Gₐ₃-, wherein xa1 and xa3 are each independently selected from the group consisting of integers from 0 to 100, preferably integers from 0 to 30; xa2 and xa4 are each independently selected from the group consisting of integers from 0 to 10, preferably integers from 0 to 6, more preferably 1 or 2; wa1, wa2, wa3, and wa4 are each independently selected from the group consisting of integers from 0 to 10, preferably integers from 0 to 6; Yₐ₁ and Yₐ₂ are each independently selected from the group consisting of O and CH₂; Gₐ₁ is selected from the group Gₓ; Gₐ₂ and Gₐ₃ are each independently selected from the group consisting of the group Gₓ and being absent;
Gₓ is selected from the group consisting of 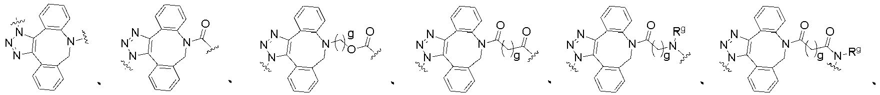 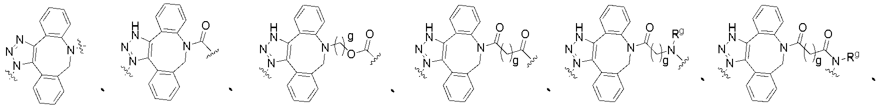 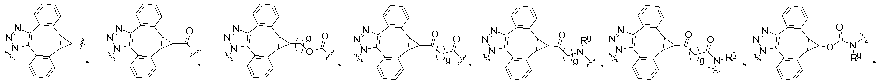 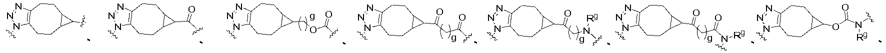 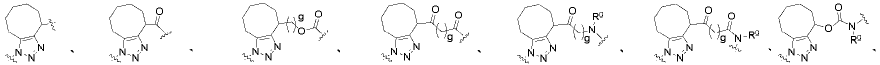 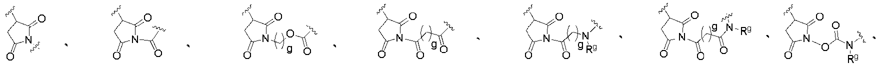 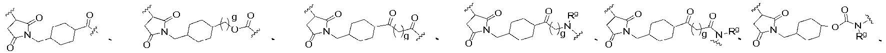 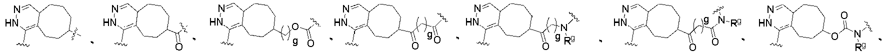 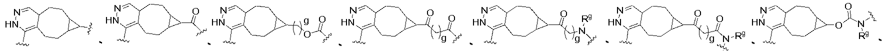 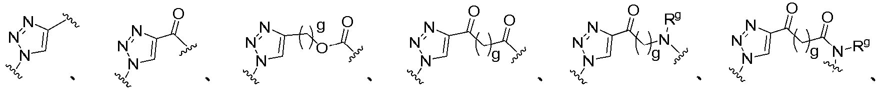 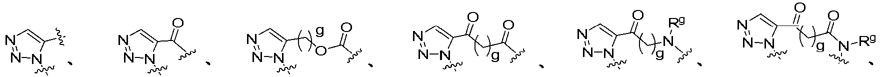 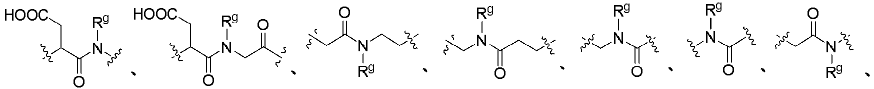 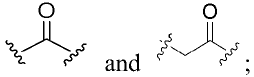 g is each independently selected from the group consisting of 0, 1, 2, 3, and 4; R^{g} is each independently selected from the group consisting of hydrogen and methyl;
G_{c1} is selected from the group consisting of -NR₆(C=O)-, -NR₆(C=S)-, -O(C=O)-, -(C=O)-, and -NR₆(C=O)CH₂-, wherein R₆ is selected from the group consisting of hydrogen and C₁-C₆ alkyl;
n is any value between 1 and 15,
E is as defined in claim 1.

15. The conjugate according to any one of claims 1 to 13, selected from the group consisting of: 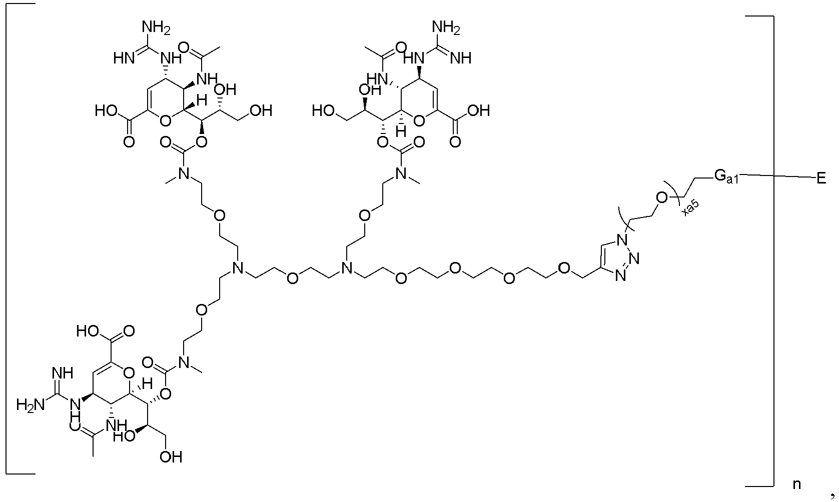 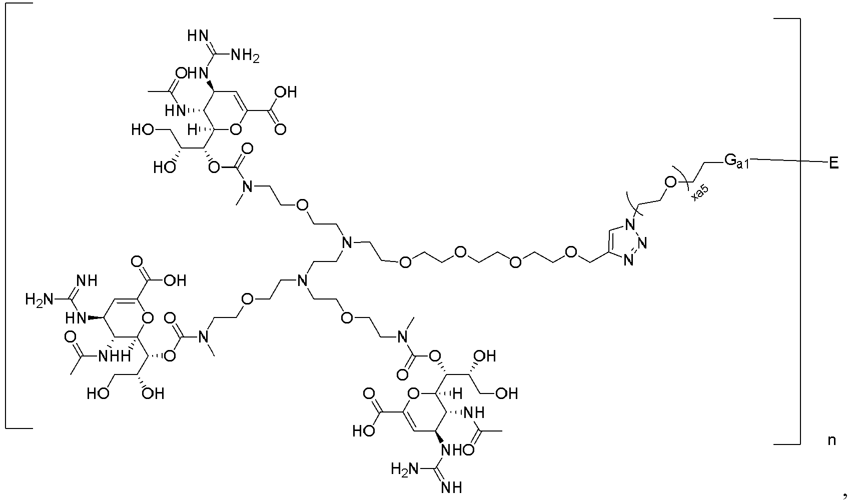 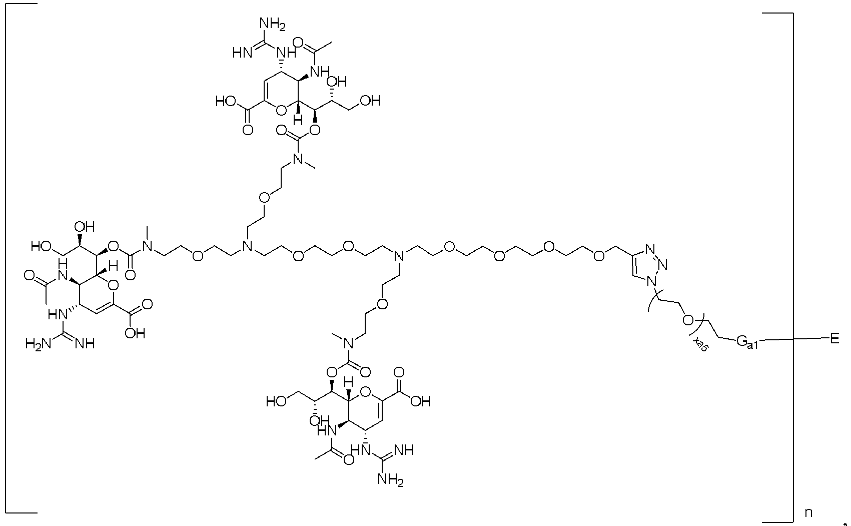 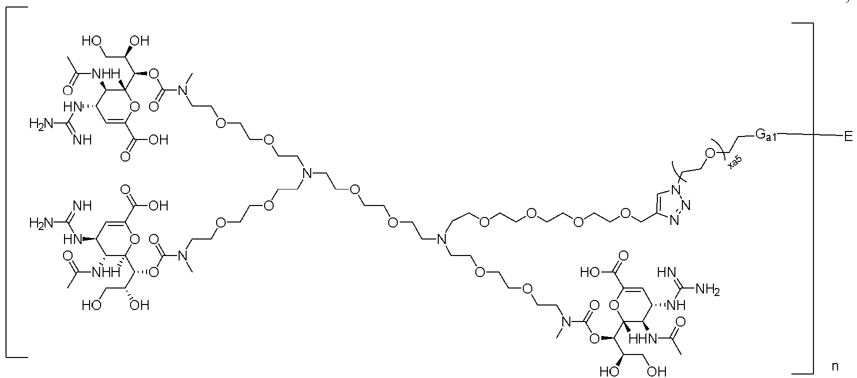 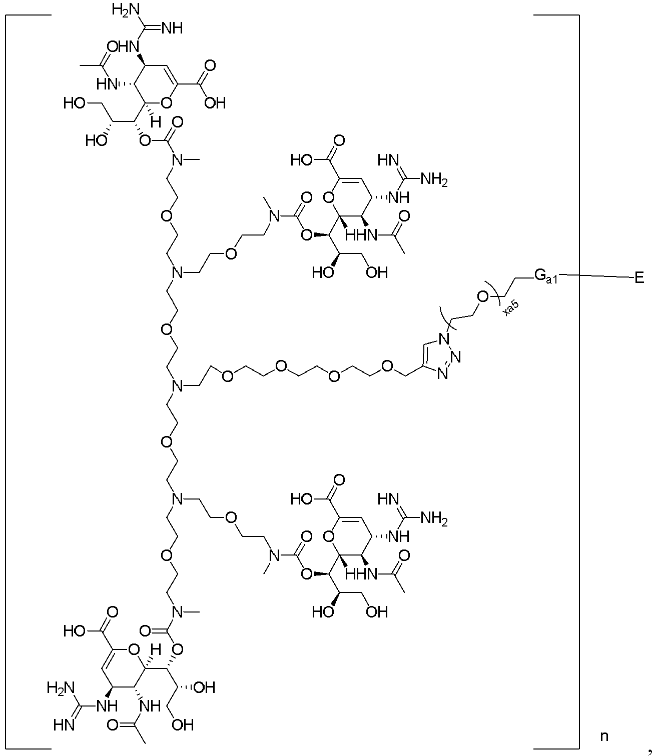 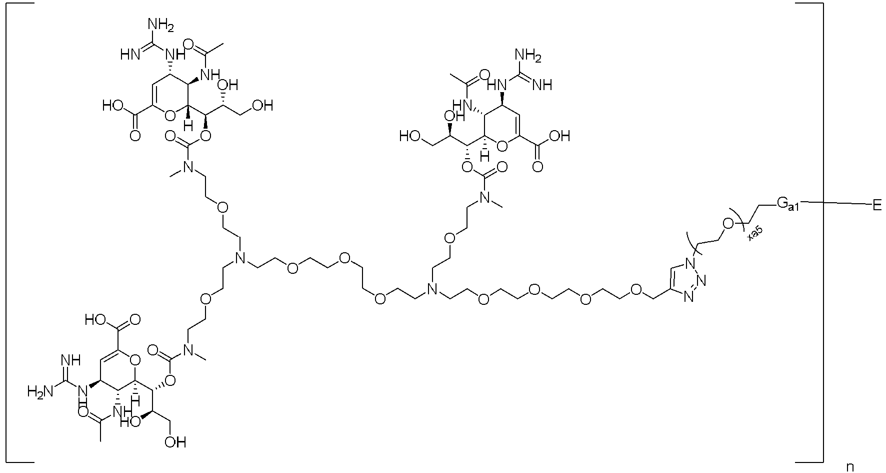 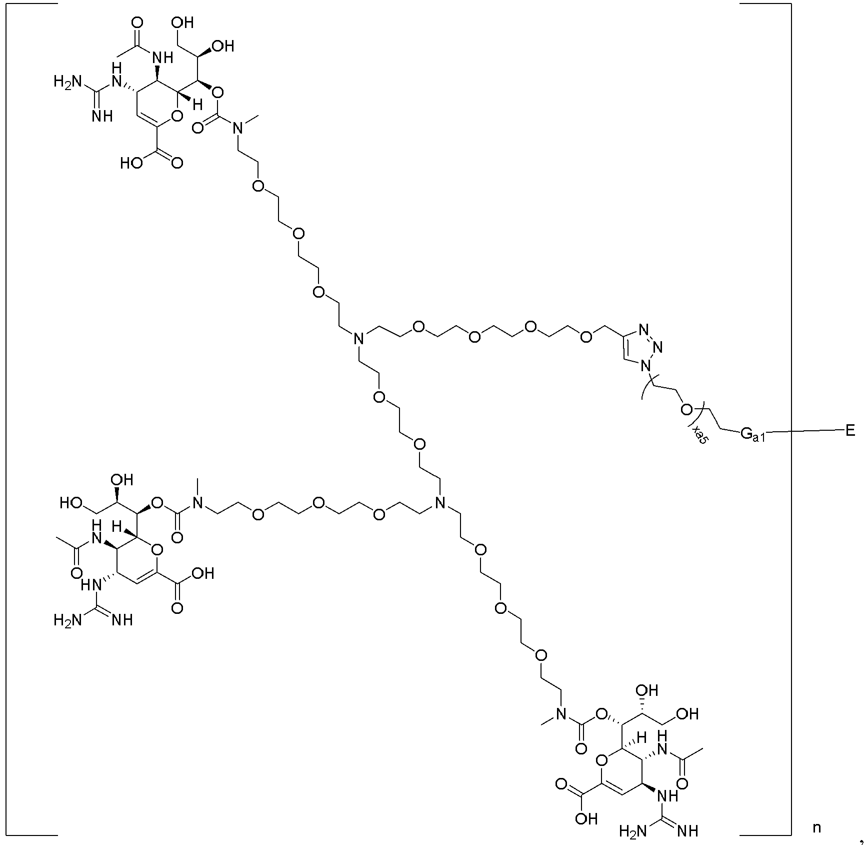 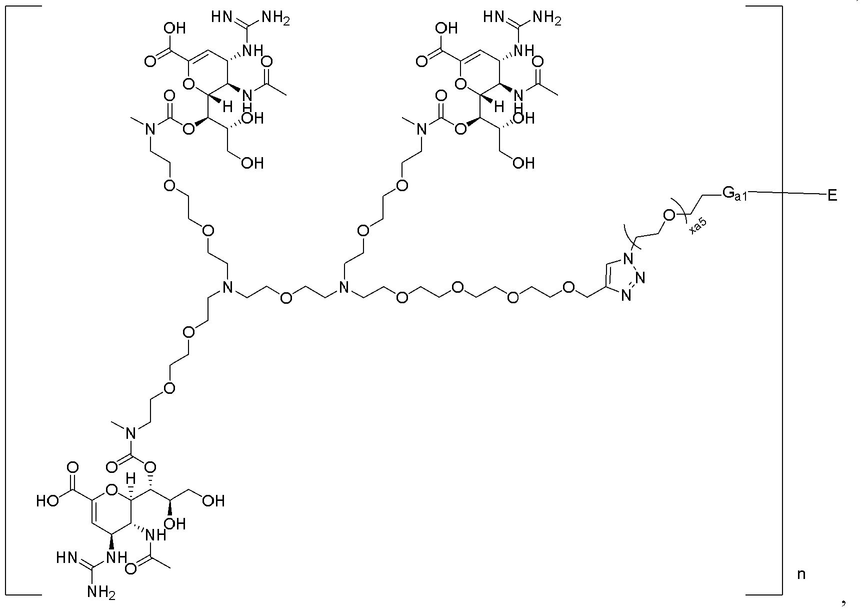 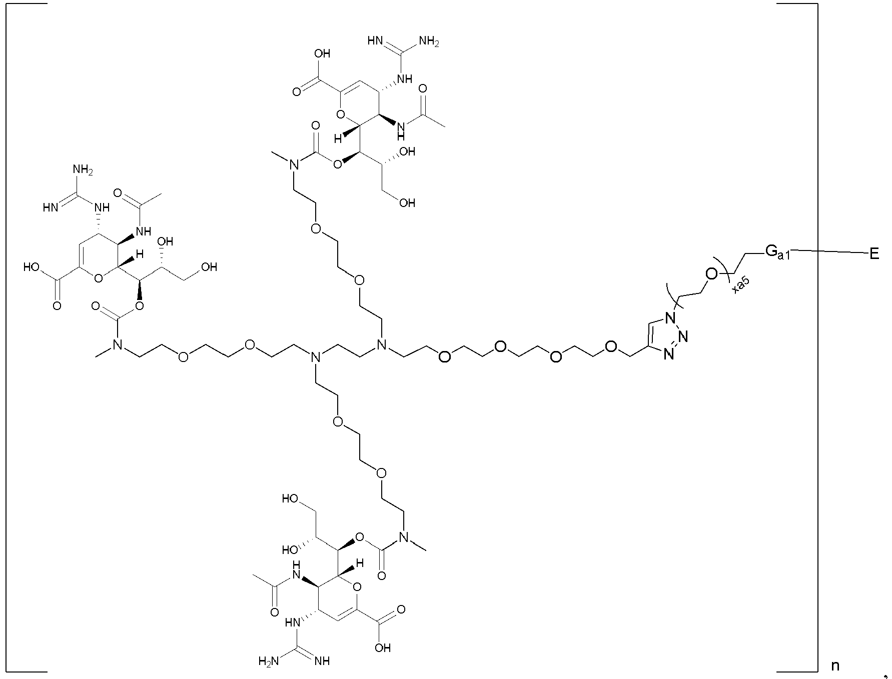 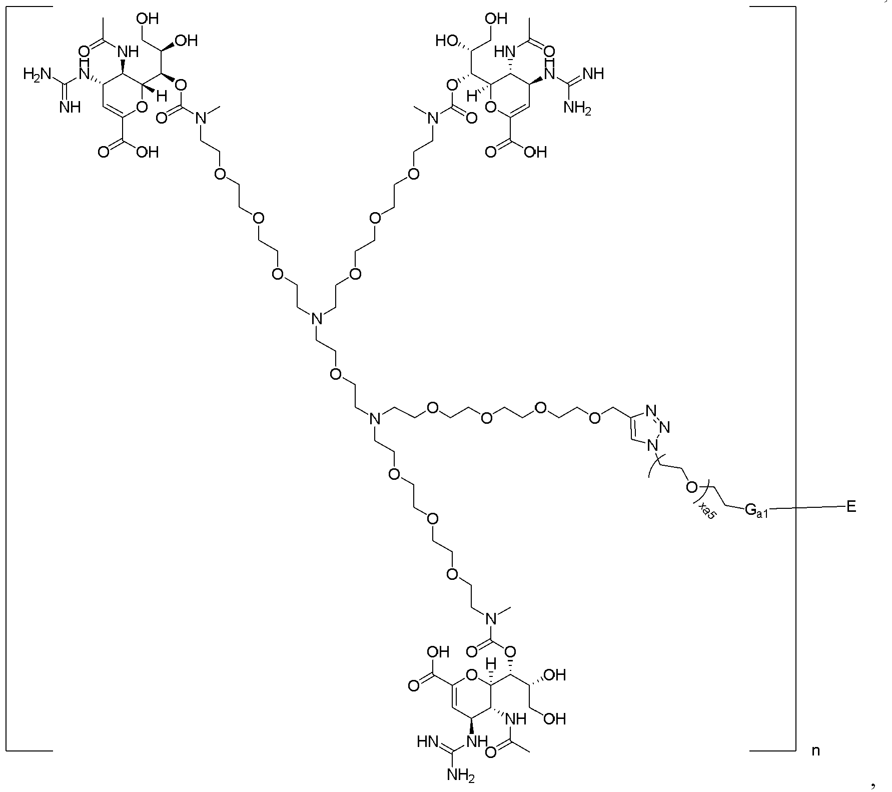 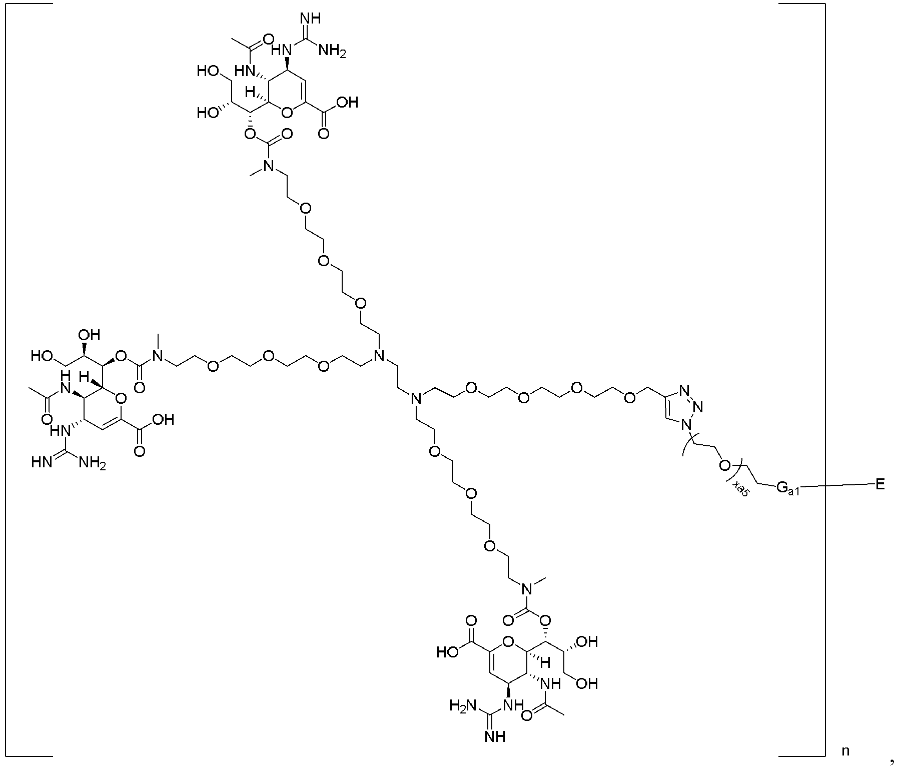 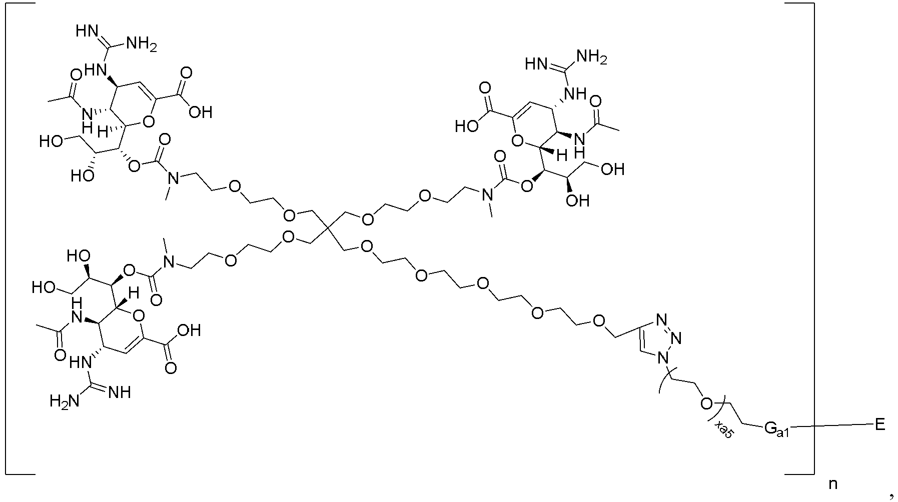 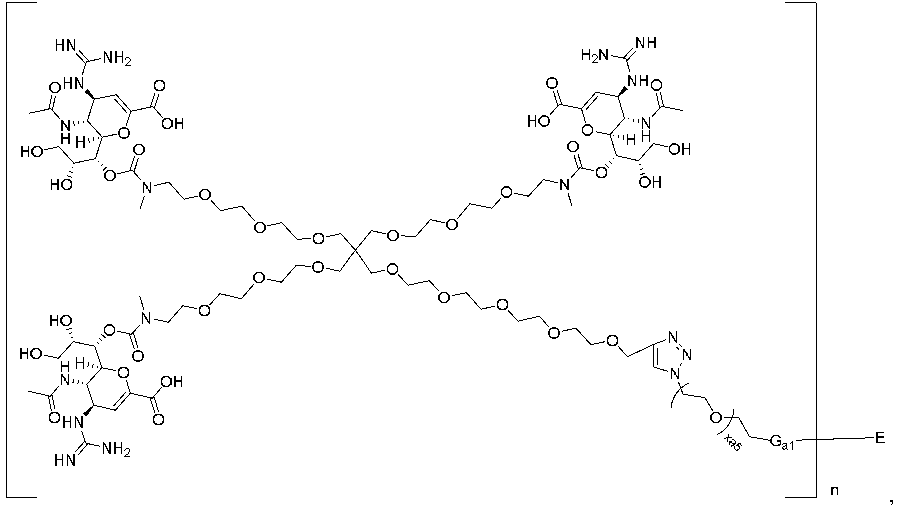 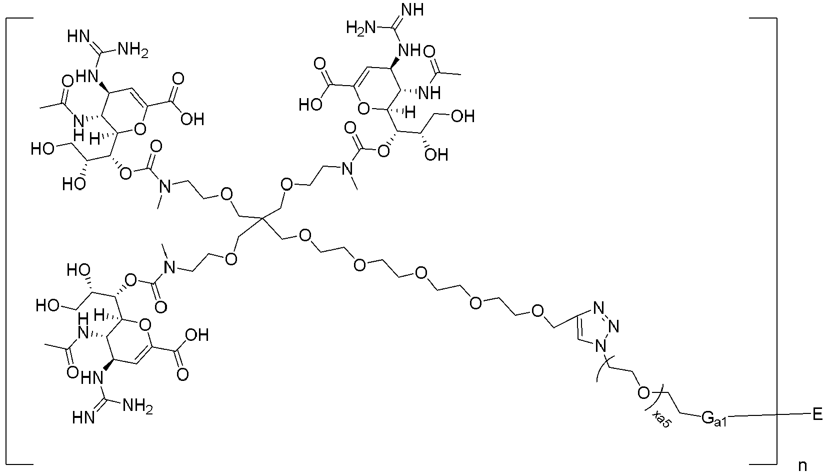 and 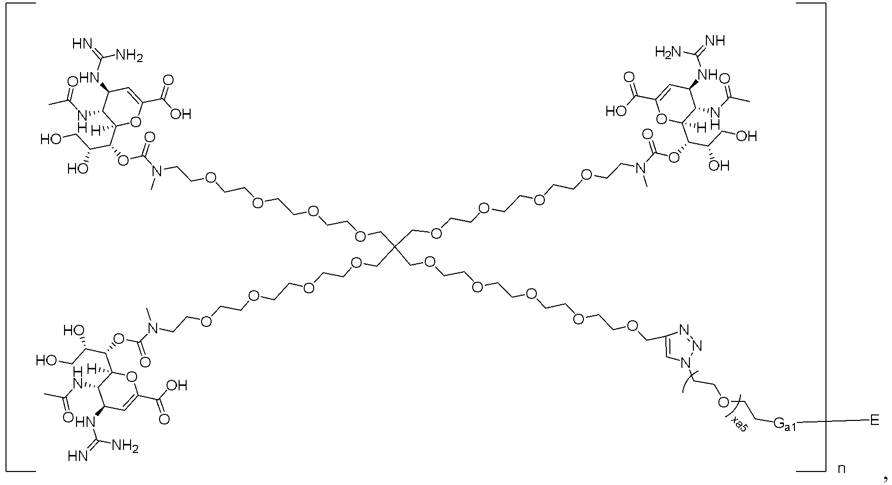 wherein,
each xa5 is independently an integer between 2 and 8,
each Gₐ₁ is independently selected from the group consisting of 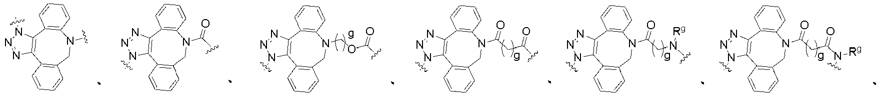 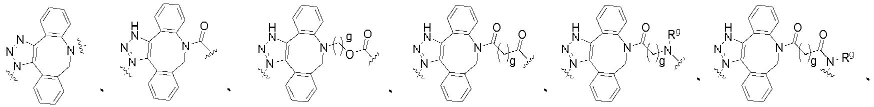 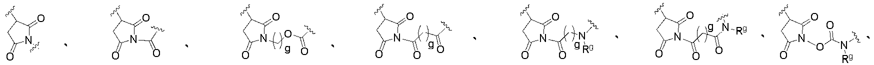 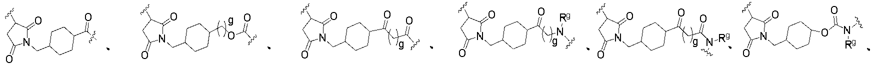 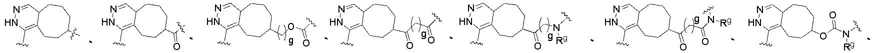 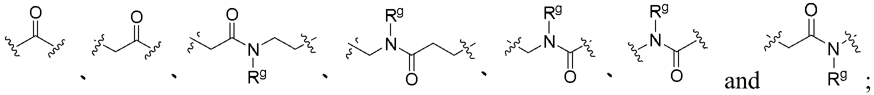
g is each independently selected from the group consisting of 0, 1, 2, 3, and 4; R^{g} is each independently selected from the group consisting of hydrogen and methyl;
n is any value between 1 and 15,
E is as defined in claim 1.

16. The conjugate according to any one of claims 1 to 15, wherein n is any value between 1 and 15, preferably any value between 2 and 12.

17. A pharmaceutical composition, comprising the conjugate according to any one of claims 1 to 16 and a pharmaceutically acceptable excipient.

18. Use of the conjugate according to any one of claims 1 to 16 or the pharmaceutical composition according to claim 17 in the preparation of a medicament for treating a viral infection, preferably the viral infection is caused by an influenza virus or a parainfluenza virus, preferably the viral infection is an influenza A, B, or C virus or a parainfluenza virus.

19. A compound represented as follows,
L₁' -L₂(-L₃-D)ₘ,
wherein,
L1' is selected from Gₓ'-(CH₂)_{wa1}-[Yₐ₁-CH₂(CH₂)ₓₐ₂]ₓₐ₁-(CH₂)_{wa2}-Gₐ₂-(CH₂)_{wa3}-[Yₐ₂-CH₂(CH₂)ₓₐ₄]ₓₐ₃-(CH₂)_{w a4}-Gₐ₃-, wherein,
xa1 and xa3 are each independently selected from the group consisting of integers from 0 to 100, preferably integers from 0 to 30;
xa2 and xa4 are each independently selected from the group consisting of integers from 0 to 10, preferably integers from 0 to 6, more preferably 1 or 2;
wa1, wa2, wa3, and wa4 are each independently selected from the group consisting of integers from 0 to 10, preferably integers from 0 to 6;
Yₐ₁ and Yₐ₂ are each independently selected from the group consisting of O and CH₂;
each Gₓ' is independently selected from the group consisting of 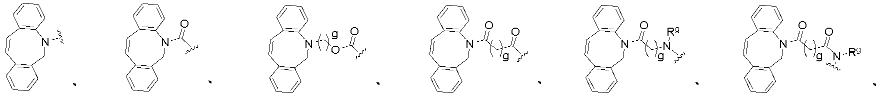 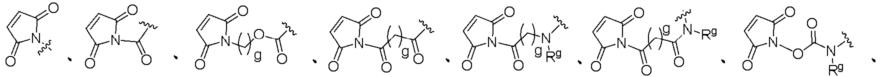 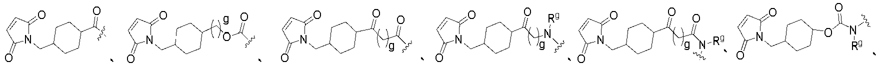 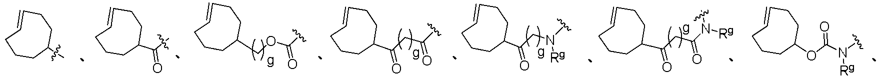 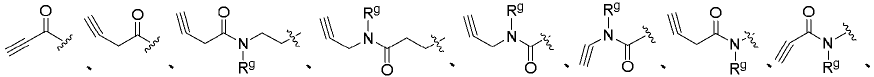 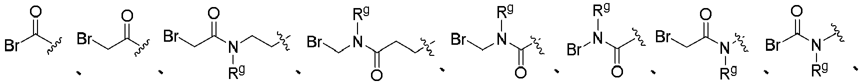 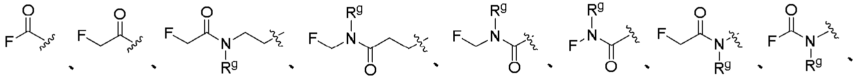 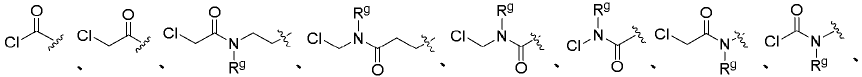 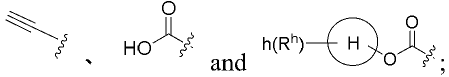
Gₐ₂ and Gₐ₃ are each independently selected from the group consisting of: 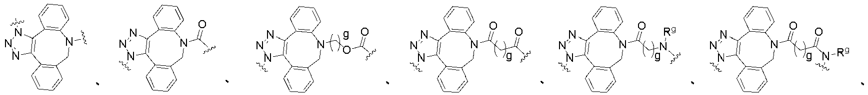 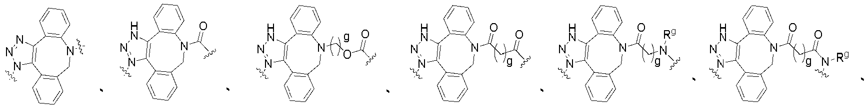 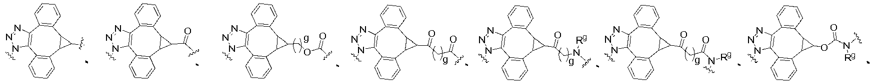 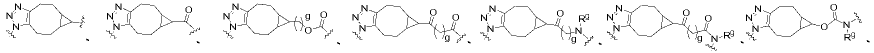 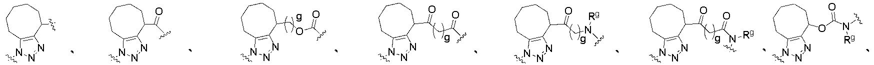 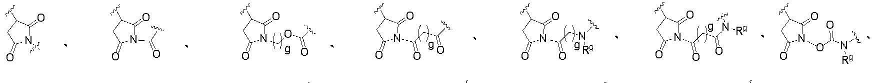 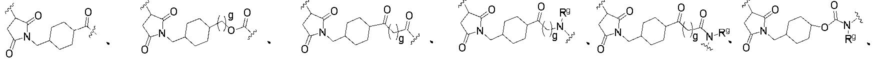 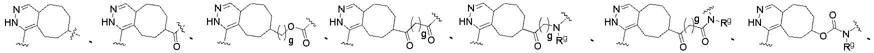 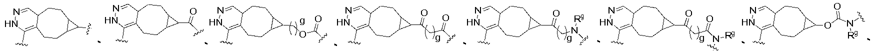 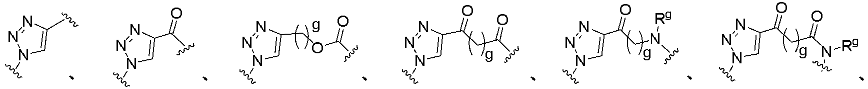 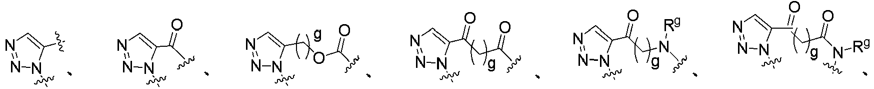 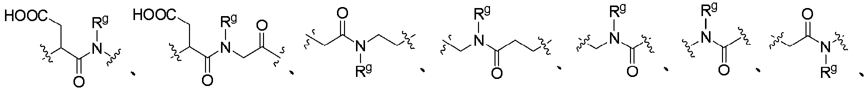 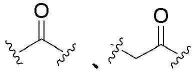 and being absent;
g is each independently selected from the group consisting of 0, 1, 2, 3, and 4; R^{g} is each independently selected from the group consisting of hydrogen and methyl;
ring H is a 5- to 10-membered heteroaryl, 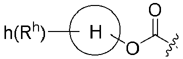 is preferably 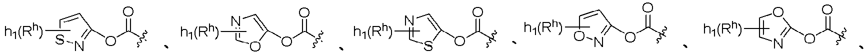 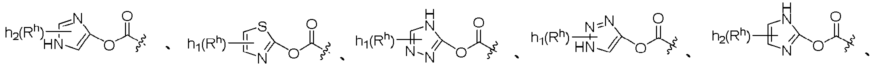 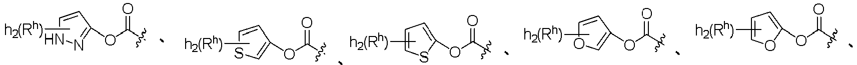 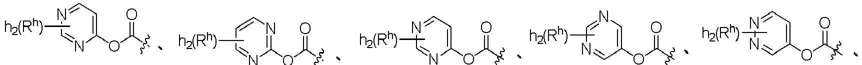 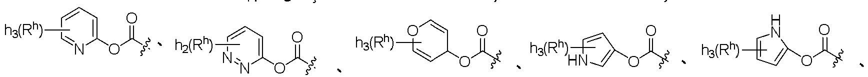 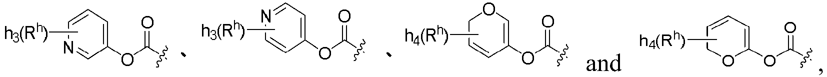 most preferably 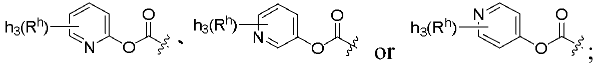
each Rₕ is independently selected from halogen;
h is selected from the group consisting of 0, 1, 2, 3, 4, and 5;
each h1 is independently selected from the group consisting of 0, 1, and 2;
each h2 is independently selected from the group consisting of 0, 1, 2, and 3;
each h3 is independently selected from the group consisting of 0, 1, 2, 3, and 4;
each h4 is independently selected from the group consisting of 0, 1, 2, 3, 4, and 5;
L₂ is selected from 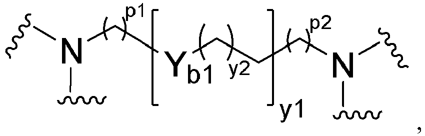
wherein, y1 is an integer from 0 to 100, preferably an integer from 0 to 30;
y2 is an integer from 0 to 10, preferably an integer from 0 to 6, more preferably 1 or 2;
Y_{b1} is selected from the group consisting of O and CH₂;
p1 and p2 are each independently selected from the group consisting of integers from 0 to 10, preferably integers from 0 to 6, more preferably 1 or 2; or 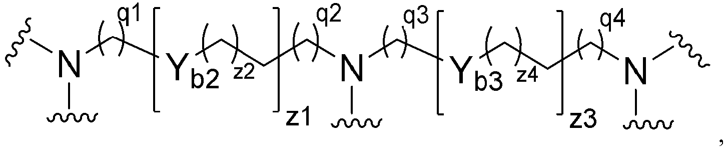
wherein, z1 is an integer from 0 to 100, preferably an integer from 0 to 30;
z2 is an integer from 0 to 10, preferably an integer from 0 to 6, more preferably 1 or 2;
Y_{b2} is selected from the group consisting of O and CH₂;
z3 is an integer from 0 to 100, preferably an integer from 0 to 30;
z4 is an integer from 0 to 10, preferably an integer from 0 to 6, more preferably 1 or 2;
Y_{b3} is selected from the group consisting of O and CH₂;
q1, q2, q3, and q4 are each independently selected from the group consisting of integers from 0 to 10, preferably integers from 0 to 6; or 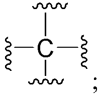
L₃ is -(CH₂)_{wc1}-[Y_{c1}-CH₂(CH₂)_{xc2}]_{xc1}-(CH₂)_{wc2}-G_{c1}-, wherein,
each xc1 is independently an integer from 0 to 100, preferably an integer from 0 to 30;
each xc2 is independently an integer from 0 to 10, preferably an integer from 0 to 6, more preferably 1 or 2;
each Y_{c1} is independently selected from the group consisting of O and CH₂;
wc1 and wc2 are each independently selected from the group consisting of integers from 0 to 10, preferably integers from 0 to 6, G_{c1} is selected from the group consisting of -NR₆(C=O)-, -NR₆(C=S)-, -O(C=O)-, -(C=O)-, -NR₆(C=O)CH₂-, and a chemical bond, wherein R₆ is selected from the group consisting of hydrogen and C₁-C₆ alkyl;
D is 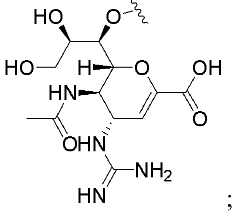
m is 3 or 4.

20. The compound according to claim 19, selected from the group consisting of: 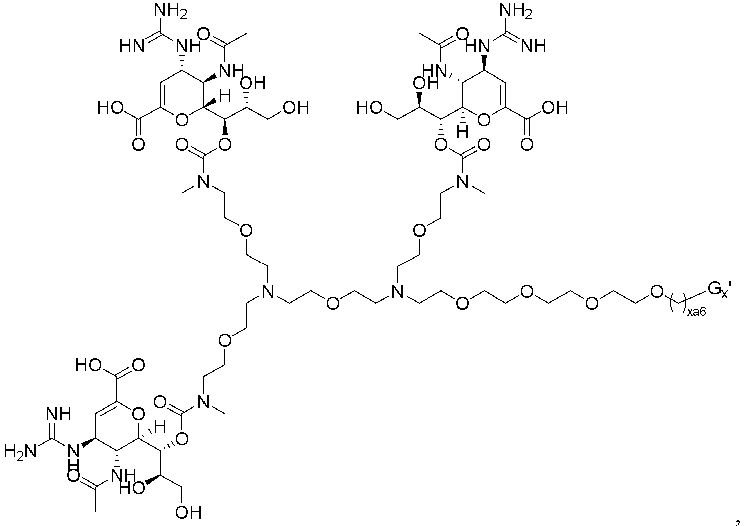 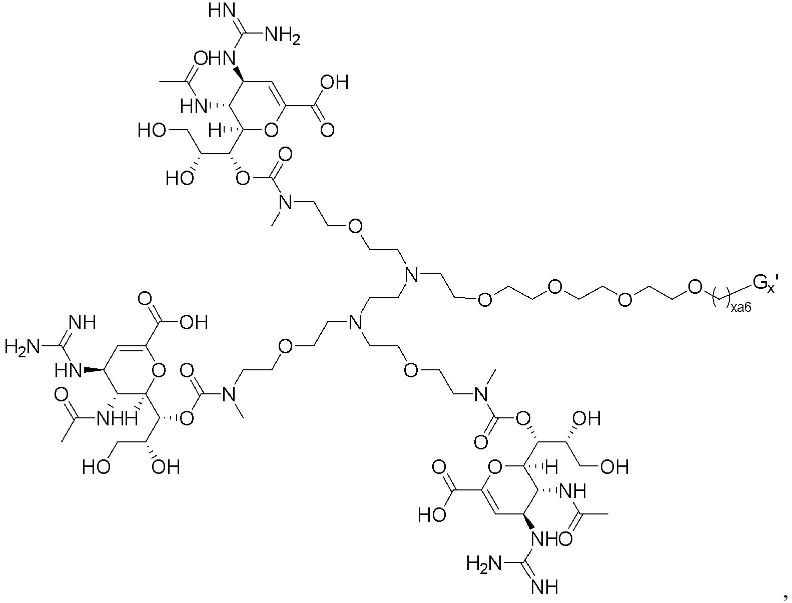 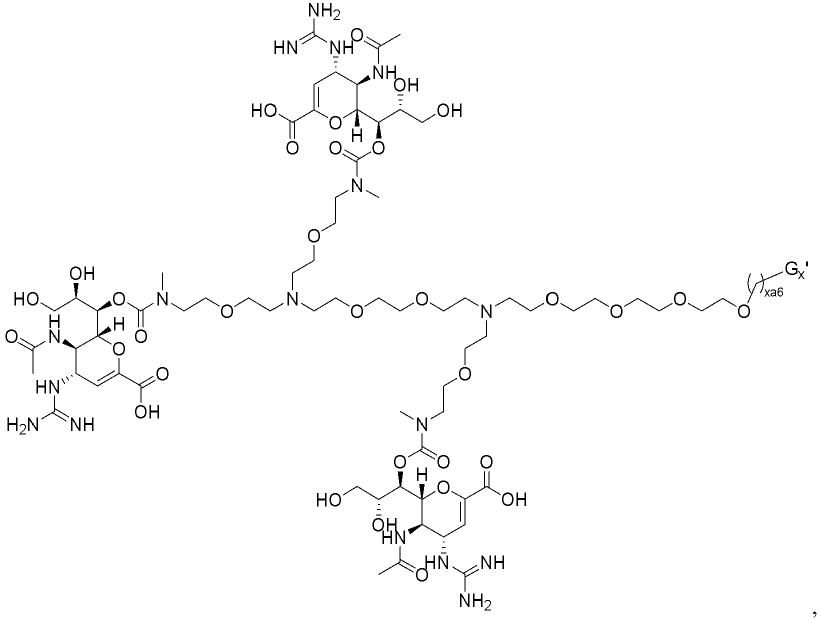 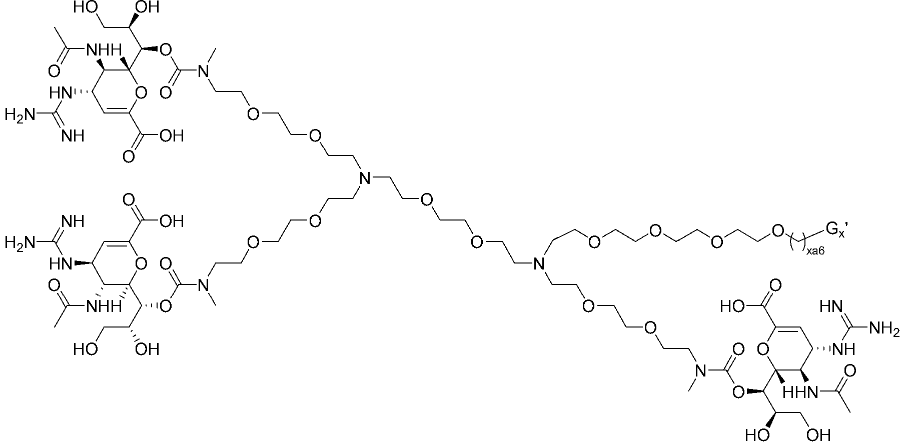 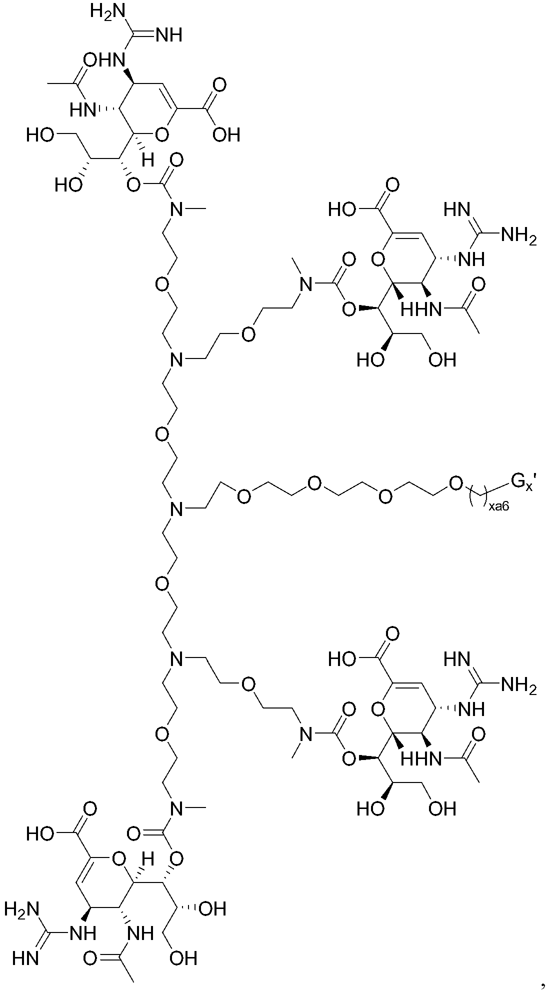 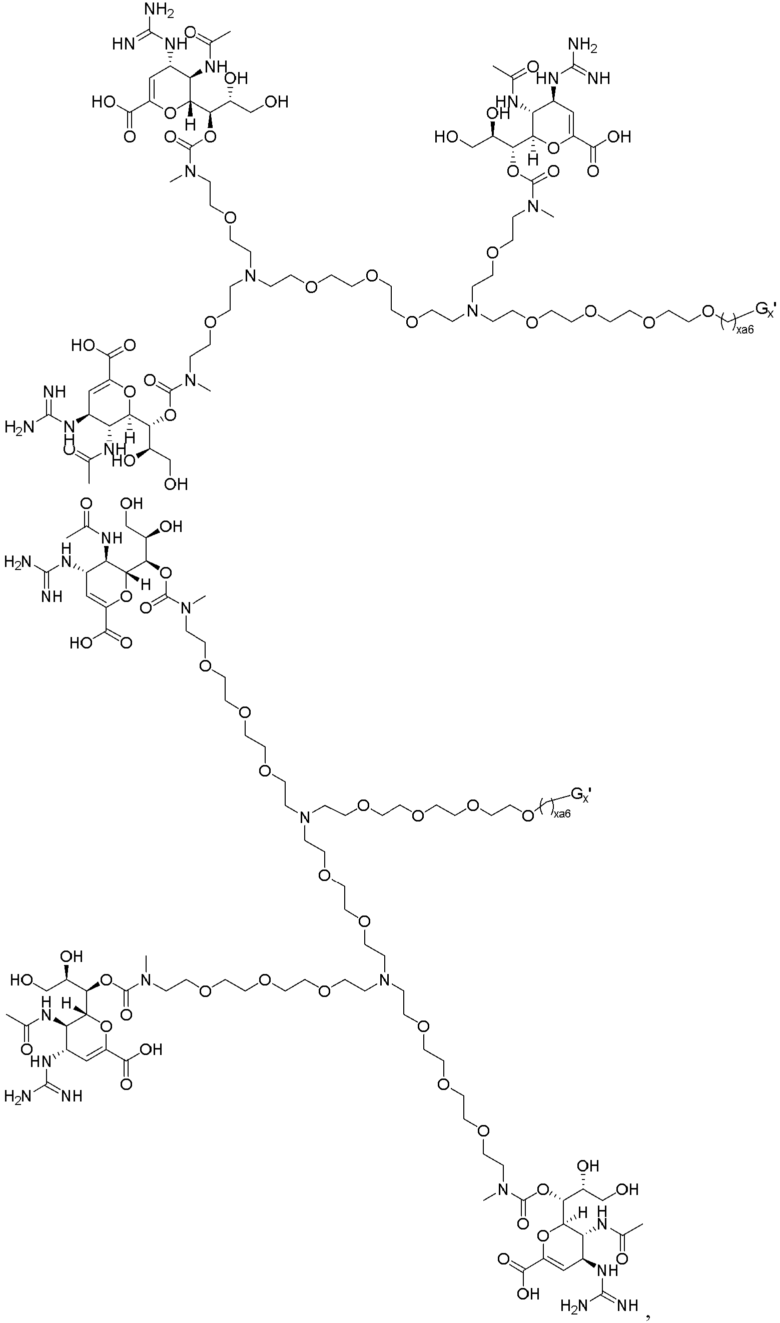 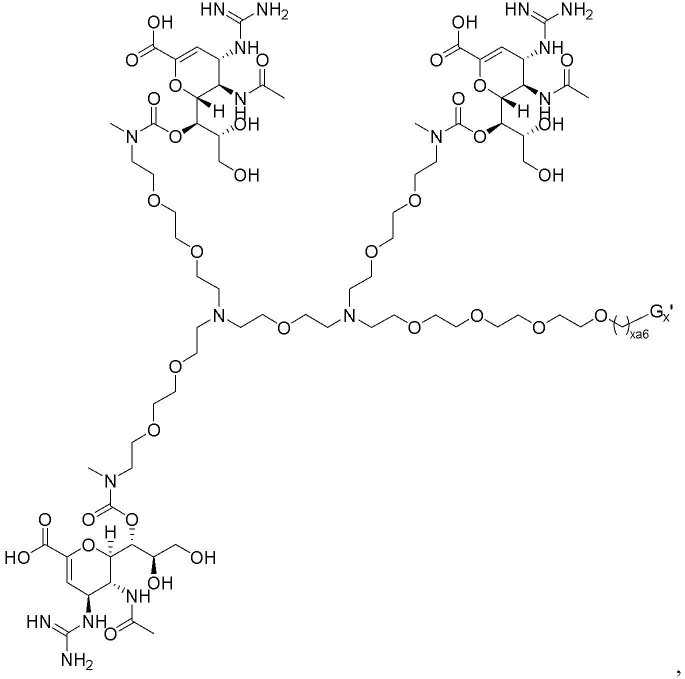 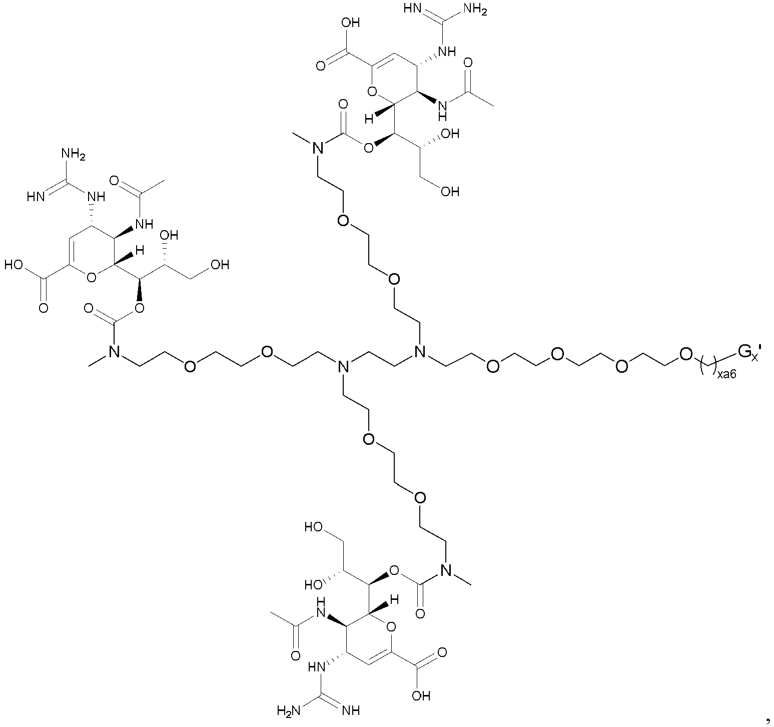 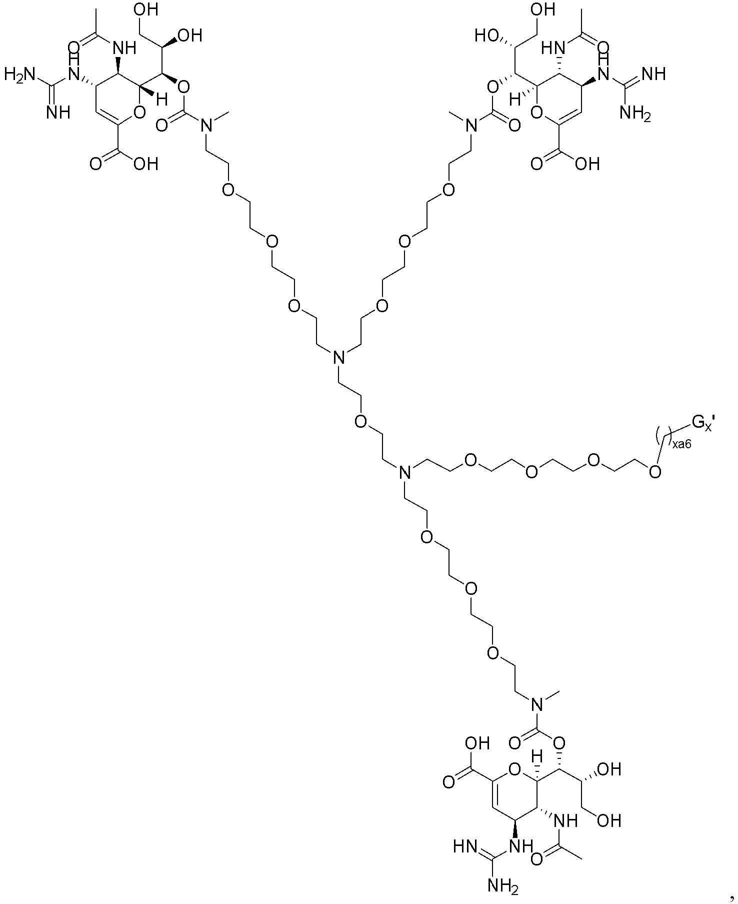 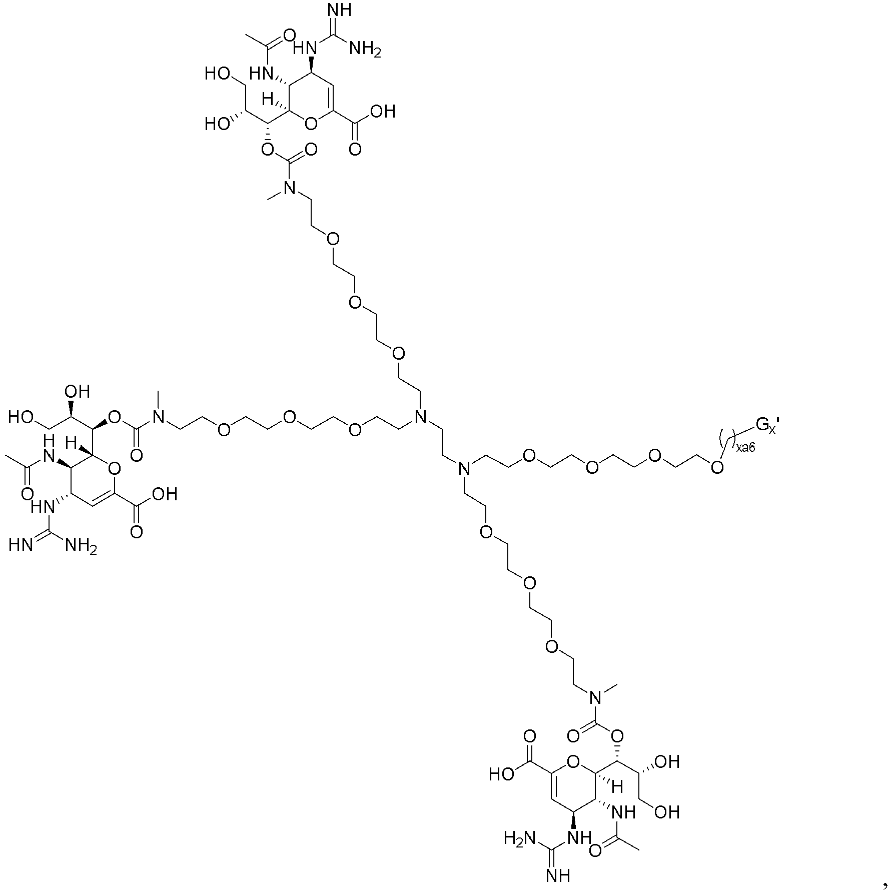 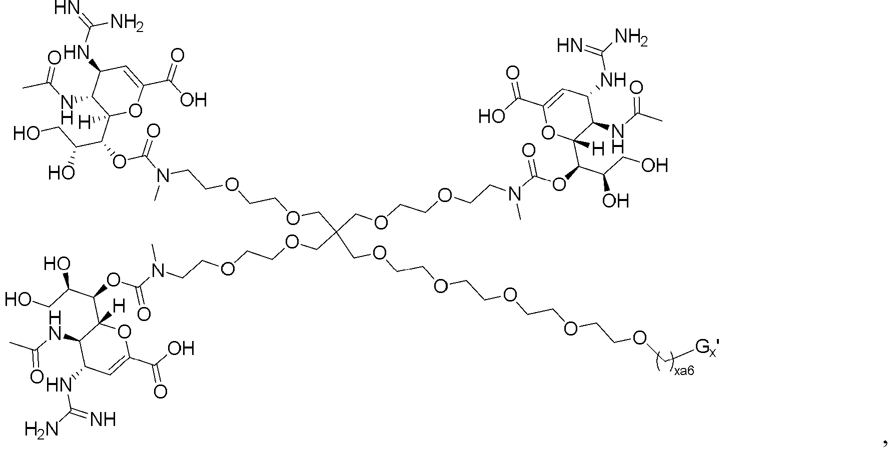 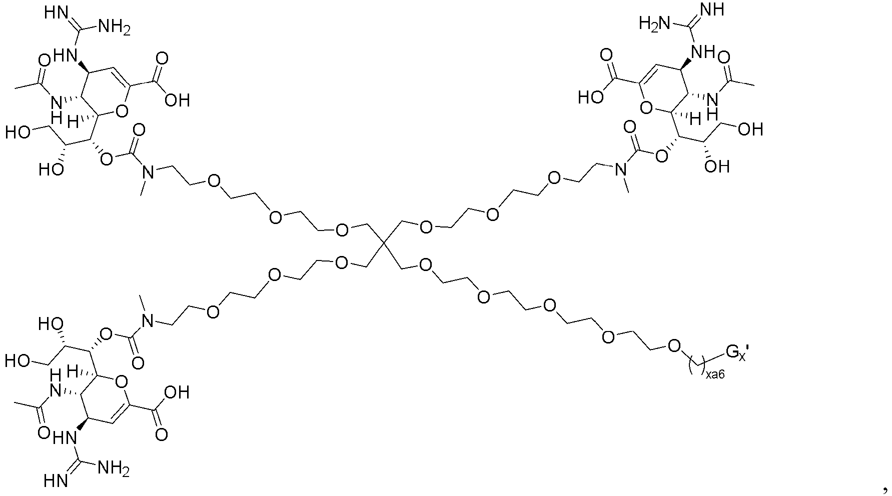 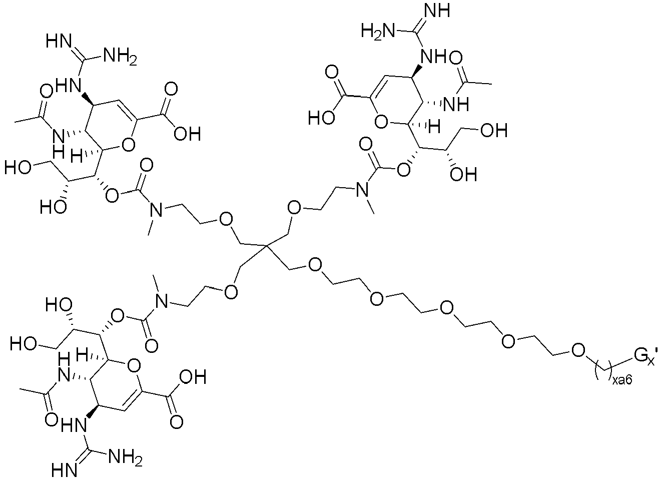 and 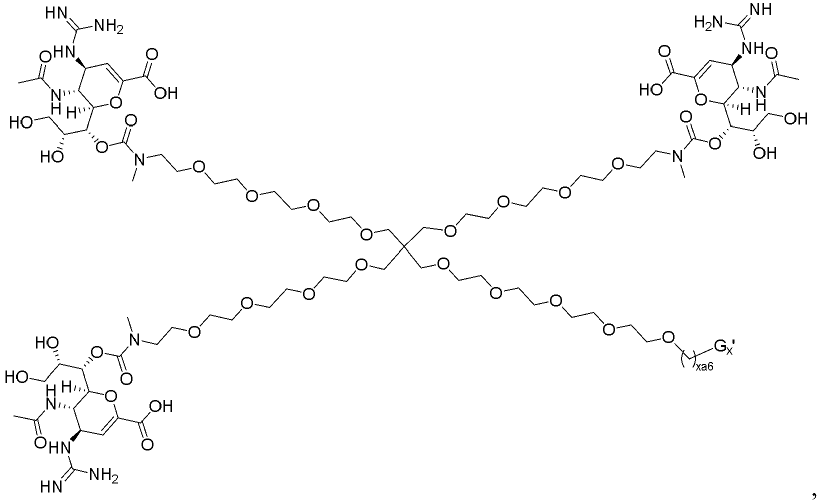 wherein,
each xa6 is independently an integer between 0 and 8,
each Gₓ' is independently selected from the group consisting of 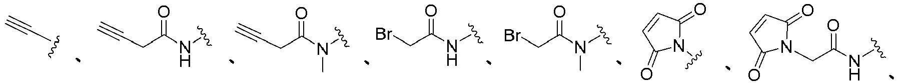 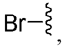 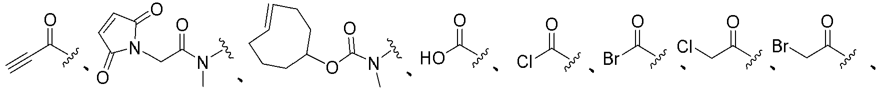 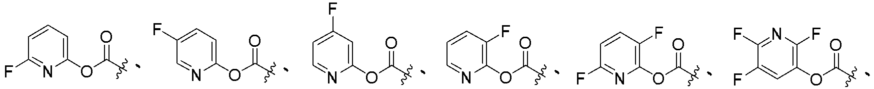 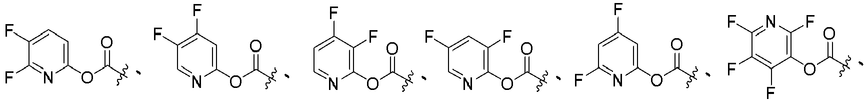 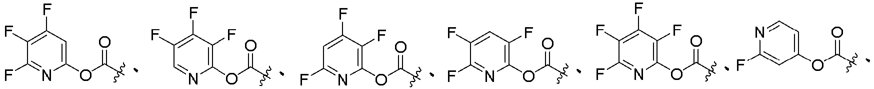 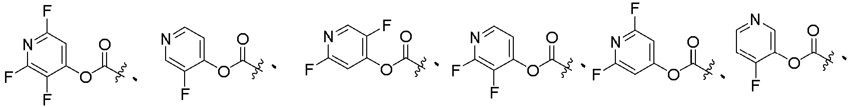 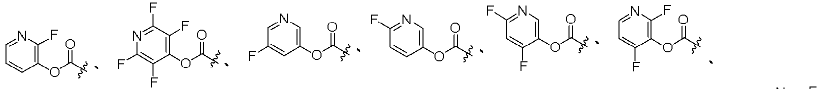 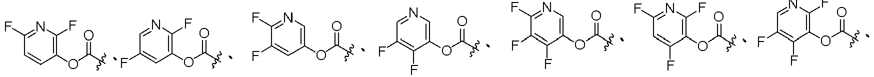 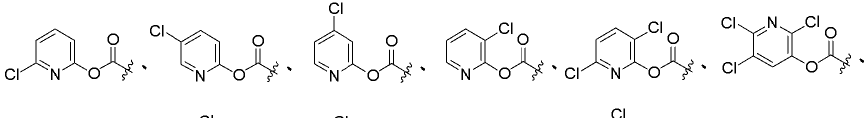 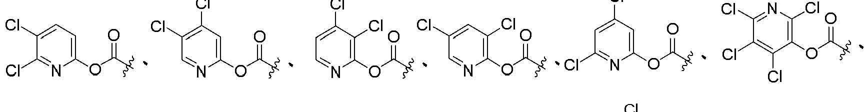 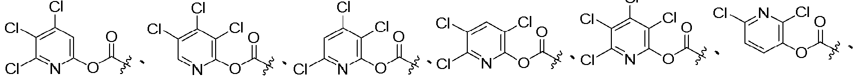 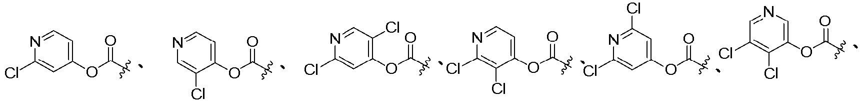 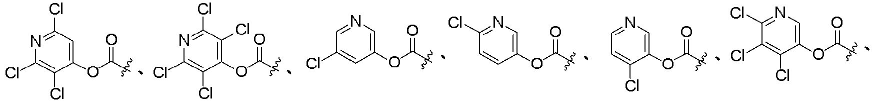 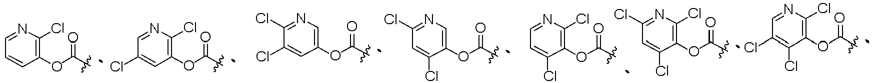 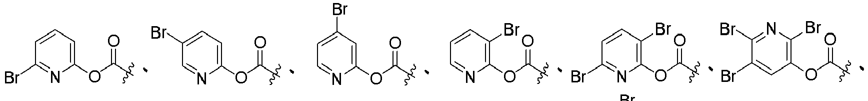 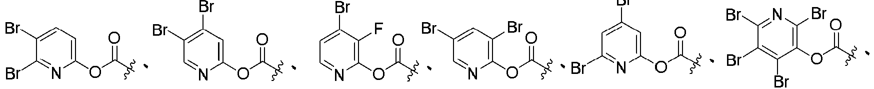 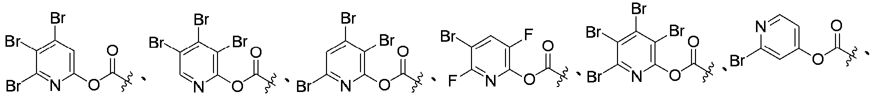 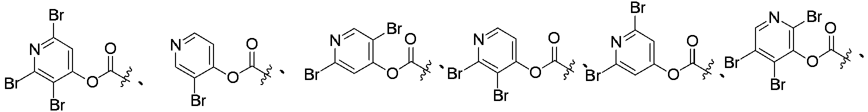 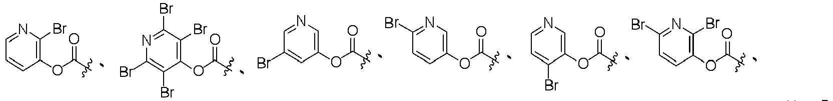 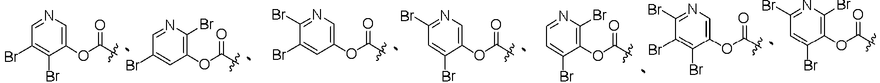 and

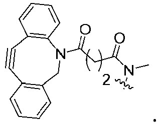

21. The compound according to claim 19 or 20, selected from the group consisting of: 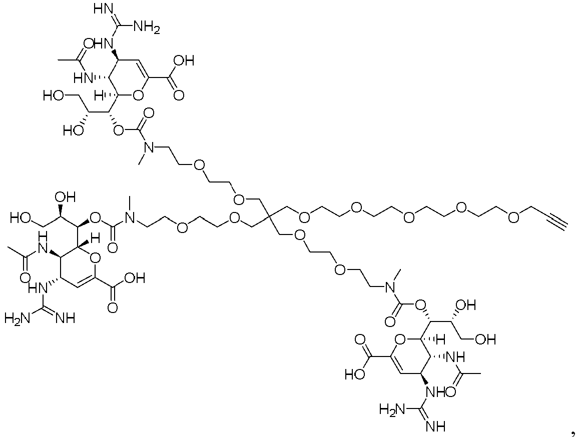 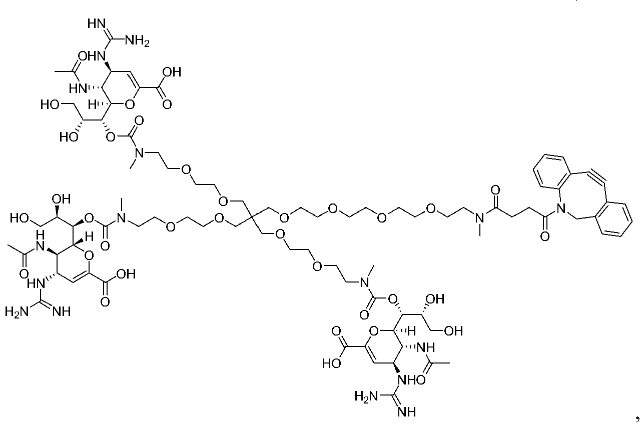 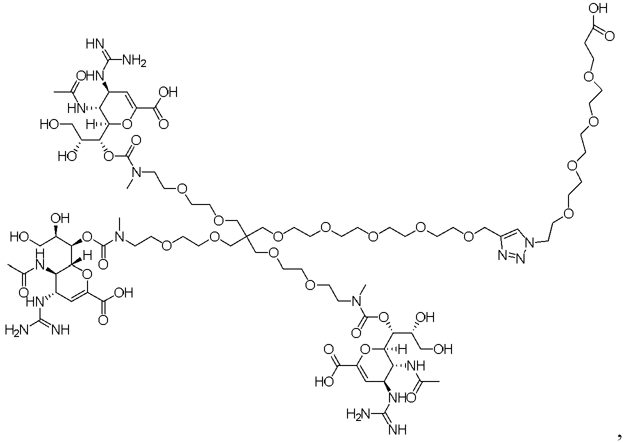 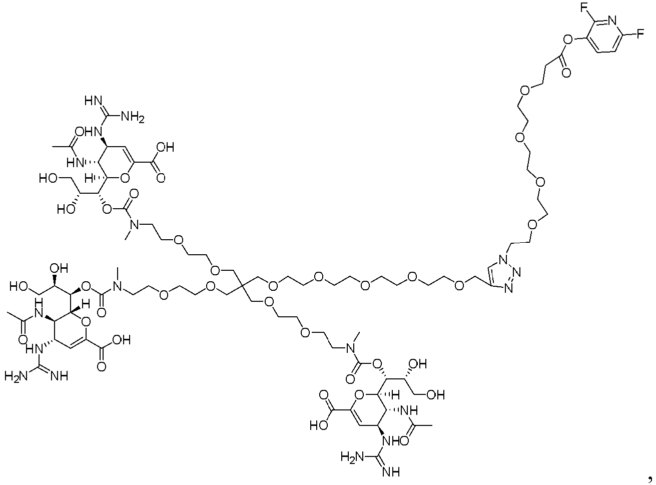 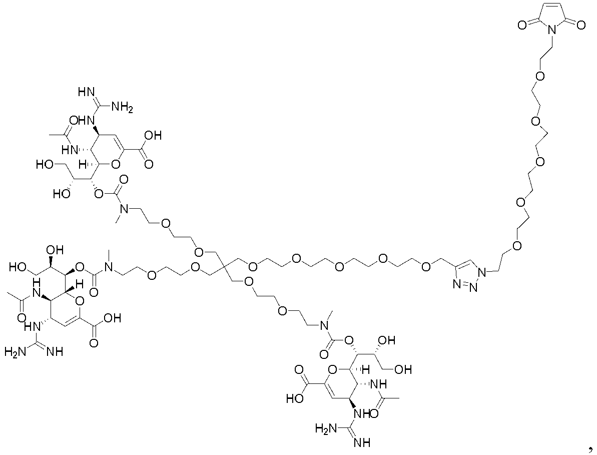 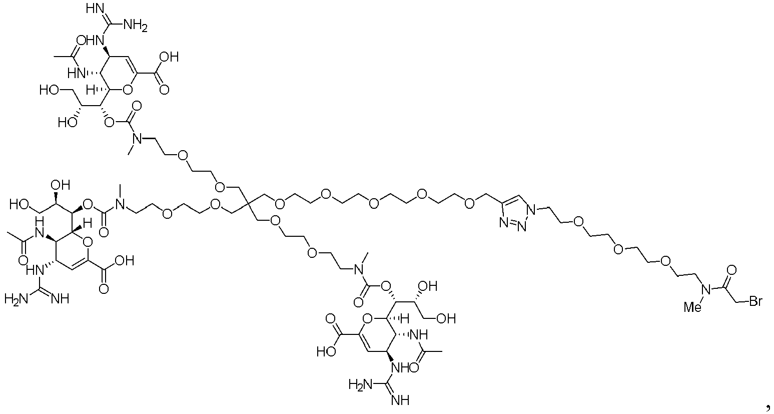 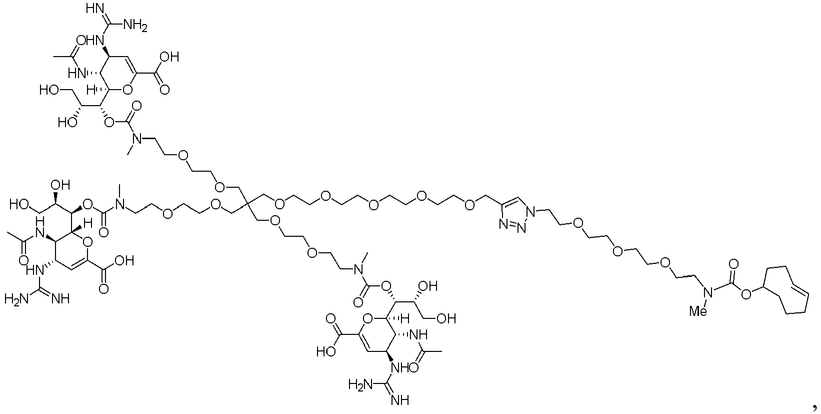 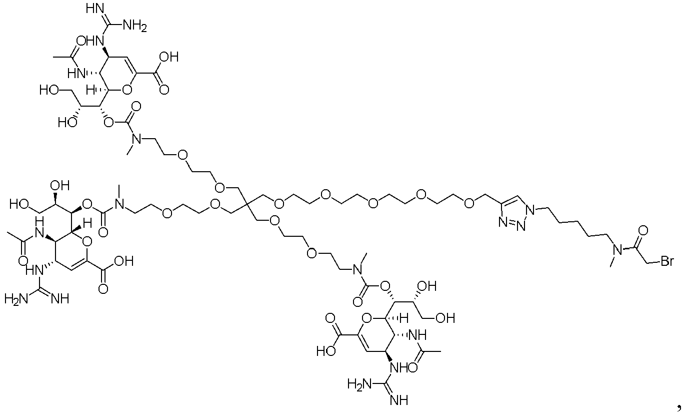 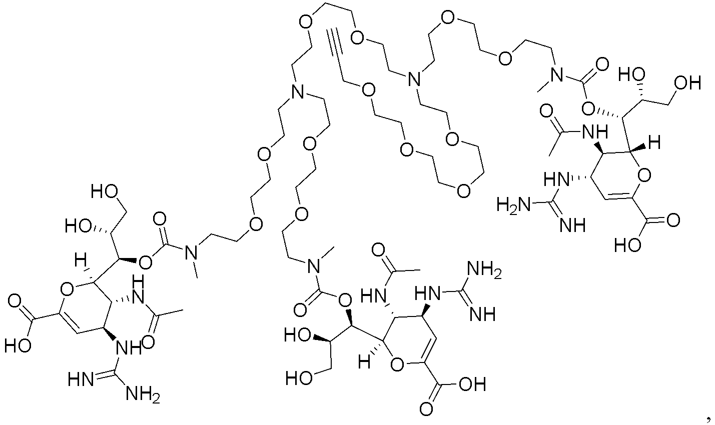 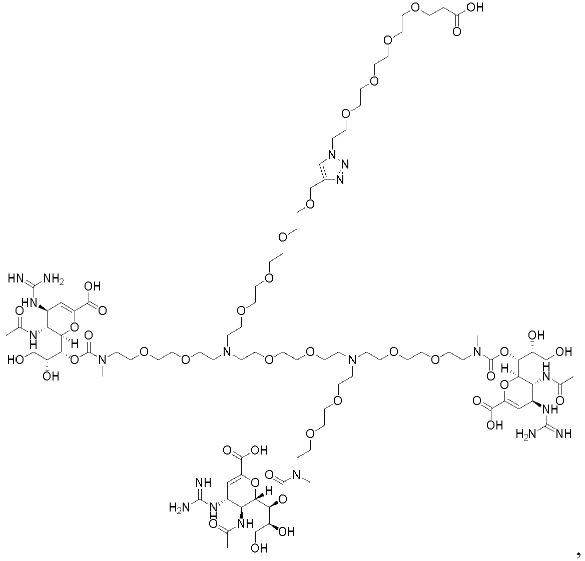 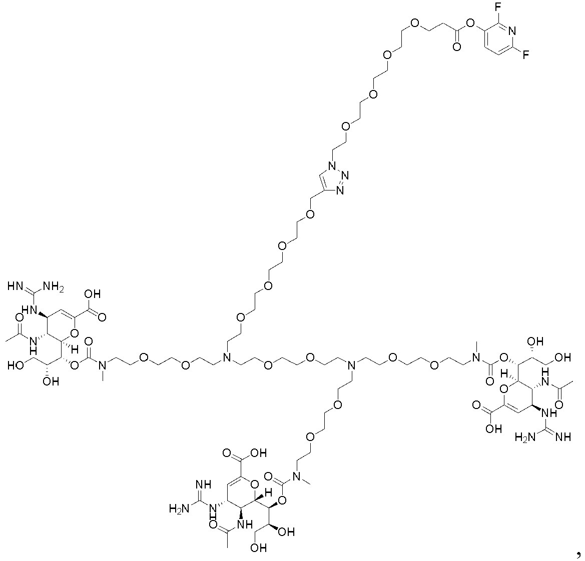 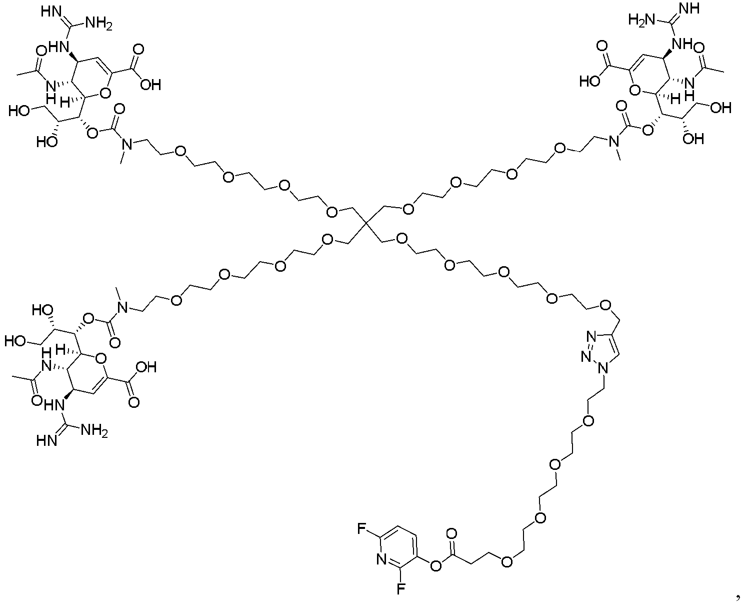 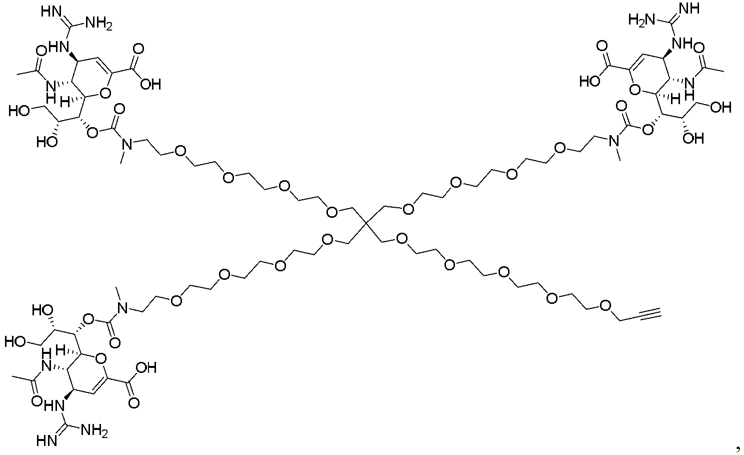 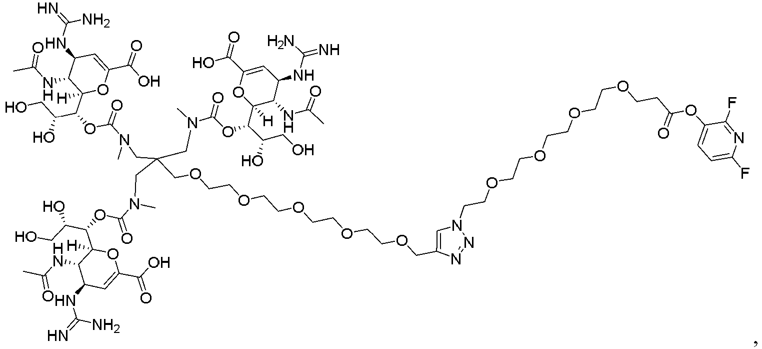 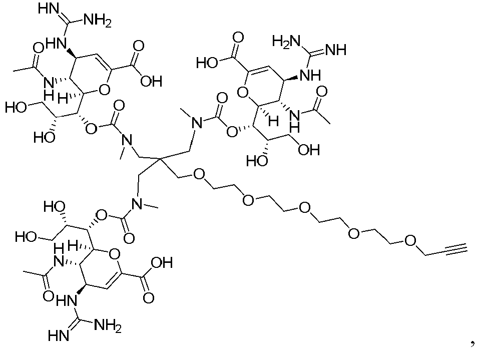 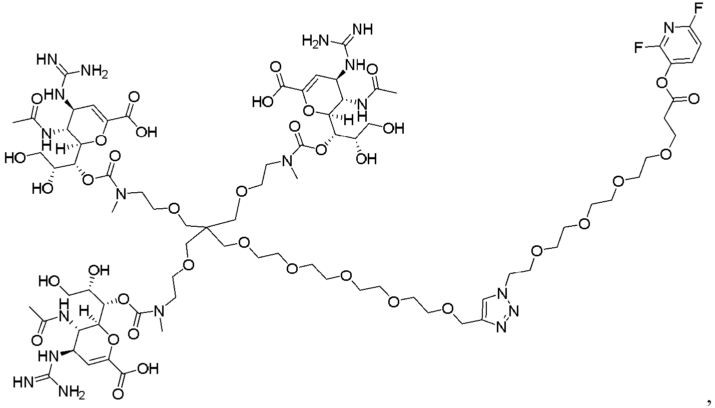 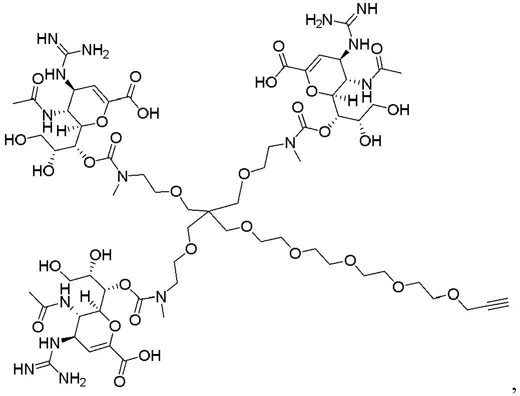 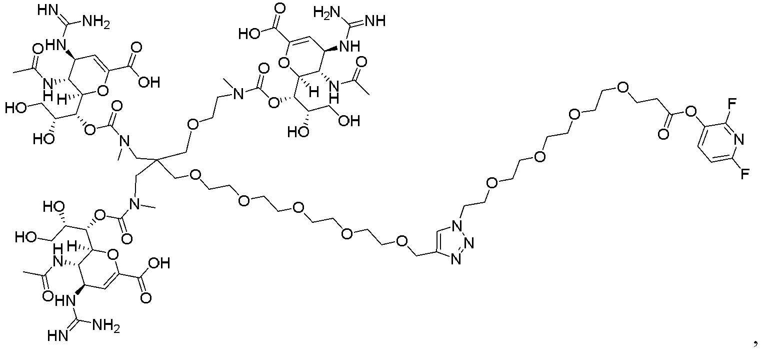 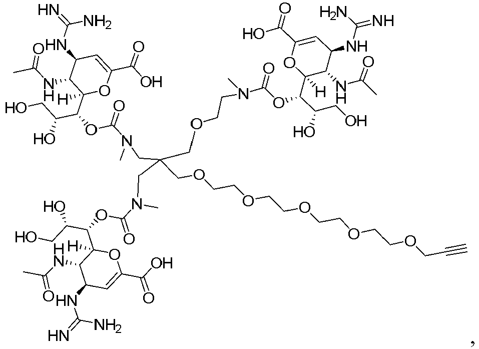 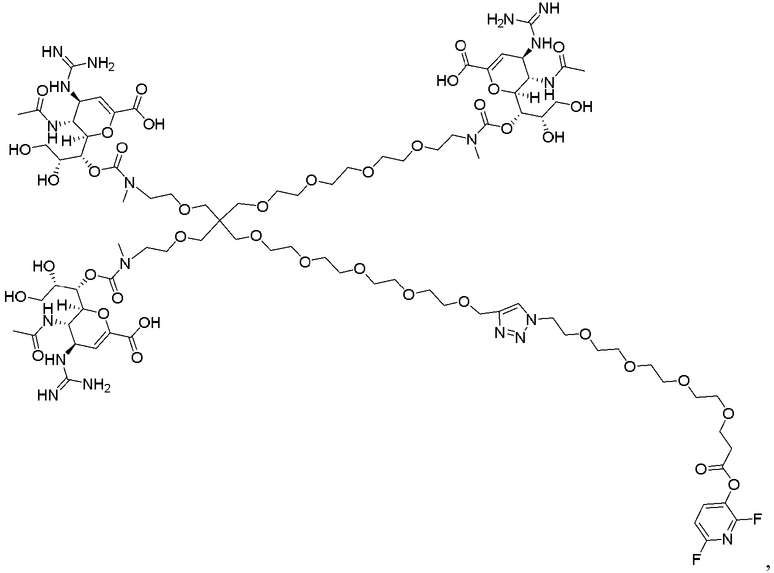 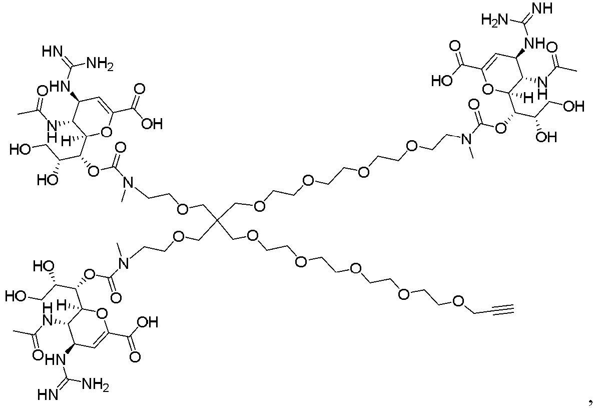 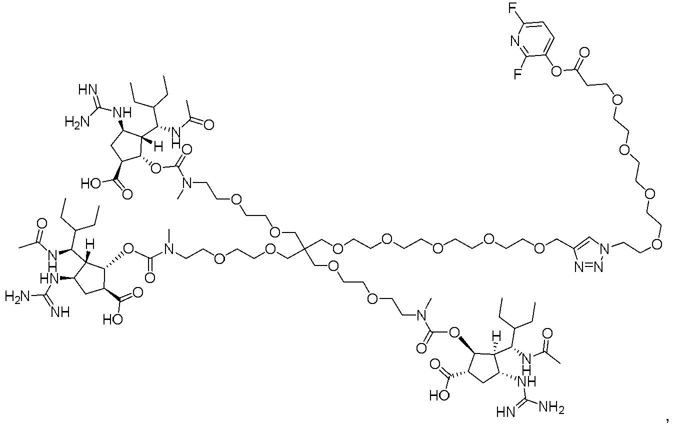 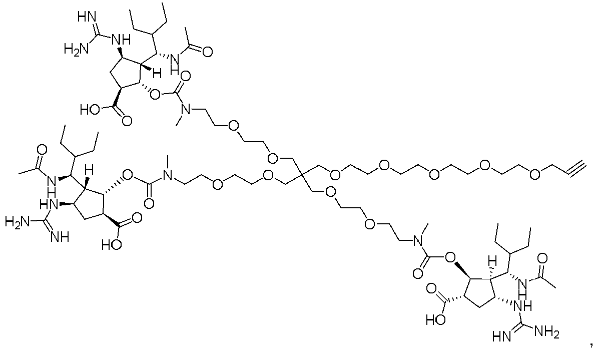 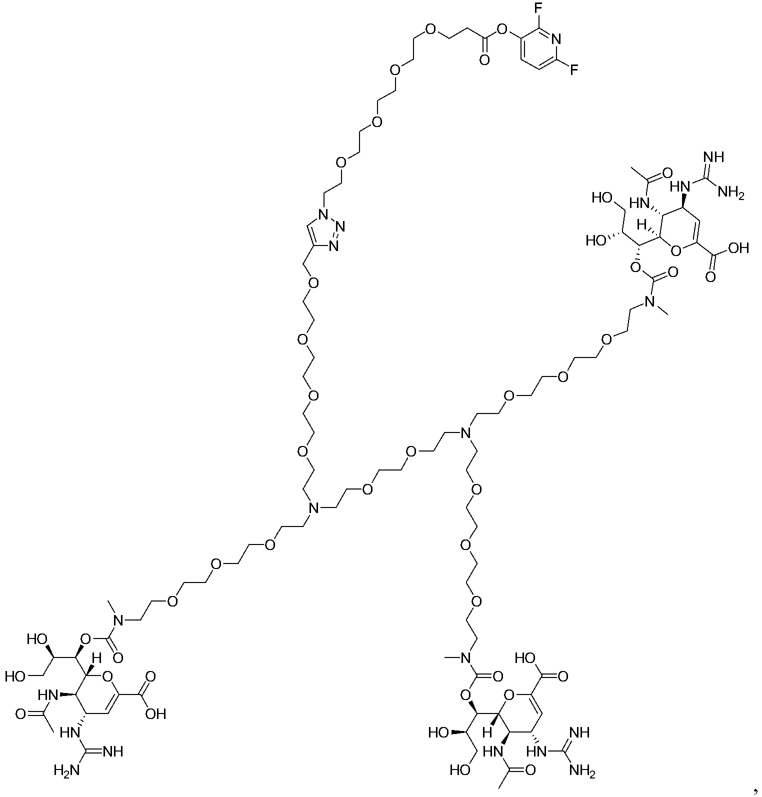 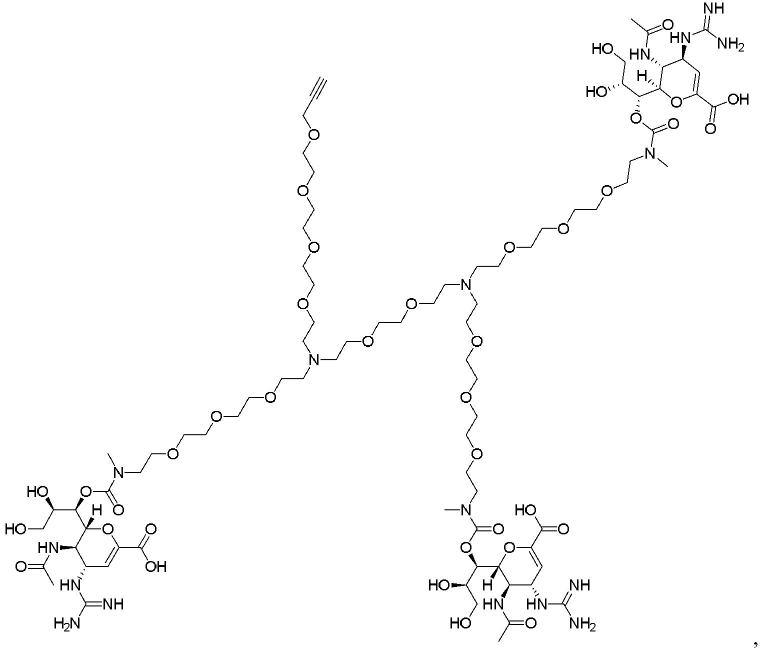 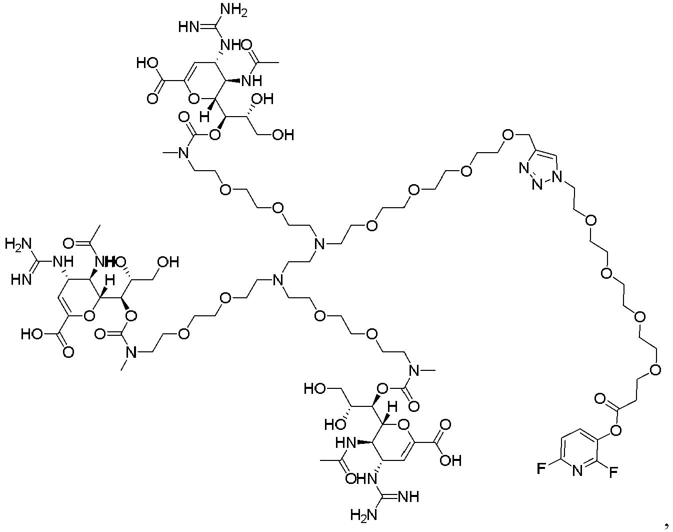 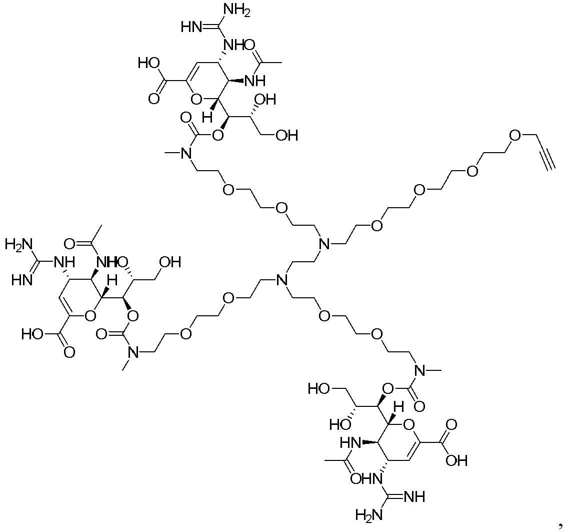 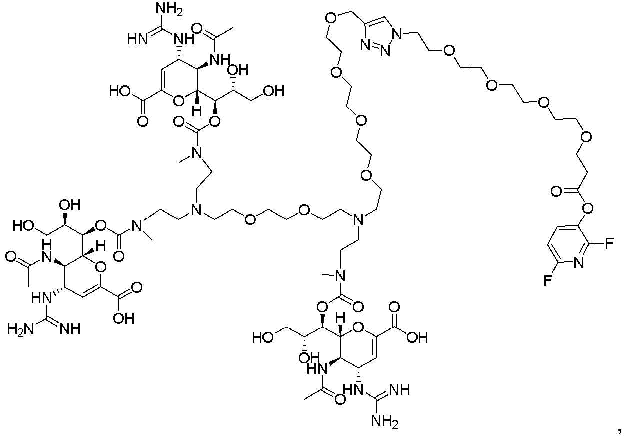 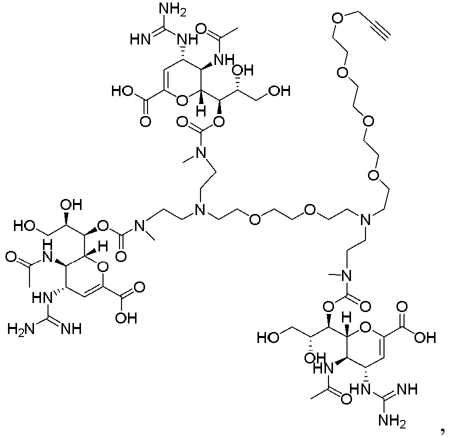 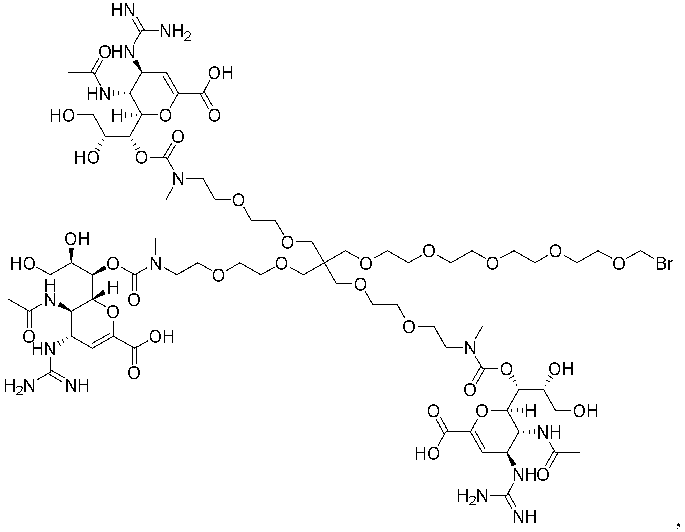 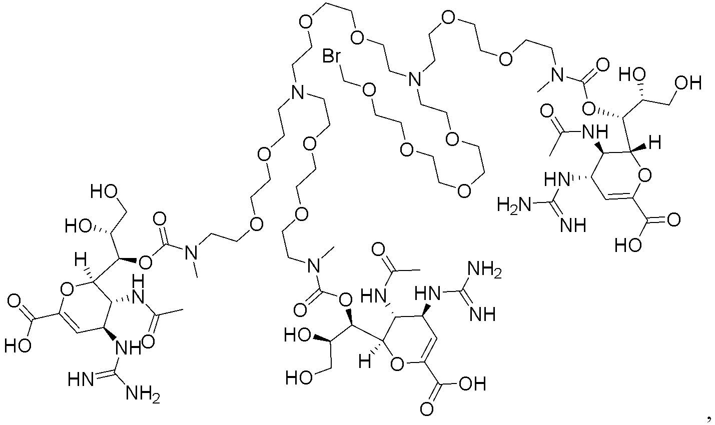 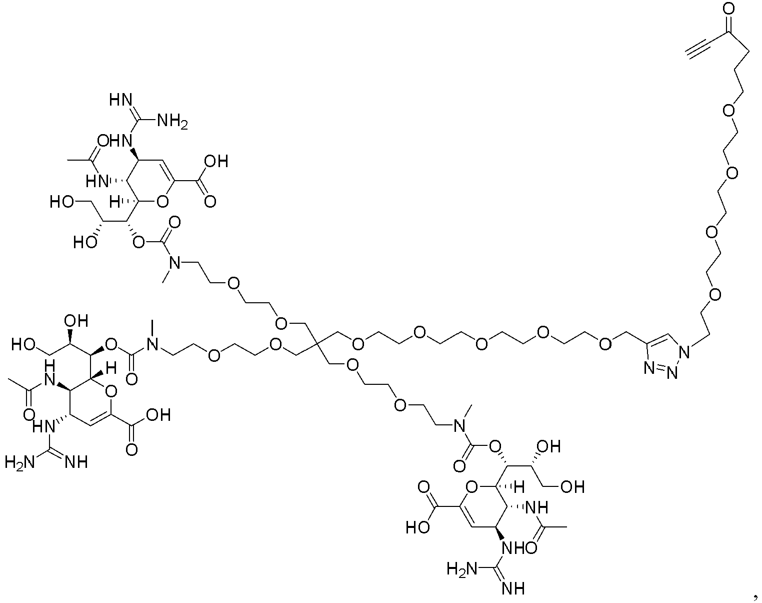 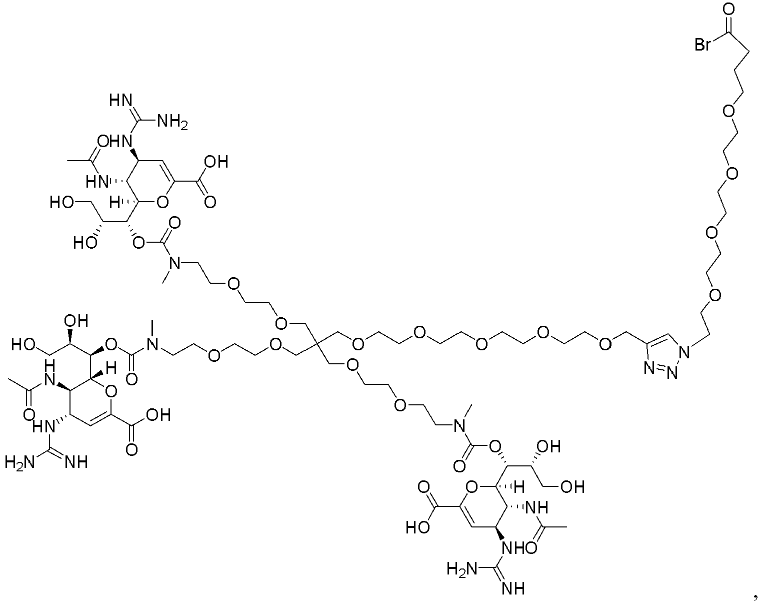 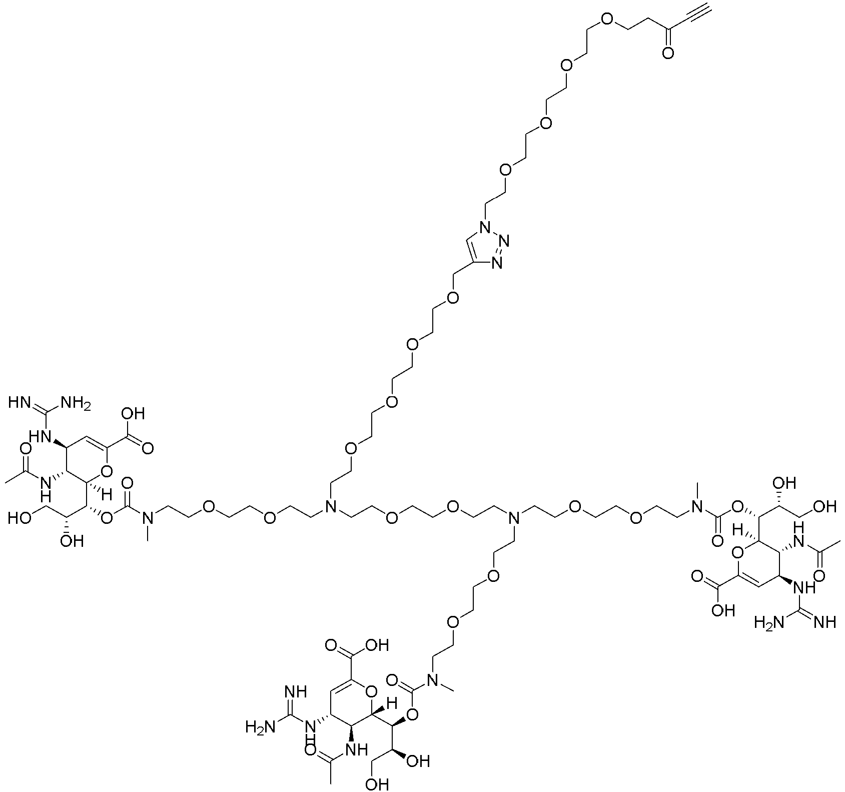 and
